# EUROPEAN PATENT APPLICATION

(11) **EP 4 502 153 A1**
(43) Date of publication of application: **05.02.2025**
(21) Application number: 23781421.5
(22) Date of filing: 30.03.2023
(51) Int. Cl.: C12N 9/12, C12N 15/113, C12N 15/10, C12N 15/86, C12N 9/22

(54) **GENE EDITING SYSTEM FOR TREATING USHER SYNDROME**

(30) Priority: 30.03.2022 KR 20220039723; 27.05.2022 KR 20220065600
(71) Applicant: GenKOre Inc., Daejeon 34141 (KR)
(72) Inventor: KIM, Yong Sam, Seoul 06767 (KR); KIM, Do Yon, Daejeon 34069 (KR)
(74) Representative: Ullrich & Naumann PartG mbB
(86) International application number: PCT/KR2023/004330
(87) International publication number: WO 2023/191570

(57) **Abstract**

The present invention relates to a gene editing system for treating Usher syndrome, to a disease treatment method using same, and the like, wherein by using the gene editing system of the present invention, an exon 13 region mutated in the Usherin (USH2A) gene is deleted with high efficiency, thereby treating type 2 Usher syndrome effectively. In addition, the gene editing system of the present invention comprises a miniaturized Cas12f1 protein-based endonuclease and an engineered guide RNA having a shorter length and improved indel efficiency, and a carrier having a limited packaging size, such as adeno-associated virus (AAV), can also be used, and therefore, in vivo or intracellular delivery efficiency can also be maximized.

## Description

### Technical Field

The present disclosure relates to the treatment of Usher syndrome using a CRISPR/Cas12f1 system. Specifically, the present disclosure relates to a gene editing system, composition, and therapeutic method based on the CRISPR/Cas12f1 system for the treatment of Usher syndrome.

This application claims priorities based on Korean Patent Application No. 10-2022-0039723, filed on March 30, 2022, and Korean Patent Application No. 10-2022-0065600, filed on May 27, 2022, the entire disclosures of which are incorporated herein by reference.

### Background Art

Usher syndrome is a rare genetic disorder that involves hearing loss and vision loss. The main symptoms of Usher syndrome are hearing loss and an eye disorder called retinitis pigmentosa, which causes night blindness and peripheral vision loss by progressive degeneration of the retina. In addition, many patients with Usher syndrome have severe balance problems. Usher syndrome is an autosomal recessive disorder characterized by congenital bilateral sensorineural hearing loss and retinitis pigmentosa, and three types thereof have been clinically reported to date. Type 1 is the most severe form, with bilateral severe to profound hearing loss and loss of vestibular function, and usually presents with night blindness, severe visual field constriction, and visual impairment before the teenage years. Type 2 has moderate to severe hearing loss and normal vestibular function, with onset of night blindness, visual field constriction, and visual impairment in the late teens or early 20s. Type 3 is rare and presents with progressive hearing loss and various vestibular dysfunctions.

Genetic heterogeneity exists depending on each clinical type. Among them, type 2 Usher syndrome is known to be caused by a mutation in the USH2A (Usherin) gene that expresses the Usherin protein which is a basement membrane protein expressed in the retina and inner ear. It is known that symptoms of type 2 (more specifically, type 2A) Usher syndrome are caused by the c.2276G>T and c.2299delG genetic mutations that occur in exon 13, which are the most common mutations in the USH2A gene. A strategy of expressing the USH2A gene whose exon 13 is deleted (for example, exon 13 skipping) to alleviate these symptoms has been proven to be effective through animal experiments conducted by researchers at Harvard Medical School and others (see Non-Patent Documents 1 and 2).

To implement this proven treatment strategies more sustainably and efficiently, treatments using the CRISPR/Cas system are being studied. However, the CRISPR/Cas system has a problem in that it has little therapeutic effect due to significantly low intracellular gene editing activity, or in vivo delivery thereof is difficult due to its relatively large molecular weight. Therefore, there is a need to develop a gene editing system that has increased intracellular gene editing activity to exhibit sufficient therapeutic effects while being miniaturized so that delivery vehicles with proven efficiency and stability, such as adeno-associated virus (AAV), can be used.

### [Prior art Document]

### [Non-patent Document]

(Non-patent Document 1) Pendse, Nachiket D et al. "In Vivo Assessment of Potential Therapeutic Approaches for USH2A-Associated Diseases." Advances in experimental medicine and biology vol. 1185 (2019): 91-96.
(Non-patent Document 2) Pendse, Nachiket D et al. "Exon 13-skipped USH2A protein retains functional integrity in mice, suggesting an exo-skipping therapeutic approach to treat USH2A-associated disease." bioRxiv 2020.02.04.934240.

### Disclosure of Invention

### Technical Problem

The object of the present disclosure is to solve the above-mentioned problems of the prior art.

In addition, an object of the present disclosure is to provide a gene editing technique for treating Usher syndrome, which exhibits improved gene editing efficiency and can be implemented as a hypercompact structure that is accommodatable in various delivery vehicles, including adeno-associated virus (AAV).

Another object of the present disclosure is to provide a CRISPR/Cas system-based gene editing technique for deleting a nucleic acid segment comprising exon 13 in the USH2A (Usherin) gene.

Yet another object of the present disclosure is to provide a method for treating Usher syndrome or delaying onset or progression thereof using a CRISPR/Cas system-based gene editing technique.

The object of the present disclosure is not limited to the above-mentioned objects. The objects of the present disclosure will become clearer from the following description and may be realized by means and combinations thereof as set forth in the claims.

### Solution to Problem

Representative configurations of the present disclosure to achieve the above-mentioned objects are as follows.

According to an aspect of the present disclosure, there is provided an editing system for a USH2A gene or a composition for editing a USH2A gene, comprising an endonuclease comprising a Cas12f1 molecule or a nucleic acid encoding the endonuclease; a first guide RNA comprising a first guide sequence capable of hybridizing to a target sequence of contiguous 15 to 30 bp in length, wherein the target sequence is located in a region 5000 bp upstream of USH2A exon 13 and is adjacent to a protospacer-adjacent motif (PAM) sequence recognized by the Cas12f1 molecule, or a nucleic acid encoding the first guide RNA; and a second guide RNA comprising a second guide sequence capable of hybridizing to a target sequence of contiguous 15 to 30 bp in length, wherein the target sequence is located in a region 14500 bp downstream of USH2A exon 13 and is adjacent to a PAM sequence recognized by the Cas12f1 molecule, or a nucleic acid encoding the second guide RNA.

In an embodiment, the system or composition may induce deletion of exon 13 in the USH2A gene in a cell.

In another embodiment, the system or composition may be for treatment of type 2A Usher syndrome.

According to another aspect of the present disclosure, there is provided a vector system, comprising at least one vector that comprises a first nucleic acid construct to which a nucleotide sequence encoding an endonuclease is operably linked, the endonuclease comprising a Cas12f1 molecule; a second nucleic acid construct to which a nucleotide sequence encoding a first guide RNA is operably linked, the first guide RNA comprising a first guide sequence capable of hybridizing to a target sequence of contiguous 15 to 30 bp in length, wherein the target sequence is located in a region 5000 bp upstream of USH2A exon 13 and is adjacent to a protospacer-adjacent motif (PAM) sequence recognized by the Cas12f1 molecule; and a third nucleic acid construct to which a nucleotide sequence encoding a second guide RNA is operably linked, the second guide RNA comprising a second guide sequence capable of hybridizing to a target sequence of contiguous 15 to 30 bp in length, wherein the target sequence is located in a region 14500 bp downstream of USH2A exon 13 and is adjacent to a PAM sequence recognized by the Cas12f1 molecule.

In an embodiment, the vector system may induce deletion of exon 13 in a USH2A gene in a cell.

In another embodiment, the nucleic acid constructs may be contained in the same or different vectors.

In yet another embodiment, the nucleic acid structures may be contained in one vector.

In still yet another embodiment, the vector may further comprise a promoter or enhancer.

In still yet another embodiment, the promoter may be, but is not limited to, U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

In still yet another embodiment, the vector may be selected from the group consisting of, but is not limited to, a retrovirus vector, a lentivirus vector, an adenovirus vector, an adeno-associated virus vector, a vaccinia virus vector, a poxvirus vector, a herpes simplex virus vector, and a phagemid vector.

In still yet another embodiment, the vector may be selected from the group consisting of, but is not limited to, plasmid, naked DNA, DNA complex, mRNA (transcript), and amplicon.

According to yet another aspect of the present disclosure, there is provided a recombinant virus produced by the vector system of the present disclosure.

According to still yet another aspect of the present disclosure, there is provided a composition comprising the system, the vector system, or the recombinant virus of the present disclosure.

In an embodiment, the composition may be a pharmaceutical composition.

According to still yet another aspect of the present disclosure, there is provided a method for inducing deletion of a segment comprising exon 13 in a USH2A gene in a cell, comprising bringing, into contact with the cell, the system, the vector system, or the recombinant virus of the present disclosure.

According to still yet another aspect of the present disclosure, there is provided a method for treating a subject having a disease associated with a mutation in exon 13 of the USH2A gene, comprising bringing, into contact with the subject, the system, the vector system, or the recombinant virus of the present disclosure.

According to still yet another aspect of the present disclosure, there is provided a method for modifying a gene of a cell, comprising bringing, into contact with the cell, the system, the vector system, or the recombinant virus of the present disclosure.

In an embodiment, the recombinant virus may be an adeno-associated virus (AAV).

In another embodiment, the cell may be a stem cell, or a cell from the eye or inner ear of a mammal, but is not limited thereto.

In yet another embodiment, the cell may be derived from a subject having Usher syndrome.

In still yet another embodiment, the bringing-into-contact may occur ex vivo or in vivo.

According to still yet another aspect of the present disclosure, there is provided a stem cell genetically modified by the method of the present disclosure.

In an embodiment, the stem cell may be for treating type 2A Usher syndrome.

According to still yet another aspect of the present disclosure, there is provided a guide RNA, comprising a spacer region, which comprises a guide sequence capable of hybridizing to a target sequence in a USH2A (Usherin) gene, and a scaffold region, wherein the guide sequence comprises (i) a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or (ii) a sequence of contiguous 15 to 20 nucleotides from a nucleotide sequence selected from the group consisting of SEQ ID NOs: 446 to 475, or a sequence that is different by 5 or fewer nucleotides in the contiguous nucleotide sequence, wherein thymine (T) is substituted with uracil (U) in the contiguous nucleotide sequence.

In an embodiment, the guide sequence may comprise a nucleotide sequence selected from the group consisting of SEQ ID NOs: 80 to 128 and SEQ ID NOs: 159 to 164, and/or the guide sequence may comprise a nucleotide sequence selected from the group consisting of SEQ ID NOs: 129 to 158 and SEQ ID NOs: 165 to 174.

According to still yet another aspect of the present disclosure, there is provided a nucleic acid molecule encoding the guide RNA of the present disclosure.

According to still yet another aspect of the present disclosure, there is provided a composition comprising at least one guide RNA of the present disclosure.

According to still yet another aspect of the present disclosure, there is provided a composition comprising at least one guide RNA and the endonuclease comprising a Cas12f1 molecule of the present disclosure.

In an embodiment, the composition may comprise two or more guide RNAs, of which (i) at least one guide RNA comprises a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, and (ii) at least one other guide RNA comprises a sequence of contiguous 15 to 20 nucleotides from a nucleotide sequence selected from the group consisting of SEQ ID NOs: 446 to 475, or a sequence that is different by 5 or fewer nucleotides in the contiguous nucleotide sequence.

Hereinafter, embodiments commonly applied to the components, such as endonuclease, guide RNA, USH2A exon 13, and the like, included in each of the system, composition, vector system, and method according to the multiple aspects of the present disclosure described above are as follows.

In an embodiment, the USH2A exon 13 may comprise at least one mutation that causes Usher syndrome.

In another embodiment, the target sequence located in a region 5000 bp upstream of USH2A exon 13 may comprise a nucleotide sequence selected from the group consisting of SEQ ID NOs: 1 to 49, and/or the target sequence located in a region 14500 bp downstream of the USH2A exon 13 may comprise a nucleotide sequence selected from the group consisting of SEQ ID NOs: 50 to 79.

In yet another embodiment, the first guide sequence may comprise a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or the second guide sequence comprise a sequence of contiguous 15 to 20 nucleotides from a nucleotide sequence selected from the group consisting of SEQ ID NOs: 446 to 475, or a sequence that is different by 5 or fewer nucleotides from the contiguous nucleotide sequence, wherein thymine (T) is substituted with uracil (U) in the contiguous nucleotide sequence.

In still yet another embodiment, the first guide sequence may comprise a nucleotide sequence selected from the group consisting of SEQ ID NOs: 80 to 128 and SEQ ID NOs: 159 to 164, and/or the second guide sequence may comprise a nucleotide sequence selected from the group consisting of SEQ ID NOs: 129 to 158 and SEQ ID NOs: 165 to 174.

In still yet another embodiment, the guide RNA, the first guide RNA, or the second guide RNA may comprise a U-rich tail sequence linked to the 3' end of the guide sequence, in which the U-rich tail is represented by 5'-(UₘV)ₙUₒ-3', wherein V is each independently A, C, or G, m and o are integers between 1 to 20, and n is an integer between 0 to 5.

In still yet another embodiment, the guide RNA, the first guide RNA, or the second guide RNA may comprise an engineered scaffold region, and the engineered scaffold region may comprise a nucleotide sequence having at least 50% sequence identity to a scaffold region of a wild-type Cas12f1 guide RNA sequence, in which the scaffold region sequentially comprises, from the 5'-end, a first stem-loop region, a second stem-loop region, a third stem-loop region, a fourth stem-loop region, and a tracrRNA-crRNA complementarity region, and comprise at least one modification selected from the group consisting of the following (1) to (4) with respect to the wild-type Cas12f1 guide RNA sequence:
(1) deletion of at least a part of the first stem-loop region; (2) deletion of at least a part of the second stem-loop region; (3) deletion of at least a part of the tracrRNA-crRNA complementarity region; and (4) replacement of one or more uracil (U) residues with A, G, or C in three or more consecutive U residues when the consecutive U residues are present in the tracrRNA-crRNA complementarity region.

In still yet another embodiment, the wild-type Cas12f1 guide RNA may comprise tracrRNA comprising the nucleotide sequence of SEQ ID NO: 175 and crRNA comprising the nucleotide sequence of SEQ ID NO: 176.

In still yet another embodiment, the scaffold region or engineered scaffold region may comprise a sequence having at least 80% sequence identity to a sequence represented by Formula (I): 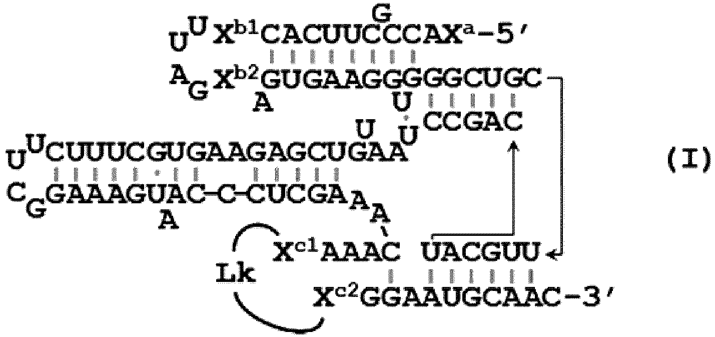 in Formula (I), X^{a} comprises the nucleotide sequence of SEQ ID NO: 178 or a nucleotide sequence having the sequence of SEQ ID NO: 178 from which 1 to 20 nucleotides are deleted, X^{b1} comprises the nucleotide sequence of SEQ ID NO: 189 or a nucleotide sequence having the sequence of SEQ ID NO: 189 from which 1 to 13 nucleotides are deleted, X^{b2} comprises the nucleotide sequence of SEQ ID NO: 193 or a nucleotide sequence having the sequence of SEQ ID NO: 193 from which 1 to 14 nucleotides are deleted, X^{c1} comprises the nucleotide sequence of SEQ ID NO: 203 or a nucleotide sequence having the sequence of SEQ ID NO: 203 from which 1 to 28 nucleotides are deleted, X^{c2} comprises the nucleotide sequence of SEQ ID NO: 222 or a nucleotide sequence having the sequence of SEQ ID NO: 222 from which 1 to 27 nucleotides are deleted, and Lk is a polynucleotide linker of 2 to 20 nucleotides in length or absent.

In still yet another embodiment, in a case where three or more consecutive uracil (U) residues are present in the X^{c1} sequence, the X^{c1} sequence may comprise a modification in which at least one U residue thereof is replaced with A, G or C.

In still yet another embodiment, the deletion in the nucleotide sequence of X^{a}, the deletion in the nucleotide sequences of X^{b1} and X^{b2}, and/or the deletion in the nucleotide sequences of X^{c1} and X^{c2} may comprise deletion of one or more pairs of complementary nucleotides.

In still yet another embodiment, the sequence 5'-X^{b1}UUAGX^{b2}-3' in Formula (I) may be selected from the group consisting of SEQ ID NOs: 198 to 202 and 5'-UUAG-3'.

In still yet another embodiment, the sequence 5'-X^{c1}-Lk-X^{c2}-3' in Formula (I) may be selected from the group consisting of SEQ ID NOs: 244 to 250 and 5'-Lk-3'.

In still yet another embodiment, the Lk may comprise a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 240), 5'-UUCAGAAAUGAA-3' (SEQ ID NO: 241), 5'-UUCAUGAAAAUGAA-3' (SEQ ID NO: 242), and 5'-UUCAUUGAAAAAUGAA-3' (SEQ ID NO: 243).

In still yet another embodiment, the scaffold region may comprise an engineered tracrRNA consisting of a nucleotide sequence selected from the group consisting of SEQ ID NOs: 251 to 296 and/or an engineered crRNA consisting of a nucleotide sequence selected from the group consisting of SEQ ID NOs: 297 to 304.

In still yet another embodiment, the guide RNA, the first guide RNA, or second guide RNA may be a dual guide RNA or a single guide RNA.

In still yet another embodiment, the guide RNA, the first guide RNA, or second guide RNA may comprise a scaffold region sequence of a nucleotide sequence selected from the group consisting of SEQ ID NOs: 313 to 350.

In still yet another embodiment, the guide RNA, the first guide RNA, or second guide RNA may comprise a scaffold region sequence of a nucleotide sequence selected from the group consisting of SEQ ID NOs: 315 to 317.

In still yet another embodiment, the Cas12f1 molecule may comprise an amino acid sequence having at least 70% sequence identity with an amino acid sequence selected from the group consisting of SEQ ID NOs: 360 to 364 and SEQ ID NOs: 370 to 377.

In still yet another embodiment, the endonuclease forms a ribonucleoprotein (RNP) with the guide RNA, the first guide RNA, or the second guide RNA.

### Advantageous Effects of Invention

Usher syndrome caused by mutations in the USH2A gene can be treated by a strategy of deleting the mutated exon 13 of the USH2A gene to induce the production of normally functioning Usherin protein. The present disclosure provides a more efficient and widely applicable gene editing system comprising a novel hypercompact nucleic acid cleavage protein, which is a Cas12f1 protein, and an engineered guide RNA which is site-specifically modified to exhibit excellent gene editing efficiency and is capable of targeting a specific region of the USH2A gene with high specificity when used together with the Cas12f1 protein, thereby effectively deleting exon 13 of the USH2A gene. In addition, the gene editing system of the present disclosure uses an endonuclease that is significantly smaller in size than conventional Cas9 protein and the like, and an engineered guide RNA that is shorter in length but exhibits excellent editing efficiency. Therefore, even when using a delivery vehicle with a very limited packaging size, such as AAV, it has the advantage of being able to include an additional configuration that increases deletion efficiency of exon 13 of the USH2A gene, since various tools necessary for editing a desired gene can be loaded into a single vector.

### Brief Description of Drawings

FIG.1 illustrates each of modification sites (MS) MS1 to MS5 in the engineered guide RNA according to an embodiment of the present disclosure.
FIG. 2A and FIG. 2B illustrate exemplary structures of the engineered single guide RNAs (sgRNAs) according to an embodiment of the present disclosure: FIG. 2A illustrates exemplary modification sites of the canonical sgRNA for Cas12f1. FIG. 2B illustrates exemplary modification sites of the mature form sgRNA for engineered Cas12f1 according to an embodiment of the present disclosure.
FIGS.3A and 3B illustrate results obtained by measuring the indel efficiency (%) of the gene editing systems comprising CWCas12f1 and engineered gRNAs having at least one modification of MS1 to MS5 in each region of the wild-type guide RNA: FIG.3A is a graph showing the indel efficiency (%) for target sequence 1 (Target-1; SEQ ID NO: 358). FIG.3B is a graph showing the indel efficiency (%) for target sequence 2 (Target-2; SEQ ID NO: 359).
FIGS.4A to 4D illustrate results obtained by measuring the indel efficiency (%) of the gene editing systems comprising CWCas12f1 and engineered gRNAs further having at least one modification of MS3 to MS5 in each region of the mature form sgRNA: Each of FIGS. 4A and 4B is a graph showing the indel efficiency (%) for target sequence 1 (Target-1; SEQ ID NO: 358), respectively. Each of FIGS. 4C and 4D is a graph showing the indel efficiency (%) for target sequence 2 (Target-2; SEQ ID NO: 359), respectively.
FIGS.5A and 5B illustrate results obtained by measuring the indel efficiency (%) of the USH2A gene editing system using three different versions of guide RNA: FIG. 5A is a graph showing the indel efficiency obtained by using gRNAs that comprise the guide sequences targeting the F region among target regions of the USH2A gene. FIG.5B is a graph showing the indel efficiency obtained by using gRNAs that comprise the guide sequences targeting the R region among target regions of the USH2A gene.
FIG.6 illustrates results obtained by confirming the indel efficiency (%) of the USH2A gene editing systems that comprise the guide RNA targeting the F region and the guide RNA targeting the R region, respectively, for which the indel efficiency was previously determined.
FIG.7 illustrates results obtained by confirming the deletion of a region comprising exon 13 in the USH2A gene in a case of using the USH2A gene editing systems that comprise specific combinations of the guide RNA targeting the F region and the guide RNA targeting the R region.
FIG.8 illustrates locations of the amplified sequences and the information of the used primer sequences in the qPCR analysis to determine deletion of exon 13 in the USH2A gene.
FIG.9 illustrates results obtained by confirming the deletion efficiency (%) of a region comprising exon 13 in the USH2A gene in a case of using the USH2A gene editing systems that comprise the guide RNA targeting the F region and the guide RNA targeting the R region in HEK293T cells (WT, wild-type guide RNA; EDIT102, positive control).
FIGS.10A to 10D illustrate results obtained by comparing the indel efficiency (%) depending on a length of the guide sequence, respectively: FIG.10A is a graph showing the indel efficiency depending on a length of the F16 guide sequence. FIG.10B is a graph showing the indel efficiency depending on a length of the FA12 guide sequence. FIG.10C is a graph showing the indel efficiency depending on a length of the R19 guide sequence. FIG.10D is a graph showing the indel efficiency depending on a length of the R40 guide sequence.
FIG.11 illustrates results obtained by confirming the indel efficiency (%) depending on a type of the U-rich tail added to the 3'-end of the guide RNA.
FIGS.12A and 12B illustrate results obtained by confirming the deletion efficiency of the USH2A gene editing system for exon 13 of the USH2A gene (WT, wild-type guide RNA; EDIT102, positive control): FIGS. 12A and 12B are graphs showing the deletion efficiency (%) for exon 13 of the USH2A gene in 661W-USH2A cell line and ARPE19/HPV16-USH2A cell line, respectively.
FIG.13 illustrates results obtained by injecting an adeno-associated virus (AAV) expressing each USH2A gene editing system into the tail vein of mice, extracting liver tissue, and confirming the indel efficiency for a target region in the USH2A gene.
FIGS.14A and 14B illustrate the Cas12f1 ver4.0-GFP vector map and the Cas12f1 ver4.1-GFP vector map used in an embodiment of the present disclosure, respectively.
FIG. 15 illustrates a schematic diagram for production of 661W-USH2A cell line.

### Modes for Carrying out Invention

The detailed description to be described later of the present disclosure will be described with reference to specific drawings with respect to specific embodiments in which the present disclosure may be practiced; however, the present disclosure is not limited thereto and, if properly described, is limited only by the appended claims, along with the full scope of equivalents to which such claims are entitled. It should be understood that various embodiments/examples of the present disclosure, although different, are not necessarily mutually exclusive. For example, a particular feature, structure, or characteristic described herein may be changed from one embodiment/example to another embodiment/example or implemented in combinations of embodiments/examples without departing from the technical spirit and scope of the present disclosure. Unless defined otherwise, technical and scientific terms used herein have the same meaning as generally used in the art to which the present disclosure belongs. For purposes of interpreting this specification, the following definitions will apply and whenever appropriate, terms used in the singular will also include the plural and vice versa.

### I. Definition

The terms "nucleic acid," "nucleotide," "nucleoside," and "base" as used herein have the meanings commonly understood by a person skilled in the art. Specifically, "nucleic acid" is a biological molecule composed of nucleotides, and is used interchangeably with polynucleotide. The nucleic acid comprises both DNA and RNA, which is double-stranded or single-stranded. "Nucleotide" is a unit composed of phosphoric acid, a pentose sugar, and a base (or nucleobase). In RNA (ribonucleic acid), the pentose sugar is ribose; and in DNA (deoxyribonucleic acid), the pentose sugar is deoxyribose. The nucleotide has one selected from adenine (A), guanine (G), cytosine (C), thymine (T), and uracil (U) as a nucleobase. Adenine, guanine, and cytosine exist both in RNA and DNA, thymine exists only in DNA, and uracil exists only in RNA. In addition, the pentose sugar and nucleobase constituting the nucleotide may be referred to as "nucleoside." The nucleoside is classified into adenosine, thymidine, cytidine, guanosine, and uridine depending on the type of nucleobase. The abbreviations for base, nucleoside, and nucleotide may be identical and may be appropriately interpreted depending on the context. For example, the sequence 5'-UUUUU-3' may be a sequence of five consecutive bases (uracil residues), a sequence of five consecutive nucleosides (uridine residues) and/or a sequence of five consecutive nucleotides (uridine monophosphate). In addition, when describing a nucleic acid, RNA, and DNA, nucleotides constituting the same are abbreviated as uridine, adenosine, thymidine, cytidine, and guanosine according to the type of nucleoside. The above abbreviation may be appropriately interpreted depending on the context. For example, RNA comprising a sequence of four consecutive uridine residues may be interpreted as RNA comprising four consecutive uridine monophosphate nucleotides. In addition, the terms nucleic acid, nucleotide, nucleoside, and base as used herein may include modified nucleic acids, nucleotides, nucleosides, and bases known in the art for improving, for example, safety or immunogenicity thereof.

The term "A, T, C, G and U" may be appropriately interpreted as a base, a nucleoside or a nucleotide in DNA or RNA, depending on the context and description. For example, when A, T, C, G, and U refer to a base, they may be interpreted as one selected from adenine, guanine, cytosine, thymine, and uracil, respectively. When A, T, C, G and U refer to a nucleoside, they may be interpreted as adenosine, thymidine, cytidine, guanosine, or uridine, respectively, and when they refer to a nucleotide in a sequence, they should be interpreted as meaning a nucleotide containing each of the nucleosides.

The term "target nucleic acid" or "target gene" refers to a nucleic acid or gene that is a subject of gene editing (for example, double-strand cleavages or deletion of a specific segment of a gene) or targeted by a gene editing system (for example, a CRISPR/Cas12f1 system). These terms may be used interchangeably and refer to the same subject. Unless otherwise defined, the target gene may be a unique gene or nucleic acid possessed by a target cell (for example, a prokaryotic cell, a eukaryotic cell, an animal cell, a mammalian cell, or a plant cell), a gene or nucleic acid of external origin, or an artificially synthesized nucleic acid or gene, and may mean single-stranded or double-stranded DNA or RNA. The target gene or target nucleic acid may be a mutated gene involved in a genetic disease. As an example, a target gene or target nucleic acid may be a human USH2A (Usherin) gene. As another example, a target gene or target nucleic acid may be a mutated human USH2A (Usherin) gene.

The term "target region" means a region of a target gene to which a guide RNA is designed to bind and in which cleavage occurs. The target region may comprise a target sequence. In addition, in double-stranded nucleic acids, the target region may refer to a region that comprises a target sequence (included in a target strand) and a sequence complementary thereto (included in a non-target strand). As an example, the target region may be a region 5000 bp upstream or a region 14500 bp downstream of exon 13 in the human USH2A (Usherin) gene.

The term "target sequence" refers to a sequence located in a target nucleic acid or a target gene, which is recognized by a guide RNA, or a sequence that can be recognized or modified by the CRISPR/Cas12f1 system or the gene editing system of the present disclosure. Specifically, the target sequence refers to a sequence complementary to a guide sequence included in a guide RNA or a sequence that binds complementarily to the guide sequence. In this specification, the strand including the target sequence is referred to as a "target strand." When the target nucleic acid or the target gene is single-stranded, the strand may be a target strand. When the target nucleic acid or the target gene is double-stranded, one of the double strands may be a target strand, and a strand complementary to the target strand may exist. The strand complementary to the target strand is referred to as a "non-target strand." The "non-target strand" comprises a PAM (Protospacer Adjacent Motif) sequence and a protospacer sequence. The PAM sequence is a sequence recognized by Cas12f1 or a variant protein thereof in the CRISPR/Cas12f1 system or the USH2A gene editing system. The protospacer sequence, which is located at the 5'-end or the 3'-end of the PAM sequence, is a sequence having complementarity to a target sequence or a sequence that forms a complementary bond with a target sequence. Correlation between the protospacer sequence and the target sequence is similar to correlation between the target sequence and the guide sequence. Due to these characteristics, in general, a guide sequence may be designed using a protospacer sequence. That is, a guide sequence which binds complementarily to a target sequence may be designed as a nucleotide sequence having the same nucleotide sequence as the protospacer sequence, and the guide sequence is designed by replacing T with U the protospacer sequence.

The term "gene editing system," "nucleic acid editing system," or "CRISPR/Cas system" refers to a complex or system comprising a nucleic acid degrading enzyme, such as a gene editing protein or an endonuclease, and a nucleic acid targeting molecule corresponding to the nucleic acid degrading enzyme, which can bind to or interact with a target gene or a target nucleic acid to cleave, edit, repair, and/or restore a target region of the target gene or target nucleic acid. Here, the nucleic acid targeting molecule may be represented by a guide RNA (gRNA), but is not limited thereto. Meanwhile, the gene editing system may exist in any form that allows editing of a target gene, for example, it may be in the form of a composition comprising a complex that comprises a nucleic acid degrading enzyme and a nucleic acid targeting molecule. Alternatively, the gene editing system may be in the form of a kit, wherein the nucleic acid degrading enzyme and the nucleic acid targeting molecule are each contained in separate compositions. Alternatively, the gene editing system may be a vector system or composition comprising at least one vector which comprises a nucleic acid encoding a nucleic acid degrading enzyme and a nucleic acid encoding a nucleic acid targeting molecule.

The term "endonuclease" may be used interchangeably with "gene editing protein," "nucleic acid editing protein," "nucleic acid degrading protein" or "nucleic acid cleavage protein," and the molecules referred to as these endonucleases or proteins refer to proteins or polypeptides that are capable of catalyzing (for example, cleaving) a region within a chain of a nucleic acid or polynucleotide (for example, double-stranded DNA, single-stranded DNA, RNA, a hybrid duplex of DNA and RNA, or synthetic DNA). In some embodiments, the molecule may refer to an (endo)nuclease that can recognize a protospacer adj acent motif (PAM) present in a target nucleic acid, that is, DNA or RNA, or a target gene, and then induce DNA double-strand breaks (DSBs) at a nucleotide sequence within or outside the target nucleotide sequence. (Endo)nucleases may cleave polynucleotides symmetrically, leaving blunt ends, or may cleave the same at non-directly facing positions, creating overhangs called sticky ends. In addition, the endonuclease, gene editing protein, or the like also referred to as an effector protein constituting a gene editing system or a nucleic acid construct for gene editing. Here, the effector protein may be a nucleic acid degrading protein capable of binding to a guide RNA (gRNA) or an engineered gRNA, or a peptide fragment capable of binding to a target nucleic acid or a target gene.

The terms "protein," "polypeptide," and "peptide" may be used interchangeably and refer to a polymer of amino acids of any length which may comprise genetically encoded and non-genetically encoded amino acids, chemically or biochemically modified or derivatized amino acids, and polypeptides having modified peptide backbones. The terms encompass all fusion proteins including, but not limited to, fusion proteins with heterologous amino acid sequences, with or without N-terminal methionine residues, fusions with heterologous and homologous leader sequences; immunologically tagged proteins, and the like.

The term "amino acid" collectively refers to the 20 types of amino acids that are synthesized through the transcription and translation of genes in an organism's body. Specifically, the amino acids comprise alanine (Ala, A), arginine (Arg, R), asparagine (Asn, N), aspartic acid (Asp, D), cysteine (Cys, C), glutamic acid (Glu, E), glutamine (Gln, Q), glycine (Gly, G), histidine (His, H), isoleucine (Ile, I), leucine (Leu, L), lysine (Lys K), methionine (Met, M), phenylalanine (Phe, F), proline (Pro, P), serine (Ser, S), threonine (Thr, T), tryptophan (Trp, W), tyrosine (Tyr, Y), and valine (Val, V). Each of these amino acids has a corresponding DNA codon and may be represented by the general amino acid one-letter or three-letter notation. The term amino acid generally refers to a standard amino acid that occurs naturally, but the object referred to by the term should be appropriately interpreted depending on the context, and may include non-naturally occurring amino acids, artificial amino acids, modified amino acids, and the like, and all other meanings that may be recognized by a person skilled in the art.

The term "guide RNA (gRNA)" refers to RNA that is capable of forming a complex with a molecule referred to as an endonuclease, a gene editing protein, a nucleic acid degrading protein, or the like, and interacting with (for example, hybridizing to, forming a complementary bond(s) with, or forming a hydrogen bond(s) with) a target nucleotide sequence, and comprises a guide sequence having sufficient complementarity with the target nucleotide sequence to cause sequence-specific binding of the complex to the target nucleotide sequence. As used herein, a guide RNA or a guide molecule may be used interchangeably.

The terms "tracrRNA (trans-activating crRNA)" and "crRNA (CRISPR RNA)" include all meanings that can be recognized by those skilled in the art in the field of gene editing technology. These terms may be used to refer to respective molecules of a dual guide RNA found in nature, and may also be used to refer to respective corresponding portions of a single guide RNA (sgRNA) in which the tracrRNA and the crRNA are connected by a linker. Unless otherwise stated, the description tracrRNA and crRNA simply means tracrRNA and crRNA that constitute a guide RNA in a gene editing system and the like.

The term "scaffold region" refers collectively to a portion of a guide RNA (gRNA) which can interact with a molecule called endonuclease, homology-directed repair protein, gene editing protein, nucleic acid degrading protein, or the like, and may be used to refer to the remaining portion of a guide RNA found in nature, excluding a spacer.

The term "stem" refers to a nucleic acid region having a secondary structure that comprises a nucleotide region capable of forming a double strand. A configuration in which a double strand is connected primarily by a region of single-stranded nucleotides (a loop region) is referred to as a "stem-loop." The terms "stem" and "stem-loop" may be used interchangeably and should be interpreted appropriately depending on the context.

The terms "guide sequence", "spacer," or "spacer sequence" may be used interchangeably, and refer to a polynucleotide within the CRISPR/Cas system which is capable of interacting with (for example, hybridizing to, forming a complementary bond(s) with, or forming a hydrogen bond(s) with) a target sequence portion. For example, the guide sequence or spacer sequence refers to 10 to 50 consecutive nucleotides linked directly or indirectly through a linker or the like to or near the 3'-end of crRNA, which constitutes a guide RNA, in a gene editing system.

The term "engineered" may be used interchangeably with "non-naturally occurring," "artificial" or "modified," and means that something is not in its natural form, state, and the like as found in nature. When the term is used with respect to an endonuclease, a gene editing protein, a nucleic acid degrading protein, Cas12f1 (CWCas12f1, Un1Cas12f1, and the like) protein or the like, it means that the endonuclease or protein is substantially free of at least one component that is found in nature or naturally occurring, or substantially contains at least one component that is not found in nature or non-naturally occurring. For example, an "engineered endonuclease" means a nuclease obtained by applying an artificial modification to the configuration (for example, amino acid sequence) of a nuclease present in nature, and may also be referred to herein as a "variant" or "mutant." The term "variant" should be understood to mean the expression of a characteristic having a pattern that deviates from the one which occurs in nature. For example, when referring to Cas12f1 or a variant protein thereof, the variant protein may mean a variant of (wild-type) Cas12f1. When the term is used with respect to a guide RNA, a guide polynucleotide or a nucleic acid molecule, it means that the guide RNA, guide polynucleotide, or nucleic acid molecule is substantially free of at least one component that is found in nature or naturally occurring, or substantially contains at least one component that is not found in nature or non-naturally occurring. For example, an "engineered guide RNA" means a gRNA obtained by applying an artificial modification to the configuration (for example, sequence) of a guide RNA (gRNA) present in nature, and may be referred to herein as an "augmented RNA."

The term "wild-type" is a term of the art understood by those skilled in the art and means a typical form of an organism, strain, gene, or characteristic as it occurs in nature to the extent that it is distinguishable from mutant or variant forms. The term "variant" or "mutant" should be understood to mean its expression of a characteristic having a pattern that deviates from the one which occurs in nature. For example, when referred to as Cas12f1 variant (protein), the variant protein may refer to a variant with respect to the wild-type Cas12f1.

The term "vector" unless otherwise specified, refers collectively to any material capable of transporting a genetic material into a cell. For example, a vector may be a DNA molecule comprising a genetic material to be delivered that is a nucleic acid encoding an endonuclease or an effector protein (Cas protein) of a gene editing system, and/or a nucleic acid encoding a guide RNA thereof; however, the vector is not limited thereto. In addition, in the present disclosure, the "vector" may be an "expression vector" including essential regulatory elements operably linked to allow the inserted gene to be expressed normally.

The term "operably linked" means, in the context of gene expression technology, that a particular component is linked to another component so as to permit the particular component function in the intended manner. For example, when a promoter sequence is operably linked to a sequence encoding a protein A, it means that the promoter is linked to the sequence encoding the protein A so as to transcribe and/or express the sequence encoding the protein A in a cell. In addition, the term includes all other meanings generally recognized by those skilled in the art and may be appropriately interpreted depending on the context.

The terms "nucleotide" and "nucleic acid" may be used interchangeably and refer to a polymeric form of nucleotides of any length, which may be either ribonucleotides or deoxynucleotides. Thus, the terms include, but are not limited to, single-, double-, or multi-stranded DNA or RNA, genomic DNA, cDNA, a DNA-RNA hybrid, or a polymer comprising purine and pyrimidine bases or other natural, chemically or biochemically modified, non-natural, or derivatized nucleotide bases. The terms "polynucleotide" and "nucleic acid" should be understood to include single-stranded (such as sense or antisense) and double-stranded polynucleotides that are applicable to the embodiment described herein.

The term "nucleic acid construct" refers to a structure that comprises, as components, a nucleotide sequence encoding an endonuclease, a nucleic acid editing protein, a nucleic acid degrading protein, or the like and/or a nucleotide sequence encoding a guide RNA, and if necessary, may further comprise nucleotide sequences encoding various types of (poly)peptides or linkers. The nucleic acid construct may be used as a component of the CRISPR/Cas system, vector system, or hypercompact gene editing system (hypercompact TaRGET system) for homology-directed repair of the present disclosure.

The term "NLS (nuclear localization sequence or signal)" refers to a signal peptide or amino acid sequence that promotes introduction of a material from outside the nucleus into the nucleus, for example, by nuclear transport. The term "NES (nuclear export sequence or signal)" refers to a signal peptide or amino acid sequence that promotes transport of a material from inside the nucleus to the outside of the nucleus, for example, by nuclear transport. The terms NLS or NES are known in the relevant art and may be clearly understood by those skilled in the art.

The term "subject" is used interchangeably with "individual" or "patient" and may be a mammal in need of prevention or treatment of Usher syndrome, such as primate (for example, human), companion animal (for example, dog and cat), domestic animal (for example, cow, pig, horse, sheep, and goat), and laboratory animal (for example, rat, mouse, and guinea pig). In an embodiment of the present disclosure, the subject is a human.

The term "treatment" generally refers to obtaining a desired pharmacological and/or physiological effect. Such an effect has a therapeutic effect in that it partially or completely cures a disease and/or harmful effects caused by the disease. Desirable therapeutic effects include, but are not limited to, prevention of occurrence or recurrence of a disease, improvement of symptoms, reduction of any direct or indirect pathological consequences of a disease, prevention of metastasis, reduction of disease progression rate, improvement or alleviation of disease state, and remission or improved prognosis. Preferably, "treatment" may refer to medical intervention for an already manifested disease or disorder. More preferably, "treatment" may be deletion of a segment comprising exon 13 in the USH2A gene or restoration of the reading frame of the USH2A gene resulting therefrom.

The term "about" refers to an amount, level, value, number, frequency, percent, dimension, size, amount, weight or length that varies by approximately 30, 25, 20, 15, 10, 9, 8, 7, 6, 5, 4, 3, 2, or 1% with respect to a reference amount, level, value, number, frequency, percent, dimension, size, amount, weight or length. For example, the term "about" may mean x ± 10% when used in relation to a value x expressed as a number or numerical value.

All technical terms used in the present disclosure, unless otherwise defined, include all meanings recognized by a person skilled in the art and are used in the same sense as generally understood, and can be interpreted appropriately depending on the context. In addition, although preferred methods or samples are described in this specification, those similar or equivalent thereto are also encompassed in the scope of the present disclosure.

### II. Usher syndrome and therapeutic strategy thereof

The most common mutations in the USH2A gene that cause type 2 (more specifically, type 2A) Usher syndrome are the c.2276G>T and c.2299delG mutations that occur in exon 13 of the USH2A gene. The c.2276G>T mutation refers to a point mutation in exon 13 of the USH2A gene where the 2276th base guanine is replaced with thymine. The c.2299delG mutation refers to deletion of the 2299th base guanine in exon 13 of the USH2A gene. These mutations result in expression of altered mRNA, thereby causing the symptoms of Usher syndrome. As a therapeutic strategy to alleviate these symptoms, a method of artificially deleting exon 13 of the USH2A gene containing the mutation has been proven to be effective through animal experiments. In order to implement this proven therapeutic strategy more sustainably and efficiently, therapeutics that incorporate various techniques are being developed, and therapeutics that utilize the CRISPR/Cas system, known as a gene scissor, are receiving particular attention. The present inventors have developed a gene editing technique that efficiently deletes exon 13 containing c.2276G>T and/or c.2299delG mutations using two guide RNAs that exhibit high specificity particularly for the USH2A locus.

Meanwhile, the present inventors increased efficiency of the CRISPR/Cas12f1 system, which is a new CRISPR/Cas system, through a previous study and named it TaRGET (Tiny nuclease augmented RNA-based Genome Editing Technology) system. The CRISPR/Cas12f1 system is a novel CRISPR/Cas system that was first reported in a previous study [see Harrington et al., Science, 362, 839-842, 2018], and despite the advantage of having a remarkably small effector protein, it has been reported that there is no or very low double-strand DNA cleavage activity, which limits its application in gene editing technology. To overcome these limitations, the present inventors have researched, developed, and completed an engineered guide RNA that has enhanced cleavage activity for double-stranded DNA (dsDNA) so that it can be utilized for gene editing (see Korean Patent Application Nos. 10-2021-0051552, 10-2021-0050093, and 10-2021-0044152, and International Application Nos. PCT/KR2021/013898, PCT/KR2021/013923, and PCT/KR2021/013933). Compared to the CRISPR/Cas9 system, the TaRGET system has a significantly smaller Cas protein, which makes it possible to solve the difficulties in loading most previously studied Cas proteins into adeno-associated virus (AAV) due to their size and the resulting difficulties in applying it as gene therapy. In addition, the TaRGET system has a feature of inducing dsDNA cleavage outside or distal to the protospacer sequence. This feature means that the dsDNA cleavage-NHEJ cycle may be repeatedly performed through additional attempts even after the first attempt of non-homologous end joining (NHEJ)-mediated indel mutation until the protospacer sequence is significantly altered. These multiple cleavage and repair processes may provide more opportunities for reliable target sequence (and protospacer sequence) cleavage, and the TaRGET system with this feature can be regarded as having excellent clinical utility in the field of gene therapy.

Based on the previous strategy for treating Usher syndrome, the present inventors introduced a novel TaRGET system for the treatment of Usher syndrome. The introduction of the TaRGET system has advantages over the existing CRISPR/Cas9 system, such as ease of loading into AAV and reliable gene editing through multiple cleavage and repair processes. Accordingly, the present inventors have developed a therapeutic agent and a therapeutic method for Usher syndrome using the TaRGET system with the above-described advantages.

Hereinafter, a USH2A gene editing system and composition for treating Usher syndrome implemented by applying the TaRGET system (for convenience, hereinafter referred to as the CRISPR/Cas12f1 system or USH2A gene editing system), a vector system, a guide RNA, and a method for treating Usher syndrome using the same are described in detail.

### III. CRISPR/Cas system for USH2A gene editing

An aspect of the present disclosure described herein relates to a CRISPR/Cas12f1 system for editing a USH2A gene (for example, a human USH2A gene) or for treating Usher syndrome. Usher syndrome is a disease caused by c.2276G>T, c.2299delG mutations occurring in exon 13 of the USH2A gene as described above. For the treatment of the disease, a viable strategy is to induce deletion of exon 13 containing the mutations that cause the disease, thereby allowing expression of a normally functioning USH2A protein.

The CRISPR/Cas12f1 system is used to delete exon 13 of the USH2A gene, and is also referred to as a USH2A gene editing system. The CRISPR/Cas12f1 system or USH2A gene editing system can more effectively delete the causative exon 13 of the USH2A gene through reliable gene editing with multiple cleavage and repair processes, thereby increasing the therapeutic effect. In addition, the CRISPR/Cas12f1 system or USH2A gene editing system is significantly smaller than the existing CRISPR/Cas9 systems, so that additional space (capacity) can be secured even when using a delivery vehicle with a limited packaging size, such as AAV, and is thus more advantageous in application as a therapeutic agent.

The CRISPR/Cas12f1 system or USH2A gene editing system according to the present disclosure comprises (i) an endonuclease comprising at least one Cas12f1 molecule (for example, Cas12f1 or a variant protein thereof) or a nucleic acid encoding the endonuclease; and (ii) at least one (for example, two) guide RNA or guide molecule, or nucleic acid encoding the same.

More specifically, the present disclosure provides an editing system for the USH2A gene comprising an endonuclease comprising Cas12f1 molecule (for example, Cas12f1 or a variant protein thereof) or a nucleic acid encoding the endonuclease; and guide RNAs comprising two or more guide sequences capable of hybridizing to target sequences in a USH2A gene, the guide RNAs being (i) a first guide RNA comprising a first guide sequence capable of hybridizing to a target sequence of contiguous 15 to 30 bp in length which is located in a region upstream of USH2A exon 13 and is adjacent to a protospacer-adjacent motif (PAM) sequence recognized by the Cas12f1 molecule, or a nucleic acid encoding the first guide RNA; and (ii) a second guide RNA comprising a second guide sequence capable of hybridizing to a target sequence of contiguous 15 to 30 bp in length, which is located in a region downstream of USH2A exon 13 and is adjacent to a protospacer-adjacent motif (PAM) sequence recognized by the Cas12f1 molecule, or a nucleic acid encoding the second guide RNA.

The CRISPR/Cas12f1 system or USH2A gene editing system may generate one or more cleavages (for example, single-stranded breaks or double-stranded breaks) near a target site of the USH2A gene (for example, a upstream region of exon 13, a downstream region of exon 13, or both regions). The one or more cleavages may occur outside the target sequence or inside the 3'-end (for example, 1 to 5 bp inward).

The two or more types of guide RNA may target an upstream region and a downstream region of exon 13 in the USH2A gene, respectively. In addition, the two or more types of guide RNA may target the intron 12 and intron 13 regions in the USH2A gene, respectively. Here, exon 13 located between the intron 12 and intron 13 regions comprises c.2276G>T, c.2299delG mutations.

In an embodiment, the USH2A gene editing system may comprise two or more guide RNAs that recognize and/or target different target sequences in the USH2A gene. Here, the different target sequences may partially overlap with each other.

In another embodiment, the guide RNA may target an adjacent region of exon 13 in the USH2A gene to generate a cleavage (for example, a single-stranded break or a double-stranded break).

In yet another embodiment, two guide RNAs may target the upstream and downstream regions of exon 13 in the USH2A gene, respectively, to generate one or more cleavages (for example, two single-stranded breaks or two double-stranded breaks).

In still yet another embodiment, two or more guide RNAs may be used to generate two or more sets of cleavages (for example, two double-stranded breaks, one double-stranded break, and one single-stranded break; or two pairs of single-stranded breaks).

For example, the system disclosed herein may induce deletion of a segment comprising exon 13 in such a way that two guide RNA molecules, which target an upstream region and a downstream region of exon 13 of the USH2A gene, respectively, generate a cleavage within the regions together with an endonuclease comprising a Cas12f1 molecule (for example, Cas12f1 or a variant protein thereof).

In still yet another embodiment, the USH2A gene editing system or the endonuclease included therein may induce a double-strand break within or outside the target sequence. Without being bound by theory, when a double-strand break is induced outside the target sequence, the target sequence and the PAM sequence are hardly modified even after the break is repaired, and thus can be recognized and cleaved again by the USH2A gene editing system. Therefore, the USH2A gene editing system may exhibit highly efficient deletion through reliable cleavage of the target sequence (and protospacer sequence) with multiple cleavage and repair processes.

In still yet another embodiment, in the system disclosed herein, the endonuclease comprising Cas12f1 molecule (for example, Cas12f1 or a variant protein thereof) and a guide RNA may be included in the form of a complex, for example, in the form of a ribonucleoprotein particle (RNP). The complex may comprise a guide RNA and two Cas12f1 proteins or variants thereof (see Satoru N. Takeda et al., Molecular Cell, 81, 1-13, (2021)). The complex may be formed by an interaction between the guide RNA and the Cas12f1 molecule.

Hereinafter, each component of the gene editing system (CRISPR/Cas12f1 system), composition, and vector system provided in the present disclosure and a method for producing the same are described in detail.

### 1. Endonuclease comprising Cas12f1 molecule

The USH2A gene editing system based on CRISPR/Cas12f1 of the present disclosure comprises an endonuclease having a Cas12f1 molecule (for example, Cas12f1 or a variant protein thereof) as an effector. The Cas12f1 molecule is a (small) endonuclease characterized by exhibiting excellent activity in cleaving a target site of a target nucleic acid and being significantly smaller in size by about 1/3 compared to the existing CRISPR/Cas9 system.

The Cas12f1 protein is one of the effector proteins named Cas14 in a previous study (see Harrington et al., Science, 362, 839-842, 2018), and is also called Cas14a1 protein. The protein referred to as Cas12f1 molecule disclosed herein may refer to a wild-type Cas12f1 protein existing in nature. In addition, the Cas12f1 molecule may be a variant of the wild-type Cas12f1 protein. The variant may also be referred to as a "Cas12f1 variant." The Cas12f1 variant may be a variant having the same function as the wild-type Cas12f1 protein, a variant of which some or all functions are modified, and/or a variant of which additional functions are added. The meaning of the Cas12f1 molecule may be appropriately interpreted depending on the context, and is interpreted in the broadest sense unless specified otherwise.

Hereinafter, the Cas12f1 molecule (including Cas12f1 and a variant protein thereof) included in the USH2A gene editing system is described in detail.

### 1.1. Wild-type Cas12f1 protein

The present inventors have confirmed that TnpB (Transposon-associated transposase B) protein derived from *Candidates Woesearchaeota* archaeon has an amino acid sequence similar to the Un1Cas12f1 protein, has a molecular weight that is about 1/3 smaller than that of an existing nucleic acid degrading protein including the Cas9 protein, which has been studied the most to date, and has a significantly higher nucleic acid cleavage efficiency for a target nucleic acid or target gene. In the present specification, the TnpB having an amino acid sequence similar to Un1Cas12f1 protein is referred to as CWCas12f1. CWCas12f1 may be collectively referred to as Cas12f1 protein together with Un1Cas12f1, and may belong to a variant of Cas12f1 in its relationship with Un1Cas12f1.

In addition, the present inventors have confirmed that engineered guide RNAs having a small size obtained by modifying the wild-type Cas12f1 guide RNA may induce excellent nucleic acid cleavage efficiency (for example, a double-strand break) together with the Cas12f1 protein such as CwCas12f1 or Un1Cas12f1. With respect to the engineered guide RNA, the entire contents described in International Application No. PCT/KR2020/014961, filed on October 29, 2020, and International Application Nos. PCT/KR2021/013933, PCT/KR2021/013898, and PCT/KR2021/013923, which were all filed on October 8, 2021, are expressly incorporated herein by reference.

The hypercompact gene editing system comprising an engineered guide RNA and a Cas12f1 molecule (for example, Cas12f1 or a variant protein thereof, such as CwCas12f1 or Un1Cas12f1) disclosed herein may be referred to as "CRISPR/Cas12f1 system" or "TaRGET system," and these terms may be used interchangeably.

It has been reported that the Cas12f1 protein forms a complex with a guide RNA such that two Cas12f1 protein molecules bind to a guide RNA in the form of a dimer, and that all or part of the domain of the Cas12f1 protein recognizes a specific part of the scaffold region of the guide RNA to form a CRISPR/Cas12f1 complex (see Takeda et al., Structure of the miniature type V-F CRISPR-Cas effector enzyme, Molecular Cell 81, 1-13, 2021; and Xiao et al., Structural basis for the dimerization-dependent CRISPR-Cas12f nuclease, bioRxiv, 2020). The Cas12f1 protein (for example, Cas12f1 protein or a variant thereof) may generate a double-stranded or single-stranded break in a target nucleic acid or a target gene. Deletion of a desired gene segment may be induced by such a double-stranded or single-stranded break.

In an embodiment, the Cas12f1 molecule may be derived from a Cas14 family (see Harrington et al., Science 362, 839-842 (2018); and US 2020/0172886 A1).

In another embodiment, the Cas12f1 molecule may be a Cas14a1 or Un1Cas12f1 protein derived from a uncultured archaeon (see Harrington et al., Science 362, 839-842 (2018); and US 2020/0172886 A1). For example, the Cas12f1 molecule (for example, Cas14a1 or Un1Cas12f1 protein) may comprise or consist of the amino acid sequence of SEQ ID NO: 364 (see Table 9).

In yet another embodiment, the Cas12f1 molecule may be TnpB (transposon-associated transposase B) protein derived from the *Candidates Woesearchaeota* archaeon. The TnpB protein is a protein conventionally known as a transposase. To date, the TnpB protein has been known only as a transposon-encoded nuclease, and it is not known whether the TnpB protein has Cas endonuclease activity. In this specification, the TnpB protein may be referred to as a variant or the like of CWCas12f1 or Un1Cas12f1, and unless otherwise stated, a protein referred to as Cas12f1 comprises CWCas12f1.

In addition, a guide RNA for the TnpB protein has also not been known. The present inventors have confirmed for the first time that TnpB variant or engineered TnpB, which is based on the TnpB protein sequence, has excellent endonuclease activity of recognizing a target nucleic acid or a target gene and cleaving a double-stranded DNA of the target site while having a similar size to a Cas12f1 protein, which belongs to the group with the smallest molecular weight among nucleic acid degrading proteins, and have constructed an engineered guide RNA that exhibits excellent gene editing activity when used together with the TnpB or variant protein thereof. For details regarding the "engineered guide RNA," see the entire contents disclosed in the following section "3. Engineered guide RNA."

In an embodiment, the Cas12f1 molecule may be CWCas12f1 protein. Here, the CWCas12f1 protein may comprise or consist of the amino acid sequence of SEQ ID NO: 360 (see Table 9).

In the present disclosure, there is provided a nucleic acid encoding the Cas12f1 molecule or an endonuclease comprising the same. The nucleic acid encoding the Cas12f1 molecule or an endonuclease comprising the same may be codon optimized so that it can be expressed in a subject (for example, a human) to which the Cas12f1 molecule or an endonuclease comprising the same is to be introduced. As a specific example, for a human codon optimized nucleotide sequence encoding the Cas12f1 molecule (CWCas12f1 or Un1Cas12f1), the nucleotide sequence of SEQ ID NO: 365 or SEQ ID NO: 369 is provided (see Example 1).

### 1.2. Cas12f1 variant protein

In another aspect, the Cas12f1 molecule (for example, Cas12f1 or a variant thereof) or an endonuclease comprising the same may comprise or consist of an amino acid sequence having at least 70% sequence identity to an amino acid sequence of Un1Cas12f1 consisting of the amino acid sequence of SEQ ID NO: 364 or CWCas12f1 protein consisting of the amino acid sequence of SEQ ID NO: 360. As an example, the Cas12f1 molecule or an endonuclease comprising the same may be or comprise a protein comprising a modified amino acid sequence having at least 70%, at least 72%, at least 74%, at least 76%, at least 78%, at least 80%, at least 82%, at least 84%, at least 86%, at least 88%, at least 88%, at least 90%, at least 91%, at least 92%, at least 93%, at least 94%, at least 95%, at least 96%, at least 97%, at least 98%, or at least 99% sequence identity to the amino acid sequence of SEQ ID NO: 360 or SEQ ID NO: 364. Such a modified protein may be referred to herein as a "Cas12f1 variant." Hereinafter, each variant is described in detail.

### (1) Cas12f1 variant (mutant)

According to one aspect of the present disclosure, the Cas12f1 molecule (for example, Cas12f1 or a variant thereof) may be Cas12f1 variant protein. The Cas12f1 variant may comprise at least one amino acid modification, such as deletion, substitution, insertion or addition, compared to the amino acid sequence of the wild-type Cas12f1 protein. For example, the Cas12f1 variant may have a sequence in which at least one amino acid residue is deleted, substituted, inserted and/or added at the C-terminus, N-terminus or within the amino acid sequence of the wild-type Cas12f1 protein, and such a Cas12f1 variant may also be referred to as a "Cas12f1 mutant."

In another embodiment, the Cas12f1 variant may be such that it has at least one random amino acid residue added to the amino acid sequence of the wild-type Cas12f1 protein. In a more specific example, the Cas12f1 variant may be a variant having at least one random amino acid residue added to the N-terminus and/or C-terminus of the amino acid sequence of wild-type Cas12f1 (for example, Un1Cas12f1 or CWCas12f1) or a variant protein thereof. The present inventors have confirmed that among the variants having amino acids added to the N-terminus and/or C-terminus of the wild-type Cas12f1 protein, there are variants having a function equivalent to the wild-type Cas12f1. For this purpose, reference may be made to Korean Patent Application No. 10-2021-0181875, the entire disclosure of which should be deemed to be incorporated herein. Preferably, the Cas12f1 variant may be such that it has 1, 2, 3, 4, 5, 6, 7, 8, 9, 10, 11, 12, 13, 14, 15, 16, 17, 18, 19, 20, 21, 22, 23, 24, 25, 26, 27, 28, 29, or 30 amino acids added to the N-terminus and/or C-terminus of wild-type Cas12f1 or the variant protein thereof. For example, the Cas12f1 variant protein may comprise an amino acid sequence in which 1 to 28 amino acids are added to the N-terminus of the amino acid sequence of wild-type Un1Cas12f1 (for example, the amino acid sequence of SEQ ID NO: 364). As a specific example of such Un1Cas12f1 variants, the present disclosure provides CWCas12f1-v1 protein (SEQ ID NO: 361), which further comprises 26 amino acids derived from the N-terminus of CasX at the N-terminus of the Un1Cas12f1 protein, CWCas12f1-v2 protein (SEQ ID NO: 362), which further comprises 28 random amino acid sequences at the N-terminus of the Un1Cas12f1 protein, and CWCas12f1-v3 protein (SEQ ID NO: 363), which further comprises 26 random amino acid sequences at the N-terminus of the Un1Cas12f1 protein. The Cas12f1 variant comprising an amino acid sequence in which 1 to 28 amino acids are added to the N-terminus of the amino acid sequence of the wild-type Un1Cas12f1 (SEQ ID NO: 364) may be defined, in another aspect, as a Cas12f1 variant comprising an amino acid sequence in which 1 to 28 amino acids are deleted or substituted at the N-terminus of the amino acid sequence of the wild-type CWCas12f1 (SEQ ID NO: 360). The specific amino acid sequences of the CWCas12f1-v1 protein (SEQ ID NO: 361), the CWCas12f1-v2 protein (SEQ ID NO: 362), and the CWCas12f1-v3 protein (SEQ ID NO: 363) are as follows:
"CWCas12f1-v1 protein", 
MEKRINKIRKKLSADNATKPVSRSGPMAKNTIITKTLKLRIVRPYNSAEVEKIVADEKNNREKIALEKNKDKVK
EACSKHLKVAAYCTTQVERNACLFCKARKLDDKFYQKLRGQFPDAVFWQEISEIFRQLQKQAAEIYNQSLIEL
YYEIFIKGKGIANASSVEHYLSDVCYTRAAELFKNAAIASGLRSKIKSNFRLKELKNMKSGLPTTKSDNFPIP
LVKQKGGQYTGFEISNHNSDFIIKIPFGRWQVKKEIDKYRPWEKFDFEQVQKSPKPISLLLSTQRRKRNKGWS
KDEGTEAEIKKVMNGDYQTSYIEVKRGSKIGEKSAWMLNLSIDVPKIDKGVDPSIIGGIDVGVKSPLVCAINN
AFSRYSISDNDLFHFNKKMFARRRILLKKNRHKRAGHGAKNKLKPITILTEKSERFRKKLIERWACEIADFFI
KNKVGTVQMENLESMKRKEDSYFNIRLRGFWPYAEMQNKIEFKLKQYGIEIRKVAPNNTSKTCSKCGHLNNYF
NFEYRKKNKFPHFKCEKCNFKENADYNAALNISNPKLKSTKEEP (SEQ ID NO: 361);
"CWCas12f1-v2 protein", 
MAGGPGAGSAAPVSSTSSLPLAALNMRVMAKNTITKTLKLRIVRPYNSAEVEKIVADEKNNREKIALEKNKDK
VKEACSKHLKVAAYCTTQVERNACLFCKARKLDDKFYQKLRGQFPDAVFWQEISEIFRQLQKQAAEIYNQSLI
ELYYEIFIKGKGIANASSVEHYLSDVCYTRAAELFKNAAIASGLRSKIKSNFRLKELKNMKSGLPTTKSDNFP
IPLVKQKGGQYTGFEISNHNSDFIIKIPFGRWQVKKEIDKYRPWEKFDFEQVQKSPKPISLLLSTQRRKRNKG
WSKDEGTEAEIKKVMNGDYQTSYIEVKRGSKIGEKSAWMLNLSIDVPKIDKGVDPSIIGGIDVGVKSPLVCAI
NNAFSRYSISDNDLFHFNKKMFARRRILLKKNRHKRAGHGAKNKLKPITILTEKSERFRKKLIERWACEIADF
FIKNKVGTVQMENLESMKRKEDSYFNIRLRGFWPYAEMQNKIEFKLKQYGIEIRKVAPNNTSKTCSKCGHLNN
YFNFEYRKKNKFPHFKCEKCNFKENADYNAALNISNPKLKSTKEEP (SEQ ID NO: 362);
"CWCas12f1-v3 protein",

MAGGPGAGSAAPVSSTSSLPLAALNMMAKNTITKTLKLRIVRPYNSAEVEKIVADEKNNREKIALEKNKDKVK
EACSKHLKVAAYCTTQVERNACLFCKARKLDDKFYQKLRGQFPDAVFWQEISEIFRQLQKQAAEIYNQSLIEL
YYEIFIKGKGIANASSVEHYLSDVCYTRAAELFKNAAIASGLRSKIKSNFRLKELKNMKSGLPTTKSDNFPIP
LVKQKGGQYTGFEISNHNSDFIIKIPFGRWQVKKEIDKYRPWEKFDFEQVQKSPKPISLLLSTQRRKRNKGWS
KDEGTEAEIKKVMNGDYQTSYIEVKRGSKIGEKSAWMLNLSIDVPKIDKGVDPSIIGGIDVGVKSPLVCAINN
AFSRYSISDNDLFHFNKKMFARRRILLKKNRHKRAGHGAKNKLKPITILTEKSERFRKKLIERWACEIADFFI
KNKVGTVQMENLESMKRKEDSYFNIRLRGFWPYAEMQNKIEFKLKQYGIEIRKVAPNNTSKTCSKCGHLNNYF
NFEYRKKNKFPHFKCEKCNFKENADYNAALNISNPKLKSTKEEP (SEQ ID NO: 363).

In the present disclosure, there is provided a nucleic acid encoding the Cas12f1 variant protein. The nucleic acid encoding the Cas12f1 variant protein may be codon optimized so that it can be expressed in a subject (for example, a human) to which the Cas12f1 variant protein is to be introduced. In a specific example, human codon optimized nucleotide sequences encoding the CWCas12f1-v1 protein, CWCas12f1-v2 protein, and CWCas12f1-v3 protein are provided below (see SEQ ID NOs: 366 to 368):
"Human codon-optimized nucleic acid encoding the CWCas12f1-v1 protein", 
5'-ATGGAAAAGAGAATCAACAAGATCAGGAAGAAGCTGAGCGCCGACAACGCCACCAAGCCTGTGTCTAGGA
GTGGCCCCCATGGCCAAGAACACAATTACAAAGACACTGAAGCTGAGGATCGTGAGACCATACAACAGCGCTG
AGGTCGAGAAGATTGTGGCTGATGAAAAGAACAACAGGGAAAAGATCGCCCTCGAGAAGAACAAGGATAAGGT
GAAGGAGGCCTGCTCTAAGCACCTGAAAGTGGCCGCCTACTGCACCACACAGGTGGAGAGGAACGCCTGTCTG
TTTTGTAAAGCTCGGAAGCTGGATGATAAGTTTTACCAGAAGCTGCGGGGCCAGTTCCCCGATGCCGTCTTTT
GGCAGGAGATTAGCGAGATCTTCAGACAGCTGCAGAAGCAGGCCGCCGAGATCTACAACCAGAGCCTGATCGA
GCTCTACTACGAGATCTTCATCAAGGGCAAGGGCATTGCCAACGCCTCCTCCGTGGAGCACTACCTGAGCGAC
GTGTGCTACACAAGAGCCGCCGAGCTCTTTAAGAACGCCGCTATCGCTTCCGGGCTGAGGAGCAAGATTAAGA
GTAACTTCCGGCTCAAGGAGCTGAAGAACATGAAGAGCGGCCTGCCCACTACAAAGAGCGACAACTTCCCAAT
TCCACTGGTGAAGCAGAAGGGGGGCCAGTACACAGGGTTCGAGATTTCCAACCACAACAGCGACTTTATTATT
AAGATCCCCTTTGGCAGGTGGCAGGTCAAGAAGGAGATTGACAAGTACAGGCCCTGGGAGAAGTTTGATTTCG
AGCAGGTGCAGAAGAGCCCCAAGCCTATTTCCCTGCTGCTGTCCACACAGCGGCGGAAGAGGAACAAGGGGTG
GTCTAAGGATGAGGGGACCGAGGCCGAGATTAAGAAAGTGATGAACGGCGACTACCAGACAAGCTACATCGAG
GTCAAGCGGGGCAGTAAGATTGGCGAGAAGAGCGCCTGGATGCTGAACCTGAGCATTGACGTGCCAAAGATTG
ATAAGGGCGTGGATCCCAGCATCATCGGAGGGATCGATGTGGGGGTCAAGAGCCCCCTCGTGTGCGCCATCAA
CAACGCCTTCAGCAGGTACAGCATCTCCGATAACGACCTGTTCCACTTTAACAAGAAGATGTTCGCCCGGCGG
AGGATTTTGCTCAAGAAGAACCGGCACAAGCGGGCCGGACACGGGGCCAAGAACAAGCTCAAGCCCATCACTA
TCCTGACCGAGAAGAGCGAGAGGTTCAGGAAGAAGCTCATCGAGAGATGGGCCTGCGAGATCGCCGATTTCTT
TATTAAGAACAAGGTCGGAACAGTGCAGATGGAGAACCTCGAGAGCATGAAGAGGAAGGAGGATTCCTACTTC
AACATTCGGCTGAGGGGGTTCTGGCCCTACGCTGAGATGCAGAACAAGATTGAGTTTAAGCTGAAGCAGTACG
GGATTGAGATCCGGAAGGTGGCCCCCAACAACACCAGCAAGACCTGCAGCAAGTGCGGGCACCTCAACAACTA
CTTCAACTTCGAGTACCGGAAGAAGAACAAGTTCCCACACTTCAAGTGCGAGAAGTGCAACTTTAAGGAGAAC
GCCGATTACAACGCCGCCCTGAACATCAGCAACCCTAAGCTGAAGAGCACTAAGGAGGAGCCC-3'
(SEQ ID NO: 366);
"Human codon-optimized nucleic acid encoding the CWCas12f1-v2 protein", 
5'-ATGGCTGGCGGACCAGGCGCAGGTAGTGCTGCGCCAGTTTCTTCAACTTCCTCCCTGCCCCTGGCTGCGC
TTAACATGCGCGTGATGGCCAAGAACACAATTACAAAGACACTGAAGCTGAGGATCGTGAGACCATACAACAG
CGCTGAGGTCGAGAAGATTGTGGCTGATGAAAAGAACAACAGGGAAAAGATCGCCCTCGAGAAGAACAAGGAT
AAGGTGAAGGAGGCCTGCTCTAAGCACCTGAAAGTGGCCGCCTACTGCACCACACAGGTGGAGAGGAACGCCT
GTCTGTTTTGTAAAGCTCGGAAGCTGGATGATAAGTTTTACCAGAAGCTGCGGGGCCAGTTCCCCGATGCCGT
CTTTTGGCAGGAGATTAGCGAGATCTTCAGACAGCTGCAGAAGCAGGCCGCCGAGATCTACAACCAGAGCCTG
ATCGAGCTCTACTACGAGATCTTCATCAAGGGCAAGGGCATTGCCAACGCCTCCTCCGTGGAGCACTACCTGA
GCGACGTGTGCTACACAAGAGCCGCCGAGCTCTTTAAGAACGCCGCTATCGCTTCCGGGCTGAGGAGCAAGAT
TAAGAGTAACTTCCGGCTCAAGGAGCTGAAGAACATGAAGAGCGGCCTGCCCACTACAAAGAGCGACAACTTC
CCAATTCCACTGGTGAAGCAGAAGGGGGGCCAGTACACAGGGTTCGAGATTTCCAACCACAACAGCGACTTTA
TTATTAAGATCCCCTTTGGCAGGTGGCAGGTCAAGAAGGAGATTGACAAGTACAGGCCCTGGGAGAAGTTTGA
TTTCGAGCAGGTGCAGAAGAGCCCCAAGCCTATTTCCCTGCTGCTGTCCACACAGCGGCGGAAGAGGAACAAG
GGGTGGTCTAAGGATGAGGGGACCGAGGCCGAGATTAAGAAAGTGATGAACGGCGACTACCAGACAAGCTACA
TCGAGGTCAAGCGGGGCAGTAAGATTGGCGAGAAGAGCGCCTGGATGCTGAACCTGAGCATTGACGTGCCAAA
GATTGATAAGGGCGTGGATCCCAGCATCATCGGAGGGATCGATGTGGGGGTCAAGAGCCCCCTCGTGTGCGCC
ATCAACAACGCCTTCAGCAGGTACAGCATCTCCGATAACGACCTGTTCCACTTTAACAAGAAGATGTTCGCCC
GGCGGAGGATTTTGCTCAAGAAGAACCGGCACAAGCGGGCCGGACACGGGGCCAAGAACAAGCTCAAGCCCAT
CACTATCCTGACCGAGAAGAGCGAGAGGTTCAGGAAGAAGCTCATCGAGAGATGGGCCTGCGAGATCGCCGAT
TTCTTTATTAAGAACAAGGTCGGAACAGTGCAGATGGAGAACCTCGAGAGCATGAAGAGGAAGGAGGATTCCT
ACTTCAACATTCGGCTGAGGGGGTTCTGGCCCTACGCTGAGATGCAGAACAAGATTGAGTTTAAGCTGAAGCA
GTACGGGATTGAGATCCGGAAGGTGGCCCCCAACAACACCAGCAAGACCTGCAGCAAGTGCGGGCACCTCAAC
AACTACTTCAACTTCGAGTACCGGAAGAAGAACAAGTTCCCACACTTCAAGTGCGAGAAGTGCAACTTTAAGG
AGAACGCCGATTACAACGCCGCCCTGAACATCAGCAACCCTAAGCTGAAGAGCACTAAGGAGGAGCCC-3'
(SEQ ID NO: 367);
"Human codon-optimized nucleic acid encoding the CWCas12f1-v3 protein",

5'-ATGGCTGGCGGACCAGGCGCAGGTAGTGCTGCGCCAGTTTCTTCAACTTCCTCCCTGCCCCTGGCTGCGC
TTAACATGATGGCCAAGAACACAATTACAAAGACACTGAAGCTGAGGATCGTGAGACCATACAACAGCGCTGA
GGTCGAGAAGATTGTGGCTGATGAAAAGAACAACAGGGAAAAGATCGCCCTCGAGAAGAACAAGGATAAGGTG
AAGGAGGCCTGCTCTAAGCACCTGAAAGTGGCCGCCTACTGCACCACACAGGTGGAGAGGAACGCCTGTCTGT
TTTGTAAAGCTCGGAAGCTGGATGATAAGTTTTACCAGAAGCTGCGGGGCCAGTTCCCCGATGCCGTCTTTTG
GCAGGAGATTAGCGAGATCTTCAGACAGCTGCAGAAGCAGGCCGCCGAGATCTACAACCAGAGCCTGATCGAG
CTCTACTACGAGATCTTCATCAAGGGCAAGGGCATTGCCAACGCCTCCTCCGTGGAGCACTACCTGAGCGACG
TGTGCTACACAAGAGCCGCCGAGCTCTTTAAGAACGCCGCTATCGCTTCCGGGCTGAGGAGCAAGATTAAGAG
TAACTTCCGGCTCAAGGAGCTGAAGAACATGAAGAGCGGCCTGCCCACTACAAAGAGCGACAACTTCCCAATT
CCACTGGTGAAGCAGAAGGGGGGCCAGTACACAGGGTTCGAGATTTCCAACCACAACAGCGACTTTATTATTA
AGATCCCCTTTGGCAGGTGGCAGGTCAAGAAGGAGATTGACAAGTACAGGCCCTGGGAGAAGTTTGATTTCGA
GCAGGTGCAGAAGAGCCCCAAGCCTATTTCCCTGCTGCTGTCCACACAGCGGCGGAAGAGGAACAAGGGGTGG
TCTAAGGATGAGGGGACCGAGGCCGAGATTAAGAAAGTGATGAACGGCGACTACCAGACAAGCTACATCGAGG
TCAAGCGGGGCAGTAAGATTGGCGAGAAGAGCGCCTGGATGCTGAACCTGAGCATTGACGTGCCAAAGATTGA
TAAGGGCGTGGATCCCAGCATCATCGGAGGGATCGATGTGGGGGTCAAGAGCCCCCTCGTGTGCGCCATCAAC
AACGCCTTCAGCAGGTACAGCATCTCCGATAACGACCTGTTCCACTTTAACAAGAAGATGTTCGCCCGGCGGA
GGATTTTGCTCAAGAAGAACCGGCACAAGCGGGCCGGACACGGGGCCAAGAACAAGCTCAAGCCCATCACTAT
CCTGACCGAGAAGAGCGAGAGGTTCAGGAAGAAGCTCATCGAGAGATGGGCCTGCGAGATCGCCGATTTCTTT
ATTAAGAACAAGGTCGGAACAGTGCAGATGGAGAACCTCGAGAGCATGAAGAGGAAGGAGGATTCCTACTTCA
ACATTCGGCTGAGGGGGTTCTGGCCCTACGCTGAGATGCAGAACAAGATTGAGTTTAAGCTGAAGCAGTACGG
GATTGAGATCCGGAAGGTGGCCCCCAACAACACCAGCAAGACCTGCAGCAAGTGCGGGCACCTCAACAACTAC
TTCAACTTCGAGTACCGGAAGAAGAACAAGTTCCCACACTTCAAGTGCGAGAAGTGCAACTTTAAGGAGAACG
CCGATTACAACGCCGCCCTGAACATCAGCAACCCTAAGCTGAAGAGCACTAAGGAGGAGCCC-3'
(SEQ ID NO: 368).

In another embodiment, the Cas12f1 variant protein may comprise an amino acid sequence in which 1 to 600 random amino acids are added to the N-terminus or C-terminus of the Cas12f1 protein. As an example, the Cas12f1 variant protein may further comprise an amino acid sequence consisting of 1 to 600 amino acids at the N-terminus or C-terminus of the amino acid sequence of the wild-type CWCas12f1 protein (for example, the amino acid sequence of SEQ ID NO: 360). Here, there is no limitation on the added sequence of 1 to 600 amino acids. For example, the added 1 to 600 amino acids may be the amino acid sequence of SEQ ID NO: 378 or SEQ ID NO: 379. Meanwhile, an NLS or NES sequence may further be included between the added sequence and the Cas12f1 variant protein. For details regarding the NLS or NES, see the entire contents described below.

In another embodiment, the Cas12f1 molecule (for example, Cas12f1 or a variant thereof) may comprise an amino acid sequence having at least 70%, at least 75%, at least 80%, at least 85%, at least 90%, or at least 95% sequence identity to an amino acid sequence selected from the group consisting of SEQ ID NOs: 360 to 364.

In another aspect, the Cas12f1 variant may be such that at least one amino acid in the amino acid sequence of the wild-type Cas12f1 protein is substituted with a different type of amino acid. Here, the substitution may be substitution of one amino acid with one other amino acid. Alternatively, the substitution may be substitution of one amino acid with multiple other amino acids, substitution of multiple amino acids with one other amino acid, or substitution of multiple amino acids with multiple other amino acids. That is, the number of amino acids being substituted and the number of substituting amino acids may be the same as or different from each other.

In another embodiment, the Cas12f1 variant may be such that at least one amino acid residue within the RuvC domain included in the wild-type Cas12f1 protein is deleted or substituted. The RuvC (or RuvC-like) domain, also referred to as an endonuclease domain, comprises a catalytic site that catalyzes nucleic acid cleavage, and is thus directly related to nucleic acid cleavage efficiency. Therefore, by mutating the RuvC domain, the Cas12f1 protein may be engineered to exhibit an equivalent or greater effect (for example, improved nucleic acid cleavage efficiency) while maintaining the same function (for example, nucleic acid cleavage function) as the wild-type Cas12f1 protein.

In another embodiment, the Cas12f1 variant may be such that it is engineered to recognize a PAM sequence other than 5'-TTTA-3' or 5'-TTTG-3'. In a more specific example, the Cas12f1 variant may comprise substitution of at least one amino acid residue selected from the group consisting of amino acids at position 170 (serine), position 174 (tyrosine), position 184 (alanine), position 188 (serine), position 191 (arginine), position 225 (glutamine), position 230 (tyrosine), position 271 (valine), and position 272 (glutamine) with respect to the wild-type sequence of CWCas12f1 (for example, amino acid sequence of SEQ ID NO: 360). Preferably, the Cas12f1 variant may comprise substitution of at least one amino acid residue selected from the group consisting of amino acids at position 170 (serine), position 188 (serine), position 191 (arginine), position 225 (glutamine), and position 272 (glutamine). More preferably, the Cas12f1 variant may comprise one or more selected from the following substitutions with respect to the wild-type sequence (for example, the amino acid sequence of SEQ ID NO: 360): S170T, S188Q, S188H, S188K, R191K, Q225T, Q225F, and Q272K (wherein T is threonine, Q is glutamine, H is histidine, K is lysine, and F is phenylalanine). As such, the specific amino acid sequence information of Cas12f1 variants with an expanded PAM sequence that can be recognized by Cas12f1 is provided in Table 1 below.

**[Table 1]**

| Name | Amino acid sequence | SEQ ID NO |
|---|---|---|
| Engineered CWCas12f1 (S 170T) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLTDVCYTRAAELFKNAAIASGLRSKIKSNFRL KELKNMKSGLPTTKSDNFPIPLVKQKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | 370 |
| Engineered CWCas12f1 (S188Q) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA | 371 |
| | NASSVEHYLSDVCYTRAAELFKNAAIAQGLRSKIKSNFRL KELKNMKSGLPTTKSDNFPIPLVKQKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | |
| Engineered CWCas12f1 (S188H) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLSDVCYTRAAELFKNAAIAHGLRSKIKSNFRL KELKNMKSGLPTTKSDNFPIPLVKQKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | 372 |
| Engineered CWCas12f1 (S188K) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLSDVCYTRAAELFKNAAIAKGLRSKIKSNFRL KELKNMKSGLPTTKSDNFPIPLVKQKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR | 373 |
| | GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | |
| Engineered CWCas12f1 (R191K) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLSDVCYTRAAELFKNAAIASGLKSKIKSNFRL KELKNMKSGLPTTKSDNFPIPLVKQKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | 374 |
| Engineered CWCas12f1 (Q225T) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLSDVCYTRAAELFKNAAIASGLRSKIKSNFRL KELKNMKSGLPTTKSDNFPIPLVKTKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE | 375 |
| | FKLKQYGIEIRKVAPNNTSKTCSKCGHLNNYFNFEYRKKN KFPHFKCEKCNFKENADYNAALNISNPKLKSTKEEP | |
| Engineered CWCas12f1 (Q225F) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLSDVCYTRAAELFKNAAIASGLRSKIKSNFRL KELKNMKSGLPTTKSDNFPIPLVKFKGGQYTGFEISNHNS DFIIKIPFGRWQVKKEIDKYRPWEKFDFEQVQKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | 376 |
| Engineered CWCas12f1 (Q272K) | MGEKSSRRRRNGKSGAWTAAITSCVGGKMAKNTITKTLK LRIVRPYNSAEVEKIVADEKNNREKIALEKNKDKVKEACS KHLKVAAYCTTQVERNACLFCKARKLDDKFYQKLRGQFP DAVFWQEISEIFRQLQKQAAEIYNQSLIELYYEIFIKGKGIA NASSVEHYLSDVCYTRAAELFKNAAIASGLRSKIKSNFRL KELKNMKSGLPTTKSDNFPIPLVKQKGGQYTGFEISNHNS DFIIKIPFGRWQVKKEIDKYRPWEKFDFEQVKKSPKPISLL LSTQRRKRNKGWSKDEGTEAEIKKVMNGDYQTSYIEVKR GSKIGEKSAWMLNLSIDVPKIDKGVDPSIIGGIDVGVKSPL VCAINNAFSRYSISDNDLFHFNKKMFARRRILLKKNRHKR AGHGAKNKLKPITILTEKSERFRKKLIERWACEIADFFIKN KVGTVQMENLESMKRKEDSYFNIRLRGFWPYAEMQNKIE FKLKQYGIEIRKVAPNNTSKTCSKCGHLNNYFNFEYRKKN KFPHFKCEKCNFKENADYNAALNISNPKLKSTKEEP | 377 |

The Cas12f1 variant may further recognize 5'-TNTN-3', 5'-TTTN-3', 5'-TGTA-3', 5'-TCTG-3', 5'-TGTG-3', or 5'-TTTC-3' as a PAM sequence (wherein N is A, T, C, or G).

In another embodiment, the Cas12f1 variant may be such that some or all of the functions of the wild-type Cas12f1 protein are modified. For example, the Cas12f1 variant may be a protein that is modified to cleave only one strand of a double strand of a target nucleic acid.

### (2) Fusion protein

According to another aspect of the present disclosure, the Cas12f1 variant may be a variant in which a domain, peptide, or protein having an additional function is added to the Cas12f1 protein or the variant thereof. Here, the Cas12f1 variant in which the domain, peptide, or protein having an additional function is added may be referred to as a "Cas12f1 fusion protein."

In an embodiment, the domain, peptide or protein having an additional function may be added to the N-terminus, C-terminus and/or within the amino acid sequence of wild-type Cas12f1 or a variant protein thereof.

In another embodiment, the domain, peptide, or protein having an additional function may be a domain, peptide, or protein having the same or different function compared to the wild-type Cas12f1 protein.

As an example, the Cas12f1 fusion protein may comprise two or more heterologous polypeptide domains, wherein one polypeptide domain comprises Cas12f1 or a variant protein thereof, and the other domain comprises a (poly)peptide having another function or activity. For example, the (poly)peptide having another function or activity may have methylase activity, demethylase activity, transcription activation activity, transcription repression activity, transcription release factor activity, histone modification activity, RNA cleavage activity, or nucleic acid binding activity.

As another example, the (poly)peptide, which has a different function or activity from Cas12f1, in the Cas12f1 fusion protein may be a tag or reporter protein for separation and/or purification. For example, the tag or reporter protein includes, but is not limited to, a tag protein such as a histidine (His) tag, a V5 tag, a FLAG tag, an influenza hemagglutinin (HA) tag, a Myc tag, a VSV-G tag, and a thioredoxin (Trx) tag; a fluorescent protein such as green fluorescent protein (GFP), yellow fluorescent protein (YFP), cyan fluorescent protein (CFP), blue fluorescent protein (BFP), HcRED, and DsRed; and a reporter protein (enzyme) such as glutathione-S-transferase (GST), horseradish peroxidase (HRP), chloramphenicol acetyltransferase (CAT), β-galactosidase, β-glucuronidase, and luciferase. In addition, the (poly)peptide having another function or activity may be, but is not limited to, a reverse transcriptase, a deaminase or another proteolytic enzyme.

As another example, the Cas12f1 molecule (for example, Cas12f1 or a variant thereof) may be such that it is fused with various enzymes that can be involved in gene expression processes within a cell. The Cas12f1 molecule fused with the enzymes may cause various quantitative and/or qualitative changes in gene expression within a cell. For example, the additionally linked various enzymes may be DNMT, TET, KRAB, DHAC, LSD, p300, M-MLV (moloney murine leukemia virus) reverse transcriptase, or a variant thereof. Cas12f1 or a variant thereof protein fused with a reverse transcriptase may also function as a prime editor.

### (3) Other additional elements

According to another aspect of the present disclosure, since the CRISPR/Cas12f1 system or USH2A gene editing system cleaves a nucleic acid at a target site of a target nucleic acid or target gene, the target site may be located in the nucleus of a cell. Accordingly, the Cas12f1 molecule (for example, Cas12f1 or a variant protein thereof) included in the CRISPR/Cas12f1 system or USH2A gene editing system may comprise one or more nuclear localization signal (NLS) sequences that localize the molecule into the nucleus. For example, one or more nuclear localization signal sequences may have a sufficient amount or activity to induce the Cas12f1 molecule to be targeted or transported into the nucleus of a eukaryotic cell (for example, a mammalian cell) in a detectable amount. For example, differences in the strength of activity may result from the number of NLSs included in the Cas12f1 molecule, the type of specific NLS(s) used, or a combination of these factors.

In an embodiment, the NLSs included in the Cas12f1 molecule of the present disclosure (for example, Cas12f1 or a variant protein thereof) may be variously selected from about 1, 2, 3, 4, 5, 6, 7, 8, 9, or 10 or more NLSs at or near the N-terminus, about 1, 2, 3, 4, 5, 6, 7, 8, 9, or 10 or more NLSs at or near the C-terminus, or combinations thereof. For example, the Cas12f1 molecule may comprise 0 or at least one NLS sequence at the N-terminus and/or 0 or at least one NLS sequence at the C-terminus. When more than one NLS sequence is present, each NLS sequence may be selected independently of the others, such that a single NLS may be present in more than one copy, and may be present in combination with more than one other NLS present in more than one copy.

In some specific embodiments, the NLS sequence is heterologous to the Cas12f1 molecule and examples thereof include, but are not limited to, the following NLS sequences:
An NLS from SV40 virus large T antigen having the amino acid sequence PKKKRKV (SEQ ID NO: 380); an NLS from nucleoplasmin (for example, a nucleoplasmin bipartite NLS having the sequence KRPAATKKAGQAKKKK (SEQ ID NO: 381)); a c-myc NLS having the amino acid sequence PAAKRVKLD (SEQ ID NO: 382) or RQRRNELKRSP (SEQ ID NO: 383); a hRNPA1 M9 NLS having the sequence NQSSNFGPMKGGNFGGRSSGPYGGGGQYFAKPRNQGGY (SEQ ID NO: 384); an IBB domain from importin-alpha having the sequence RMRIZFKNKGKDTAELRRRRVEVSVELRKAKKDEQILKRRNV (SEQ ID NO: 385); an NLS sequence derived from the sequences VSRKRPRP (SEQ ID NO: 386) and PPKKARED (SEQ ID NO: 387) of a myoma T protein; the sequence PQPKKKPL (SEQ ID NO: 388) of a human p53; the sequence SALIKKKKKMAP (SEQ ID NO: 389) of a mouse c-abl IV; the sequences DRLRR (SEQ ID NO: 390) and PKQKKRK (SEQ ID NO: 391) of an influenza virus NS1; the sequence RKLKKKIKKL (SEQ ID NO: 392) of a hepatitis virus delta antigen; the sequence REKKKFLKRR (SEQ ID NO: 393) of a mouse Mx1 protein; the sequence KRKGDEVDGVDEVAKKKSKK (SEQ ID NO: 394) of a human poly(ADP-ribose) polymerase; or the sequence RKCLQAGMNLEARKTKK (SEQ ID NO: 395) of the steroid hormone receptor (human) glucocorticoid.

In another embodiment, the Cas12f1 variant having an NLS added to the wild-type Cas12f1 protein may comprise or consist of the amino acid sequence of SEQ ID NO: 396:
"Un1Cas12f1 protein having an NLS added,"

PKKKRKVGIHGVPAAMAKNTITKTLKLRIVRPYNSAEVEKIVADEKNNREKIALEKN
KDKVKEACSKHLKVAAYCTTQVERNACLFCKARKLDDKFYQKLRGQFPDAVFWQEI
SEIFRQLQKQAAEIYNQSLIELYYEIFIKGKGIANASSVEHYLSDVCYTRAAELFKNAA
IASGLRSKIKSNFRLKELKNMKSGLPTTKSDNFPIPLVKQKGGQYTGFEISNHNSDFIIK
IPFGRWQVKKEIDKYRPWEKFDFEQVQKSPKPISLLLSTQRRKRNKGWSKDEGTEAE
IKKVMNGDYQTSYIEVKRGSKIGEKSAWMLNLSIDVPKIDKGVDPSIIGGIDVGVKSP
LVCAINNAFSRYSISDNDLFHFNKKMFARRRILLKKNRHKRAGHGAKNKLKPITILTE
KSERFRKKLIERWACEIADFFIKNKVGTVQMENLESMKRKEDSYFNIRLRGFWPYAE

MQNKIEFKLKQYGIEIRKVAPNNTSKTCSKCGHLNNYFNFEYRKKNKFPHFKCEKCN
FKENADYNAALNISNPKLKSTKEEPKRPAATKKAGQAKKKK (SEQ ID NO: 396).

In another embodiment, the Cas12f1 or the variant protein thereof may comprise NES (a nuclear export signal). The NES sequence refers to a peptide of a certain length or a sequence thereof that acts as a kind of "tag" by attaching to a protein to be transported when transporting a material inside the nucleus of a cell to the outside of the nucleus by nuclear transport.

### 1.3. PAM sequence of Cas12f1 molecule

In some embodiments, the following two conditions are required for the CRISPR/Cas12f1 system or USH2A gene editing system of the present disclosure to be located at a target site in a target gene or target nucleic acid and to accurately cleave a nucleic acid at the target site.

First, there must be a nucleotide sequence of a certain length that can be recognized by Cas12f1 molecule (for example, Cas12f1 or a variant thereof) in the target gene or target nucleic acid. In addition, around the nucleotide sequence of a certain length, there must be a sequence that can bind complementarily to a guide sequence (for example, a first guide sequence or a second guide sequence) included in the guide RNA (gRNA) according to the present disclosure. In other words, when the Cas12f1 molecule recognizes the nucleotide sequence of a certain length and a guide sequence (spacer) portion included in the guide RNA (gRNA) complementarily binds to a sequence portion around the nucleotide sequence of a certain length, it is possible to accurately cleave (or edit) a nucleic acid at a target site in a target nucleic acid or target gene. Here, the nucleotide sequence of a certain length recognized by the Cas12f1 molecule is called a protospacer adjacent motif (PAM) sequence. The PAM sequence is a unique sequence determined depending on the Cas12f1 molecule. This means that when determining a target sequence of a complex consisting of the Cas12f1 molecule and the gRNA within the gene editing system, the target sequence must be determined within sequences adjacent to the PAM sequence.

The PAM sequence of the Cas12f1 molecule (for example, Cas12f1 or a variant protein thereof) may be a T-rich sequence. More specifically, the PAM sequence may be 5'-TTTN-3', wherein N is one of deoxythymidine (T), deoxyadenosine (A), deoxycytidine (C), or deoxyguanosine (G).

In an embodiment, the PAM sequence of the Cas12f1 molecule may be 5'-TTTA-3', 5'-TTTT-3', 5'-TTTC-3', or 5'-TTTG-3'. Preferably, the PAM sequence of the Cas12f1 molecule may be 5'-TTTA-3' or 5'-TTTG-3'.

In another embodiment, the PAM sequence of the Cas12f1 molecule may be different from the PAM sequence of the wild-type Cas12f1 protein. For example, the Cas12f1 variant may be such that it is engineered to recognize a PAM sequence other than 5'-TTTA-3' or 5'-TTTG-3'.

### 2. Engineered guide RNA

As disclosed herein, the CRISPR/Cas12f1 system or USH2A gene editing system comprises at least one engineered guide RNA or a nucleic acid encoding the same. Therefore, according to another aspect of the present disclosure, there is provided an engineered guide RNA comprising a guide sequence capable of hybridizing with a target sequence in a USH2A gene, wherein the guide RNA comprises a scaffold region and a spacer region (or a guide region), and the guide sequence is contained in the spacer region.

The engineered guide RNA allows the USH2A gene editing system to target to a specific region in the USH2A gene. The guide RNA of the CRISPR/Cas12f1 system or USH2A gene editing system according to the present disclosure may be based on Cas12f1 guide RNA found in nature or an engineered Cas12f1 guide RNA. The Cas12f1 guide RNA found in nature or the engineered Cas12f1 guide RNA comprises tracrRNA (trans-activating CRISPR RNA) and crRNA (CRISPR RNA). Here, the crRNA comprises a part of a scaffold region and a spacer region, and the spacer region comprises a guide sequence capable of complementarily binding to a target sequence. The tracrRNA comprises a part of a scaffold region and may hybridize with or be directly linked to the crRNA. The scaffold region of the Cas12f1 guide RNA comprises a function of interacting with a Cas12f1 molecule.

According to another aspect of the present disclosure, there is provided a guide RNA comprising a spacer region, which comprises a guide sequence capable of hybridizing with a target sequence in a USH2A (Usherin) gene, and a scaffold region, wherein the guide RNA is (i) a first guide RNA comprising a first guide sequence capable of hybridizing to a target sequence of contiguous 15 to 30 bp in length, which is present in a region 5,000 bp upstream of USH2A exon 13 and is adj acent to a protospacer-adj acent motif (PAM) sequence recognized by the Cas12f1 molecule; or (ii) a second guide RNA comprising a second guide sequence capable of hybridizing to a target sequence of contiguous 15 to 30 bp in length, which is present in a region 14,500 bp downstream of USH2A exon 13 and is adjacent to a protospacer-adjacent motif (PAM) sequence recognized by Cas12f1 molecule. Here, the meaning of "adjacent to a PAM sequence" includes both the 5'-end direction and the 3'-end direction of the PAM sequence.

According to yet another aspect of the present disclosure, there is provided a guide RNA comprising a spacer region, which comprises a guide sequence capable of hybridizing with a target sequence in a USH2A (Usherin) gene, and a scaffold region, wherein the guide sequence comprises (i) a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or (ii) a sequence of contiguous 15 to 20 nucleotides from a nucleotide sequence selected from the group consisting of SEQ ID NOs: 446 to 475, or a sequence that is different by 5 or fewer nucleotides from the contiguous nucleotide sequence, wherein thymine (T) in the contiguous nucleotide sequence is substituted with uracil (U).

Hereinafter, the target gene of the guide RNA used in the USH2A gene editing system, the spacer region of the guide RNA, the scaffold region, and engineering thereof are described in detail.

### 2.1. Target gene of guide RNA

Type 2 (more specifically, type 2A) Usher syndrome is known to be caused by the c.2276G>T mutation and/or the c.2299delG mutation that occur in the exon 13 region of the USH2A gene. These mutations cause expression of altered mRNA, resulting in expression of abnormal Usherin protein or inhibition of expression of normally functioning Usherin protein. Therefore, for the treatment of type 2 (more specifically, type 2A) Usher syndrome, the USH2A gene was selected as a target subject, that is, a target gene, of the gene editing system of the present disclosure.

The USH2A gene may comprise the c.2276G>T mutation and/or the c.2299delG mutation. The "USH2A gene" targeted by the gene editing system of the present disclosure may be a USH2A gene comprising the c.2276G>T mutation and/or the c.2299delG mutation. Here, the USH2A gene comprising the c.2276G>T mutation and/or the c.2299delG mutation is also referred to as an "abnormal USH2A gene," a "USH2A gene mutant" or a "USH2A gene (c.2276G>T and/or c.2299delG)," and these terms may be used interchangeably. In addition, a USH2A gene that does not contain the c.2276G>T mutation and/or the c.2299delG mutation, a USH2A gene that normally expresses the Usherin protein, or a USH2A gene that expresses the Usherin protein with normal function may be referred to as a "normal USH2A gene," a "normal functioning USH2A gene," or a "functional USH2A gene," and these terms may be used interchangeably.

In an embodiment, the target gene may be a human USH2A gene. The human USH2A gene is located at positions 215,622,891 to 216,423,448 on the reverse strand of chromosome 1. The reference sequence for the human USH2A gene is known in the art (see Ensembl: ENSG00000042781).

In this specification, a sequence present in a target gene (for example, USH2A gene) that can be targeted (or recognized) by the gene editing system of the present disclosure or hybridized with the guide RNA of the present disclosure is referred to as a "target sequence." A specific region in a target gene that comprises at least one target sequence is referred to as a "target region."

### (1) Target region

For the treatment of Usher syndrome, the gene editing system of the present disclosure may target the USH2A gene. More specifically, the USH2A gene editing system may target a certain region in the USH2A gene. The certain region in the USH2A gene is referred to as a target region in relation to the gene editing system of the present disclosure, and the target region comprises a target sequence that hybridizes with or binds complementarily to a guide RNA constituting the gene editing system.

The certain region of the USH2A gene, that is, the target region, may be an upstream region and/or a downstream region of exon 13 comprising the c.2276G>T mutation and/or the c.2299delG mutation.

Throughout this specification, the "upstream region of exon 13" refers to a region located in the 5'-end direction of exon 13 based on the coding strand in the double-stranded DNA of the USH2A gene (the nucleotide sequence of the coding strand is based on the reference sequence of the human USH2A gene [Ensembl: ENSG00000042781]). In addition, the "downstream region of exon 13" refers to a region located in the 3'-end direction of exon 13 based on the coding strand in the double-stranded DNA of the USH2A gene. Therefore, based on the template strand in the double-stranded DNA of the USH2A gene, a region located in the 3'-end direction of exon 13 may be referred to as an upstream region of exon 13, and a region located in the 5'-end direction of exon 13 may be referred to as a downstream region of exon 13. That is, in this specification, the terms "upstream region" and "downstream region" are used as concepts including both the coding strand of a double-stranded DNA and the template strand that is a complementary sequence thereto (or antiparallel sequence).

In an embodiment, the upstream region of exon 13 may be a 5'-end region of the USH2A gene linked to the 5'-end of exon 13 of the USH2A gene. Alternatively, the upstream region of exon 13 may be a region between the 3'-end of exon 12 and the 5'-end of exon 13 of the USH2A gene. In an embodiment, the downstream region may be a 3'-end region of the USH2A gene linked to the 3'-end of exon 13 of the USH2A gene. Alternatively, the downstream region may be a region between the 3'-end of exon 13 and the 5'-end of exon 14 of the USH2A gene.

In another embodiment, the target region may be intron 12 or a region comprising the same and/or intron 13 or a region comprising the same in the USH2A gene.

In another embodiment, the target region may be a region 5000bp, 4000bp, 3700bp, 3600bp, 3500bp, 3400bp, 3300bp, 3200bp, 3100bp, 3000bp, 2900bp, 2800bp, 2700bp, 2600bp, 2500bp, 2400bp, 2300bp, 2200bp, 2100bp, 2000bp, 1900bp, 1800bp, 1700bp, 1600bp, 1500bp, 1400bp, 1300bp, 1200bp, 1100bp, or 1000bp upstream of exon 13 of the USH2A gene. In addition, the target region is a region 15000bp, 14500bp, 14000bp, 13500bp, 13000bp, 12500bp, 12000bp, 11500bp, 11000bp, 10500bp, 10000bp, 9500bp, 9000bp, 8500bp, 8000bp, 7500bp, 7000bp, 6500bp, 6000bp, 5500bp, 5000bp, 4500bp, 4000bp, 3500bp, 3000bp, 2900bp, 2800bp, 2700bp, 2600bp, 2500bp, 2400bp, 2300bp, 2200bp, 2100bp, 2000bp, 1900bp, 1800bp, 1700bp, 1600bp, 1500bp, 1400bp, 1300bp, 1200bp, 1100bp, or 1000bp downstream of exon 13 of the USH2A gene.

The target region is a double-stranded DNA, and the two strands may be referred to as the "target strand" and the "non-target strand," respectively. Here, the "target strand" is a strand that comprises a target sequence and interacts with (for example, hybridizes with) the guide RNA included in the gene editing system of the present disclosure.

The "target strand" refers to a strand comprising a target sequence. When the target gene is single-stranded, the strand may be a target strand. Alternatively, when the target gene is double-stranded, one of the double strands may be a target strand, and a strand complementary to the target strand may exist. Here, the strand complementary to the target strand is referred to as a "non-target strand."

The "non-target strand" is a strand complementary to the target strand, and comprises a "PAM (Protospacer Adjacent Motif) sequence" and a "protospacer sequence." The PAM sequence is a sequence recognized by Cas12f1 or a variant protein thereof of the gene editing system of the present disclosure. The protospacer sequence is a sequence located adjacent to the PAM sequence, for example, located at the 5'-end or the 3'-end, and is a sequence complementary to the target sequence or a sequence that binds complementarily to the target sequence. Correlation between the protospacer sequence and the target sequence is similar to correlation between the target sequence and the guide sequence. Due to these characteristics, in general, a guide sequence may be designed using a protospacer sequence. That is, when designing a guide sequence that binds complementarily to a target sequence, the guide sequence may be designed as a nucleotide sequence having the same nucleotide sequence as the protospacer sequence. Here, the guide sequence is designed by replacing T with U in the protospacer sequence.

It should be understood that a particular strand referred to as a non-target strand in a double-stranded DNA is not always a non-target strand and is relative in its relationship to the target strand. For example, when one strand of a double-stranded DNA comprising a target sequence, which can be hybridized with either guide sequence is referred to as a target strand, the other DNA strand may be referred to as a non-target strand; and when the other guide sequence can hybridize with the strand referred to as the non-target strand, the DNA strand referred to as the non-target strand is referred to as a target strand in relation to the other guide sequence, and thus the DNA strand referred to as a target strand is referred to as the non-target strand. A "protospacer sequence" is a sequence that has complementarity to a target sequence or a sequence that forms a complementary bond with a target sequence.

In an embodiment, the target region comprising the target sequence may comprise a protospacer sequence selected from the group consisting of SEQ ID NOs: 397 to 475. In addition, the target sequence may be a sequence complementary to a protospacer sequence selected from the group consisting of SEQ ID NOs: 397 to 475 within the target region.

### (2) Target sequence

The term "target sequence" refers to a sequence present in a target gene or a target region, which is recognized by a guide RNA of the gene editing system of the present disclosure, or is to be modified by the gene editing system. Specifically, the target sequence is a sequence present in the target region described above, which is complementary to a guide RNA included in the USH2A gene editing system or to a guide sequence included in the guide RNA or binds complementarily thereto.

In an embodiment, the target sequence may be a sequence of 15 to 40 nucleotides. For example, the target sequence may be a sequence of 15 to 20, 15 to 25, 15 to 30, 15 to 35 or 15 to 40 nucleotides. In addition, the target sequence may be a sequence of 20 to 25, 20 to 30, 20 to 35 or 20 to 40 nucleotides. In addition, the target sequence may be a sequence of 25 to 30, 25 to 35 or 25 to 40 nucleotides. In addition, the target sequence may be a sequence of 30 to 35 or 30 to 40 nucleotides. In addition, the target sequence may be a sequence of 35 to 40 nucleotides. As another example, the target sequence may be a sequence of 15, 16, 17, 18, 19, 20, 21, 22, 23, 24, 25, 26, 27, 28, 29, 30, 31, 32, 33, 34, 35, 36, 37, 38, 39 or 40 nucleotides.

In another embodiment, the target sequence may be a sequence of 15 to 40 nucleotides present in an upstream region of exon 13 comprising the c.2276G>T mutation and/or the c.2299delG mutation. In addition, the target sequence may be a sequence of 15 to 40 nucleotides present in the region between the 3'-end of exon 12 and the 5'-end of exon 13 of the USH2A gene. In addition, the target sequence may be a sequence of 15 to 40 nucleotides present in the intron 12 region of the USH2A gene. In addition, the target sequence may be a sequence of 15 to 40 nucleotides present in a 5000bp, 4000bp, 3700bp, 3600bp, 3500bp, 3400bp, 3300bp, 3200bp, 3100bp, 3000bp, 2900bp, 2800bp, 2700bp, 2600bp, 2500bp, 2400bp, 2300bp, 2200bp, 2100bp, 2000bp, 1900bp, 1800bp, 1700bp, 1600bp, 1500bp, 1400bp, 1300bp, 1200bp, 1100bp or 1000bp region connected to the 5'-end of exon 13 of the USH2A gene. In addition, the target sequence may comprise a nucleotide sequence selected from the group consisting of SEQ ID NOs: 1 to 49. A specific example of the target sequence according to the present embodiment is provided in Table 2 below. For convenience, the upstream region is referred to as the F region, which is an abbreviation for the front region.

**[Table 2]**

| No. | Name (Oligo) | Target sequence (5'→3') | SEQ ID NO |
|---|---|---|---|
| 1 | GK-USH2A-F02 | CATTCAAGATAGACGAGACA | 1 |
| 2 | GK-USH2A-F03 | TACTGCAGATGATACGAACA | 2 |
| 3 | GK-USH2A-F05 | TAGGGGGCCAATCTTACTCT | 3 |
| 4 | GK-USH2A-F06 | GTTGTATATTAAAGCTAAAT | 4 |
| 5 | GK-USH2A-F07 | CATCGCAAACAGTTGTATAT | 5 |
| 6 | GK-USH2A-F09 | GGAGCTCTTTTTCTCTTTAA | 6 |
| 7 | GK-USH2A-F10 | TTTTAACAAATGTGCTCATT | 7 |
| 8 | GK-USH2A-F12 | TACTCAGCTTAACCTTTTAT | 8 |
| 9 | GK-USH2A-F13 | TAATAAAAGGTTAAGCTGAGTA | 9 |
| 10 | GK-USH2A-F15 | GATCTTAAATGTTCTCACCC | 10 |
| 11 | GK-USH2A-F16 | TTTGATATATGTACACATTA | 11 |
| 12 | GK-USH2A-F17 | CAGCTTCACGAAGGTATAAT | 12 |
| 13 | GK-USH2A-F22 | TCCTTTAAATAGAAGTAATA | 13 |
| 14 | GK-USH2A-F23 | TCTGACAAGTAAGGTTATTC | 14 |
| 15 | GK-USH2A-F24 | GGTATTACAAGGCAAAGAAA | 15 |
| 16 | GK-USH2A-F25 | GAATAGTAAATGTTTAGATG | 16 |
| 17 | GK-USH2A-F26 | TAAAGGAAGTATTTTGCATC | 17 |
| 18 | GK-USH2A-F27 | TACTTCCTTTAGATAGTTTC | 18 |
| 19 | GK-USH2A-F30 | TTCAAGCTATAATTGCAATT | 19 |
| 20 | GK-USH2A-FA01 | CATTTTCCCATCCTCACCTTT | 20 |
| 21 | GK-USH2A-FA02 | CAACTGTTTGCGATGAACTTCA | 21 |
| 22 | GK-USH2A-FA03 | TCTTTGCATTAAGTAATAAT | 22 |
| 23 | GK-USH2A-FA04 | TTTTTAATTATTACTTAATG | 23 |
| 24 | GK-USH2A-FA05 | TATGTAATTCTACTATAATTT | 24 |
| 25 | GK-USH2A-FA06 | TTGCTAAGAGATTAGATCT | 25 |
| 26 | GK-USH2A-FA07 | TTTATAATGTGTACATATAT | 26 |
| 27 | GK-USH2A-FA08 | CAAAACATCATGTTGTCTGCCA | 27 |
| 28 | GK-USH2A-FA09 | CTTCACGAAGGTATAATTAAA | 28 |
| 29 | GK-USH2A-FA10 | GGTGAGTCATTCATCACTGT | 29 |
| 30 | GK-USH2A-FA11 | TTTATTTTCCTTATTGAAAT | 30 |
| 31 | GK-USH2A-FA12 | TATATATGTATATATATGGA | 31 |
| 32 | GK-USH2A-FA13 | CATATGTAGAAAAGCATTTCC | 32 |
| 33 | GK-USH2A-FA14 | TTTAATTTCAATAAGGAAAA | 33 |
| 34 | GK-USH2A-FA15 | GTTAACAATACAGTTATTTT | 34 |
| 35 | GK-USH2A-FA16 | GTAGACCAATTTTAATAGTT | 35 |
| 36 | GK-USH2A-FA17 | GATTCATATCATATCAGTTT | 36 |
| 37 | GK-USH2A-FA18 | TATGACTCATTTTGAACTAT | 37 |
| 38 | GK-USH2A-FA19 | CCACTATTGCTGCAAATTT | 38 |
| 39 | GK-USH2A-FA20 | GGAATATGTATGGCATATT | 39 |
| 40 | GK-USH2A-FA21 | TAAGCACTGTGCATATTTT | 40 |
| 41 | GK-USH2A-FA22 | CTTATTTTAAGATTAATTTT | 41 |
| 42 | GK-USH2A-FA23 | TTTCCAAATATCCATGAATT | 42 |
| 43 | GK-USH2A-FA24 | CAGAGATTTAAGTTTAGGTGA | 43 |
| 44 | GK-USH2A-FA25 | TGACTCAGAACATACCTCTT | 44 |
| 45 | GK-USH2A-FA26 | TTTATCATTTTCAATTAATA | 45 |
| 46 | GK-USH2A-FA27 | TGATAAAATAGAGGAGCATA | 46 |
| 47 | GK-USH2A-FA28 | TTTTATTTATATTAATTACT | 47 |
| 48 | GK-USH2A-FA29 | TAAGTGTATATGCTGTTTTCA | 48 |
| 49 | GK-USH2A-FA30 | CATGGATATTTGGAAACTATC | 49 |

In yet another embodiment, the target sequence may be a sequence of 15 to 40 nucleotides present in a downstream region of exon 13 comprising the c.2276G>T mutation and/or the c.2299delG mutation. In addition, the target sequence may be a sequence of 15 to 40 nucleotides present in a region between the 3'-end of exon 13 and the 5'-end of exon 14 of the USH2A gene. In addition, the target sequence may be a sequence of 15 to 40 nucleotides present in the intron 13 region of the USH2A gene. In addition, the target sequence may be a sequence of 15 to 40 nucleotides present in a 15000bp, 14500bp, 14000bp, 13500bp, 13000bp, 12500bp, 12000bp, 11500bp, 11000bp, 10500bp, 10000bp, 9500bp, 9000bp, 8500bp, 8000bp, 7500bp, 7000bp, 6500bp, 6000bp, 5500bp, 5000bp, 4500bp, 4000bp, 3500bp, 3000bp, 2900 bp, 2800 bp, 2700 bp, 2600 bp, 2500 bp, 2400 bp, 2300 bp, 2200 bp, 2100 bp, 2000 bp, 1900 bp, 1800 bp, 1700 bp, 1600 bp, 1500 bp, 1400 bp, 1300 bp, 1200 bp, 1100 bp, or 1000 bp region connected to the 3'-end of exon 13 of the USH2A gene. In addition, the target sequence may comprise a nucleotide sequence selected from the group consisting of SEQ ID NOs: 50 to 79. A specific example of the target sequence according to the present embodiment is provided in Table 3 below. For convenience, the above downstream region is referred to as the R region, which is an abbreviation for the rear region.

**[Table 3]**

| No. | Name (Oligo) | Target sequence (5'→3') | SEQ ID NO |
|---|---|---|---|
| 1 | GK-USH2A-R01 | GGAGAAGTTACCTAAGTTAA | 50 |
| 2 | GK-USH2A-R02 | GCTTCTACAAATTTTATTTC | 51 |
| 3 | GK-USH2A-R04 | CCGATCGGCTGAGTTTTATC | 52 |
| 4 | GK-USH2A-R05 | CTCAATTTCTACACTTGAAG | 53 |
| 5 | GK-USH2A-R07 | CATTGTATGGATATTCAACT | 54 |
| 6 | GK-USH2A-R08 | GTTGAATATCCATACAATGC | 55 |
| 7 | GK-USH2A-R09 | TGATGAACTAAATCTCTGAA | 56 |
| 8 | GK-USH2A-R10 | CAATTCTAGGTATTTCTATA | 57 |
| 9 | GK-USH2A-R11 | GAATTGTTTCCACATGCCAT | 58 |
| 10 | GK-USH2A-R13 | TCCACATGCCATCAAATTAA | 59 |
| 11 | GK-USH2A-R14 | CTGTTTAATCTCATTATATA | 60 |
| 12 | GK-USH2A-R17 | CTTACATTTAAGATTTTAAC | 61 |
| 13 | GK-USH2A-R18 | CTCTGAGTTATATGGGTCTA | 62 |
| 14 | GK-USH2A-R19 | TCTACTCCTTCTCTGGCAAG | 63 |
| 15 | GK-USH2A-R20 | TTGCCAGAGAAGGAGTAGAA | 64 |
| 16 | GK-USH2A-R22 | TCTTACACACTGACCAATGC | 65 |
| 17 | GK-USH2A-R23 | TCTTTTTGTGATGTAAGTAT | 66 |
| 18 | GK-USH2A-R24 | TATTATAACTAGATACTCCA | 67 |
| 19 | GK-USH2A-R26 | TGTGGCTGGTGGTAGAATTA | 68 |
| 20 | GK-USH2A-R27 | TATAACTAAGAGGTAGCTAA | 69 |
| 21 | GK-USH2A-R29 | CTCAGAGGTAACCAACCAAA | 70 |
| 22 | GK-USH2A-R30 | TTGGCTCAGAGGTAACCAAC | 71 |
| 23 | GK-USH2A-R31 | CCAGGGGTGTCACGTACTTA | 72 |
| 24 | GK-USH2A-R32 | CTACCTGATGAAATGGTCCC | 73 |
| 25 | GK-USH2A-R34 | TGAAAGGATTAACCTGAAGG | 74 |
| 26 | GK-USH2A-R35 | GAGACAAAGGACTTTGTTGC | 75 |
| 27 | GK-USH2A-R36 | TCCTTTGTCTCCTACACAGT | 76 |
| 28 | GK-USH2A-R38 | TTAGATATCTGGTAGGTGTA | 77 |
| 29 | GK-USH2A-R39 | GTCTTATGCATGGTGTAGAT | 78 |
| 30 | GK-USH2A-R40 | TATACATCCTTCTTTCTAAG | 79 |

### 2.2. Spacer region comprising guide sequence

The engineered guide RNA (gRNA) according to an embodiment of the present disclosure comprises a sequence portion that enables the gRNA to locate a target nucleic acid, that is, one or more guide sequences that recognize, bind to, or target a target sequence in the USH2A gene as described above. More specifically, the guide sequence may be a sequence that can hybridize with or bind complementarily to a target sequence. In this section, for the "target sequence," see the entire contents described in the above section "(2) Target sequence."

The sequence referred to herein as a "guide sequence" or a "spacer sequence" is a sequence complementary to a target sequence in a target gene, and is linked to the 3'-end of the crRNA repeat sequence. In an embodiment, a guide sequence portion of the crRNA may bind complementarily to a target gene (for example, the USH2A gene). In another embodiment, a guide sequence portion of the crRNA may bind complementarily to a part of the target sequence in the target gene. For example, when the target nucleic acid is a double-stranded DNA, the guide sequence may be a sequence complementary to a target sequence included in a target strand of the double-stranded DNA. Here, when the target nucleic acid is a double-stranded DNA, the guide sequence may comprise a sequence homologous to a protospacer sequence included in a non-target strand of the double-stranded DNA. Specifically, the guide sequence may have the same nucleotide sequence as the protospacer sequence, except that each of the thymine (T) residues included in the nucleotide sequence is replaced with uracil (U). As an example, the guide sequence may comprise an RNA sequence corresponding to the DNA sequence of the protospacer. As a more specific example, the guide sequence may comprise an RNA sequence corresponding to the DNA sequence of one protospacer selected within the upstream region of USH2A exon 13 and/or an RNA sequence corresponding to the DNA sequence of one protospacer selected within the downstream region of USH2A exon 13.

The guide sequence may be a sequence of 15 to 40 nucleotides. In an embodiment, the guide sequence may be a sequence of 15 to 20, 15 to 25, 15 to 30, 15 to 35, or 15 to 40 nucleotides. In addition, the guide sequence may be a sequence of 20 to 25, 20 to 30, 20 to 35, or 20 to 40 nucleotides. In addition, the guide sequence may be a sequence of 25 to 30, 25 to 35, or 25 to 40 nucleotides. In addition, the guide sequence may be a sequence of 30 to 35 or 30 to 40 nucleotides. In addition, the guide sequence may be a sequence of 35 to 40 nucleotides. In another embodiment, the guide sequence may be a sequence of 15, 16, 17, 18, 19, 20, 21, 22, 23, 24, 25, 26, 27, 28, 29, 30, 31, 32, 33, 34, 35, 36, 37, 38, 39, or 40 nucleotides.

In yet another embodiment, the guide sequence may be a sequence that forms a complementary bond with the target sequence, wherein the complementary bond may optionally include at least one mismatch bond. For example, the guide sequence may be a sequence that forms a complementary bond with the target sequence, wherein the complementary bond may include 0 to 5 mismatches.

In still yet another embodiment, the guide sequence may be a complementary sequence to the target sequence, wherein the complementary sequence may include a sequence of 0 to 5 mismatched nucleotides to the target sequence. The guide sequence may be a sequence having at least 70% sequence complementarity to the target sequence. Unless stated otherwise, "complementary" may mean including 0 to 5 mismatches or having at least 70% complementarity, and should be interpreted appropriately depending on the context. When the target sequence is DNA, for an adenosine (A) present in the target sequence, the guide sequence may comprise a uridine (U) residue that can form a complementary bond to A.

In an embodiment, the guide sequence may be a sequence that is at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95% or at least 75% to 100% complementary to the target sequence. Specifically, the guide sequence may be a sequence that is at least 80% to 85%, at least 80% to 90%, at least 80% to 95%, at least 80% to 100%, at least 85% to 90%, at least 85% to 95%, or at least 85% to 100% complementary to the target sequence. More specifically, the guide sequence may be a sequence that is at least 90% to 95%, at least 90% to 100%, or at least 95% to 100% complementary to the target sequence. Even more specifically, the guide sequence may be a sequence that is at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% complementary to the target sequence.

In yet another embodiment, the guide sequence may be a sequence identical with or similar to the protospacer sequence. Alternatively, the guide sequence may be a sequence having sequence identity or sequence similarity to the protospacer sequence. Here, the sequence identity or sequence similarity may be at least 70% or more. Here, for thymidine (T) present in the protospacer sequence, the guide sequence may comprise uridine (U) instead of thymidine (T).

In another embodiment, the guide sequence may be a sequence identical with or similar to the protospacer sequence. The guide sequence may have at least 70% sequence identity to the protospacer sequence. For thymine (T) present in the protospacer sequence, the guide sequence may comprise uracil (U) instead of thymine (T).

In an embodiment, the guide sequence may have at least 70% to 75%, at least 70% to 80%, at least 70% to 85%, at least 70% to 90%, at least 70% to 95%, at least 70% to 100%, at least 75% to 80%, at least 75% to 85%, at least 75% to 90%, at least 75% to 95%, or at least 75% to 100% sequence identity or similarity to the protospacer sequence. Specifically, the guide sequence may have at least 80% to 85%, at least 80% to 90%, at least 80% to 95%, at least 80% to 100%, at least 85% to 90%, at least 85% to 95%, or at least 85% to 100% sequence identity or similarity to the protospacer sequence. More specifically, the guide sequence may have at least 90% to 95%, at least 90% to 100%, or at least 95% to 100% identity or similarity to the protospacer sequence. Even more specifically, the guide sequence may have at least 70, 71, 72, 73, 74, 75, 76, 77, 78, 79, 80, 81, 82, 83, 84, 85, 86, 87, 88, 89, 90, 91, 92, 93, 94, 95, 96, 97, 98, 99, or 100% identity or similarity to the protospacer sequence.

In a more specific embodiment, the USH2A gene editing system according to the present disclosure may comprise a first guide RNA comprising a first guide sequence, a second guide RNA comprising a second guide sequence, or the first guide RNA and the second guide RNA.

In another embodiment, the first guide sequence may be a sequence of 15 to 40 nucleotides that binds complementarily to a target sequence present in an upstream region of exon 13 in the USH2A gene comprising the c.2276G>T mutation and/or the c.2299delG mutation. Here, the upstream region is a region 5000bp, 4000bp, 3700bp, 3600bp, 3500bp, 3400bp, 3300bp, 3200bp, 3100bp, 3000bp, 2900bp, 2800bp, 2700bp, 2600bp, 2500bp, 2400bp, 2300bp, 2200bp, 2100bp, 2000bp, 1900bp, 1800bp, 1700bp, 1600bp, 1500bp, 1400bp, 1300bp, 1200bp, 1100 bp, or 1000 bp upstream of the USH2A gene exon 13. In addition, the target sequence may comprise a nucleotide sequence selected from the group consisting of SEQ ID NOs: 1 to 49.

In yet another embodiment, the first guide sequence may be a sequence of 15 to 40 nucleotides that binds complementarily to a target sequence present in a region between the 3'-end of exon 12 and the 5'-end of exon 13 of the USH2A gene. In addition, the first guide sequence may be a sequence of 15 to 40 nucleotides that binds complementarily to a target sequence present in the intron 12 region of the USH2A gene. In addition, the first guide sequence may be a sequence of 15 to 40 nucleotides that binds complementarily to a target sequence present in a 5000bp, 4000bp, 3700bp, 3600bp, 3500bp, 3400bp, 3300bp, 3200bp, 3100bp, 3000bp, 2900bp, 2800bp, 2700bp, 2600bp, 2500bp, 2400bp, 2300bp, 2200bp, 2100bp, 2000bp, 1900bp, 1800bp, 1700bp, 1600bp, 1500bp, 1400bp, 1300 bp, 1200 bp, 1100 bp or 1000 bp region connected to the 5'-end of exon 13 of the USH2A gene.

In still yet another embodiment, the first guide sequence may be a sequence of 15 to 40 nucleotides that binds complementarily to one or more target sequences selected from the group consisting of SEQ ID NOs: 1 to 49.

In still yet another embodiment, the first guide sequence may comprise or consist of a sequence that is hybridizable with or complementary to a target sequence that is complementary to a nucleotide sequence selected from the group consisting of SEQ ID NOs: 397 to 445 within a region 5000 bp upstream of USH2A exon 13.

In still yet another embodiment, the first guide sequence may comprise or consist of a sequence of contiguous 15 to 22 nucleotides from a nucleotide sequence selected from the group consisting of SEQ ID NOs: 397 to 445, wherein thymine (T) is substituted with uracil (U) in the contiguous nucleotide sequence.

In still yet another embodiment, the first guide sequence may comprise or consist of a nucleotide sequence selected from the group consisting of SEQ ID NOs: 80 to 128 and SEQ ID NOs: 159 to 164. The first guide sequence having any one of the nucleotide sequences of SEQ ID NOs: 80 to 128 is provided in Table 4 below, and the first guide sequence having any one of the nucleotide sequences of SEQ ID NOs: 159 to 164 is provided in Table 15. For convenience, the upstream region is referred to as the F region, which is an abbreviation for the front region.

**[Table 4]**

| No. | Name | Guide sequence (5'→3') | SEQ ID NO |
|---|---|---|---|
| 1 | GUIDE-USH2A-F02 | UGUCUCGUCUAUCUUGAAUG | 80 |
| 2 | GUIDE-USH2A-F03 | UGUUCGUAUCAUCUGCAGUA | 81 |
| 3 | GUIDE-USH2A-F05 | AGAGUAAGAUUGGCCCCCUA | 82 |
| 4 | GUIDE-USH2A-F06 | AUUUAGCUUUAAUAUACAAC | 83 |
| 5 | GUIDE-USH2A-F07 | AUAUACAACUGUUUGCGAUG | 84 |
| 6 | GUIDE-USH2A-F09 | UUAAAGAGAAAAAGAGCUCC | 85 |
| 7 | GUIDE-USH2A-F10 | AAUGAGCACAUUUGUUAAAA | 86 |
| 8 | GUIDE-USH2A-F12 | AUAAAAGGUUAAGCUGAGUA | 87 |
| 9 | GUIDE-USH2A-F13 | UACUCAGCUUAACCUUUUAUUA | 88 |
| 10 | GUIDE-USH2A-F15 | GGGUGAGAACAUUUAAGAUC | 89 |
| 11 | GUIDE-USH2A-F16 | UAAUGUGUACAUAUAUCAAA | 90 |
| 12 | GUIDE-USH2A-F17 | AUUAUACCUUCGUGAAGCUG | 91 |
| 13 | GUIDE-USH2A-F22 | UAUUACUUCUAUUUAAAGGA | 92 |
| 14 | GUIDE-USH2A-F23 | GAAUAACCUUACUUGUCAGA | 93 |
| 15 | GUIDE-USH2A-F24 | UUUCUUUGCCUUGUAAUACC | 94 |
| 16 | GUIDE-USH2A-F25 | CAUCUAAACAUUUACUAUUC | 95 |
| 17 | GUIDE-USH2A-F26 | GAUGCAAAAUACUUCCUUUA | 96 |
| 18 | GUIDE-USH2A-F27 | GAAACUAUCUAAAGGAAGUA | 97 |
| 19 | GUIDE-USH2A-F30 | AAUUGCAAUUAUAGCUUGAA | 98 |
| 20 | GUIDE-USH2A-FA01 | AAAGGUGAGGAUGGGAAAAUG | 99 |
| 21 | GUIDE-USH2A-FA02 | UGAAGUUCAUCGCAAACAGUUG | 100 |
| 22 | GUIDE-USH2A-FA03 | AUUAUUACUUAAUGCAAAGA | 101 |
| 23 | GUIDE-USH2A-FA04 | CAUUAAGUAAUAAUUAAAAA | 102 |
| 24 | GUIDE-USH2A-FA05 | AAAUUAUAGUAGAAUUACAUA | 103 |
| 25 | GUIDE-USH2A-FA06 | AGAUCUAAUCUCUUAGCAA | 104 |
| 26 | GUIDE-USH2A-FA07 | AUAUAUGUACACAUUAUAAA | 105 |
| 27 | GUIDE-USH2A-FA08 | UGGCAGACAACAUGAUGUUUUG | 106 |
| 28 | GUIDE-USH2A-FA09 | UUUAAUUAUACCUUCGUGAAG | 107 |
| 29 | GUIDE-USH2A-FA10 | ACAGUGAUGAAUGACUCACC | 108 |
| 30 | GUIDE-USH2A-FA11 | AUUUCAAUAAGGAAAAUAAA | 109 |
| 31 | GUIDE-USH2A-FA12 | UCCAUAUAUAUACAUAUAUA | 110 |
| 32 | GUIDE-USH2A-FA13 | GGAAAUGCUUUUCUACAUAUG | 111 |
| 33 | GUIDE-USH2A-FA14 | UUUUCCUUAUUGAAAUUAAA | 112 |
| 34 | GUIDE-USH2A-FA15 | AAAAUAACUGUAUUGUUAAC | 113 |
| 35 | GUIDE-USH2A-FA16 | AACUAUUAAAAUUGGUCUAC | 114 |
| 36 | GUIDE-USH2A-FA17 | AAACUGAUAUGAUAUGAAUC | 115 |
| 37 | GUIDE-USH2A-FA18 | AUAGUUCAAAAUGAGUCAUA | 116 |
| 38 | GUIDE-USH2A-FA19 | AAAUUUGCAGCAAUAGUGG | 117 |
| 39 | GUIDE-USH2A-FA20 | AAUAUGCCAUACAUAUUCC | 118 |
| 40 | GUIDE-USH2A-FA21 | AAAAUAUGCACAGUGCUUA | 119 |
| 41 | GUIDE-USH2A-FA22 | AAAAUUAAUCUUAAAAUAAG | 120 |
| 42 | GUIDE-USH2A-FA23 | AAUUCAUGGAUAUUUGGAAA | 121 |
| 43 | GUIDE-USH2A-FA24 | UCACCUAAACUUAAAUCUCUG | 122 |
| 44 | GUIDE-USH2A-FA25 | AAGAGGUAUGUUCUGAGUCA | 123 |
| 45 | GUIDE-USH2A-FA26 | UAUUAAUUGAAAAUGAUAAA | 124 |
| 46 | GUIDE-USH2A-FA27 | UAUGCUCCUCUAUUUUAUCA | 125 |
| 47 | GUIDE-USH2A-FA28 | AGUAAUUAAUAUAAAUAAAA | 126 |
| 48 | GUIDE-USH2A-FA29 | UGAAAACAGCAUAUACACUUA | 127 |
| 49 | GUIDE-USH2A-FA30 | GAUAGUUUCCAAAUAUCCAUG | 128 |

In another embodiment, the second guide sequence may be a sequence of 15 to 40 nucleotides that binds complementarily to a target sequence present in a downstream region of exon 13 in the USH2A gene comprising the c.2276G>T mutation and/or the c.2299delG mutation. Here, the downstream region is a region 15000bp, 14500bp, 14000bp, 13500bp, 13000bp, 12500bp, 12000bp, 11500bp, 11000bp, 10500bp, 10000bp, 9500bp, 9000bp, 8500bp, 8000bp, 7500bp, 7000bp, 6500bp, 6000bp, 5500bp, 5000bp, 4500bp, 4000bp, 3500bp, 3000bp, 2900bp, 2800 bp, 2700 bp, 2600 bp, 2500 bp, 2400 bp, 2300 bp, 2200 bp, 2100 bp, 2000 bp, 1900 bp, 1800 bp, 1700 bp, 1600 bp, 1500 bp, 1400 bp, 1300 bp, 1200 bp, 1100 bp, or 1000 bp downstream of the USH2A gene exon 13. In addition, the target sequence may comprise a nucleotide sequence selected from the group consisting of SEQ ID NOs: 50 to 79.

In yet another embodiment, the second guide sequence may be a sequence of 15 to 40 nucleotides that binds complementarily to a target sequence present in a region between the 3 '-end of exon 12 and the 5'-end of exon 13 of the USH2A gene. In addition, the second guide sequence may be a sequence of 15 to 40 nucleotides that binds complementarily to a target sequence present in the intron 13 region of the USH2A gene. In addition, the second guide sequence may be a sequence of 15 to 40 nucleotides that binds complementarily to a target sequence present in a 15000bp, 14500bp, 14000bp, 13500bp, 13000bp, 12500bp, 12000bp, 11500bp, 11000bp, 10500bp, 10000bp, 9500bp, 9000bp, 8500bp, 8000bp, 7500bp, 7000bp, 6500bp, 6000bp, 5500bp, 5000bp, 4500bp, 4000bp, 3500bp, 3000bp, 2900 bp, 2800 bp, 2700 bp, 2600 bp, 2500 bp, 2400 bp, 2300 bp, 2200 bp, 2100 bp, 2000 bp, 1900 bp, 1800 bp, 1700 bp, 1600 bp, 1500 bp, 1400 bp, 1300 bp, 1200 bp, 1100 bp or 1000 bp region connected to the 3'-end of exon 13 of the USH2A gene.

In still yet another embodiment, the second guide sequence may be a sequence of 15 to 40 nucleotides that binds complementarily to one or more target sequences selected from the group consisting of SEQ ID NOs: 50 to 79.

In still yet another embodiment, the second guide sequence may comprise or consist of a sequence that is hybridizable with or complementary to a target sequence that is complementary to a nucleotide sequence selected from the group consisting of SEQ ID NOs: 446 to 475 that is within a region 5000 bp upstream of USH2A exon 13.

In still yet another embodiment, the second guide sequence may comprise or consist of a sequence of contiguous 15 to 20 nucleotides selected from the group consisting of SEQ ID NOs: 446 to 475, wherein thymine (T) in the contiguous nucleotide sequence is substituted with uracil (U).

In still yet another embodiment, the second guide sequence may comprise or consist of a nucleotide sequence selected from the group consisting of SEQ ID NOs: 129 to 158 and SEQ ID NOs: 165 to 174. The second guide sequence having a nucleotide sequence of any one of SEQ ID NOs: 129 to 158 is provided in Table 5 below, and the second guide sequence having a nucleotide sequence of any one of SEQ ID NOs: 165 to 174 is provided in Table 15. For convenience, the downstream region is referred to as the R region, which is an abbreviation for the rear region.

**[Table 5]**

| No. | Name | Guide sequence (5'→3') | SEQ ID NO |
|---|---|---|---|
| 1 | GUIDE-USH2A-R01 | UUAACUUAGGUAACUUCUCC | 129 |
| 2 | GUIDE-USH2A-R02 | GAAAUAAAAUUUGUAGAAGC | 130 |
| 3 | GUIDE-USH2A-R04 | GAUAAAACUCAGCCGAUCGG | 131 |
| 4 | GUIDE-USH2A-R05 | CUUCAAGUGUAGAAAUUGAG | 132 |
| 5 | GUIDE-USH2A-R07 | AGUUGAAUAUCCAUACAAUG | 133 |
| 6 | GUIDE-USH2A-R08 | GCAUUGUAUGGAUAUUCAAC | 134 |
| 7 | GUIDE-USH2A-R09 | UUCAGAGAUUUAGUUCAUCA | 135 |
| 8 | GUIDE-USH2A-R10 | UAUAGAAAUACCUAGAAUUG | 136 |
| 9 | GUIDE-USH2A-R11 | AUGGCAUGUGGAAACAAUUC | 137 |
| 10 | GUIDE-USH2A-R13 | UUAAUUUGAUGGCAUGUGGA | 138 |
| 11 | GUIDE-USH2A-R14 | UAUAUAAUGAGAUUAAACAG | 139 |
| 12 | GUIDE-USH2A-R17 | GUUAAAAUCUUAAAUGUAAG | 140 |
| 13 | GUIDE-USH2A-R18 | UAGACCCAUAUAACUCAGAG | 141 |
| 14 | GUIDE-USH2A-R19 | CUUGCCAGAGAAGGAGUAGA | 142 |
| 15 | GUIDE-USH2A-R20 | UUCUACUCCUUCUCUGGCAA | 143 |
| 16 | GUIDE-USH2A-R22 | GCAUUGGUCAGUGUGUAAGA | 144 |
| 17 | GUIDE-USH2A-R23 | AUACUUACAUCACAAAAAGA | 145 |
| 18 | GUIDE-USH2A-R24 | UGGAGUAUCUAGUUAUAAUA | 146 |
| 19 | GUIDE-USH2A-R26 | UAAUUCUACCACCAGCCACA | 147 |
| 20 | GUIDE-USH2A-R27 | UUAGCUACCUCUUAGUUAUA | 148 |
| 21 | GUIDE-USH2A-R29 | UUUGGUUGGUUACCUCUGAG | 149 |
| 22 | GUIDE-USH2A-R30 | GUUGGUUACCUCUGAGCCAA | 150 |
| 23 | GUIDE-USH2A-R31 | UAAGUACGUGACACCCCUGG | 151 |
| 24 | GUIDE-USH2A-R32 | GGGACCAUUUCAUCAGGUAG | 152 |
| 25 | GUIDE-USH2A-R34 | CCUUCAGGUUAAUCCUUUCA | 153 |
| 26 | GUIDE-USH2A-R35 | GCAACAAAGUCCUUUGUCUC | 154 |
| 27 | GUIDE-USH2A-R36 | ACUGUGUAGGAGACAAAGGA | 155 |
| 28 | GUIDE-USH2A-R38 | UACACCUACCAGAUAUCUAA | 156 |
| 29 | GUIDE-USH2A-R39 | AUCUACACCAUGCAUAAGAC | 157 |
| 30 | GUIDE-USH2A-R40 | CUUAGAAAGAAGGAUGUAUA | 158 |

Meanwhile, the guide sequence (the first guide sequence and/or the second guide sequence) may be present at the 5'-end of the crRNA. Here, a U-rich tail may be added to the 5'-end of the guide sequence. For details regarding the U-rich tail, see the entire contents described in the section "(2) Modification at modification site 2 (MS2)" as described below.

### 2.3. Scaffold region and engineering thereof

The guide RNA (gRNA) comprises a scaffold region and a spacer region as described above, wherein the scaffold region interacts with the Cas12f1 molecule (for example, Cas12f1 or a variant protein thereof) to contribute to formation of a CRISPR/Cas12f1 complex. The scaffold region may comprise a crRNA scaffold sequence and a tracrRNA scaffold sequence, and may be located at or bound to the 5'-end of the guide region.

The scaffold region may be composed of a dual scaffold sequence or a single scaffold sequence. When composed of a dual scaffold sequence, the scaffold sequence is composed of two different molecules, wherein the two molecules may comprise a crRNA scaffold sequence and a tracrRNA scaffold sequence, respectively. When the scaffold region is composed of a dual scaffold sequence, the guide RNA may also be composed of two molecules as a dual guide RNA. That is, the crRNA and tracrRNA in the dual guide RNA may exist independently of each other. In addition, when the scaffold region is composed of a single scaffold sequence, the scaffold sequence may be composed of a single molecule, for example, it may include a tracrRNA scaffold sequence, a linker, and a crRNA scaffold sequence. When the scaffold region is composed of a single scaffold sequence, the guide RNA may also be composed of a single molecule as a single guide RNA. Here, the single guide RNA may be such that crRNA is linked directly or via a linker to tracrRNA. For example, the single guide RNA may have a structure of 5'-(tracrRNA)-(linker)-(crRNA)-3'.

Meanwhile, since no naturally occurring gRNA has been found for CWCas12f1 according to an embodiment of the present disclosure, it was desired to produce an optimal gRNA exhibiting highly efficient targeting and editing activity not only for Un1Cas12f1 and Cas12f1 variant proteins but also for CWCas12f1 protein. From this perspective, the naturally occurring gRNA for CWCas12f1 protein may be a wild-type gRNA found in nature for wild-type Un1Cas12f1, which is similar in size to the CWCas12f1 protein. That is, in the present disclosure, the "wild-type" gRNA for Cas12f1 protein was used to mean "basic" or "canonical" gRNA.

The wild-type gRNA includes two structures in which a part of tracrRNA (tracrRNA anti-repeat) and a part of crRNA repeat (crRNA repeat) are complementarily bound to form a duplex, which are conveniently referred to as R:AR1 (crRNA repeat-tracrRNA anti-repeat duplex 1) and R:AR2 (crRNA repeat-tracrRNA anti-repeat duplex 2) portions. The wild-type guide RNA may comprise (i) at least one stem-loop region, (ii) a tracrRNA-crRNA complementarity, and optionally (iii) a region comprising three or more, four or more, or five or more consecutive uracil (U) residues.

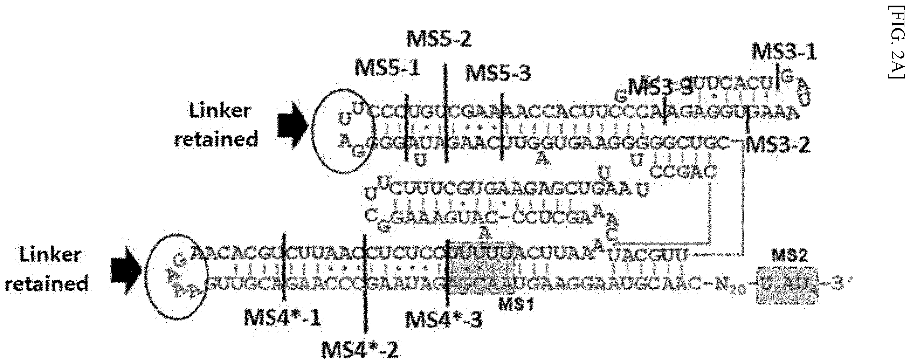

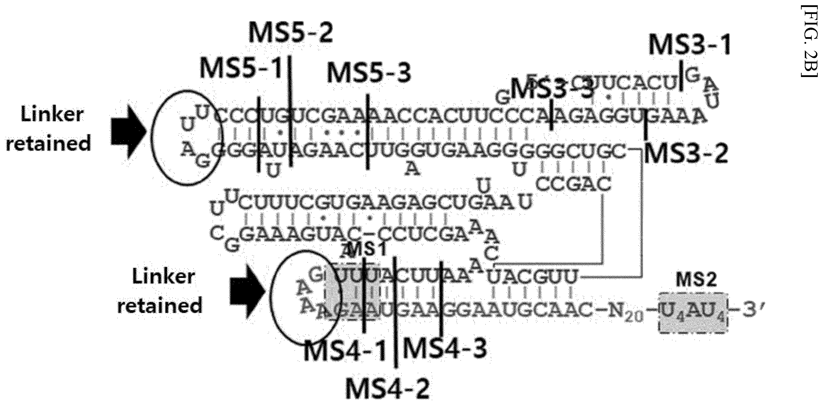

Specifically, the scaffold region of the wild-type guide RNA may sequentially comprise, from the 5'-end, a first stem-loop region, a second stem-loop region, a third stem-loop region, a fourth stem-loop region, and a fifth stem-loop region (or a fifth stem region or a tracrRNA-crRNA complementarity region). For example, referring to FIG. 2, the scaffold region of the wild-type dual guide RNA comprises five stem regions, that is, a first stem-loop region (stem 1), a second stem-loop region (stem 2), a third stem-loop region (stem 3), a fourth stem-loop region (stem 4), and a fifth stem region (stem 5 (including R:AR2)), from the 5'-end. In the present specification, the region comprising stem 5 (R:AR2) is also referred to as a tracrRNA-crRNA complementarity region. Meanwhile, in the present disclosure, the regions subdivided into stem or stem-loop region, tracrRNA-crRNA complementarity region, and the like, do not encompass all regions of the scaffold sequence, and the scaffold sequence may further comprise other regions or sequences that do not correspond to the subdivided regions.

More specifically, the wild-type gRNA may comprise a wild-type tracrRNA having the nucleotide sequence of SEQ ID NO: 175, or a wild-type crRNA having the nucleotide sequence of SEQ ID NO: 176. In addition, the wild-type gRNA may be fused in the form of a single guide RNA to become a single guide RNA (sgRNA) having the nucleotide sequence of SEQ ID NO: 177. Representative sequences of the wild-type tracrRNA, crRNA, and sgRNA are presented in Table 6.

**[Table 6]**

| Name | Nucleotide sequence (5'→3') | SEQ ID NO |
|---|---|---|
| Wild-type tracrRNA | CUUCACUGAUAAAGUGGAGAACCGCUUCACCAAAA GCUGUCCCUUAGGGGAUUAGAACUUGAGUGAAGGU GGGCUGCUUGCAUCAGCCUAAUGUCGAGAAGUGCU UUCUUCGGAAAGUAACCCUCGAAACAAAUUCAUUU UUCCUCUCCAAUUCUGCACAA | 175 |
| Wild-type crRNA | GUUGCAGAACCCGAAUAGACGAAUGAAGGAAUGCA AC | 176 |
| Canonical sgRNA | CUUCACUGAUAAAGUGGAGAACCGCUUCACCAAAA GCUGUCCCuuagGGGAUUAGAACUUGAGUGAAGGUG GGCUGCUUGCAUCAGCCUAAUGUCGAGAAGUGCUU UCUUCGGAAAGUAACCCUCGAAACAAAUUCAUUUU UCCUCUCCAAUUCUGCACAAgaaaGUUGCAGAACCCG AAUAGacgaaUGAAGGAAUGCAACNNNNNNNNNNNNNN NNNNNNN | 177 |

The sequence indicated by 'NNNNNNNNNNNNNNNNNNNN' in Table 6 refers to a guide sequence (spacer sequence) having any length (for example, 15 to 40 nucleotides in length) that can hybridize with a target sequence within a target gene (for example, USH2A gene).

In an embodiment, the guide RNA (for example, the first guide RNA and/or the second guide RNA) for Cas12f1 or a variant protein thereof of the present disclosure is characterized in that it is an engineered guide RNA in which a new configuration is added to a wild-type guide RNA found in nature, or the canonical structure is modified (for example, deleted and/or substituted).

In a more specific embodiment, the engineered gRNA (for example, the first gRNA and/or the second gRNA) is an engineered gRNA comprising a sequence having a wild-type gRNA sequence in which at least one nucleotide has been substituted, deleted, inserted, or added, and wherein the sequence excluding the guide sequence has at least 50%, 50%, 55%, 60%, 65%, 70%, 75%, 80%, 85%, 90%, 91%, 92%, 93%, 94%, 95%, 96%, 97%, 98%, 99% or 95% sequence identity to the wild-type Cas12f1 gRNA. In the context of RNA, nucleic acids, or polypeptides, the term "sequence identity" refers to a value determined by comparing two sequences that are optimally aligned over a comparison window, in which a sequence portion of RNA, nucleic acid, and the like within the comparison window may comprise insertions or deletions (that is, gaps) relative to the reference sequence to achieve optimal alignment.

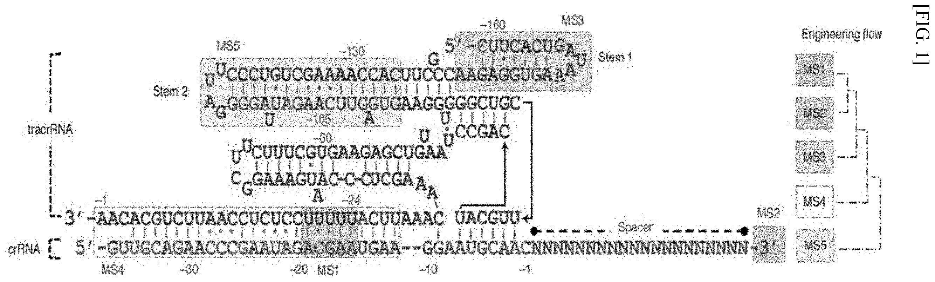

Hereinafter, the structures of wild-type and engineered gRNA and modifications thereof will be described in detail for each of the five modification sites. The modification site is abbreviated as "MS" throughout this specification, and the numbers following "modification site" or "MS" are sequentially assigned depending on engineering flow of each modification site according to an embodiment. However, this does not mean that engineering (modification) at a modification site with a later number necessarily includes engineering (modification) at a modification site with an earlier number. FIG. 1 illustrates modification sites MS1 to MS5 included in the engineered guide RNA according to an embodiment of the present disclosure on the wild-type guide RNA sequence.

In an embodiment, among the subdivided regions of the gRNA as described above, the first stem-loop region comprising modification site 3 (MS3), the second stem-loop region comprising modification site 5 (MS5), and the tracrRNA-crRNA complementarity region (the fifth stem region or the fifth stem-loop region) comprising modification site 1 (MS1) and modification site 4 (MS4) may be defined as corresponding to or included in regions marked by single dotted line boxes with different shades of color in FIG. 1. In addition, the third stem-loop region may be defined as corresponding to or included in the G(-90)-C(-74) sequence in FIG. 1, and the fourth stem-loop region may be defined as corresponding to or included in the U(-68)-A(-35) sequence in FIG. 1.

The modifications applied to the engineered guide RNA (gRNA) of the present disclosure are ultimately intended to achieve high gene editing efficiency while deriving a gRNA that is shorter in length. That is, the modifications disclosed in the present disclosure are intended to produce an engineered gRNA of a shorter length having equal or improved recognition/cleavage efficiency for a target nucleic acid compared to the wild-type gRNA of a longer length, thereby allowing more space to be allocated to other components (for example, additional guide RNAs, shRNAs for suppressing specific gene expression, and the like) for various purposes or uses within the packaging limit (about 4.7 kb) of a delivery vehicle such as adeno-associated virus (AAV). This provides a highly efficient gene editing effect that could not be achieved with the existing CRISPR/Cas system.

Therefore, the engineered gRNA provided in the present disclosure basically comprises a sequence having the wild-type Cas12f1 gRNA sequence in which one or more nucleotides are substituted, deleted, inserted, or added. Here, for the engineered gRNA, a portion thereof excluding the guide sequence may have a sequence identity of 50% or more, 60% or more, 70% or more, 80% or more, 85% or more, 90% or more, or 95% or more with the wild-type Cas12f1 gRNA.

In an embodiment, compared to a wild-type Cas12f1 gRNA comprising (i) at least one stem-loop region, (ii) a tracrRNA-crRNA complementarity region and optionally (iii) a region comprising three or more, four or more, or five or more consecutive uracil (U) residues, the engineered gRNA of the present disclosure may comprise at least one modification selected from the group consisting of (a) deletion of at least a part of the at least one stem-loop region; (b) deletion of at least a part of the tracrRNA-crRNA complementarity region; (c) replacement of one or more of uracil (U) residues when three or more, four or more, or five or more consecutive uracil (U) residues are present; and (d) addition of one or more uridine residues to the 3'-end of the crRNA sequence.

In another embodiment, the engineered guide RNA may comprise at least one modification selected from the group consisting of (a1) deletion of at least a part of the first stem-loop region; (a2) deletion of at least a part of the second stem-loop region; (b) deletion of at least a part of the tracrRNA-crRNA complementarity region; (c) replacement of one or more U with A, G or C in three or more, four or more, or five or more consecutive uracil (U) residues when the consecutive U residues are present in the tracrRNA-crRNA complementarity region; and (d) addition of a U-rich tail to the 3'-end of the crRNA sequence, wherein a sequence of the U-rich tail is represented by 5'-(UₘV)ₙUₒ-3', wherein V is each independently A, C, or G, m and o are integers between 1 to 20, and n is an integer between 0 to 5.

In another embodiment, the engineered guide RNA may comprise a scaffold region comprising a (scaffold) sequence represented by Formula (I).

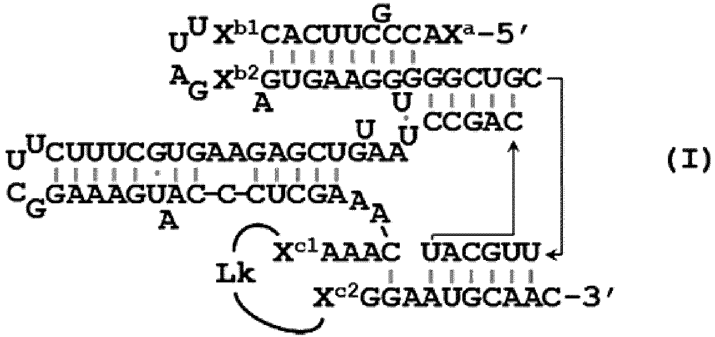

In Formula (I), X^{a}, X^{b1}, X^{b2}, X^{c1}, and X^{c2} each independently consist of 0 to 35 (poly)nucleotides, and Lk is a polynucleotide linker of 2 to 20 nucleotides or is absent.

[In Formula (I), the black solid line represents a chemical bond (for example, a phosphodiester bond) between nucleotides, and the gray thick line represents a complementary bond between nucleotides.]

In Formula (I), in a case where X^{a}, X^{b1}, X^{b2}, X^{c1}, or X^{c2} consists of 0 nucleotides, it is interpreted to mean that X^{a}, X^{b1}, X^{b2}, X^{c1}, or X^{c2} is absent.

In addition, in Formula (I), in a case where X^{a}, X^{b1}, X^{b2}, X^{c1}, or X^{c2} consists of 0 nucleotides or is absent, it is interpreted that if there are two or more nucleotides linked through X^{a}, X^{b1}, X^{b2}, X^{c1}, or X^{c2}, these nucleotides are directly linked to each other in any way. For example, in Formula (I), in a case where X^{b1} consists of 0 nucleotides or is absent, the nucleotide directly linked to the 5'-end of X^{b1} and the nucleotide directly linked to the 3'-end of X^{b1} may be directly linked, for example, by a phosphodiester bond.

In an embodiment, X^{a} may be absent or a (poly)nucleotide having a stem-loop conformation. In another embodiment, X^{a} may consist of 0 to 20 (poly)nucleotides.

In an embodiment, X^{b1} and X^{b2} may be (poly)nucleotides capable of complementary binding. In another embodiment, X^{b1} may consist of 0 to 13 (poly)nucleotides, or X^{b2} may consist of 0 to 14 (poly)nucleotides.

In an embodiment, X^{c1} and X^{c2} may be (poly)nucleotides capable of complementary binding. In another embodiment, X^{c1} may consist of 0 to 28 (poly)nucleotides, or X^{c2} may consist of 0 to 27 (poly)nucleotides.

In an embodiment, Lk is a polynucleotide linker of 2 to 20, 2 to 15, 2 to 10, or 2 to 8 nucleotides, or is absent.

In another embodiment, the scaffold region of the engineered gRNA may be a gRNA consisting of a scaffold sequence represented by Formula (I) or having at least 80%, at least 85%, at least 90%, or at least 95% sequence identity with the sequence. Here, the sequence identity with Formula (I) is based on the sequence excluding the regions indicated by the symbols.

When referring to the scaffold region of the wild-type guide RNA, the first stem-loop region of the scaffold sequence may be a region corresponding to or comprising X^{a} in Formula (I). The second stem-loop region of the scaffold sequence may be a region corresponding to or comprising X^{b1} and X^{b2} in Formula (I). For example, the second stem-loop region comprising X^{b1} and X^{b2} may be a region corresponding to the sequence 5'-CCGCUUCAC-X^{b1}-uuag-X^{b2}-AGUGAAGGUG-3'. The third stem region of the scaffold sequence may be a region corresponding to or comprising the sequence 5'-GGCUGCUUGCAUCAGCC-3' in Formula (I). The fourth stem-loop region of the scaffold sequence may be a region corresponding to or comprising the sequence 5'-UCGAGAAGUGCUUUCUUCGGAAAGUAACCCUCGA-3' in Formula (I). In addition, the tracrRNA-crRNA complementarity region (the fifth stem(-loop) region) of the scaffold sequence may be a region corresponding to X^{c1} and X^{c2} in Formula (I).

Hereinafter, modifications at respective modification sites in the engineered gRNA will be described in detail.

### (1) Modification at modification site 1 (MS1)

This section describes a modification at MS1 (FIG. 1). In an embodiment, wild-type tracrRNA (for example, SEQ ID NO: 175), which may be included in a naturally occurring guide RNA (gRNA). may have a sequence containing five consecutive uracil (U) residues therein. This poses a problem in that, in a case of attempting to express the wild-type tracrRNA in a cell using a vector or the like, such a sequence acts as a transcription termination signal under certain conditions, thereby causing unintended early termination of transcription. That is, in a case where the sequence containing five consecutive U residues acts as a transcription termination signal, normal or complete expression of the tracrRNA is inhibited, and formation of normal or complete gRNA is also inhibited, which consequently decreases gene editing (for example, deletion of exon 13) efficiency of the USH2A gene editing system of the present disclosure.

Therefore, in order to solve the above-mentioned problem, the engineered gRNA may be such that at least one uracil (U) of three or more, four or more, or five or more consecutive U residues, preferably four or five U residues, which are contained in the wild-type tracrRNA (for example, SEQ ID NO: 175), is artificially modified into another nucleotide such as A, C, T, or G.

In an embodiment, the engineered gRNA is provided which comprises a modification in which at least one of three or more, four or more, or five or more consecutive U residues is substituted with a different type of nucleotide in a region containing three or more, four or more, or five or more consecutive U residues, referred to as MS1. As an example, the three or more, four or more, or five or more consecutive U residues may be present in the tracrRNA-crRNA complementarity region of the tracrRNA, wherein a modification may be made by substituting at least one of the three or more, preferably four or more, or five or more U residues with A, G, or C such that no sequence with three or more, preferably four or more, or five or more consecutive U residues exists.

Here, it is preferable that the sequence within the tracrRNA-crRNA complementarity region of crRNA, which corresponds to the sequence to be modified, is also modified together. In an embodiment, when there is the sequence 5'-ACGAA-3' within the tracrRNA-crRNA complementarity region of crRNA, which forms a partial complementary bond with the sequence 5'-UUUUU-3' within the tracrRNA-crRNA complementarity region of tracrRNA, this sequence may be replaced with 5'-NGNNN-3'. Here, N is each independently A, C, G, or U.

In an embodiment, the engineered gRNA of Formula (I) may comprise a modification in which one or more of the U residues are substituted with A, G, or C, when three or more, four or more, or five or more consecutive uracil (U) residues are present in the X^{c1} sequence. For example, when the sequence 5'-UUUUU-3' is present in the X^{c1} sequence, the sequence may be replaced with 5'-NNNCN-3', wherein N is each independently A, C, G, or U. As a more specific example, the sequence 5'-UUUUU-3' in the X^{c1} sequence may be replaced by any one nucleotide sequence selected from the group consisting of the following sequences; however, the replacing sequence is not limited to the following sequences as long as it prevents appearance of a sequence containing three or more, preferably four or more or five or more consecutive U residues: 5'-UUUCU-3', 5'-GUUCU-3', 5'-UCUCU-3', 5'-UUGCU-3', 5'-UUUCC-3', 5'-GCUCU-3', 5'-GUUCC-3', 5'-UCGCU-3', 5'-UCUCC-3', 5'-UUGCC-3', 5'-GCGCU-3', 5'-GCUCC-3', 5'-GUGCC-3', 5'-UCGCC-3', 5'-GCGCC-3', and 5'-GUGCU-3'.

In another embodiment, in the engineered gRNA of Formula (I), the X^{c2} sequence comprises a region in which at least a part of the sequence forms a complementary bond with the X^{c1} sequence (also referred to as a tracrRNA-crRNA complementarity region), wherein a corresponding sequence in the X^{c2} sequence, which forms at least one complementary bond with 3 or more, 4 or more, or 5 or more consecutive U residues present in the X^{c1} sequence, may also be modified. For example, when the sequence 5'-ACGAA-3' is present in the X^{c2} sequence of Formula (I), the sequence may be replaced with 5'-NGNNN-3', wherein N is each independently A, C, G, or U. As a more specific example, the sequence 5'-ACGAA-3' in the X^{c1} sequence of Formula (I) may be replaced by any one nucleotide sequence selected from the group consisting of the following sequences; however, the replacing sequence is not limited to the following sequences: 5'-AGGAA-3', 5'-AGCAA-3', 5'-AGAAA-3', 5'-AGCAU-3', 5'-AGCAG-3', 5'-AGCAC-3', 5'-AGCUA-3', 5'-AGCGA-3', 5'-AGCCA-3', 5'-UGCAA-3', 5'-UGCUA-3', 5'-UGCGA-3', 5'-UGCCA-3', 5'-GGCAA-3', 5'-GGCUA-3', 5'-GGCGA-3', 5'-GGCCA-3', 5'-CGCAA-3', 5'-CGCUA-3', 5'-CGCGA-3', and 5'-CGCCA-3'.

In another embodiment, when a sequence containing 3 or more, 4 or more, or 5 or more consecutive U residues in the X^{c1} sequence of Formula (I) is modified to another sequence, it is preferred that the corresponding nucleotides in the X^{c2} sequence (that is, at least some of which forms a complementary bond therewith) are modified so that they can form a complementary bond with the modified nucleotides. For example, when the sequence 5'-UUUUU-3' in the X^{c1} sequence is modified to 5'-GUGCU-3', it is preferred that the sequence 5'-ACGAA-3' in the X^{c2} sequence is modified to 5'-AGCAA-3'; however, complementary bonding is not necessarily required.

### (2) Modification at modification site 2 (MS2)

This section describes a modification at MS2 (FIG. 1). In an embodiment, the engineered guide RNA (gRNA) may be obtained by adding a new configuration to the gRNA found in nature, and may be such that one or more uridine residues are added to the 3'-end of the crRNA sequence, more specifically, the 3'-end of the spacer sequence included in the crRNA. Here, the 3'-end of the crRNA sequence may be the 3'-end of the guide sequence (spacer). In the present disclosure, the one or more uridine residues added to the 3'-end are also referred to herein as a "U-rich tail." The engineered gRNA comprising one or more uridines or a U-rich tail added to the 3'-end serves to increase nucleic acid cleavage or indel efficiency of the hypercompact CRISPR/Cas12f1 system for a target gene or target nucleic acid.

The term "U-rich tail" as used herein may refer not only to an RNA sequence itself that is rich in uridine (U), but also a DNA sequence encoding the same, and this may be appropriately interpreted depending on the context. The present inventors have experimentally elucidated the structure and effects of the U-rich tail sequence in detail. The U-rich tail sequence will be described in more detail with specific embodiments.

In an embodiment, the U-rich tail sequence may be represented by Ux, wherein x may be 1, 2, 3, 4, 5, 6, 7, 8, 9, 10, 11, 12, 13, 14, 15, 16, 17, 18, 19, or 20. As an example, x may be an integer within a range of two numerical values selected from the numerical values listed above. For example, x may be an integer between 1 and 6. As another example, x may be an integer between 1 and 20. In an embodiment, x may be an integer of 20 or higher.

In another embodiment, the U-rich tail sequence is represented by 5'-(UₘV)ₙUₒ-3', wherein V may be each independently A, C or G, m and o may be integers from 1 to 20, and n may be an integer from 0 to 5. As an example, n may be 0, 1, or 2. As an example, m and o may be each independently 1, 2, 3, 4, 5, 6, 7, 8, 9, or 10.

In yet another embodiment, the U-rich tail sequence may be a U-rich tail represented by 5'-(UₘV)ₙUₒ-3', wherein (i) n is 0, o is an integer between 1 and 6, or (ii) V is each independently A or G, m and o are each independently an integer between 3 and 6, and n is an integer between 1 and 3. In a specific example, the U-rich tail may consist of any one sequence selected from the group consisting of 5'-U-3', 5'-UU-3', 5'-UUU-3', 5'-UUUU-3', 5'-UUUUU-3', 5'-UUUUUU-3', 5'-UUURUUU-3', 5'-UUURUUURUUU-3', 5'-UUUURU-3', 5'-UUUURUU-3', 5'-UUUURUUU-3', 5'-UUUURUUUU-3', 5'-UUUURUUUUU-3', and 5'-UUUURUUUUUU-3', wherein R is A or G. For example, the U-rich tail may be a sequence consisting of or comprising the sequence 5'-UUUUUUUUUU-3' (SEQ ID NO: 351), 5'-UUAUUUAUUU-3' (SEQ ID NO: 352), 5'-UUUCUAUUUU-3' (SEQ ID NO: 353), or 5'-UUAUGUUUUU-3' (SEQ ID NO: 354).

In still yet another embodiment, the U-rich tail sequence may comprise a modified uridine repeat sequence that contains a non-uridine ribonucleoside (A, C, or G) for every 1 to 5 repetitions of uridine. The modified uridine repeat sequence is particularly useful in a case of designing a vector that expresses an engineered crRNA. In an embodiment, the U-rich tail sequence may comprise a sequence in which UV, UUV, UUUV, UUUUV, and/or UUUUUV are repeated one or more times. Here, V is one of A, C or G.

In addition, the U-rich tail sequence may be a combination of the sequence represented by Ux and the sequence represented by 5'-(Uₘ)ₙ-3'. In an embodiment, the U-rich tail sequence may be represented by (U)n1-V1-(U)n2-V2-Ux. Here, V1 and V2 are each one of adenine (A), cytidine (C), and guanine (G). Here, n1 and n2 may each be an integer between 1 and 4. Here, x may be an integer between 1 and 20. In addition, the U-rich tail sequence may have a length of 1 nt, 2 nts, 3 nts, 4 nts, 5 nts, 6 nts, 7 nts, 8 nts, 9 nts, 10 nts, 11 nts, 12 nts, 13 nts, 14 nts, 15 nts, 16 nts, 17 nts, 18 nts, 19 nts, or 20 nts. In an embodiment, the U-rich tail sequence may have a length of 20 nts or longer.

In still yet another embodiment, when the engineered gRNA is expressed in a cell, the U-rich tail may be expressed as one or more sequences due to premature termination of transcription. For example, according to an embodiment, when a gRNA intended to contain a U-rich tail of the sequence 5'-UUUUAUUUUUU-3' is transcribed in a cell, four or more or five or more T residues may act as a termination sequences, and thus gRNAs containing a U-rich tail such as 5'-UUUUAUUUU-3', 5'-UUUUAUUUUUU-3', or 5'-UUUUAUUUUUU-3' may be produced simultaneously. Therefore, in the present disclosure, a U-rich tail containing four or more U residues may be understood to also include a U-rich tail sequence having a shorter length than the intended length.

In still yet another embodiment, the U-rich tail sequence may comprise additional nucleotides other than uridine, depending on the environment where the gene editing system of the present disclosure is actually used and expression environment, such as the internal environment of a eukaryotic cell or a prokaryotic cell.

### (3) Modification at modification site 3 (MS3)

This section describes a modification at MS3 (FIG. 1). As described above, MS3 refers to a region (which may be referred to as the first stem-loop region) that comprises at least a part of the nucleotides forming a stem-loop structure within a complex of the gRNA with an effector protein. The MS3 may comprise a region that does not interact with the effector protein when the gRNA and effector protein form a complex. The modification at MS3 involves removal of at least a part of first stem-loop region near the 5'-end of tracrRNA.

In an embodiment, the engineered gRNA comprises a modification in which at least a part of first stem-loop region (for example, the sequence of SEQ ID NO: 178) is deleted.

In another embodiment, the engineered gRNA comprises a modification in which at least a part of first stem-loop region on tracrRNA is deleted, wherein at least a part of the first stem-loop region to be deleted may consist of 1 to 20 nucleotides. Specifically, at least a part of the first stem-loop region may consist of 2 to 20, 3 to 20, 4 to 20, 5 to 20, 6 to 20, 7 to 20, 8 to 20, 9 to 20, 10 to 20, 11 to 20, 12 to 20, 13 to 20, 14 to 20, 15 to 20, 16 to 20, 17 to 20, 18 to 20, 19, or 20 nucleotides.

In yet another embodiment, the MS3 or the first stem-loop region is a portion corresponding to the polynucleotide indicated by X^{a} of Formula (I), wherein due to a modification in which at least a part of the first stem-loop region is deleted, X^{a} may consist of 0 to 35 (poly)nucleotides, preferably 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1 or 0 (poly)nucleotides.

In an embodiment, in the scaffold sequence of Formula (I), X^{a} may comprise the nucleotide sequence of SEQ ID NO: 178 or may comprise a nucleotide sequence having at least a part thereof, preferably a nucleotide sequence having the sequence of SEQ ID NO: 178 from which 1 to 20 nucleotides are deleted. For example, the nucleotide deletion may involve random deletion of at least 1, 2, 3, 5, 6, 7, 8, 9, 10, 11, 12, 13, 14 15, 16, 17, 18, 19, or 20 nucleotides from the sequence of SEQ ID NO: 178. As a preferred example, the nucleotide deletion may involve sequential deletion of at least 1, 2, 3, 5, 6, 7, 8, 9, 10, 11, 12, 13, 14 15, 16, 17, 18, 19, or 20 nucleotides from the 5'-end of the sequence of SEQ ID NO: 178. More specifically, X^{a} of Formula (I) may comprise or consist of 5'-CUUCACUGAUAAAGUGGAGA-3' (SEQ ID NO: 178), 5'-UUCACUGAUAAAGUGGAGA-3' (SEQ ID NO: 179), 5'-UCACUGAUAAAGUGGAGA-3' (SEQ ID NO: 180), 5'-CACUGAUAAAGUGGAGA-3' (SEQ ID NO: 181), 5'-ACUGAUAAAGUGGAGA-3' (SEQ ID NO: 182), 5'-CUGAUAAAGUGGAGA-3' (SEQ ID NO: 183), 5'-UGAUAAAGUGGAGA-3' (SEQ ID NO: 184), 5'-GAUAAAGUGGAGA-3' (SEQ ID NO: 185), 5'-AUAAAGUGGAGA-3' (SEQ ID NO: 186), 5'-UAAAGUGGAGA-3' (SEQ ID NO: 187), 5'-AAAGUGGAGA-3' (SEQ ID NO: 188), 5'-AAGUGGAGA-3', 5'-AGUGGAGA-3', 5'-GUGGAGA-3', 5'-UGGAGA-3', 5'-GGAGA-3', 5'-GAGA-3', 5'-AGA-3', 5'-GA-3', or 5'-A-3', or X^{a} may be absent.

### (4) Modification at modification site 4 (MS4)

This section describes a modification at MS4 (FIG. 1). MS4 refers to a region spanning the 3'-end of tracrRNA and the 5'-end of crRNA, or, in a case of a single guide RNA form, a region where the sequence corresponding to tracrRNA and the sequence corresponding to crRNA form at least partial complementary bonding. MS4 may comprise at least a part of the sequence referred to as the tracrRNA-crRNA complementarity region (which may also be referred to as the fifth stem region). In the present disclosure, the tracrRNA-crRNA complementarity region may comprise both modification site 1 (MS1) and modification site 4 (MS4). The modification at MS4 comprises deletion of at least a part of the tracrRNA-crRNA complementarity region. The tracrRNA-crRNA complementarity region may comprise a part of tracrRNA and a part of crRNA. In this regard, the tracrRNA-crRNA complementarity region may comprise nucleotides such that partial nucleotides contained in tracrRNA can form complementary bonds with partial nucleotides contained in crRNA within a complex of gRNA with the nucleic acid degrading protein, and may comprise nucleotides adjacent thereto. The tracrRNA-crRNA complementarity region of tracrRNA may comprise a region that does not interact with the nucleic acid degrading protein within a complex of gRNA with the nucleic acid degrading protein.

In some embodiments, the engineered gRNA comprises deletion of at least a part of the tracrRNA-crRNA complementarity region in tracrRNA, deletion of at least a part of the tracrRNA-crRNA complementarity region in crRNA, or deletion of at least a part of the tracrRNA-crRNA complementarity region in both the tracrRNA and the crRNA.

In an embodiment, the tracrRNA-crRNA complementarity region may comprise the nucleotide sequence of SEQ ID NO: 203 and/or the nucleotide sequence of SEQ ID NO: 222.

In another embodiment, the tracrRNA-crRNA complementarity region may further comprise a linker (for example, a polynucleotide) linking the 3'-end of the tracrRNA and the 5'-end of the crRNA.

In an embodiment, the engineered gRNA comprises a modification in which a part of the tracrRNA-crRNA complementarity region is deleted, wherein the part of the complementary region to be deleted may consist of 1 to 54 nucleotides.

In another embodiment, the engineered gRNA comprises a modification in which the entire tracrRNA-crRNA complementarity region is deleted, wherein the entire complementary region to be deleted may consist of 55 nucleotides.

Specifically, at least a part of the tracrRNA-crRNA complementarity region may consist of 3 to 55, 5 to 55, 7 to 55, 9 to 55, 11 to 55, 13 to 55, 15 to 55, 17 to 55, 19 to 55, 21 to 55, 23 to 55, 25 to 55, 27 to 55, 29 to 55, 31 to 55, 33 to 55, 35 to 55, 37 to 55, 39 to 55, or 41 to 55 nucleotides, preferably 42 to 55, 43 to 55, 44 to 55, 45 to 55, 46 to 55, 47 to 55, 48 to 55, 49 to 55, 50 to 55, 51 to 55, 52 to 55, 53 to 55, 54, or 55 nucleotides.

In another embodiment, MS4 or the tracrRNA-crRNA complementarity region is a region corresponding to the polynucleotide indicated by X^{c1} and X^{c2} in Formula (I), in which due to the modification where at least a part of the tracrRNA-crRNA complementarity region is deleted, X^{c1} and X^{c2} may each independently consist of 0 to 35 (poly)nucleotides.

Preferably, X^{c1} may consist of 0 to 28, 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides. In addition, preferably, X^{c2} may consist of 0 to 27, 0 to 26, 0 to 25, 0 to 24, 0 to 23, 0 to 22, 0 to 21, 0 to 20, 0 to 19, 0 to 18, 0 to 17, 0 to 16, 0 to 15, 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides.

In an embodiment, in the scaffold sequence of Formula (I), X^{c1} may comprise the nucleotide sequence of SEQ ID NO: 203 or a nucleotide sequence having the sequence of SEQ ID NO: 203 from which 1 to 28 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, 27, or 28 nucleotides from the 5'-end of the sequence of SEQ ID NO: 203. More specifically, X^{c1} may comprise or consist of 5'-UUCAUUUUUCCUCUCCAAUUCUGCACAA-3' (SEQ ID NO: 203), 5'-UUCAUUUUUCCUCUCCAAUUCUGCACA-3' (SEQ ID NO: 204), 5'-UUCAUUUUUCCUCUCCAAUUCUGCAC-3' (SEQ ID NO: 205), 5'-UUCAUUUUUCCUCUCCAAUUCUGCA-3' (SEQ ID NO: 206), 5'-UUCAUUUUUCCUCUCCAAUUCUGC-3' (SEQ ID NO: 207), 5'-UUCAUUUUUCCUCUCCAAUUCUG-3' (SEQ ID NO: 208), 5'-UUCAUUUUUCCUCUCCAAUUCU-3' (SEQ ID NO: 209), 5'-UUCAUUUUUCCUCUCCAAUUC-3' (SEQ ID NO: 210), 5'-UUCAUUUUUCCUCUCCAAUU-3' (SEQ ID NO: 211), 5'-UUCAUUUUUCCUCUCCAAU-3' (SEQ ID NO: 212), 5'-UUCAUUUUUCCUCUCCAA-3' (SEQ ID NO: 213), 5'-UUCAUUUUUCCUCUCCA-3' (SEQ ID NO: 214), 5'-UUCAUUUUUCCUCUCC-3' (SEQ ID NO: 215), 5'-UUCAUUUUUCCUCUC-3' (SEQ ID NO: 216), 5'-UUCAUUUUUCCUCU-3' (SEQ ID NO: 217), 5'-UUCAUUUUUCCUC-3' (SEQ ID NO: 218), 5'-UUCAUUUUUCCU-3' (SEQ ID NO: 219), 5'-UUCAUUUUUCC-3' (SEQ ID NO: 220), 5'-UUCAUUUUUC-3' (SEQ ID NO: 221), 5'-UUCAUUUUU-3', 5'-UUCAUUUU-3', 5'-UUCAUUU-3', 5'-UUCAUU-3', 5'-UUCAU-3', 5'-UUCA-3', 5'-UUC-3', 5'-UU-3', or 5'-U-3', or X^{c1} may be absent.

Here, in a case where there is a region containing 3, 4, or 5 or more uracil (U) residues in the sequence of X^{c1} from which some nucleotides have been removed, the modification at MS1 as described above may also apply. For details about MS1, see the section "(1) Modification at modification site 1 (MS1)."

In yet another embodiment, in the scaffold sequence of Formula (I), X^{c2} may comprise the nucleotide sequence of SEQ ID NO: 222 or a nucleotide sequence having the sequence of SEQ ID NO: 222 from which 1 to 27 nucleotides are deleted. Preferably, the nucleotide deletion may involve sequential removal of at least 1, 2, 3, 5, 6, 7, 8, 9, 10, 11, 12, 13, 14, 15, 16, 17, 18, 19, 20, 21, 22, 23, 24, 25, 26, or 27 nucleotides from the 5'-end of the sequence of SEQ ID NO: 222. More specifically, X^{c2} may comprise or consist of 5'-GUUGCAGAACCCGAAUAGACGAAUGAA-3' (SEQ ID NO: 222), 5'-UUGCAGAACCCGAAUAGACGAAUGAA-3' (SEQ ID NO: 223), 5'-UGCAGAACCCGAAUAGACGAAUGAA-3' (SEQ ID NO: 224), 5'-GCAGAACCCGAAUAGACGAAUGAA-3' (SEQ ID NO: 225), 5'-CAGAACCCGAAUAGACGAAUGAA-3' (SEQ ID NO: 226), 5'-AGAACCCGAAUAGACGAAUGAA-3' (SEQ ID NO: 227), 5'-GAACCCGAAUAGACGAAUGAA-3' (SEQ ID NO: 228), 5'-AACCCGAAUAGACGAAUGAA-3' (SEQ ID NO: 229), 5'-ACCCGAAUAGACGAAUGAA-3' (SEQ ID NO: 230), 5'-CCCGAAUAGACGAAUGAA-3' (SEQ ID NO: 231), 5'-CCGAAUAGACGAAUGAA-3' (SEQ ID NO: 232), 5'-CGAAUAGACGAAUGAA-3' (SEQ ID NO: 233), 5'-GAAUAGACGAAUGAA-3' (SEQ ID NO: 234), 5'-AAUAGACGAAUGAA-3' (SEQ ID NO: 235), 5'-AUAGACGAAUGAA-3' (SEQ ID NO: 236), 5'-UAGACGAAUGAA-3' (SEQ ID NO: 237), 5'-AGACGAAUGAA-3' (SEQ ID NO: 238), 5'-GACGAAUGAA-3' (SEQ ID NO: 239), 5'-ACGAAUGAA-3', 5'-CGAAUGAA-3', 5'-GAAUGAA-3', 5'-AAUGAA-3', 5'-AUGAA-3', 5'-UGAA-3', 5'-GAA-3', 5'-AA-3', or 5'-A-3', or X^{c2} may be absent.

Here, in a case where there is a sequence corresponding a sequence containing 3 or more, or 3, 4, or 5 or more uracil (U) residues in the sequence of X^{c2} from which some nucleotides have been removed, the modification at MS1 as described above may also apply. For details regarding MS1, see the section "(1) Modification at modification site 1 (MS1)."

In the scaffold sequence of Formula (I), the regions corresponding to X^{c1} and X^{c2} may each independently undergo the above-described modification. However, MS4 or the tracrRNA-crRNA complementarity region is a region where tracrRNA and crRNA form complementary bonds. For the tracrRNA and the crRNA to function as a dual guide RNA, it is preferable that the position and number of nucleotides to be deleted in each of X^{c1} and X^{c2} be identical with or similar to each other. That is, in order to preserve complementarity between the X^{c1} and X^{c2} sequences, in a case of sequentially deleting nucleotides from the 3'-end of tracrRNA in MS4 (tracrRNA-crRNA complementarity region), it is preferable to sequentially delete nucleotides from the 5'-end of crRNA. In an embodiment according to this viewpoint, deletion of the X^{c1} and X^{c2} nucleotide sequences may involve deletion of one or more complementary nucleotide pairs.

In an embodiment, the 3'-end of X^{c1} and the 5'-end of X^{c2} in the scaffold sequence of Formula (I) may be linked by a linker (Lk) so that the gRNA is modified into a single guide RNA (sgRNA) form. Lk is a sequence that physically or chemically connects tracrRNA and crRNA, and may be a polynucleotide sequence having a length of 1 to 30 nucleotides. In an embodiment, Lk may be a sequence of 1 to 5, 5 to 10, 10 to 15, 2 to 20, 15 to 20, 20 to 25, or 25 to 30 nucleotides. For example, Lk may be, but is not limited to, 5'-GAAA-3'. As another example, Lk may be a linker comprising or consisting of 5'-UUAG-3', 5'-UGAAAA-3', 5'-UUGAAAAA-3', 5'-UUCGAAAGAA-3' (SEQ ID NO: 240), 5'-UUCAGAAAUGAA-3' (SEQ ID NO: 241), 5'-UUCAUGAAAAUGAA-3' (SEQ ID NO: 242), or 5'-UUCAUUGAAAAAUGAA-3' (SEQ ID NO: 243).

Meanwhile, while it is possible to use a linker (Lk) to make a single guide RNA (sgRNA), it is also possible to directly connect the 3'-end of tracrRNA, of which a partial sequence has been removed, to the 5'-end of crRNA of which a partial sequence has been removed.

In another embodiment, a case where X^{c1} and X^{c2} in the scaffold sequence of Formula (I) are linked by a linker may be indicated by 5'-X^{c1}-Lk-X^{c2}-3' as in Formula (I), and the 5'-X^{c1}-Lk-X^{c2}-3' may be any one nucleotide sequence selected from the group consisting of SEQ ID NOs: 244 to 250 and 5'-Lk-3' (a form in which both X^{c1} and X^{c2} are deleted), but is not limited thereto.

### (5) Modification at modification site 5 (MS5)

This section describes a modification at MS1 (FIG. 1). As described above, MS5 corresponds to a region located toward the 3'-end of tracrRNA, which is referred to as the second stem-loop region. The second stem-loop region may comprise nucleotides that form a stem structure within a complex of the guide RNA (gRNA) with nucleic acid editing protein, and may comprise nucleotides adjacent thereto. Here, the stem or stem-loop structure is distinct from the stem included in the above-described first stem-loop region.

### In an embodiment, the second stem-loop region may comprise the nucleotide sequence of SEQ ID NO: 189 and/or the nucleotide sequence of SEQ ID NO: 193.

In another embodiment, MS5 or the second stem-loop region is a region comprising a (poly)nucleotide (comprising a loop of 5'-UUAG-3') that is adjacent to the polynucleotide indicated by X^{b1} and X^{b2} in Formula (I), in which due to the modification where at least the part of the second stem region is deleted, X^{b1} and X^{b2} may each independently consist of 0 to 35 (poly)nucleotides.

In an embodiment, the engineered gRNA comprises a modification in which at least a part of the second stem-loop region is deleted.

In another embodiment, the engineered gRNA comprises deletion of at least a part of the second stem-loop region, wherein at least a part of the second stem-loop region to be deleted may consist of 1 to 27 nucleotides. Specifically, the at least a part of the second stem region may consist of 2 to 27, 3 to 27, 4 to 27, 5 to 27, 6 to 27, 7 to 27, 8 to 27, 9 to 27, 10 to 27, 11 to 27, 12 to 27, 13 to 27, 14 to 27, 15 to 27, 16 to 27, 17 to 27, 18 to 27, 19 to 27, 20 to 27, 21 to 27, 22 to 27, 23 to 27, 24 to 27, 25 to 27, 26, or 27 nucleotides.

Preferably, X^{b1} in Formula (I) may consist of 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides. In addition, preferably, X^{b2} may consist of 0 to 14, 0 to 13, 0 to 12, 0 to 11, 0 to 10, 0 to 9, 0 to 8, 0 to 7, 0 to 6, 0 to 5, 0 to 4, 0 to 3, 0 to 2, 1, or 0 (poly)nucleotides.

In an embodiment, in the scaffold sequence of Formula (I), X^{b1} may comprise the nucleotide sequence of SEQ ID NO: 189 or a nucleotide sequence having the sequence of SEQ ID NO: 189 from which 1 to 13 nucleotides are deleted. Preferably, the nucleotide deletion may involve sequential removal of at least 1, 2, 3, 5, 6, 7, 8, 9, 10, 11, 12, or 13 nucleotide from the 5'-end of the sequence of SEQ ID NO: 189. More specifically, X^{b1} may comprise or consist of 5'-CAAAAGCUGUCCC-3' (SEQ ID NO: 189), 5'-CAAAAGCUGUCC-3' (SEQ ID NO: 190), 5'-CAAAAGCUGUC-3' (SEQ ID NO: 191), 5'-CAAAAGCUGU-3' (SEQ ID NO: 192), 5'-CAAAAGCUG-3', 5'-CAAAAGCU-3', 5'-CAAAAGC-3', 5'-CAAAAG-3', 5'-CAAAA-3', 5'-CAAA-3', 5'-CAA-3', 5'-CA-3', or 5'-C-3', or X^{b1} may be absent.

In another embodiment, in the scaffold sequence of Formula (I), X^{b2} may comprise the nucleotide sequence of SEQ ID NO: 193 or a nucleotide sequence having the sequence of SEQ ID NO: 193 from which 1 to 14 nucleotides are deleted. Preferably, the nucleotide deletion may involve sequential removal of at least 1, 2, 3, 5, 6, 7, 8, 9, 10, 11, 12, 13, or 14 nucleotides from the 5'-end of the sequence of SEQ ID NO: 193. More specifically, X^{b2} may comprise or consist of 5'-GGGAUUAGAACUUG-3' (SEQ ID NO: 193), 5'-GGAUUAGAACUUG-3' (SEQ ID NO: 194), 5'-GAUUAGAACUUG-3' (SEQ ID NO: 195), 5'-AUUAGAACUUG-3' (SEQ ID NO: 196), 5'-UUAGAACUUG-3' (SEQ ID NO: 197), 5'-UAGAACUUG-3', 5'-AGAACUUG-3', 5'-GAACUUG-3', 5'-AACUUG-3', 5'-ACUUG-3', 5'-CUUG-3', 5'-UUG-3', 5'-UG-3', or 5'-G-3', or X^{b2} may be absent.

In the scaffold sequence of Formula (I), the regions corresponding to X^{b1} and X^{b2} may be each independently modified. However, for normal preservation of the stem-loop structure, it is preferable that the position and number of nucleotides to be deleted in each of X^{b1} and X^{b2} be identical with or similar to each other. For example, in a case of sequentially deleting nucleotides from the 5'-end direction in X^{b1}, it is preferable to sequentially delete nucleotides from the 3'-end direction in X^{b2}. In an embodiment according to this viewpoint, the deletion of the nucleotide sequences X^{b1} and X^{b2} may be deletion of one or more complementary nucleotide pairs.

In another embodiment, a sequence of the loop portion connecting X^{b1} and X^{b2} in the scaffold sequence of Formula (I) is indicated by 5'-UUAG-3', and this may be replaced with another sequence such as 5'-NNNN-3' and '5-NNN-3', if necessary. Here, N is each independently A, C, G, or U. For example, the 5'-NNNN-3' may be 5'-GAAA-3', and the '5-NNN-3' may be 5'-CGA-3'.

For example, in the scaffold sequence of Formula (I), a sequence of the loop portion connecting X^{b1} and X^{b2} is 5'-UUAG-3', and the sequence 5'-X^{b1}UUAG X^{b2}-3' in Formula (I) may comprise or consist of any one nucleotide sequence selected from the group consisting of SEQ ID NOs: 198 to 202 and 5'-UUAG-3' (a form in which both X^{b1} and X^{b2} are deleted).

### (6) Examples of gRNAs to which modifications at modification sites 1 to 5 have been applied

The engineered guide RNA (for example, the engineered first guide RNA and/or the engineered second guide RNA) included in the USH2A gene editing system of the present disclosure may comprise modifications at two or more of the above-mentioned modification sites 1 (MS1) to 5 (MS5).

In an embodiment, the engineered guide RNA may comprise one or more modifications selected from the group consisting of (a1) deletion of at least a part of the first stem-loop region; (a2) deletion at least a part of the second stem-loop region; (b) deletion of at least a part of the tracrRNA-crRNA complementarity region; (c) replacement of one or more uracil (U) residues with A, G, or C in three or more consecutive U residues when the consecutive U residues are present in the tracrRNA-crRNA complementarity region; and (d) addition of a U-rich tail to the 3'-end of the crRNA sequence. The U-rich tail sequence may be represented by 5'-(UₘV)ₙUₒ-3', wherein V is each independently A, C, or G, m and o are integers between 1 to 20, and n is an integer between 0 to 5.

For example, the engineered guide RNA may comprise (d) addition of a U-rich tail to the 3'-end of the crRNA sequence and (c) replacement of one or more uracil (U) residues with A, G, or C in three or more, four or more, or five or more consecutive U residues when the consecutive U residues are present in the tracrRNA-crRNA complementarity region.

As another example, the engineered guide RNA may comprise (d) addition of a U-rich tail to the 3'-end of the crRNA sequence, (c) replacement of one or more U with A, G or C in three or more, four or more, or five or more consecutive U residues when the consecutive U residues are present in the tracrRNA-crRNA complementarity region, and (a1) deletion of at least a part of first stem-loop region.

As yet another example, the engineered guide RNA may comprise (d) addition of a U-rich tail to the 3'-end of the crRNA sequence, (c) replacement of one or more U with A, G or C in three or more, four or more, or five or more consecutive U residues when the consecutive U residues are present in the tracrRNA-crRNA complementarity region, and (a1) deletion of at least a part of first stem-loop region.

As another example, the engineered guide RNA may comprise (d) addition of a U-rich tail to the 3'-end of the crRNA sequence, (a1) deletion of at least a part of the first stem-loop region, and (b) deletion of at least a part of the tracrRNA-crRNA complementarity region, wherein the engineered guide RNA may further comprise replacement of one or more U with A, G or C in three or more, four or more, or five or more consecutive U residues when the consecutive U residues are present in the tracrRNA-crRNA complementarity region containing partial deletion.

As another example, the engineered guide RNA may comprise (d) addition of a U-rich tail to the 3'-end of the crRNA sequence, (a1) deletion of at least a part of the first stem-loop region, (b) deletion of at least a part of the tracrRNA-crRNA complementarity region, and (a2) deletion of at least a part of the second stem-loop region, wherein the engineered guide RNA may further comprise replacement of one or more U with A, G or C in three or more, four or more, or five or more consecutive U residues when the consecutive U residues are present in the tracrRNA-crRNA complementarity region containing partial deletion.

As an example of tracrRNA to which modifications at the plurality of modification sites (MS) as described above have been applied, there is provided an engineered tracrRNA comprising the nucleotide sequence of any one of SEQ ID NOs: 251 to 296.

Specifically, the engineered tracrRNA may comprise or consist of the nucleotide sequence of SEQ ID NO: 251 (MS1), SEQ ID NO: 252 (MS1/MS3-1), SEQ ID NO: 253 (MS1/MS3-2), SEQ ID NO: 254 (MS1/MS3-3), SEQ ID NO: 255 (MS1/MS4*-1), SEQ ID NO: 256 (MS1/MS4*-2), SEQ ID NO: 257 (MS1/MS4*-3), SEQ ID NO: 258 (MS1/MS5-1), SEQ ID NO: 259 (MS1/MS5-2), SEQ ID NO: 260 (MS1/MS5-3), SEQ ID NO: 261 (MS1/MS3-3/MS4*-1), SEQ ID NO: 262 (MS1/MS3-3/MS4*-2), SEQ ID NO: 263 (MS1/MS3-3/MS4*-3), SEQ ID NO: 264 (MS1/MS4*-2/MSS-1), SEQ ID NO: 265 (MS1/MS4*-2/MSS-2), SEQ ID NO: 266 (MS1/MS4*-2/MSS-3), SEQ ID NO: 267 (MS1/MS3-3/MS5-1), SEQ ID NO: 268 (MS1/MS3-3/MS5-2), SEQ ID NO: 269 (MS1/MS3-3/MS5-3), SEQ ID NO: 270 (MS1/MS3-3/MS4*-2/MSS-3), SEQ ID NO: 271 (mature form, MF), SEQ ID NO: 272 (MF/MS3-1), SEQ ID NO: 273 (MF/MS3-2), SEQ ID NO: 274 (MF/MS3-3), SEQ ID NO: 275 (MF/MS4-1), SEQ ID NO: 276 (MF/MS4-2), SEQ ID NO: 277 (MF/MS4-3), SEQ ID NO: 278 (MF/MS5-1), SEQ ID NO: 279 (MF/MS5-2), SEQ ID NO: 280 (MF/MS5-3), SEQ ID NO: 281 (MF/MS5), SEQ ID NO: 282 (MF/MS3-3/MS4-1), SEQ ID NO: 283 (MF/MS3-3/MS4-2), SEQ ID NO: 284 (MF/MS3-3/MS4-3), SEQ ID NO: 285 (MF/MS4-3/MS5-1), SEQ ID NO: 286 (MF/MS4-3/MS5-2), SEQ ID NO: 287 (MF/MS4-3/MS5-3), SEQ ID NO: 288 (MF/MS4-3/MS5-F), SEQ ID NO: 289 (MF/MS3-3/MS5-1), SEQ ID NO: 290 (MF/MS3-3/MS5-2), SEQ ID NO: 291 (MF/MS3-3/MS5-3), SEQ ID NO: 292 (MF/MS3-3/MS5), SEQ ID NO: 293 (MF/MS3-3/MS4-3/MS5-3), SEQ ID NO: 294 (MF/MS3-3/MS4-1/MS5), SEQ ID NO: 295 (MF/MS3-3/MS4-2/MS5), or SEQ ID NO: 296 (MF/MS3-3/MS4-3/MS5).

As a more specific example, exemplary sequences of the engineered tracrRNA, which has one or more modifications at any one or more of the modification sites selected from MS1, MS3, MS4, and MS5, are provided in Table 7 below. Such an engineered tracrRNA constitutes part of the scaffold sequence of the scaffold region.

**[Table 7]**

| tracrRNA | Nucleotide sequence | SEQ ID NO |
|---|---|---|
| MS1 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAGUGCUCCUCUCCAAUUCUGC ACAA | 251 |
| MS1/MS3-1 | GAUAAAGUGGAGAACCGCUUCACCAAAAGCUGUCCCUUA GGGGAUUAGAACUUGAGUGAAGGUGGGCUGCUUGCAUCA GCCUAAUGUCGAGAAGUGCUUUCUUCGGAAAGUAACCCU CGAAACAAAUUCAGUGCUCCUCUCCAAUUCUGCACAA | 252 |
| MS1/MS3-2 | UGGAGAACCGCUUCACCAAAAGCUGUCCCUUAGGGGAUU AGAACUUGAGUGAAGGUGGGCUGCUUGCAUCAGCCUAAU GUCGAGAAGUGCUUUCUUCGGAAAGUAACCCUCGAAACA AAUUCAGUGCUCCUCUCCAAUUCUGCACAA | 253 |
| MS1/MS3-3 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUCAG UGCUCCUCUCCAAUUCUGCACAA | 254 |
| MS1/MS4*-1 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAGUGCUCCUCUCCAAUUC | 255 |
| MS1/MS4*-2 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAGUGCUCCUCUC | 256 |
| MS1/MS4*-3 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAGUGCU | 257 |
| MS1/MS5-1 | CUUCACUGAUAAAGUGGAGAACCGCUUCACCAAAAGCUG UUUAGAUUAGAACUUGAGUGAAGGUGGGCUGCUUGCAUC AGCCUAAUGUCGAGAAGUGCUUUCUUCGGAAAGUAACCC UCGAAACAAAUUCAGUGCUCCUCUCCAAUUCUGCACAA | 258 |
| MS1/MS5-2 | CUUCACUGAUAAAGUGGAGAACCGCUUCACCAAAAGCUU AGGAACUUGAGUGAAGGUGGGCUGCUUGCAUCAGCCUAA UGUCGAGAAGUGCUUUCUUCGGAAAGUAACCCUCGAAAC AAAUUCAGUGCUCCUCUCCAAUUCUGCACAA | 259 |
| MS1/MS5-3 | CUUCACUGAUAAAGUGGAGAACCGCUUCACCAAUUAGUU | 260 |
| | GAGUGAAGGUGGGCUGCUUGCAUCAGCCUAAUGUCGAGA AGUGCUUUCUUCGGAAAGUAACCCUCGAAACAAAUUCAG UGCUCCUCUCCAAUUCUGCACAA | |
| MS1/MS3-3/MS4*-1 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUCAG UGCUCCUCUCCAAUUC | 261 |
| MS1/MS3-3/MS4*-2 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUCAG UGCUCCUCUC | 262 |
| MS1/MS3-3/MS4*-3 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUCAG UGCU | 263 |
| MS1/MS4*-2/MS5-1 | CUUCACUGAUAAAGUGGAGAACCGCUUCACCAAAAGCUG UUUAGAUUAGAACUUGAGUGAAGGUGGGCUGCUUGCAUC AGCCUAAUGUCGAGAAGUGCUUUCUUCGGAAAGUAACCC UCGAAACAAAUUCAGUGCUCCUCUC | 264 |
| MS1/MS4*-2/MS5-2 | CUUCACUGAUAAAGUGGAGAACCGCUUCACCAAAAGCUU AGGAACUUGAGUGAAGGUGGGCUGCUUGCAUCAGCCUAA UGUCGAGAAGUGCUUUCUUCGGAAAGUAACCCUCGAAAC AAAUUCAGUGCUCCUCUC | 265 |
| MS1/MS4*-2/MS5-3 | CUUCACUGAUAAAGUGGAGAACCGCUUCACCAAUUAGUU GAGUGAAGGUGGGCUGCUUGCAUCAGCCUAAUGUCGAGA AGUGCUUUCUUCGGAAAGUAACCCUCGAAACAAAUUCAG UGCUCCUCUC | 266 |
| MS1/MS3-3/MS5-1 | ACCGCUUCACCAAAAGCUGUUUAGAUUAGAACUUGAGUG AAGGUGGGCUGCUUGCAUCAGCCUAAUGUCGAGAAGUGC UUUCUUCGGAAAGUAACCCUCGAAACAAAUUCAGUGCUCC UCUCCAAUUCUGCACAA | 267 |
| MS1/MS3-3/MS5-2 | ACCGCUUCACCAAAAGCUUAGGAACUUGAGUGAAGGUGG GCUGCUUGCAUCAGCCUAAUGUCGAGAAGUGCUUUCUUC GGAAAGUAACCCUCGAAACAAAUUCAGUGCUCCUCUCCAA UUCUGCACAA | 268 |
| MS1/MS3-3/MS5-3 | ACCGCUUCACCAAUUAGUUGAGUGAAGGUGGGCUGCUUG CAUCAGCCUAAUGUCGAGAAGUGCUUUCUUCGGAAAGUA ACCCUCGAAACAAAUUCAGUGCUCCUCUCCAAUUCUGCAC AA | 269 |
| MS1/MS3-3/MS4*-2/MS5-3 | ACCGCUUCACCAAUUAGUUGAGUGAAGGUGGGCUGCUUG CAUCAGCCUAAUGUCGAGAAGUGCUUUCUUCGGAAAGUA ACCCUCGAAACAAAUUCAGUGCUCCUCUC | 270 |
| Mature Form(MF) | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAUUU | 271 |
| MF/MS3-1 | GAUAAAGUGGAGAACCGCUUCACCAAAAGCUGUCCCUUA GGGGAUUAGAACUUGAGUGAAGGUGGGCUGCUUGCAUCA GCCUAAUGUCGAGAAGUGCUUUCUUCGGAAAGUAACCCU CGAAACAAAUUCAUUU | 272 |
| MF/MS3-2 | UGGAGAACCGCUUCACCAAAAGCUGUCCCUUAGGGGAUU AGAACUUGAGUGAAGGUGGGCUGCUUGCAUCAGCCUAAU GUCGAGAAGUGCUUUCUUCGGAAAGUAACCCUCGAAACA AAUUCAUUU | 273 |
| MF/MS3-3 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUCAU UU | 274 |
| MF/MS4-1 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUCAU | 275 |
| MF/MS4-2 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAAUUC | 276 |
| MF/MS4-3 | CUUCACUGAUAAAGUGGAGAACCGCUUCACCAAAAGCUG UCCCUUAGGGGAUUAGAACUUGAGUGAAGGUGGGCUGCU UGCAUCAGCCUAAUGUCGAGAAGUGCUUUCUUCGGAAAG UAACCCUCGAAACAAA | 277 |
| MF/MS5-1 | CUUCACUGAUAAAGUGGAGAACCGCUUCACCAAAAGCUG UUUAGAUUAGAACUUGAGUGAAGGUGGGCUGCUUGCAUC AGCCUAAUGUCGAGAAGUGCUUUCUUCGGAAAGUAACCC UCGAAACAAAUUCAUUU | 278 |
| MF/MS5-2 | CUUCACUGAUAAAGUGGAGAACCGCUUCACCAAAAGCUU UAGAGAACUUGAGUGAAGGUGGGCUGCUUGCAUCAGCCU AAUGUCGAGAAGUGCUUUCUUCGGAAAGUAACCCUCGAA ACAAAUUCAUUU | 279 |
| MF/MS5-3 | CUUCACUGAUAAAGUGGAGAACCGCUUCACCAAUUAGUU GAGUGAAGGUGGGCUGCUUGCAUCAGCCUAAUGUCGAGA AGUGCUUUCUUCGGAAAGUAACCCUCGAAACAAAUUCAU UU | 280 |
| MF/MS5 | CUUCACUGAUAAAGUGGAGAACCGCUUCACUUAGAGUGA AGGUGGGCUGCUUGCAUCAGCCUAAUGUCGAGAAGUGCU | 281 |
| | UUCUUCGGAAAGUAACCCUCGAAACAAAUUCAUUU | |
| MF/MS3-3/MS4-1 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUCAU | 282 |
| MF/MS3-3/MS4-2 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAAUUC | 283 |
| MF/MS3-3/MS4-3 | ACCGCUUCACCAAAAGCUGUCCCUUAGGGGAUUAGAACUU GAGUGAAGGUGGGCUGCUUGCAUCAGCCUAAUGUCGAGA AGUGCUUUCUUCGGAAAGUAACCCUCGAAACAAA | 284 |
| MF/MS4-3/MS5-1 | CUUCACUGAUAAAGUGGAGAACCGCUUCACCAAAAGCUG UUUAGAUUAGAACUUGAGUGAAGGUGGGCUGCUUGCAUC AGCCUAAUGUCGAGAAGUGCUUUCUUCGGAAAGUAACCC UCGAAACAAA | 285 |
| MF/MS4-3/MS5-2 | CUUCACUGAUAAAGUGGAGAACCGCUUCACCAAAAGCUU UAGAGAACUUGAGUGAAGGUGGGCUGCUUGCAUCAGCCU AAUGUCGAGAAGUGCUUUCUUCGGAAAGUAACCCUCGAA ACAAA | 286 |
| MF/MS4-3/MS5-3 | CUUCACUGAUAAAGUGGAGAACCGCUUCACCAAUUAGUU GAGUGAAGGUGGGCUGCUUGCAUCAGCCUAAUGUCGAGA AGUGCUUUCUUCGGAAAGUAACCCUCGAAACAAA | 287 |
| MF/MS4-3/MS5 | CUUCACUGAUAAAGUGGAGAACCGCUUCACUUAGAGUGA AGGUGGGCUGCUUGCAUCAGCCUAAUGUCGAGAAGUGCU UUCUUCGGAAAGUAACCCUCGAAACAAA | 288 |
| MF/MS3-3/MS5-1 | ACCGCUUCACCAAAAGCUGUUUAGAUUAGAACUUGAGUG AAGGUGGGCUGCUUGCAUCAGCCUAAUGUCGAGAAGUGC UUUCUUCGGAAAGUAACCCUCGAAACAAAUUCAUUU | 289 |
| MF/MS3-3/MS5-2 | ACCGCUUCACCAAAAGCUUUAGAGAACUUGAGUGAAGGU GGGCUGCUUGCAUCAGCCUAAUGUCGAGAAGUGCUUUCU UCGGAAAGUAACCCUCGAAACAAAUUCAUUU | 290 |
| MF/MS3-3/MS5-3 | ACCGCUUCACCAAUUAGUUGAGUGAAGGUGGGCUGCUUG CAUCAGCCUAAUGUCGAGAAGUGCUUUCUUCGGAAAGUA ACCCUCGAAACAAAUUCAUUU | 291 |
| MF/MS3-3/MS5 | ACCGCUUCACUUAGAGUGAAGGUGGGCUGCUUGCAUCAG CCUAAUGUCGAGAAGUGCUUUCUUCGGAAAGUAACCCUC GAAACAAAUUCAUUU | 292 |
| MF/MS3-3/MS4-3/MS5-3 | ACCGCUUCACCAAUUAGUUGAGUGAAGGUGGGCUGCUUG CAUCAGCCUAAUGUCGAGAAGUGCUUUCUUCGGAAAGUA ACCCUCGAAACAAA | 293 |
| MF/MS3-3/MS4-1/MS5 | ACCGCUUCACUUAGAGUGAAGGUGGGCUGCUUGCAUCAG CCUAAUGUCGAGAAGUGCUUUCUUCGGAAAGUAACCCUC GAAACAAAUUCAU | 294 |
| MF/MS3-3/MS4-2/MS5 | ACCGCUUCACUUAGAGUGAAGGUGGGCUGCUUGCAUCAG CCUAAUGUCGAGAAGUGCUUUCUUCGGAAAGUAACCCUC GAAACAAAUUC | 295 |
| MF/MS3-3/MS4-3/MS5 | ACCGCUUCACUUAGAGUGAAGGUGGGCUGCUUGCAUCAG CCUAAUGUCGAGAAGUGCUUUCUUCGGAAAGUAACCCUC GAAACAAA | 296 |

In addition, as an example of crRNA to which modifications at the plurality of modification sites (MS) as described above have been applied, there is provided an engineered crRNA comprising the nucleotide sequence of any one of SEQ ID NOs: 297 to 312.

Specifically, the engineered crRNA of the present disclosure may comprise or consist of the nucleotide of SEQ ID NO: 297 (MS1), SEQ ID NO: 298 (MS1/MS4*-1), SEQ ID NO: 299 (MS1/MS4*-2), SEQ ID NO: 300 (MS1/MS4*-3), SEQ ID NO: 301 (mature form; MF), SEQ ID NO: 302 (MF/MS4-1), SEQ ID NO: 303 (MF/MS4-2), SEQ ID NO: 304 (MF/MS4-3), SEQ ID NO: 305 (MS1/MS2), SEQ ID NO: 306 (MS1/MS2/MS4M), SEQ ID NO: 307 (MS1/MS2/MS4*-2), SEQ ID NO: 308 (MS1/MS2/MS4*-3), SEQ ID NO: 309 (MF/MS2), SEQ ID NO: 310 (MF/MS2/MS4-1), SEQ ID NO: 311 (MF/MS2/MS4-2), or SEQ ID NO: 312 (MF/MS2/MS4-3).

In some embodiments, exemplary sequences of the engineered crRNA, which has one or more modifications at any one or more modification sites selected from MS1, MS2, and MS4 are provided in Table 8 below.

**[Table 8]**

| tracrRNA | Nucleotide sequence | SEQ ID NO |
|---|---|---|
| MS1 | GUUGCAGAACCCGAAUAGAGCAAUGAAGGAAUGCAAC | 297 |
| MS1/MS4*-1 | GAACCCGAAUAGAGCAAUGAAGGAAUGCAAC | 298 |
| MS1/MS4*-2 | GAAUAGAGCAAUGAAGGAAUGCAAC | 299 |
| MS1/MS4*-3 | AGCAAUGAAGGAAUGCAAC | 300 |
| MF | GAAUGAAGGAAUGCAAC | 301 |
| MF/MS4-1 | AUGAAGGAAUGCAAC | 302 |
| MF/MS4-2 | GAAGGAAUGCAAC | 303 |
| MF/MS4-3 | GGAAUGCAAC | 304 |
| MS1/MS2 | GUUGCAGAACCCGAAUAGAGCAAUGAAGGAAUGCAACNN | 305 |
| | NNNNNNNNNNNNNNNNNNNUUUUAUUUUUU | |
| MS1/MS2/ MS4*-1 | GAACCCGAAUAGAGCAAUGAAGGAAUGCAACNNNNNNNN NNNNNNNNNNNNUUUUAUUUUUU | 306 |
| MS1/MS2/ MS4*-2 | GAAUAGAGCAAUGAAGGAAUGCAACNNNNNNNNNNNNNN NNNNNNUUUUAUUUUUU | 307 |
| MS1/MS2/ MS4*-3 | AGCAAUGAAGGAAUGCAACNNNNNNNNNNNNNNNNNNNN UUUUAUUUUUU | 308 |
| MF/MS2 | GAAUGAAGGAAUGCAACNNNNNNNNNNNNNNNNNNNNUU UUAUUUUUU | 309 |
| MF/MS2/M S4-1 | AUGAAGGAAUGCAACNNNNNNNNNNNNNNNNNNNNUUUU AUUUUUU | 310 |
| MF/MS2/M S4-2 | GAAGGAAUGCAACNNNNNNNNNNNNNNNNNNNNUUUUAU UUUUU | 311 |
| MF/MS2/M S4-3 | GGAAUGCAACNNNNNNNNNNNNNNNNNNNNUUUUAUUUU UU | 312 |

In Table 8, indication of a guide sequence (spacer) is omitted from all crRNA sequences omit unless necessary, and the sequence indicated by 'NNNNNNNNNNNNNNNNNNNN' indicates any guide sequence (spacer) that can hybridize with a target sequence in a target gene (for example, USH2A gene). The guide sequence may be appropriately designed by a person skilled in the art depending on a desired target gene and/or a target sequence in the target gene as described above, and therefore is not limited to a specific sequence of a particular length.

In another embodiment, the scaffold region of the engineered gRNA may comprise tracrRNA comprising or consisting of any one nucleotide sequence selected from the group consisting of SEQ ID NOs: 251 to 296; and crRNA comprising or consisting of any one nucleotide sequence selected from the group consisting of SEQ ID NOs: 297 to 304.

In another embodiment, the first guide RNA or the second guide RNA of the present disclosure may comprise a sequence of a scaffold region of a nucleotide sequence selected from the group consisting of SEQ ID NOs: 313 to 350. Here, the scaffold region of the nucleotide sequence means the remaining region excluding the spacer region present at the 3'-end portion of crRNA (for example, the region indicated by 5'-NNNNNNNNNNNNNNNNNN-3' in the nucleotide sequence of any one of SEQ ID NOs: 313 to 350).

In another embodiment, when the engineered gRNA of the present disclosure is in the form of a single guide RNA (sgRNA), the scaffold region of the engineered sgRNA may comprise or consist of any one nucleotide sequence selected from the group consisting of SEQ ID NOs: 313 to 350. Here, the 5'-NNNNNNNNNNNNNNNNNNNN-3', 5'-NNNNNNNNNNNNNNNNNNNUUUUAUUUU-3', or 5'-NNNNNNNNNNNNNNNNNNNUUUUAUUUUU-3' sequence present at the 3'-end of SEQ ID NOs: 313 to 350 is excluded.

For example, the engineered sgRNA may be sgRNA of SEQ ID NO: 313 comprising a modification at MS1, sgRNA of SEQ ID NO: 314 comprising modifications at MS1/MS2, sgRNA of SEQ ID NO: 315 comprising modifications at MS1/MS2/MS3, sgRNA of SEQ ID NO: 316 comprising modifications at MS2/MS3/MS4, or sgRNA of SEQ ID NO: 317 comprising modifications at MS2/MS3/MS4/MS5. Here, in the nucleotide sequences of SEQ ID NOs: 313 to 317, the sequence indicated by 5'-NNNNNNNNNNNNNNNNNN-3' refers to a guide sequence. For details regarding the guide sequence, see the entire contents described in section "2.2. Spacer region comprising guide sequence."

In another specific example, the engineered sgRNA may be sgRNA comprising or consisting of the nucleotide sequence of SEQ ID NO: 318 (MS1/MS3-1), SEQ ID NO: 319 (MS1/MS3-2), SEQ ID NO: 320 (MS1/MS3-3), SEQ ID NO: 321 (MS1/MS4*-1), SEQ ID NO: 322 (MS1/MS4*-2), SEQ ID NO: 323 (MS1/MS4*-3), SEQ ID NO: 324 (MS1/MS5-1), SEQ ID NO: 325 (MS1/MS5-2), SEQ ID NO: 326 (MS1/MS5-3), SEQ ID NO: 327 (MS1/MS2/MS4*-2), SEQ ID NO: 328 (MS1/MS3-3/MS4*-2), SEQ ID NO: 329 (MS1/MS2/MS5-3), SEQ ID NO: 330 (MS1/MS3-3/MS5-3), SEQ ID NO: 331 (MS1/MS4*-2/MS5-3), SEQ ID NO: 332 (MS1/MS2/MS3-3/MS4*-2), SEQ ID NO: 333 (MS1/MS2/MS3-3/MS5-3), SEQ ID NO: 334 (MS1/MS2/MS4*-2/MSS-3), SEQ ID NO: 335 (MS1/MS3-3/MS4*-2/MS5-3), or SEQ ID NO: 336 (MS1/MS2/MS3-3/MS4*-2/MSS-3). Here, the sequence indicated by 5'-NNNNNNNNNNNNNNNNNN-3' in the nucleotide sequences of SEQ ID NOs: 318 to 336 refers to a guide sequence. For details regarding the guide sequence, see the entire contents described in section "2.2. Spacer region comprising guide sequence."

In addition, the sgRNA may be sgRNA comprising or consisting of the nucleotide sequence of SEQ ID NO: 337, which is a mature form (abbreviated as MF) of sgRNA.

In another embodiment, there is provided an exemplary sgRNA which comprises partial modification of the nucleotide sequence of the MF sgRNA. Specifically, the MF sgRNA may be sgRNA comprising or consisting of the nucleotide sequence of SEQ ID NO: 338 (MS3-1), SEQ ID NO: 339 (MS3-2), SEQ ID NO: 340 (MS3-3), SEQ ID NO: 341 (MS4-1), SEQ ID NO: 342 (MS4-2), SEQ ID NO: 343 (MS4-3), SEQ ID NO: 344 (MS5-1), SEQ ID NO: 345 (MS5-2), SEQ ID NO: 346 (MS5-3), SEQ ID NO: 347 (MS3-3/MS4-3), SEQ ID NO: 348 (MS3-3/MS5-3), SEQ ID NO: 349 (MS4-3/MS5-3), or SEQ ID NO: 350 (MS3-3/MS4-3/MS5-3). Here, the sequence indicated by 5'-NNNNNNNNNNNNNNNNNN-3' in the nucleotide sequences of the SEQ ID NOs: 337 to 350 refers to a guide sequence. For details regarding the guide sequence, see the entire contents described in section "2.2. Spacer region comprising guide sequence."

In a preferred embodiment, the engineered sgRNA may consist of the nucleotide sequence of SEQ ID NO: 315 (Cas12f1 ver3.0), SEQ ID NO: 316 (Cas12f1 ver4.0), or SEQ ID NO: 317 (Cas12f1 ver4.1). Here, in the nucleotide sequences of SEQ ID NO: 315, SEQ ID NO: 316, and SEQ ID NO: 317, the sequence indicated by 5'-NNNNNNNNNNNNNNNNNN-3' refers to a guide sequence. For details regarding the guide sequence, see the entire contents described in section "2.2. Spacer region comprising guide sequence."

### (7) Additional sequence

The engineered tracrRNA of the present disclosure may optionally further comprise an additional sequence. The additional sequence may also be located at the 3'-end of the engineered tracrRNA. In addition, the additional sequence may be located at the 5'-end of the engineered tracrRNA. For example, the additional sequence may be located at the 5'-end of the first stem-loop region.

The additional sequence may consist of 1 to 40 nucleotides. In an embodiment, the additional sequence may be any nucleotide sequence or a randomly arranged nucleotide sequence. For example, the additional sequence may be 5'-AUAAAGGUGA-3' (SEQ ID NO: 355).

In addition, the additional sequence may be a known nucleotide sequence. As an example, the additional sequence may be a hammerhead ribozyme nucleotide sequence. Here, the hammerhead ribozyme nucleotide sequence may be 5'-CUGAUGAGUCCGUGAGGACGAAACGAGUAAGCUCGUC-3' (SEQ ID NO: 356) or 5'-CUGCUCGAAUGAGCAAAGCAGGAGUGCCUGAGUAGUC-3' (SEQ ID NO: 357). The sequences listed above are merely examples, and the additional sequence is not limited thereto.

### (8) Chemical modification

In some embodiments, the engineered tracrRNA or engineered crRNA included in the engineered gRNA may have chemical modification in at least one or more nucleotides, if necessary. Here, the chemical modification may be a modification in various covalent bonds that may occur in a nucleotide base and/or sugar portion.

In an embodiment, the chemical modification may be methylation, halogenation, acetylation, phosphorylation, phosphorothioate (PS) linkage, locked nucleic acid (LNA), 2'-O-methyl 3'phosphorothioate (MS) or 2'-O-methyl 3'thioPACE (MSP). The above example is a simple example and the modification is not limited thereto.

In a case of using the hypercompact gene editing system comprising a complex of the engineered gRNA with Cas12f1 (CWCas12f1 or Un1Cas12f1) or a variant complex thereof of the present disclosure, indel efficiency for a target gene or target nucleic acid in a cell is significantly improved compared to a case of using the guide RNA found in nature, so that a large-scale deletion effect may be exhibited.

Above all, the engineered gRNA may involve optimized length for high efficiency and resulting cost reduction in gRNA synthesis, creation of additional space or capacity in a case of being inserted into a viral vector, normal expression of tracrRNA, increased expression of operable gRNA, increased gRNA stability, increased stability of complex of gRNA with gene editing protein, induction of formation of complex of gRNA with gene editing protein at high efficiency, increased cleavage efficiency of target nucleic acid by hypercompact USH2A gene editing system comprising complex of gRNA with gene editing protein, and increased deletion efficiency for a specific region in a desired gene by such a system. Accordingly, in a case of using the above-described engineered gRNA for Cas12f1 or a variant protein thereof, it is possible to overcome the limitations of the above-mentioned prior art, thereby cleaving a gene with high efficiency in a cell and editing (for example, deleting) a specific region in a gene with high efficiency.

In addition, the engineered gRNA has a short length compared to gRNA found in nature, and thus has high applicability in the field of gene editing technology. Using the engineered gRNA, the hypercompact gene editing system comprising a complex of the gRNA with gene editing protein has advantages of being very small in size and having excellent editing efficiency, which allows the system to be utilized in various gene editing technologies.

### 2.4. Single guide RNA or dual guide RNA

The engineered guide RNA according to the embodiment of the present disclosure may be a single guide RNA or dual guide RNA. The dual guide RNA means that the guide RNA is composed of two RNA molecules: tracrRNA and crRNA. The single guide RNA (sgRNA) means that the 3'-end of tracrRNA and the 5'-end of crRNA are connected via a linker.

In an embodiment, the engineered single guide RNA (sgRNA) may further comprise a linker sequence, and the tracrRNA sequence and the crRNA sequence may be connected via the linker sequence. Preferably, this may include a case where the 3'-end of the tracrRNA-crRNA complementarity sequence in the tracrRNA and the 5'-end of the tracrRNA-crRNA complementarity sequence in the crRNA, which are contained in the engineered scaffold sequence, may be connected via a linker. More preferably, the tracrRNA-crRNA complementarity regions of the tracrRNA and the crRNA may be connected to each other, at the 3'-end of the tracrRNA and the 5'-end of the crRNA, by the linker 5'-GAAA-3'. For details regarding the linker, see the description of Lk of Formula (I).

In an embodiment, a sequence of the single guide RNA is such that the tracrRNA sequence, the linker sequence, the crRNA sequence, and the U-rich tail sequence are sequentially linked in a 5' to 3' direction. A part of the tracrRNA sequence and at least a part of the CRISPR RNA repeat sequence included in the crRNA sequence have sequences complementary to each other.

In addition, the engineered guide RNA according to the embodiment of the present disclosure may be a dual guide RNA in which tracrRNA and crRNA form separate RNA molecules. Here, a part of the tracrRNA and a part of the crRNA may have sequences complementary to each other so that a double-stranded RNA is formed. More specifically, in the dual guide RNA, a part containing the 3'-end of the tracrRNA and a part containing the CRISPR RNA repeat sequence of the crRNA may form a double strand. The engineered guide RNA may bind to Cas12f1 or a variant protein thereof to form a complex of the guide RNA with the protein. This complex recognizes a target sequence complementary to the guide sequence included in the crRNA sequence, which allows for editing of a target gene or target nucleic acid comprising the target sequence.

In an embodiment, the tracrRNA sequence may comprise a complementary sequence having 0 to 20 mismatches with the CRISPR RNA repeat sequence. Preferably, the tracrRNA sequence may comprise a complementary sequence having 0 to 8 or 8 to 12 mismatches with the CRISPR RNA repeat sequence.

### 3. Factors inhibiting non-homologous end joining activity

As disclosed herein, the USH2A gene editing system may further comprise additional components for achieving the purpose (for example, deletion of exon 13 in the USHA2A gene) in addition to the engineered guide RNA and Cas12f1 molecule (for example, Cas12f1 or a variant protein thereof) as described above. For example, the USH2A gene editing system may further comprise a factor capable of inhibiting or reducing non-homologous end joining (NHEJ) activity. The factor may be, for example, a molecule that inhibits expression of a gene involved in NHEJ, or a nucleic acid encoding the molecule. Without being bound by any particular theory, for example, inhibition or reduction of NHEJ activity may result in promotion of a homology-directed repair (HDR) mediated pathway. The factor may be used to inhibit/reduce NHEJ activity or promote/increase or decrease HDR activity.

The term "non-homologous end joining (NHEJ)" refers to a mechanism that repairs a double-strand break in a nucleotide sequence by direct ligation of the broken ends without the requirement for a homologous template (as opposed to homology-directed repair, which requires a homologous sequence to induce healing of a double-strand break in a nucleotide sequence). NHEJ often leads to loss (deletion) of a nucleotide sequence near the double-strand break site.

In an embodiment, the CRISPR/Cas12f1 system of the present disclosure for deleting a segment comprising exon 13 in the USH2A gene may comprise a molecule that suppresses expression of a gene involved in non-homologous end joining. This may achieve improved deletion efficiency for the segment comprising exon 13.

In another embodiment, the molecule that suppresses expression may be a small molecule or an inhibitory nucleic acid. The expression-suppressing molecule may be, for example, but is not limited to, an interfering nucleic acid (for example, short interfering RNA (siRNA), double-stranded RNA (dsRNA), micro-RNA (miRNA), short hairpin RNA (shRNA) specific for a gene transcript) or an antisense oligonucleotide.

In yet another embodiment, the expression-suppressing molecule may be targeted to enzymes involved in NHEJ, HDR, or upstream regulation thereof by post-translational modification, for example, via phosphorylation, ubiquitylation, and/or sumoylation.

In mammalian cells, the "canonical" or "classical" NHEJ pathway (C-NHEJ) requires several factors, including DNA-PK, Ku70-80, Artemis, ligase IV (Lig4), XRCC4, CLF, and Pol µ, to repair double-strand breaks (see Kasparek & Humphrey Seminars in Cell & Dev. Biol. 22:886-897, 2011).

In an embodiment, to inhibit the C-NHEJ pathway in a cell, the USH2A gene editing system of the present disclosure may be modified to reduce or eliminate expression or activity of a factor involved in the NHEJ pathway. For example, the USH2A gene editing system may further comprise a factor capable of reducing or eliminating expression or activity of one or more selected from the group consisting of MRE11, RAD50, NBS1, DNA-PK, CtIP, Ku70, Ku80, Artemis (DCLRE1C), Ligase IV (Lig4), PNKP, XRCC4, XLF (XRCC4-like factor), ATM (ATM Serine/Threonine Kinase), CHK1/CHK2, CURLY LEAF (CLF), and Pol Mu (POLM).

In mammals, in addition to C-NHEJ, an alternative NHEJ (A-NHEJ) pathway exists, which is known to require different factors.

In another embodiment, to inhibit the A-NHEJ pathway in a cell, the USH2A gene editing system of the present disclosure may be modified to reduce or eliminate expression or activity of a factor involved in the NHEJ pathway. For example, the USH2A gene editing system may further comprise a factor capable of reducing or eliminating the expression or activity of one or more selected from the group consisting of XRCC1, PARP (for example, PARP1), Lig1, and Lig3.

In an embodiment, the gene involved in non-homologous end joining may be one or more selected from the group consisting of ATM1, XRCC4, XLF, XRCC6, LIG4, and DCLRE1C.

In another embodiment, the gene involved in non-homologous terminal joining may be one or more selected from the group consisting of XRCC6 and DCLRE1C.

In an embodiment, the inhibitory molecule may be shRNA, siRNA, miRNA, or antisense oligonucleotide. In another embodiment, the inhibitory molecule may be shRNA.

In yet another embodiment, the shRNA molecule may be a molecule that inhibits expression of one or more genes selected from the group consisting of XRCC6 and DCLRE1C. Specifically, the shRNA molecule may be one or more selected from the group consisting of shXRCC6 and shDCLRE1C.

### 4. Nucleic acid encoding component(s) of the USH2A gene editing system

Since each component of the CRISPR/Cas12f1 system or USH2A gene editing system provided in the present disclosure is intended to be expressed within a cell, according to another aspect of the present disclosure, there is provided a nucleic acid or polynucleotide encoding each component of the gene editing system. Here, the nucleic acid or polynucleotide may be a synthetic nucleotide sequence.

Specifically, for the nucleic acid or polynucleotide, there is provided a nucleotide sequence encoding the nucleic acid editing protein (or endonuclease), the guide RNA (for example, the engineered guide RNA comprising a spacer region comprising a guide sequence capable of hybridizing with a target sequence in a USH2A gene and a scaffold region), and/or the molecule that inhibits expression of a gene involved in non-homologous end joining, included in the gene editing system to be expressed. In an embodiment, the nucleic acid may be DNA or RNA (for example, mRNA). The nucleic acid or polynucleotide encoding each component of the gene editing system are disclosed herein as representative examples, or the nucleotide sequence thereof may be readily determined by a person skilled in the art by referring to the specific sequence of each component.

In an embodiment, the nucleic acid or polynucleotide may comprise a human codon-optimized nucleotide sequence encoding the Cas12f1 molecule (for example, Cas12f1 or a variant protein thereof). The term "codon optimization" refers to a process of modifying a native nucleic acid sequence for enhanced expression in a cell of interest by replacing at least one codon in the native sequence with a codon, which is used more frequently or most frequently in a gene of the target cell, while maintaining its native amino acid sequence. Different species have specific biases for specific codons for specific amino acids, and codon bias (differences in codon usage between organisms) is often correlated with translation efficiency of an mRNA, which is considered to be dependent on the nature of codons being translated and availability of specific tRNA molecules. Predominance of tRNA selected in a cell generally reflects the most frequently used codon in peptide synthesis. Thus, genes may be tailored for optimal gene expression in a given organism based on codon optimization.

For example, the nucleic acid encoding the human codon optimized CWCas12f1 protein or a variant thereof may comprise or consist of a sequence selected from SEQ ID NOs: 365 to 368. In addition, the nucleic acid encoding the human codon optimized Un1Cas12f1 protein may comprise or consist of the sequence of SEQ ID NO: 364.

In another embodiment, the nucleic acid or polynucleotide may be DNA or RNA that exists in nature, or may be a modified nucleic acid in which a chemical modification has occurred in at least a part of the nucleic acid or polynucleotide. For example, the nucleic acid or polynucleotide may be one in which one or more nucleotides have been chemically modified. Here, the chemical modification may include any modification of nucleic acids known to those skilled in the art.

### IV. Vector system for expression of USA2A gene editing system

As disclosed herein, there is provided a vector system for editing or altering a USH2A gene (for example, a human USH2A gene). Since the disclosed vector system allows each component of the above-described USH2A gene editing system (or CRISPR/Cas12f1 system) to be expressed in a cell, a nucleic acid construct (for example, a nucleotide sequence) included in the vector system comprises at least one nucleotide sequence encoding each component of the USH2A gene editing system. In addition, since the disclosed vector system allows each component of the USH2A gene editing system to be expressed in a cell, all effects and advantages that are achieved or can be achieved by the USH2A gene editing system are applied as is.

In the disclosed vector system, each nucleic acid construct is capable of expressing each component of the USH2A gene editing system in a cell. The vector system enables editing of the USH2A gene (for example, deletion of a segment comprising exon 13) in a cell.

In the vector system disclosed herein, for the nucleotide sequence of each nucleic acid construct and the components expressed thereby, see the entire contents described in the section "III. CRISPR/Cas system for USH2A gene editing."

In order to use the USH2A gene editing system disclosed herein for editing a USH2A gene (for example, deletion of a segment comprising exon 13), a method may be used in which one or more vectors comprising nucleotide sequences encoding respective components of the USH2A gene editing system is introduced directly or through an appropriate delivery means such as a virus into a target cell and the respective components of the gene editing system are allowed to be expressed in the target cell. Preferably, for editing a USH2A gene (for example, deletion of a segment comprising exon 13), the nucleotide sequences encoding respective components of the gene editing system described above may be operably linked and included in a single vector.

In an embodiment, the nucleotide sequences encoding one or more components of the USH2A gene editing system may be present in two or more vectors, wherein the two or more vectors may be the same or different vectors.

In another embodiment, the nucleotide sequences encoding one or more components of the USH2A gene editing system may be present in a single vector.

In addition, the vector system of the present disclosure may comprise, in addition to the components of the USH2A gene editing system, a nucleotide sequence encoding an additional expression element that is desired to be expressed as needed by a person skilled in the art. For example, the additional expression element may be a tag. Specifically, the additional expression element may be a herbicide resistance gene such as glyphosate, glufosinate ammonium, or phosphinothricin, an antibiotic resistance gene such as ampicillin, kanamycin, G418, bleomycin, hygromycin, or chloramphenicol.

In another embodiment, the vector system may comprise one or more regulatory and/or control components so that it is directly expressed in a cell. Specifically, the regulatory and/or control components may include, but are not limited to, a promoter, an enhancer, an intron, a polyadenylation signal, a Kozak consensus sequence, an internal ribosome entry site (IRES), a splice acceptor, a 2A sequence, and/or a replication origin. The replication origin may be, but is not limited to, an f1 origin of replication, an SV40 origin of replication, a pMB 1 origin of replication, an adeno origin of replication, an AAV origin of replication, and/or a BBV origin of replication.

In another embodiment, in order to express, in a cell, the nucleotide sequences encoding the gene editing system of the present disclosure included in the vector system, a promoter sequence is operably linked to the sequence encoding each component so that an RNA transcription factor can be activated in the cell. The promoter sequence may be designed differently depending on the corresponding RNA transcription factor or expression environment, and is not limited as long as it can properly express the components of the gene editing system of the present disclosure in a cell.

For example, the promoter sequence may be a promoter that promotes transcription of RNA polymerase RNA Pol I, Pol II, or Pol III. Specifically, the promoter may be one of U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

In another embodiment, when a sequence of the vector comprises the promoter sequence, transcription of a sequence operably linked to the promoter is induced by an RNA transcription factor. The vector may comprise a termination signal that induces termination of transcription of the RNA transcription factor. The termination signal may vary depending on the type of the promoter sequence. Specifically, when the promoter is a U6 or H1 promoter, the promoter recognizes a TTTTT (T5) or TTTTTT (T6) sequence, which is a thymidine (T) repeat sequence, as a termination signal.

The sequence of the engineered guide RNA provided in the present disclosure may comprise a U-rich tail sequence at its 3'-end. Accordingly, the sequence encoding the engineered guide RNA comprises a T-rich sequence corresponding to the U-rich tail sequence at its 3'-end. As described above, some promoter sequences recognize a thymidine (T) repeat sequence, for example, a sequence consisting of five or more consecutive thymidine (T) residues as a termination signal, and therefore, in some cases, the T-rich sequence may be recognized as a termination signal. In other words, when the vector sequence provided in the present specification comprises a sequence encoding the engineered guide RNA, a sequence encoding the U-rich tail sequence included in the engineered gRNA sequence may be used as a termination signal.

In an embodiment, when the vector sequence comprises a U6 or H1 promoter sequence and a sequence encoding the engineered guide RNA operably linked thereto, a sequence portion that encodes the U-rich tail sequence included in the guide RNA sequence may be recognized as a termination signal. Specifically, the U-rich tail sequence may comprise a sequence consisting of five or more consecutive uridine (U) residues.

In an embodiment, the vector may be a viral vector. Specifically, the viral vector may be one or more selected from the group consisting of a retrovirus vector, a lentivirus vector, an adenovirus vector, an adeno-associated virus vector, a vaccinia virus vector, a poxvirus vector, a herpes simplex virus vector, and a phagemid vector. Preferably, the viral vector may be an adeno-associated virus vector. In addition, the viral vector includes, but is not limited to, a SIN lentivirus vector, a retrovirus vector, a foamy virus vector, an adenovirus vector, an adeno-associated virus (AAV) vector, a hybrid vector and/or a plasmid transposon (for example, the Sleeping Beauty transposon system), or an integrase-based vector system.

In another embodiment, the vector may be a non-viral vector. Specifically, the non-viral vector may be one or more selected from the group consisting of, but not limited to, plasmid, naked DNA, DNA complex, mRNA (transcript), and amplicon. For example, the plasmid may be selected from the group consisting of pcDNA series, pSC101, pGV1106, pACYC177, ColE1, pKT230, pME290, pBR322, pUC8/9, pUC6, pBD9, pHC79, pIJ61, pLAFR1, pHV14, pGEX series, pET series, and pUC19.

The term "naked DNA" refers to DNA (for example, histone-free DNA) that encodes a protein, such as Cas12f1 or a variant thereof of the present disclosure, cloned into a suitable expression vector (for example, plasmid) in an appropriate orientation for expression.

The term "amplicon", when used with respect to a nucleic acid, means a product of copying the nucleic acid, wherein the product has a nucleotide sequence that is identical with or complementary to at least a portion of the nucleotide sequence of the nucleic acid. For example, an amplicon may be produced by any of a variety of amplification methods that use a nucleic acid or an amplicon thereof, as a template including polymerase extension, polymerase chain reaction (PCR), rolling circle amplification (RCA), multi-displacement amplification (MDA), ligation extension, or ligation chain reaction. The amplicon may be a nucleic acid molecule having a single copy of a particular nucleotide sequence (for example, a PCR product) or multiple copies of the nucleotide sequence (for example, a concatemeric product of RCA).

The vector disclosed herein may be designed in the form of a linear or circular vector. In a case where the vector is a linear vector, RNA transcription is terminated at the 3'-end even if a sequence of the linear vector does not separately comprise a termination signal. However, in a case where the vector is a circular vector, RNA transcription is not terminated unless a sequence of the circular vector separately comprises a termination signal. Therefore, when using a circular vector, a termination signal corresponding to a transcription factor related to each promoter sequence has to be included in order for the vector to express an intended target.

In an embodiment, the viral vector or non-viral vector may be delivered by a delivery system such as liposomes, polymeric nanoparticles (for example, lipid nanoparticles), oil-in-water nanoemulsions, or combinations thereof, or in the form of a virus.

### V. Virus expressing USA2A gene editing system

There is provided a recombinant virus or recombinant viral particle produced by the vector system disclosed herein.

In an embodiment, the viral vector may be, for example, one or more viral vectors selected from the group consisting of a retrovirus vector, a lentivirus vector, an adenovirus vector, an adeno-associated virus vector, a vaccinia virus vector, a poxvirus vector, a herpes simplex virus vector, and a phagemid vector. Preferably, the viral vector may be an adeno-associated virus vector.

In another embodiment, the virus may be selected from the group consisting of retrovirus, lentivirus, adenovirus, adeno-associated virus, vaccinia virus, poxvirus, herpes simplex virus, and phage.

In yet another embodiment, the phage may be selected from the group consisting of λgt4λB, λ-Charon, λΔz1, and M13.

In order to efficiently deliver the USH2A gene editing system of the present disclosure into a target cell or target site via a virus, in particular, an adeno-associated virus (AAV), it is important to design a size of the nucleotide sequence encoding all components of the editing system to be within 4.7 kb that is a packaging limit of AAV. This has an advantage in that in a case where the CRISPR/Cas12f1 system of the present disclosure is used, a very small size of the hypercompact nucleic acid editing protein and two engineered gRNAs included in the system allows sufficient packaging by an AAV delivery vehicle even if additional regulatory molecules (for example, molecules that suppress genes involved in mechanism of non-homologous end joining) are further included.

### VI. Composition for USH2A gene editing

As disclosed herein, there is provided a composition comprising each component of the gene editing system, one or two or more vectors of the vector system, or the viru. The disclosed composition may be a pharmaceutical composition. In addition, the pharmaceutical composition may be used for the prevention or treatment of Usher syndrome.

In an embodiment, the pharmaceutical composition may be for editing the USH2A gene (for example, deleting a segment comprising exon 13 in the USH2A gene). In addition, the pharmaceutical composition may be for treating Usher syndrome or delaying onset or progression thereof.

In an embodiment, the pharmaceutical composition may be formulated according to the mode of administration to be used. For example, in a case where the pharmaceutical composition is an injectable pharmaceutical composition, it may be desirable to use an isotonic agent. An additive for isotonicity may generally include sodium chloride, dextrose, mannitol, sorbitol, and lactose. In an embodiment, isotonic solutions such as phosphate buffered saline are preferred. A stabilizer may include gelatin and albumin. In an embodiment, a vasoconstrictor is added to the formulation.

In another embodiment, the composition may further comprise a pharmaceutically acceptable excipient. The pharmaceutically acceptable excipient may be a functional molecule that acts as a vehicle, an adjuvant, a carrier, or a diluent. The pharmaceutically acceptable excipient may be a gene transfer enhancer (which may include a surfactant) such as an immune stimulating complex (ISCOMS), Freund's incomplete adjuvant, a LPS analogue (including monophosphoryl lipid A), a muramyl peptide, a quinone analogue, a vesicle such as squalene and squalane, hyaluronic acid, a lipid, a liposome, a calcium ion, a viral protein, a polyanion, a polycation, or a nanoparticle, or other known gene transfer facilitating agent.;

In another embodiment, the composition may comprise a gene transfer enhancer. The gene transfer enhancer may be a polyanion, a polycation (including poly-L-glutamic acid (LGS)), or a lipid. The gene transfer enhancer is poly-L-glutamic acid, and more preferably, the poly-L-glutamic acid may be present in the composition for genome editing of skeletal muscle or cardiac muscle at a concentration of less than 6 mg/ml. The gene transfer enhancer may also include a surfactant, such as an immune stimulating complex (ISCOMS), Freund's incomplete adjuvant, a LPS analogue (including monophosphoryl lipid A), a muramyl peptide, a quinone analogue and a vesicle, such as squalene and squalane; and hyaluronic acid may also be used.

In an embodiment, the composition comprising one or more vectors included in the above-described vector system may comprise a gene transfer enhancer, such as a lipid, a liposome (including lecithin liposomes, or other liposomes known in the art), a DNA-liposome mixture, a calcium ion, a viral protein, a polyanion, a polycation, or a nanoparticle, or other known gene transfer enhancer. Preferably, the gene transfer enhancer is a polyanion, a polycation (for example, poly-L-glutamic acid (LGS)), or a lipid.

An actual dosage of the (pharmaceutical) composition may vary greatly depending on various factors, such as the choice of vector, the target cell, organism, or tissue, the condition of the subject to be treated, the degree of transformation/modification sought, the route of administration, the method of administration, the form of transformation/modification sought, and the like. The administration may be performed by a route of administration selected from subretinal administration, subcutaneous administration, intradermal administration, intraocular administration, intravitreal administration, intratumoral administration, intranodal administration, intramedullary administration, intramuscular administration, intravenous administration, intralymphatic administration, and intraperitoneal administration. The pharmaceutical composition may further comprise a carrier (water, saline, ethanol, glycerol, lactose, sucrose, calcium phosphate, gelatin, dextran, agar, pectin, peanut oil, sesame oil, and the like), a diluent, a pharmaceutically acceptable carrier (for example, phosphate buffered saline), a pharmaceutically acceptable excipient, and/or other compounds known in the art.

For example, delivery for the treatment of a disease may be via AAV. A therapeutically effective dosage for in vivo delivery of AAV to a human may be a saline solution in a range of about 20 ml to about 50 ml containing about 1×10¹⁰ to about 1×10¹⁰⁰ AAV per ml of solution. The dosage may be adjusted to balance the therapeutic benefit against any adverse effects.

### VII. Method for editing USH2A gene

As disclosed herein, there is provided a method for editing a USH2A gene using the USH2A gene editing system, vector system, composition, or virus of the present disclosure. Specifically, editing of the USH2A gene may involve inducing deletion of a segment comprising exon 13 in the USH2A gene.

In an embodiment, a length of the segment comprising exon 13 may be 640 bp to 19 kb. For example, a length of the segment may be 640 bp to 18 kb, 640 bp to 17 kb, 640 bp to 16 kb, 640 bp to 15 kb, 640 bp to 14 kb, 640 bp to 13 kb, 640 bp to 12 kb, 640 bp to 11 kb, 640 bp to 10 kb, 640 bp to 9 kb, 640 bp to 8 kb, 640 bp to 7 kb, 640 bp to 6 kb, 640 bp to 5.5 kb, 640 bp to 5 kb, 640 bp to 4.5 kb, 640 bp to 4 kb, 640 bp to 3.5 kb, 640 bp to 3 kb, 640 bp to 2.5 kb, 640 bp to 2 kb, 640 bp to 1.5 kb, 640 bp to 1 kb; 700 bp to 18 kb, 1 kb to 17 kb, 1.3 kb to 16 kb, 1.7 kb to 15 kb, 2 kb to 14 kb, 2.3 kb to 13 kb, 2.7 kb to 12 kb, 3 kb to 11 kb, 3.3 kb to 10 kb, 3.7 kb to 9 kb, 4 kb to 8 kb, 4.3 kb to 7 kb, 4.7 kb to 6 kb, 5 kb to 5.5 kb; 640 bp to 5 kb, 700 bp to 5 kb, 1 kb to 5 kb, 1.5 kb to 5 kb, 2 kb to 5 kb, 3 kb to 5 kb, or 4 kb to 5 kb, but is not limited thereto. It is clear that a length of the segment comprising exon 13 may be appropriately determined or understood by a person skilled in the relevant art.

The disclosed method comprises bring, into contact with a cell, the USH2A gene editing system, vector system, composition or (recombinant) virus of the present disclosure. Here, the cell may be a cell derived from a subject having Usher syndrome. Furthermore, the cell may be a stem cell or a mammalian eye or inner ear cell. However, the method of the present disclosure is not limited to the above-mentioned cells.

In an embodiment, the stem cells may be induced pluripotent stem cells (iPSCs) or dedifferentiated stem cells. The induced pluripotent stem cells refer to genetically initialized adult cells that exhibit a similar state (for example, similar differentiation potential) to pluripotent stem cells similar to embryonic stem cells (ESCs). The induced pluripotent stem cells may be, for example, stem cells produced by artificially dedifferentiating cells derived from a subject having Usher syndrome. The production of such dedifferentiated stem cells is well known in the art (see, for example, Ying Wang et al., Scalable Production of Human Erythrocytes from Induced Pluripotent Stem Cells, 2016, https://doi.org/10.1101/050021).

Therefore, according to another aspect of the present disclosure, there is provided a stem cell genetically modified by the method disclosed herein. Specifically, the genetic modification may be deletion of exon 13 in the USH2A gene in the stem cell.

In an embodiment, the genetically modified stem cell may be for treating type 2 (for example, type 2A) Usher syndrome.

In addition, the disclosed method comprises bringing, into contact with a subject, the USH2A gene editing system, vector system, composition or (recombinant) virus, wherein the subject may be a subject having a disease associated with an exon 13 mutation of the USH2A gene.

By the above-described methods, it is possible to induce deletion of a segment comprising exon 13 in the USH2A gene in a cell, and/or to treat a subject having a disease associated with a mutation in exon 13 of the USH2A gene, and/or to alter the USH2A gene in a cell.

In an embodiment, the bringing-into-contact with a cell may comprise delivering or introducing, into the cell, the USH2A gene editing system, vector system, composition, or virus of the present disclosure.

The nucleic acid or nucleic acid construct (for example, a vector) of the present disclosure may be delivered or introduced, for example, by *in vivo* electroporation, liposomes, nanoparticles, or DNA injection or DNA vaccination, with or without a recombinant vector.

The vector system of the present disclosure may be delivered or introduced by a virus, such as a retrovirus, a lentivirus, an adenovirus, an adeno-associated virus, a vaccinia virus, a poxvirus, a herpes simplex virus or a phage. Specifically, the system may be contained in a packaging virus and delivered into a cell in the form of a virus produced by the packaging virus.

Specifically, the bringing-into-contact, delivery, or introduction may be made by a method of electroporation, gene gun, sonoporation, magnetofection, nanoparticles, and/or transient cell compression or squeezing method. When the cell is a eukaryotic cell, cationic liposome method, lithium acetate-DMSO, lipid-mediated transfection, calcium phosphate precipitation, lipofection, polyethyleneimine (PEI)-mediated transfection, DEAE-dextran-mediated transfection, and/or nanoparticle-mediated nucleic acid delivery (see Panyam et al., Adv Drug Deliv Rev. 2012 Sep 13. pii: S0169-409X(12)00283-9) may be used.

In another embodiment, the bringing-into-contact, delivery, or introduction may be performed *in vitro, in vivo,* or *ex vivo.*

In an embodiment, the cell may be a plant cell, a non-human animal cell, or a human cell. In addition, the cell may be a eukaryotic cell or a prokaryotic cell. In addition, the cell may be a cell of a patient with Usher syndrome. Furthermore, the cell can be a cell of a patient with type 2 (more specifically, type 2A) Usher syndrome.

Furthermore, as described herein, there is provided a method for treating Usher syndrome (for example, Usher syndrome type 2) comprising administering to a subject the USH2A gene editing system, vector system, composition, or virus of the present disclosure

In an embodiment, the subject may be a subject having Usher syndrome (for example, Usher syndrome type 2), such as a mammal including a human.

In another embodiment, the USH2A gene editing system, vector system, composition, or virus of the present disclosure may be administered directly to the eye or inner ear of a subject.

### [Embodiments]

### [Embodiment 1]

An editing system for a USH2A gene, comprising:
an endonuclease comprising a Cas12f1 molecule or a nucleic acid encoding the endonuclease;
a first guide RNA comprising a first guide sequence capable of hybridizing to a target sequence of contiguous 15 to 30 bp in length, wherein the target sequence is located in a region 5000 bp upstream of USH2A exon 13 and is adjacent to a protospacer-adjacent motif (PAM) sequence recognized by the Cas12f1 molecule, or a nucleic acid encoding the first guide RNA; and
a second guide RNA comprising a second guide sequence capable of hybridizing to a target sequence of contiguous 15 to 30 bp in length, wherein the target sequence is located in a region 14500 bp downstream of USH2Aexon 13 and is adjacent to a PAM sequence recognized by the Cas12f1 molecule, or a nucleic acid encoding the second guide RNA.

### [Embodiment 2]

The system of the above-described embodiment, wherein
the system induces deletion of exon 13 in the USH2A gene in a cell.

### [Embodiment 3]

The system of any one of the above-described embodiments, wherein
the system is for treatment of type 2A Usher syndrome.

### [Embodiment 4]

The system of any one of the above-described embodiments, wherein
the USH2A exon 13 comprises at least one mutation that causes Usher syndrome.

### [Embodiment 5]

The system of any one of the above-described embodiments, wherein
the target sequence located in a region 5000 bp upstream of USH2A exon 13 comprises a nucleotide sequence selected from the group consisting of SEQ ID NOs: 1 to 49, and/or
the target sequence located in a region 14500 bp downstream of the USH2A exon 13 comprises a nucleotide sequence selected from the group consisting of SEQ ID NOs: 50 to 79.

### [Embodiment 6]

The system of any one of the above-described embodiments, wherein
the first guide sequence comprises a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or
the second guide sequence comprises a sequence of contiguous 15 to 20 nucleotides in a nucleotide sequence selected from the group consisting of SEQ ID NOs: 446 to 475, or a sequence that is different by 5 or fewer nucleotides from the contiguous nucleotide sequence, wherein thymine (T) is substituted with uracil (U) in the contiguous nucleotide sequence.

### [Embodiment 7]

The system of any one of the above-described embodiments, wherein
the first guide sequence comprises a nucleotide sequence selected from the group consisting of SEQ ID NOs: 80 to 128 and SEQ ID NOs: 159 to 164, and/or
the second guide sequence comprises a nucleotide sequence selected from the group consisting of SEQ ID NOs: 129 to 158 and SEQ ID NOs: 165 to 174.

### [Embodiment 8]

The system of any one of the above-described embodiments, wherein
the first or second guide RNA comprises a U-rich tail sequence linked to the 3' end of the guide sequence, in which the U-rich tail is represented by 5'-(UₘV)ₙUₒ-3', wherein V is each independently A, C, or G, m and o are integers between 1 to 20, and n is an integer between 0 to 5.

### [Embodiment 9]

The system of any one of the above-described embodiments, wherein
the first or second guide RNA comprises an engineered scaffold region, and the engineered scaffold region comprises a nucleotide sequence having at least 50% sequence identity to a scaffold region of a wild-type Cas12f1 guide RNA sequence, in which the scaffold region sequentially comprises, from the 5'-end, a first stem-loop region, a second stem-loop region, a third stem-loop region, a fourth stem-loop region, and a tracrRNA-crRNA complementarity region, and comprises at least one modification selected from the group consisting of the following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

### [Embodiment 10]

The system of any one of the above-described embodiments, wherein
the wild-type Cas12f1 guide RNA comprises tracrRNA comprising the nucleotide sequence of SEQ ID NO: 175 and crRNA comprising the nucleotide sequence of SEQ ID NO: 176.

### [Embodiment 11]

The system of any one of the above-described embodiments, wherein
the engineered scaffold region comprises a sequence having at least 80% sequence identity to a sequence represented by Formula (I): 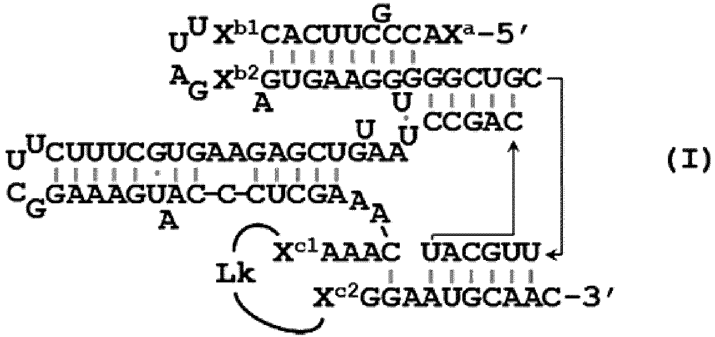 in Formula (I),
X^{a} comprises the nucleotide sequence of SEQ ID NO: 178 or a nucleotide sequence having the sequence of SEQ ID NO: 178 from which 1 to 20 nucleotides are deleted,
X^{b1} comprises the nucleotide sequence of SEQ ID NO: 189 or a nucleotide sequence having the sequence of SEQ ID NO: 189 from which 1 to 13 nucleotides are deleted,
X^{b2} comprises the nucleotide sequence of SEQ ID NO: 193 or a nucleotide sequence having the sequence of SEQ ID NO: 193 from which 1 to 14 nucleotides are deleted,
X^{c1} comprises the nucleotide sequence of SEQ ID NO: 203 or a nucleotide sequence having the sequence of SEQ ID NO: 203 from which 1 to 28 nucleotides are deleted,
X^{c2} comprises the nucleotide sequence of SEQ ID NO: 222 or a nucleotide sequence having the sequence of SEQ ID NO: 222 from which 1 to 27 nucleotides are deleted, and
Lk is a polynucleotide linker of 2 to 20 nucleotides in length or absent.

### [Embodiment 12]

The system of any one of the above-described embodiments, wherein
in a case where three or more consecutive uracil (U) residues are present in the X^{c1} sequence, the X^{c1} sequence comprises a modification in which at least one U residue thereof is replaced with A, G or C.

### [Embodiment 13]

The system of any one of the above-described embodiments, wherein
the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

### [Embodiment 14]

The system of any one of the above-described embodiments, wherein
the sequence 5'-X^{b1}UUAGX^{b2}-3' in Formula (I) is selected from the group consisting of SEQ ID NOs: 198 to 202 and 5'-UUAG-3'.

### [Embodiment 15]

The system of any one of the above-described embodiments, wherein
the sequence 5'-X^{c1}-Lk-X^{c2}-3' in Formula (I) is selected from the group consisting of SEQ ID NOs: 244 to 250 and 5'-Lk-3'.

### [Embodiment 16]

The system of any one of the above-described embodiments, wherein
the scaffold region comprises an engineered tracrRNA consisting of a nucleotide sequence selected from the group consisting of SEQ ID NOs: 251 to 296 and/or
an engineered crRNA consisting of a nucleotide sequence selected from the group consisting of SEQ ID NOs: 297 to 304.

### [Embodiment 17]

The system of any one of the above-described embodiments, wherein
the first or second guide RNA is a dual guide RNA or a single guide RNA.

### [Embodiment 18]

The system of any one of the above-described embodiments, wherein
the first or second guide RNA comprises a scaffold region sequence of a nucleotide sequence selected from the group consisting of SEQ ID NOs: 313 to 350.

### [Embodiment 19]

The system of any one of the above-described embodiments, wherein
the Cas12f1 molecule comprises an amino acid sequence having at least 70% sequence identity with an amino acid sequence selected from the group consisting of SEQ ID NOs: 360 to 364 and SEQ ID NOs: 370 to 377.

### [Embodiment 20]

The system of any one of the above-described embodiments, wherein
the endonuclease forms a ribonucleoprotein (RNP) with the first guide RNA or the second guide RNA.

### [Embodiment 21]

A vector system, comprising at least one vector that comprises:
a first nucleic acid construct to which a nucleotide sequence encoding an endonuclease is operably linked, the endonuclease comprising a Cas12f1 molecule;
a second nucleic acid construct to which a nucleotide sequence encoding a first guide RNA is operably linked, the first guide RNA comprising a first guide sequence capable of hybridizing to a target sequence of contiguous 15 to 30 bp in length, wherein the target sequence is located in a region 5000 bp upstream of USH2A exon 13 and is adjacent to a protospacer-adjacent motif (PAM) sequence recognized by the Cas12f1 molecule; and
a third nucleic acid construct to which a nucleotide sequence encoding a second guide RNA is operably linked, the second guide RNA comprising a second guide sequence capable of hybridizing to a target sequence of contiguous 15 to 30 bp in length, wherein the target sequence is located in a region 14500 bp downstream of USH2A exon 13 and is adjacent to a PAM sequence recognized by the Cas12f1 molecule.

### [Embodiment 22]

The vector system of any one of the above-described embodiments, wherein
the vector system induces deletion of exon 13 in a USH2A gene in a cell.

### [Embodiment 23]

The vector system of any one of the above-described embodiments, wherein
the USH2A exon 13 comprises at least one mutation that causes Usher syndrome.

### [Embodiment 24]

The vector system of any one of the above-described embodiments, wherein
the nucleic acid constructs are contained in the same or different vectors.

### [Embodiment 25]

The vector system of any one of the above-described embodiments, wherein
the nucleic acid structures are contained in one vector.

### [Embodiment 26]

The vector system of any one of the above-described embodiments, wherein
the target sequence located in a region 5000 bp upstream of USH2A exon 13 comprises a nucleotide sequence selected from the group consisting of SEQ ID NOs: 1 to 49, and/or
the target sequence located in a region 14500 bp downstream of USH2A exon 13 comprises a nucleotide sequence selected from the group consisting of SEQ ID NOs: 50 to 79.

### [Embodiment 27]

The vector system of any one of the above-described embodiments, wherein
the first guide sequence comprises a sequence of contiguous 15 to 22 nucleotides from a nucleotide sequence selected from the group consisting of SEQ ID NOs: 397 to 445, or a sequence that is different by 5 or fewer nucleotides in the contiguous nucleotide sequence, wherein thymine (T) in the contiguous nucleotide sequence is substituted with uracil (U), and/or
the second guide sequence comprises a sequence of contiguous 15 to 20 nucleotides in a nucleotide sequence selected from the group consisting of SEQ ID NOs: 446 to 475, or a sequence that is different by 5 or fewer nucleotides in the contiguous nucleotide sequence, wherein thymine (T) is substituted with uracil (U) in the contiguous nucleotide sequence.

### [Embodiment 28]

The vector system of any one of the above-described embodiments, wherein
the first guide sequence comprises a nucleotide sequence selected from the group consisting of SEQ ID NOs: 80 to 128 and SEQ ID NOs: 159 to 164, and/or
the second guide sequence comprises a nucleotide sequence selected from the group consisting of SEQ ID NOs: 129 to 158 and SEQ ID NOs: 165 to 174.

### [Embodiment 29]

The vector system of any one of the above-described embodiments, wherein
the first or second guide RNA comprises a U-rich tail sequence linked to the 3' end of the guide sequence, in which the U-rich tail is represented by 5'-(UₘV)ₙUₒ-3', wherein V is each independently A, C, or G, m and o are integers between 1 to 20, and n is an integer between 0 to 5.

### [Embodiment 30]

The vector system of any one of the above-described embodiments, wherein
the first or second guide RNA comprises an engineered scaffold region, wherein the engineered scaffold region comprises a nucleotide sequence having at least 50% sequence identity to a scaffold region of a wild-type Cas12f1 guide RNA sequence, which sequentially comprises a first stem-loop region, a second stem-loop region, a third stem-loop region, a fourth stem-loop region and a tracrRNA-crRNA complementarity region from the 5'-end, and wherein the system comprises at least one modification selected from the group consisting of the following (1) to (4):
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

### [Embodiment 31]

The vector system of any one of the above-described embodiments, wherein
the wild-type Cas12f1 guide RNA comprises tracrRNA comprising the nucleotide sequence of SEQ ID NO: 175 and crRNA comprising the nucleotide sequence of SEQ ID NO: 176.

### [Embodiment 32]

The vector system of any one of the above-described embodiments, wherein
the engineered scaffold region comprises a sequence having at least 80% sequence identity to a sequence represented by Formula (I): 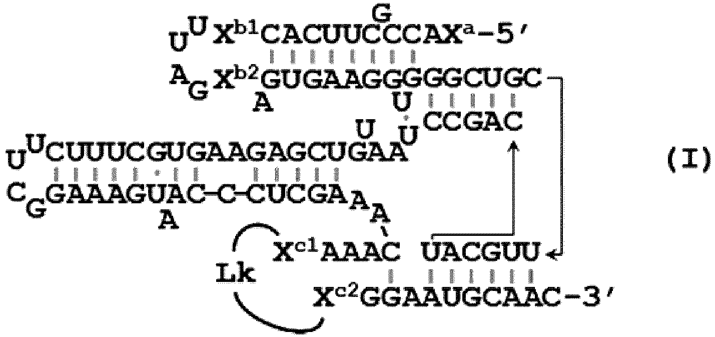 in Formula (I),
X^{a} comprises the nucleotide sequence of SEQ ID NO: 178 or a nucleotide sequence having the sequence of SEQ ID NO: 178 from which 1 to 20 nucleotides are deleted,
X^{b1} comprises the nucleotide sequence of SEQ ID NO: 189 or a nucleotide sequence having the sequence of SEQ ID NO: 189 from which 1 to 13 nucleotides are deleted,
X^{b2} comprises the nucleotide sequence of SEQ ID NO: 193 or a nucleotide sequence having the sequence of SEQ ID NO: 193 from which 1 to 14 nucleotides are deleted,
X^{c1} comprises the nucleotide sequence of SEQ ID NO: 203 or a nucleotide sequence having the sequence of SEQ ID NO: 203 from which 1 to 28 nucleotides are deleted,
X^{c2} comprises the nucleotide sequence of SEQ ID NO: 222 or a nucleotide sequence having the sequence of SEQ ID NO: 222 from which 1 to 27 nucleotides are deleted, and
Lk is a polynucleotide linker of 2 to 20 nucleotides in length or absent.

### [Embodiment 33]

The vector system of any one of the above-described embodiments, wherein
in a case where three or more consecutive uracil (U) residues are present in the X^{c1} sequence, the system comprises a modification in which at least one U residue thereof is replaced with A, G or C.

### [Embodiment 34]

The vector system of any one of the above-described embodiments, wherein
the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

### [Embodiment 35]

The vector system of any one of the above-described embodiments, wherein
the sequence 5'-X^{b1}UUAGX^{b2}-3' in Formula (I) is selected from the group consisting of SEQ ID NOs: 198 to 202 and 5'-UUAG-3'.

### [Embodiment 36]

The vector system of any one of the above-described embodiments, wherein
the sequence 5'-X^{c1}-Lk-X^{c2}-3' in Formula (I) is selected from the group consisting of SEQ ID NOs: 244 to 250 and 5'-Lk-3'.

### [Embodiment 37]

The vector system of any one of the above-described embodiments, wherein
the Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 240), 5'-UUCAGAAAUGAA-3' (SEQ ID NO: 241), 5'-UUCAUGAAAAUGAA-3' (SEQ ID NO: 242), and 5'-UUCAUUGAAAAAUGAA-3' (SEQ ID NO: 243).

### [Embodiment 38]

The vector system of any one of the above-described embodiments, wherein
the scaffold region comprises an engineered tracrRNA consisting of a nucleotide sequence selected from the group consisting of SEQ ID NOs: 251 to 296 and/or
an engineered crRNA consisting of a nucleotide sequence selected from the group consisting of SEQ ID NOs: 297 to 304.

### [Embodiment 39]

The vector system of any one of the above-described embodiments, wherein
the first or second guide RNA comprises a scaffold region sequence of a nucleotide sequence selected from the group consisting of SEQ ID NOs: 313 to 350.

### [Embodiment 40]

The vector system of any one of the above-described embodiments, wherein
the Cas12f1 molecule comprises an amino acid sequence having at least 70% sequence identity with an amino acid sequence selected from the group consisting of SEQ ID NOs: 360 to 364 and SEQ ID NOs: 370 to 377.

### [Embodiment 41]

The vector system of any one of the above-described embodiments, wherein
the vector further comprises a promoter or enhancer.

### [Embodiment 42]

The vector system of any one of the above-described embodiments, wherein
the promoter is U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

### [Embodiment 43]

The vector system of any one of the above-described embodiments, wherein
the vector is selected from the group consisting of a retrovirus vector, a lentivirus vector, an adenovirus vector, an adeno-associated virus vector, a vaccinia virus vector, a poxvirus vector, a herpes simplex virus vector, and a phagemid vector.

### [Embodiment 44]

The vector system of any one of the above-described embodiments, wherein
the vector is selected from the group consisting of plasmid, naked DNA, DNA complex, mRNA (transcript), and amplicon.

### [Embodiment 45]

A recombinant virus produced by the vector system of any one of the above-described embodiments.

### [Embodiment 46]

A composition comprising the system of any one of the above-described embodiments, the vector system of any one of the above-described embodiments, or the recombinant virus of any one of the above-described embodiments.

### [Embodiment 47]

The composition of any one of the above-described embodiments, wherein
the composition is a pharmaceutical composition.

### [Embodiment 48]

A method for inducing deletion of a segment comprising exon 13 in a USH2A gene in a cell, comprising
bringing, into contact with the cell, the system of any one of the above-described embodiments, the vector system of any one of the above-described embodiments, or the recombinant virus of any one of the above-described embodiments.

### [Embodiment 49]

A method for treating a subject having a disease associated with a mutation in exon 13 of the USH2A gene, comprising
bringing, into contact with the cell, the system of any one of the above-described embodiments, the vector system of any one of the above-described embodiments, or the recombinant virus of any one of the above-described embodiments.

### [Embodiment 50]

A method for modifying a gene of a cell, comprising
bringing, into contact with the cell, the system of any one of the above-described embodiments, the vector system of any one of the above-described embodiments, or the recombinant virus of any one of the above-described embodiments.

### [Embodiment 51]

The method of any one of the above-described embodiments, wherein
the recombinant virus is an adeno-associated virus (AAV).

### [Embodiment 52]

The method of any one of the above-described embodiments, wherein
the cell is a stem cell, or a cell from the eye or inner ear of a mammal.

### [Embodiment 53]

The method of any one of the above-described embodiments, wherein
the cell is derived from a subject having Usher syndrome.

### [Embodiment 54]

The method of any one of the above-described embodiments, wherein
the bringing-into-contact occurs ex vivo or in vivo.

### [Embodiment 55]

A stem cell genetically modified by the method of any one of the above-described embodiments.

### [Embodiment 56]

The stem cell of any one of the above-described embodiments, wherein
the stem cell is for treating type 2A Usher syndrome.

### [Embodiment 57]

A guide RNA, comprising a spacer region, which comprises a guide sequence capable of hybridizing to a target sequence in a USH2A (Usherin) gene, and a scaffold region, wherein
the guide sequence comprises (i) a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or (ii) a sequence of contiguous 15 to 20 nucleotides from a nucleotide sequence selected from the group consisting of SEQ ID NOs: 446 to 475, or a sequence that is different by 5 or fewer nucleotides in the contiguous nucleotide sequence, wherein thymine (T) is substituted with uracil (U) in the contiguous nucleotide sequence.

### [Embodiment 58]

The guide RNA of any one of the above-described embodiments, wherein
the guide sequence comprises a nucleotide sequence selected from the group consisting of SEQ ID NOs: 80 to 128 and SEQ ID NOs: 159 to 164, and/or
the guide sequence comprises a nucleotide sequence selected from the group consisting of SEQ ID NOs: 129 to 158 and SEQ ID NOs: 165 to 174.

### [Embodiment 59]

The guide RNA of any one of the above-described embodiments, wherein
the guide RNA comprises a U-rich tail sequence linked to the 3' end of the guide sequence, in which the U-rich tail is represented by 5'-(UₘV)ₙUₒ-3', wherein V is each independently A, C, or G, m and o are integers between 1 to 20, and n is an integer between 0 to 5.

### [Embodiment 60]

The guide RNA of any one of the above-described embodiments, wherein
the scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region and a tracrRNA-crRNA complementarity region, and at least one modification selected from the group consisting of the following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

### [Embodiment 61]

The guide RNA of any one of the above-described embodiments, wherein
the wild-type Cas12f1 guide RNA comprises tracrRNA comprising the nucleotide sequence of SEQ ID NO: 175 and crRNA comprising the nucleotide sequence of SEQ ID NO: 176.

### [Embodiment 62]

The guide RNA of any one of the above-described embodiments, wherein
the scaffold region comprises a sequence having at least 80% sequence identity to a sequence represented by Formula (I): 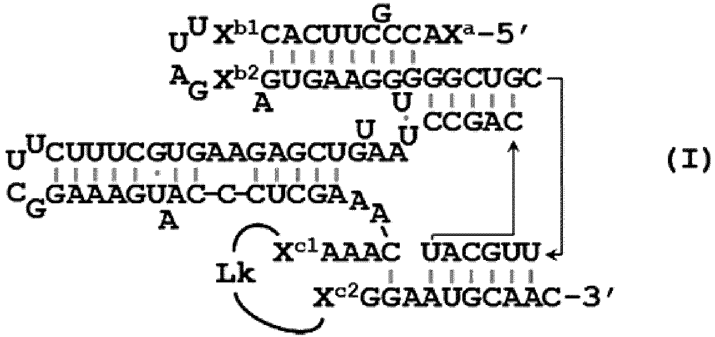 in Formula (I),
X^{a} comprises the nucleotide sequence of SEQ ID NO: 178 or a nucleotide sequence having the sequence of SEQ ID NO: 178 from which 1 to 20 nucleotides are deleted,
X^{b1} comprises the nucleotide sequence of SEQ ID NO: 189 or a nucleotide sequence having the sequence of SEQ ID NO: 189 from which 1 to 13 nucleotides are deleted,
X^{b2} comprises the nucleotide sequence of SEQ ID NO: 193 or a nucleotide sequence having the sequence of SEQ ID NO: 193 from which 1 to 14 nucleotides are deleted,
X^{c1} comprises the nucleotide sequence of SEQ ID NO: 203 or a nucleotide sequence having the sequence of SEQ ID NO: 203 from which 1 to 28 nucleotides are deleted,
X^{c2} comprises the nucleotide sequence of SEQ ID NO: 222 or a nucleotide sequence having the sequence of SEQ ID NO: 222 from which 1 to 27 nucleotides are deleted, and
Lk is a polynucleotide linker of 2 to 20 nucleotides in length or absent.

### [Embodiment 63]

The guide RNA of any one of the above-described embodiments, wherein
in a case where three or more consecutive uracil (U) residues are present in the X^{c1} sequence, the X^{c1} sequence comprises a modification in which at least one U residue thereof is replaced with A, G or C.

### [Embodiment 64]

The guide RNA of any one of the above-described embodiments, wherein
the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

### [Embodiment 65]

The guide RNA of any one of the above-described embodiments, wherein
the sequence 5'-X^{b1}UUAGX^{b2}-3' in Formula (I) is selected from the group consisting of SEQ ID NOs: 198 to 202 and 5'-UUAG-3'.

### [Embodiment 66]

The guide RNA of any one of the above-described embodiments, wherein
the sequence 5'-X^{c1}-Lk-X^{c2}-3' in Formula (I) is selected from the group consisting of SEQ ID NOs: 244 to 250 and 5'-Lk-3'.

### [Embodiment 67]

The guide RNA of any one of the above-described embodiments, wherein
the scaffold region comprises an engineered tracrRNA consisting of a nucleotide sequence selected from the group consisting of SEQ ID NOs: 251 to 296 and/or
an engineered crRNA consisting of a nucleotide sequence selected from the group consisting of SEQ ID NOs: 297 to 304.

### [Embodiment 68]

The guide RNA of any one of the above-described embodiments, wherein
the guide RNA is a single guide RNA.

### [Embodiment 69]

The guide RNA of any one of the above-described embodiments, wherein
the guide RNA comprises a scaffold region sequence of a nucleotide sequence selected from the group consisting of SEQ ID NOs: 313 to 350.

### [Embodiment 70]

The guide RNA of any one of the above-described embodiments, wherein
the guide RNA comprises a scaffold region sequence of a nucleotide sequence selected from the group consisting of SEQ ID NOs: 315 to 317.

### [Embodiment 71]

A nucleic acid molecule encoding the guide RNA of any one of the above-described embodiments.

### [Embodiment 72]

A composition comprising at least one guide RNA of any one of the above-described embodiments.

### [Embodiment 73]

A composition comprising at least one guide RNA of any one of the above-described embodiments and an endonuclease comprising a Cas12f1 molecule.

### [Embodiment 74]

The composition of any one of the above-described embodiments, wherein
the composition comprises two or more guide RNAs, of which (i) at least one guide RNA comprises a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, and/or (ii) at least one other guide RNA comprises a sequence of contiguous 15 to 20 nucleotides from a nucleotide sequence selected from the group consisting of SEQ ID NOs: 446 to 475, or a sequence that is different by 5 or fewer nucleotides in the contiguous nucleotide sequence.

Hereinafter, the present disclosure will be described in more detail by way of the following examples. It will be apparent to those skilled in the art that these examples are intended only to illustrate the content disclosed by the present specification, and that the scope of the content disclosed by the present specification is not limited by these examples.

### Examples

### Example 1. Engineering guide RNA for Cas12f1

### Example 1.1. Wild-type Cas12f1 protein and human codon-optimized nucleic acid encoding thereof

The gene editing system of the present disclosure comprises, as one component, an endonuclease comprising a wild-type Cas12f1 (CWCas12f1 or Un1Cas12f1) protein or a variant protein thereof. In some other aspects, the gene editing system comprises an engineered guide RNA (gRNA) which is combined with the endonuclease and exhibits high gene editing efficiency. The engineered gRNA is artificially modified to exhibit improved gene editing efficiency while being shorter than the wild-type gRNA. For the development of such a gRNA, a plurality of engineered gRNAs comprising various modifications and combinations thereof were produced based on the gene editing system comprising the wild-type Cas12f1 protein, and the gene editing efficiency of the respective gRNAs was tested. Here, the Cas12f1 protein may be a protein comprising or consisting of the amino acid sequence of SEQ ID NO: 360 or SEQ ID NO: 364 as presented in Table 9.

**[Table 9]**

| Name | Amino acid sequence | SEQ ID NO |
|---|---|---|
| CWCas12f1 protein | MGEKSSRRRRNGKSGAWTAAITSCVGGKMAKNTITKTL KLRIVRPYNSAEVEKIVADEKNNREKIALEKNKDKVKEA CSKHLKVAAYCTTQVERNACLFCKARKLDDKFYQKLRG QFPDAVFWQEISEIFRQLQKQAAEIYNQSLIELYYEIFIKGK GIANASSVEHYLSDVCYTRAAELFKNAAIASGLRSKIKSN FRLKELKNMKSGLPTTKSDNFPIPLVKQKGGQYTGFEISN HNSDFIIKIPFGRWQVKKEIDKYRPWEKFDFEQVQKSPKP ISLLLSTQRRKRNKGWSKDEGTEAEIKKVMNGDYQTSYI EVKRGSKIGEKSAWMLNLSIDVPKIDKGVDPSIIGGIDVG VKSPLVCAINNAFSRYSISDNDLFHFNKKMFARRRILLKK NRHKRAGHGAKNKLKPITILTEKSERFRKKLIERWACEIA DFFIKNKVGTVQMENLESMKRKEDSYFNIRLRGFWPYAE MQNKIEFKLKQYGIEIRKVAPNNTSKTCSKCGHLNNYFNF EYRKKNKFPHFKCEKCNFKENADYNAALNISNPKLKSTK EEP | 360 |
| Un1Cas12f1 protein | MAKNTITKTLKLRIVRPYNSAEVEKIVADEKNNREKIALE KNKDKVKEACSKHLKVAAYCTTQVERNACLFCKARKLD DKFYQKLRGQFPDAVFWQEISEIFRQLQKQAAEIYNQSLI ELYYEIFIKGKGIANASSVEHYLSDVCYTRAAELFKNAAI ASGLRSKIKSNFRLKELKNMKSGLPTTKSDNFPIPLVKQK GGQYTGFEISNHNSDFIIKIPFGRWQVKKEIDKYRPWEKF DFEQVQKSPKPISLLLSTQRRKRNKGWSKDEGTEAEIKK VMNGDYQTSYIEVKRGSKIGEKSAWMLNLSIDVPKIDKG VDPSIIGGIDVGVKSPLVCAINNAFSRYSISDNDLFHFNKK MFARRRILLKKNRHKRAGHGAKNKLKPITILTEKSERFRK KLIERWACEIADFFIKNKVGTVQMENLESMKRKEDSYFNI RLRGFWPYAEMQNKIEFKLKQYGIEIRKVAPNNTSKTCSK CGHLNNYFNFEYRKKNKFPHFKCEKCNFKENADYNAAL NISNPKLKSTKEEP | 364 |

In order to construct the gene editing system expressed in human cells and a nucleic acid construct encoding each component of the system, a codon optimization program was used to obtain human-codon optimized genes for CWCas12f1 and Un1Cas12f1 proteins. As an example, thus obtained nucleotide sequences of human-codon optimized nucleic acids for CWCas12f1 and Un1Cas12f1 proteins are as follows:
"Human codon-optimized nucleic acid encoding the CWCas12f1 protein", 
5'-ATGGGGGAGAAAAGTTCCCGCCGCCGACGGAATGGAAAAAGCGGTGCGTGGACTGCTGCTATAACAAGCT
GTGTTGGGGGTAAGATGGCCAAGAACACAATTACAAAGACACTGAAGCTGAGGATCGTGAGACCATACAACAG
CGCTGAGGTCGAGAAGATTGTGGCTGATGAAAAGAACAACAGGGAAAAGATCGCCCTCGAGAAGAACAAGGAT
AAGGTGAAGGAGGCCTGCTCTAAGCACCTGAAAGTGGCCGCCTACTGCACCACACAGGTGGAGAGGAACGCCT
GTCTGTTTTGTAAAGCTCGGAAGCTGGATGATAAGTTTTACCAGAAGCTGCGGGGCCAGTTCCCCGATGCCGT
CTTTTGGCAGGAGATTAGCGAGATCTTCAGACAGCTGCAGAAGCAGGCCGCCGAGATCTACAACCAGAGCCTG
ATCGAGCTCTACTACGAGATCTTCATCAAGGGCAAGGGCATTGCCAACGCCTCCTCCGTGGAGCACTACCTGA
GCGACGTGTGCTACACAAGAGCCGCCGAGCTCTTTAAGAACGCCGCTATCGCTTCCGGGCTGAGGAGCAAGAT
TAAGAGTAACTTCCGGCTCAAGGAGCTGAAGAACATGAAGAGCGGCCTGCCCACTACAAAGAGCGACAACTTC
CCAATTCCACTGGTGAAGCAGAAGGGGGGCCAGTACACAGGGTTCGAGATTTCCAACCACAACAGCGACTTTA
TTATTAAGATCCCCTTTGGCAGGTGGCAGGTCAAGAAGGAGATTGACAAGTACAGGCCCTGGGAGAAGTTTGA
TTTCGAGCAGGTGCAGAAGAGCCCCAAGCCTATTTCCCTGCTGCTGTCCACACAGCGGCGGAAGAGGAACAAG
GGGTGGTCTAAGGATGAGGGGACCGAGGCCGAGATTAAGAAAGTGATGAACGGCGACTACCAGACAAGCTACA
TCGAGGTCAAGCGGGGCAGTAAGATTGGCGAGAAGAGCGCCTGGATGCTGAACCTGAGCATTGACGTGCCAAA
GATTGATAAGGGCGTGGATCCCAGCATCATCGGAGGGATCGATGTGGGGGTCAAGAGCCCCCTCGTGTGCGCC
ATCAACAACGCCTTCAGCAGGTACAGCATCTCCGATAACGACCTGTTCCACTTTAACAAGAAGATGTTCGCCC
GGCGGAGGATTTTGCTCAAGAAGAACCGGCACAAGCGGGCCGGACACGGGGCCAAGAACAAGCTCAAGCCCAT
CACTATCCTGACCGAGAAGAGCGAGAGGTTCAGGAAGAAGCTCATCGAGAGATGGGCCTGCGAGATCGCCGAT
TTCTTTATTAAGAACAAGGTCGGAACAGTGCAGATGGAGAACCTCGAGAGCATGAAGAGGAAGGAGGATTCCT
ACTTCAACATTCGGCTGAGGGGGTTCTGGCCCTACGCTGAGATGCAGAACAAGATTGAGTTTAAGCTGAAGCA
GTACGGGATTGAGATCCGGAAGGTGGCCCCCAACAACACCAGCAAGACCTGCAGCAAGTGCGGGCACCTCAAC
AACTACTTCAACTTCGAGTACCGGAAGAAGAACAAGTTCCCACACTTCAAGTGCGAGAAGTGCAACTTTAAGG
AGAACGCCGATTACAACGCCGCCCTGAACATCAGCAACCCTAAGCTGAAGAGCACTAAGGAGGAGCCC-3'
(SEQ ID NO: 365);
"Human codon-optimized nucleic acid encoding the Un1Cas12f1 protein",

5'-ATGGCCAAGAACACAATTACAAAGACACTGAAGCTGAGGATCGTGAGACCATACAACAGCGCTGAGGTCG
AGAAGATTGTGGCTGATGAAAAGAACAACAGGGAAAAGATCGCCCTCGAGAAGAACAAGGATAAGGTGAAGGA
GGCCTGCTCTAAGCACCTGAAAGTGGCCGCCTACTGCACCACACAGGTGGAGAGGAACGCCTGTCTGTTTTGT
AAAGCTCGGAAGCTGGATGATAAGTTTTACCAGAAGCTGCGGGGCCAGTTCCCCGATGCCGTCTTTTGGCAGG
AGATTAGCGAGATCTTCAGACAGCTGCAGAAGCAGGCCGCCGAGATCTACAACCAGAGCCTGATCGAGCTCTA
CTACGAGATCTTCATCAAGGGCAAGGGCATTGCCAACGCCTCCTCCGTGGAGCACTACCTGAGCGACGTGTGC
TACACAAGAGCCGCCGAGCTCTTTAAGAACGCCGCTATCGCTTCCGGGCTGAGGAGCAAGATTAAGAGTAACT
TCCGGCTCAAGGAGCTGAAGAACATGAAGAGCGGCCTGCCCACTACAAAGAGCGACAACTTCCCAATTCCACT
GGTGAAGCAGAAGGGGGGCCAGTACACAGGGTTCGAGATTTCCAACCACAACAGCGACTTTATTATTAAGATC
CCCTTTGGCAGGTGGCAGGTCAAGAAGGAGATTGACAAGTACAGGCCCTGGGAGAAGTTTGATTTCGAGCAGG
TGCAGAAGAGCCCCAAGCCTATTTCCCTGCTGCTGTCCACACAGCGGCGGAAGAGGAACAAGGGGTGGTCTAA
GGATGAGGGGACCGAGGCCGAGATTAAGAAAGTGATGAACGGCGACTACCAGACAAGCTACATCGAGGTCAAG
CGGGGCAGTAAGATTGGCGAGAAGAGCGCCTGGATGCTGAACCTGAGCATTGACGTGCCAAAGATTGATAAGG
GCGTGGATCCCAGCATCATCGGAGGGATCGATGTGGGGGTCAAGAGCCCCCTCGTGTGCGCCATCAACAACGC
CTTCAGCAGGTACAGCATCTCCGATAACGACCTGTTCCACTTTAACAAGAAGATGTTCGCCCGGCGGAGGATT
TTGCTCAAGAAGAACCGGCACAAGCGGGCCGGACACGGGGCCAAGAACAAGCTCAAGCCCATCACTATCCTGA
CCGAGAAGAGCGAGAGGTTCAGGAAGAAGCTCATCGAGAGATGGGCCTGCGAGATCGCCGATTTCTTTATTAA
GAACAAGGTCGGAACAGTGCAGATGGAGAACCTCGAGAGCATGAAGAGGAAGGAGGATTCCTACTTCAACATT
CGGCTGAGGGGGTTCTGGCCCTACGCTGAGATGCAGAACAAGATTGAGTTTAAGCTGAAGCAGTACGGGATTG
AGATCCGGAAGGTGGCCCCCAACAACACCAGCAAGACCTGCAGCAAGTGCGGGCACCTCAACAACTACTTCAA
CTTCGAGTACCGGAAGAAGAACAAGTTCCCACACTTCAAGTGCGAGAAGTGCAACTTTAAGGAGAACGCCGAT
TACAACGCCGCCCTGAACATCAGCAACCCTAAGCTGAAGAGCACTAAGGAGGAGCCC-3'
(SEQ ID NO: 369).

The exemplified sequence was used as a nucleic acid encoding the endonuclease (gene editing protein) in the gene editing system to test the indel efficiency according to the modification of gRNA.

Meanwhile, the nucleic acid construct expressing each component of the gene editing system was prepared by the following method: The nucleic acid construct used in this example comprises a gene sequence of human codon-optimized Cas12f1. PCR amplification was performed using the gene sequence as a template, and cloning was performed according to a desired cloning sequence for a vector having a promoter capable of expression in a eukaryotic system and a poly(A) signal sequence by the Gibson assembly method. The sequence of a plasmid vector obtained after cloning was finally identified by the Sanger sequencing method.

### Example 1.2. Engineering of guide RNA and selection of optimal guide RNA

### Example 1.2.1. Design of engineered guide RNA

In a case where a length of the guide RNA (gRNA) can be shortened while maintaining the same indel activity, it may have advantages such as overcoming the packaging limitations of adeno-associated virus (AAV). Furthermore, in a case where the indel activity can be further improved, applicability in various gene editing fields including therapeutics may increase. The engineered guide RNA for Cas12f1 or a variant protein thereof used in the USH2A gene editing system of the present disclosure is a gRNA found in nature with a new configuration added thereto or a part of its structure or sequence modified. The engineered gRNA may comprise a sequence in which one or more nucleotides are substituted, deleted, inserted, or added to the wild-type Cas12f1 guide RNA sequence.

FIG. 1 illustrates a wild-type guide RNA for wild-type Cas12f1 and MS1 to MS5, which are sites (modification sites (MS), hereinafter abbreviated as MS) to which various modifications can be applied based on a wild-type gRNA sequence existing in nature for production of a gRNA that allows the USH2A gene editing system of the present disclosure to have highly efficient gene editing activity. FIGS. 2A and 2B illustrate exemplary modification sites for producing an engineered single guide RNA (engineered sgRNA) in which various modifications in MS1 to MS5 are combined (for example, exemplary modification sites corresponding to MS3 are indicated by MS3-1, MS3-2, and MS3-3, respectively). FIG. 2A illustrates modification sites of a canonical sgRNA for Cas12f1, and FIG. 2B illustrates modification sites of a mature form sgRNA for Cas12f1.

In this embodiment, as described in detail in the section "2.3. Scaffold region and engineering thereof," engineered gRNAs exhibiting highly efficient gene editing ability for the endonuclease comprising Cas12f1 or a variant protein thereof were produced, and exemplary sequences thereof are provided in Table 10. The gRNAs disclosed herein are representative examples of engineered gRNAs used in the USH2A gene editing system of the present disclosure, and the gRNAs that can be used in the gene editing system of the present disclosure are not limited to the exemplified sequences.

**[Table 10]**

| gRNA | Sequence (5'→3') | SEQ ID NO |
|---|---|---|
| Canonical sgRNA | CUUCACUGAUAAAGUGGAGAACCGCUUCACCAAAAG CUGUCCCuuagGGGAUUAGAACUUGAGUGAAGGUGGG CUGCUUGCAUCAGCCUAAUGUCGAGAAGUGCUUUCU UCGGAAAGUAACCCUCGAAACAAAUUCAUUUUUCCU CUCCAAUUCUGCACAAgaaaGUUGCAGAACCCGAAUAG acgaaUGAAGGAAUGCAACNNNNNNNNNNNNNNNNNN NN | 177 |
| MS1 | CUUCACUGAUAAAGUGGAGAACCGCUUCACCAAAAG CUGUCCCUUAGGGGAUUAGAACUUGAGUGAAGGUGG GCUGCUUGCAUCAGCCUAAUGUCGAGAAGUGCUUUC UUCGGAAAGUAACCCUCGAAACAAAUUCAGUGCUCC UCUCCAAUUCUGCACAAgaaaGUUGCAGAACCCGAAUA GAGCAAUGAAGGAAUGCAACNNNNNNNNNNNNNNNN NNNN | 313 |
| MS1/MS2 | CUUCACUGAUAAAGUGGAGAACCGCUUCACCAAAAG CUGUCCCUUAGGGGAUUAGAACUUGAGUGAAGGUGG GCUGCUUGCAUCAGCCUAAUGUCGAGAAGUGCUUUC UUCGGAAAGUAACCCUCGAAACAAAUUCAGUGCUCC UCUCCAAUUCUGCACAAgaaaGUUGCAGAACCCGAAUA GAGCAAUGAAGGAAUGCAACNNNNNNNNNNNNNNNN NNNNUUUUAUUUUUU | 314 |
| MS1/MS2/MS3 (ver3.0) | ACCGCUUCACCAAAAGCUGUCCCUUAGGGGAUUAGA ACUUGAGUGAAGGUGGGCUGCUUGCAUCAGCCUAAU GUCGAGAAGUGCUUUCUUCGGAAAGUAACCCUCGAA ACAAAUUCAGUGCUCCUCUCCAAUUCUGCACAAgaaaG UUGCAGAACCCGAAUAGAGCAAUGAAGGAAUGCAAC NNNNNNNNNNNNNNNNNNNNUUUUAUUUUUU | 315 |
| MS2/MS3/MS4 (ver4.0) | ACCGCUUCACCAAAAGCUGUCCCUUAGGGGAUUAGA ACUUGAGUGAAGGUGGGCUGCUUGCAUCAGCCUAAU GUCGAGAAGUGCUUUCUUCGGAAAGUAACCCUCGAA ACAAAgaaaGGAAUGCAACNNNNNNNNNNNNNNNNNN NNUUUUAUUUUUU | 316 |
| MS2/MS3/MS4/M S5 (ver4.1) | ACCGCUUCACUUAGAGUGAAGGUGGGCUGCUUGCAU CAGCCUAAUGUCGAGAAGUGCUUUCUUCGGAAAGUA ACCCUCGAAACAAAgaaaGGAAUGCAACNNNNNNNNN | 317 |
| | NNNNNNNNNNUUUUAUUUUUU | |
| MS1/MS3-1 | GAUAAAGUGGAGAACCGCUUCACCAAAAGCUGUCCC UUAGGGGAUUAGAACUUGAGUGAAGGUGGGCUGCUU GCAUCAGCCUAAUGUCGAGAAGUGCUUUCUUCGGAA AGUAACCCUCGAAACAAAUUCAGUGCUCCUCUCCAA UUCUGCACAAgaaaGUUGCAGAACCCGAAUAGAGCAAU GAAGGAAUGCAACNNNNNNNNNNNNNNNNNNNN | 318 |
| MS1/MS3-2 | UGGAGAACCGCUUCACCAAAAGCUGUCCCUUAGGGG AUUAGAACUUGAGUGAAGGUGGGCUGCUUGCAUCAG CCUAAUGUCGAGAAGUGCUUUCUUCGGAAAGUAACC CUCGAAACAAAUUCAGUGCUCCUCUCCAAUUCUGCA CAAgaaaGUUGCAGAACCCGAAUAGAGCAAUGAAGGA AUGCAACNNNNNNNNNNNNNNNNNNNN | 319 |
| MS1/MS3-3 | ACCGCUUCACCAAAAGCUGUCCCUUAGGGGAUUAGA ACUUGAGUGAAGGUGGGCUGCUUGCAUCAGCCUAAU GUCGAGAAGUGCUUUCUUCGGAAAGUAACCCUCGAA ACAAAUUCAGUGCUCCUCUCCAAUUCUGCACAAgaaaG UUGCAGAACCCGAAUAGAGCAAUGAAGGAAUGCAAC NNNNNNNNNNNNNNNNNNNN | 320 |
| MS1/MS4*-1 | CUUCACUGAUAAAGUGGAGAACCGCUUCACCAAAAG CUGUCCCUUAGGGGAUUAGAACUUGAGUGAAGGUGG GCUGCUUGCAUCAGCCUAAUGUCGAGAAGUGCUUUC UUCGGAAAGUAACCCUCGAAACAAAUUCAGUGCUCC UCUCCAAUUCgaaaGAACCCGAAUAGAGCAAUGAAGGA AUGCAACNNNNNNNNNNNNNNNNNNNN | 321 |
| MS1/MS4*-2 | CUUCACUGAUAAAGUGGAGAACCGCUUCACCAAAAG CUGUCCCUUAGGGGAUUAGAACUUGAGUGAAGGUGG GCUGCUUGCAUCAGCCUAAUGUCGAGAAGUGCUUUC UUCGGAAAGUAACCCUCGAAACAAAUUCAGUGCUCC UCUCgaaaGAAUAGAGCAAUGAAGGAAUGCAACNNNN NNNNNNNNNNNNNNNN | 322 |
| MS1/MS4*-3 | CUUCACUGAUAAAGUGGAGAACCGCUUCACCAAAAG CUGUCCCUUAGGGGAUUAGAACUUGAGUGAAGGUGG GCUGCUUGCAUCAGCCUAAUGUCGAGAAGUGCUUUC UUCGGAAAGUAACCCUCGAAACAAAUUCAGUGCUgaaa AGCAAUGAAGGAAUGCAACNNNNNNNNNNNNNNNNN NNN | 323 |
| MS1/MS5-1 | CUUCACUGAUAAAGUGGAGAACCGCUUCACCAAAAG CUGUuuagAUUAGAACUUGAGUGAAGGUGGGCUGCUU GCAUCAGCCUAAUGUCGAGAAGUGCUUUCUUCGGAA AGUAACCCUCGAAACAAAUUCAGUGCUCCUCUCCAA UUCUGCACAAgaaaGUUGCAGAACCCGAAUAGAGCAAU | 324 |
| | GAAGGAAUGCAACNNNNNNNNNNNNNNNNNNNN | |
| MS1/MS5-2 | CUUCACUGAUAAAGUGGAGAACCGCUUCACCAAAAG CuuagGAACUUGAGUGAAGGUGGGCUGCUUGCAUCAG CCUAAUGUCGAGAAGUGCUUUCUUCGGAAAGUAACC CUCGAAACAAAUUCAGUGCUCCUCUCCAAUUCUGCA CAAgaaaGUUGCAGAACCCGAAUAGAGCAAUGAAGGA AUGCAACNNNNNNNNNNNNNNNNNNNN | 325 |
| MS1/MS5-3 | CUUCACUGAUAAAGUGGAGAACCGCUUCACCAAuuag UUGAGUGAAGGUGGGCUGCUUGCAUCAGCCUAAUGU CGAGAAGUGCUUUCUUCGGAAAGUAACCCUCGAAAC AAAUUCAGUGCUCCUCUCCAAUUCUGCACAAgaaaGUU GCAGAACCCGAAUAGAGCAAUGAAGGAAUGCAACNN NNNNNNNNNNNNNNNNNN | 326 |
| MS1/MS2/MS4*-2 | CUUCACUGAUAAAGUGGAGAACCGCUUCACCAAAAG CUGUCCCUUAGGGGAUUAGAACUUGAGUGAAGGUGG GCUGCUUGCAUCAGCCUAAUGUCGAGAAGUGCUUUC UUCGGAAAGUAACCCUCGAAACAAAUUCAGUGCUCC UCUCgaaaGAAUAGAGCAAUGAAGGAAUGCAACNNNN NNNNNNNNNNNNNNNNUUUUAUUUU | 327 |
| MS1/MS3-3/MS4*-2 | ACCGCUUCACCAAAAGCUGUCCCUUAGGGGAUUAGA ACUUGAGUGAAGGUGGGCUGCUUGCAUCAGCCUAAU GUCGAGAAGUGCUUUCUUCGGAAAGUAACCCUCGAA ACAAAUUCAGUGCUCCUCUCgaaaGAAUAGAGCAAUGA AGGAAUGCAACNNNNNNNNNNNNNNNNNNNN | 328 |
| MS1/MS2/MS5-3 | CUUCACUGAUAAAGUGGAGAACCGCUUCACCAAuuag UUGAGUGAAGGUGGGCUGCUUGCAUCAGCCUAAUGU CGAGAAGUGCUUUCUUCGGAAAGUAACCCUCGAAAC AAAUUCAGUGCUCCUCUCCAAUUCUGCACAAgaaaGUU GCAGAACCCGAAUAGAGCAAUGAAGGAAUGCAACNN NNNNNNNNNNNNNNNNNNUUUUAUUUU | 329 |
| MS1/MS3-3/MS5-3 | ACCGCUUCACCAAuuagUUGAGUGAAGGUGGGCUGCU UGCAUCAGCCUAAUGUCGAGAAGUGCUUUCUUCGGA AAGUAACCCUCGAAACAAAUUCAGUGCUCCUCUCCA AUUCUGCACAAgaaaGUUGCAGAACCCGAAUAGAGCAA UGAAGGAAUGCAACNNNNNNNNNNNNNNNNNNNN | 330 |
| MS1/MS4*-2/MS5-3 | CUUCACUGAUAAAGUGGAGAACCGCUUCACCAAuuag UUGAGUGAAGGUGGGCUGCUUGCAUCAGCCUAAUGU CGAGAAGUGCUUUCUUCGGAAAGUAACCCUCGAAAC AAAUUCAGUGCUCCUCUCgaaaGAAUAGAGCAAUGAA GGAAUGCAACNNNNNNNNNNNNNNNNNNNN | 331 |
| MS1/MS2/MS3-3/MS4*-2 | ACCGCUUCACCAAAAGCUGUCCCuuagGGGAUUAGAAC UUGAGUGAAGGUGGGCUGCUUGCAUCAGCCUAAUGU | 332 |
| | CGAGAAGUGCUUUCUUCGGAAAGUAACCCUCGAAAC AAAUUCAGUGCUCCUCUCgaaaGAAUAGAGCAAUGAA GGAAUGCAACNNNNNNNNNNNNNNNNNNNNUUUUAU UUU | |
| MS1/MS2/MS3-3/MS5-3 | ACCGCUUCACCAAuuagUUGAGUGAAGGUGGGCUGCU UGCAUCAGCCUAAUGUCGAGAAGUGCUUUCUUCGGA AAGUAACCCUCGAAACAAAUUCAGUGCUCCUCUCCA AUUCUGCACAAgaaaGUUGCAGAACCCGAAUAGAGCAA UGAAGGAAUGCAACNNNNNNNNNNNNNNNNNNNNUU UUAUUUU | 333 |
| MS1/MS2/MS4*-2/MS5-3 | CUUCACUGAUAAAGUGGAGAACCGCUUCACCAAuuag UUGAGUGAAGGUGGGCUGCUUGCAUCAGCCUAAUGU CGAGAAGUGCUUUCUUCGGAAAGUAACCCUCGAAAC AAAUUCAGUGCUCCUCUCgaaaGAAUAGAGCAAUGAA GGAAUGCAACNNNNNNNNNNNNNNNNNNNNUUUUAU UUU | 334 |
| MS1/MS3-3/MS4*-2/MS5-3 | ACCGCUUCACCAAuuagUUGAGUGAAGGUGGGCUGCU UGCAUCAGCCUAAUGUCGAGAAGUGCUUUCUUCGGA AAGUAACCCUCGAAACAAAUUCAGUGCUCCUCUCgaaa GAAUAGAGCAAUGAAGGAAUGCAACNNNNNNNNNNN NNNNNNNNN | 335 |
| MS1/MS2/MS3-3/MS4*-2/MS5-3 | ACCGCUUCACCAAuuagUUGAGUGAAGGUGGGCUGCU UGCAUCAGCCUAAUGUCGAGAAGUGCUUUCUUCGGA AAGUAACCCUCGAAACAAAUUCAGUGCUCCUCUCgaaa GAAUAGAGCAAUGAAGGAAUGCAACNNNNNNNNNNN NNNNNNNNNUUUUAUUUU | 336 |

In addition, a mature form gRNA was produced by removing a portion of the sequence corresponding to MS4, which is one of the modification sites, from the canonical sgRNA. Exemplary sequences of the mature form gRNA are shown in Table 11.

**[Table 11]**

| gRNA | Sequence (5'→3') | SEQ ID NO |
|---|---|---|
| Mature form gRNA | CUUCACUGAUAAAGUGGAGAACCGCUUCACCAAAAG [?] CUGUCCCuuagGGGAUUAGAACUUGAGUGAAGGUGGG CUGCUUGCAUCAGCCUAAUGUCGAGAAGUGCUUUCU UCGGAAAGUAACCCUCGAAACAAAUUCAUUUgaaaGA AUGAAGGAAUGCAACNNNNNNNNNNNNNNNNNNNN | 337 |
| MS3-1 | GAUAAAGUGGAGAACCGCUUCACCAAAAGCUGUCCC [?] uuagGGGAUUAGAACUUGAGUGAAGGUGGGCUGCUUG CAUCAGCCUAAUGUCGAGAAGUGCUUUCUUCGGAAA | 338 |
| | GUAACCCUCGAAACAAAUUCAUUUgaaaGAAUGAAGG AAUGCAACNNNNNNNNNNNNNNNNNNNN | |
| MS3-2 | UGGAGAACCGCUUCACCAAAAGCUGUCCCuuagGGGAU UAGAACUUGAGUGAAGGUGGGCUGCUUGCAUCAGCC UAAUGUCGAGAAGUGCUUUCUUCGGAAAGUAACCCU CGAAACAAAUUCAUUUgaaaGAAUGAAGGAAUGCAAC NNNNNNNNNNNNNNNNNNNN | 339 |
| MS3-3 | ACCGCUUCACCAAAAGCUGUCCCuuagGGGAUUAGAAC UUGAGUGAAGGUGGGCUGCUUGCAUCAGCCUAAUGU CGAGAAGUGCUUUCUUCGGAAAGUAACCCUCGAAAC AAAUUCAUUUgaaaGAAUGAAGGAAUGCAACNNNNNN NNNNNNNNNNNNNN | 340 |
| MS4-1 | CUUCACUGAUAAAGUGGAGAACCGCUUCACCAAAAG CUGUCCCuuagGGGAUUAGAACUUGAGUGAAGGUGGG CUGCUUGCAUCAGCCUAAUGUCGAGAAGUGCUUUCU UCGGAAAGUAACCCUCGAAACAAAUUCAUgaaaAUGA AGGAAUGCAACNNNNNNNNNNNNNNNNNNNN | 341 |
| MS4-2 | CUUCACUGAUAAAGUGGAGAACCGCUUCACCAAAAG CUGUCCCuuagGGGAUUAGAACUUGAGUGAAGGUGGG CUGCUUGCAUCAGCCUAAUGUCGAGAAGUGCUUUCU UCGGAAAGUAACCCUCGAAACAAAUUCgaaaGAAGGA AUGCAACNNNNNNNNNNNNNNNNNNNN | 342 |
| MS4-3 | CUUCACUGAUAAAGUGGAGAACCGCUUCACCAAAAG CUGUCCCuuagGGGAUUAGAACUUGAGUGAAGGUGGG CUGCUUGCAUCAGCCUAAUGUCGAGAAGUGCUUUCU UCGGAAAGUAACCCUCGAAACAAAgaaaGGAAUGCAAC NNNNNNNNNNNNNNNNNNNN | 343 |
| MS5-1 | CUUCACUGAUAAAGUGGAGAACCGCUUCACCAAAAG CUGUuuagAUUAGAACUUGAGUGAAGGUGGGCUGCUU GCAUCAGCCUAAUGUCGAGAAGUGCUUUCUUCGGAA AGUAACCCUCGAAACAAAUUCAUUUgaaaGAAUGAAG GAAUGCAACNNNNNNNNNNNNNNNNNNNN | 344 |
| MS5-2 | CUUCACUGAUAAAGUGGAGAACCGCUUCACCAAAAG CUuuagAGAACUUGAGUGAAGGUGGGCUGCUUGCAUC AGCCUAAUGUCGAGAAGUGCUUUCUUCGGAAAGUAA CCCUCGAAACAAAUUCAUUUgaaaGAAUGAAGGAAUG CAACNNNNNNNNNNNNNNNNNNNN | 345 |
| MS5-3 | CUUCACUGAUAAAGUGGAGAACCGCUUCACCAAuuag UUGAGUGAAGGUGGGCUGCUUGCAUCAGCCUAAUGU CGAGAAGUGCUUUCUUCGGAAAGUAACCCUCGAAAC AAAUUCAUUUgaaaGAAUGAAGGAAUGCAACNNNNNN NNNNNNNNNNNNNN | 346 |
| MS3-3/MS4-3 | ACCGCUUCACCAAAAGCUGUCCCuuagGGGAUUAGAAC UUGAGUGAAGGUGGGCUGCUUGCAUCAGCCUAAUGU CGAGAAGUGCUUUCUUCGGAAAGUAACCCUCGAAAC AAAgaaaGGAAUGCAACNNNNNNNNNNNNNNNNNNNN | 347 |
| MS3-3/MS5-3 | ACCGCUUCACCAAuuagUUGAGUGAAGGUGGGCUGCU UGCAUCAGCCUAAUGUCGAGAAGUGCUUUCUUCGGA AAGUAACCCUCGAAACAAAUUCAUUUgaaaGAAUGAA GGAAUGCAACNNNNNNNNNNNNNNNNNNNN | 348 |
| MS4-3/MSS-3 | CUUCACUGAUAAAGUGGAGAACCGCUUCACCAAuuag UUGAGUGAAGGUGGGCUGCUUGCAUCAGCCUAAUGU CGAGAAGUGCUUUCUUCGGAAAGUAACCCUCGAAAC AAAgaaaGGAAUGCAACNNNNNNNNNNNNNNNNNNNN | 349 |
| MS3-3/MS4-3/MS5-3 | ACCGCUUCACCAAuuagUUGAGUGAAGGUGGGCUGCU UGCAUCAGCCUAAUGUCGAGAAGUGCUUUCUUCGGA AAGUAACCCUCGAAACAAAgaaaGGAAUGCAACNNNN NNNNNNNNNNNNNNNN | 350 |

The sequence indicated by 'NNNNNNNNNNNNNNNNNNNN' in Tables 10 and 11 refers to a guide sequence (spacer sequence) having any length that can hybridize with a target sequence in a target gene (for example, USH2A gene). The guide sequence can be appropriately designed by a person skilled in the art depending on a desired target gene and/or a target sequence in the target gene, and therefore is not limited to a specific sequence of a particular length.

### Example 1.2.2. Comparison of indel activity of engineered guide RNAs

An insertion or deletion (indel) may occur in a target gene or target nucleic acid due to nucleic acid cleavage. The indel is generated by non-homologous end joining (NHEJ), in which two sticky ends formed by a double-strand break and the like repeatedly come into frequent contact with each other to repair or restore double-strand breaks in DNA, resulting in partial insertion and/or deletion (insertion and deletion) of a nucleotide sequence at the NHEJ repair site. As a result, the nucleic acid editing, in which one or more bases are deleted and/or added, may occur in a target gene or target nucleic acid due to target nucleic acid cleavage caused by the gene editing system.

In this example, it was intended to identify that the engineered guide RNA causes superior target nucleic acid cleavage activity in the CWCas12f1 protein-based gene editing system, as compared with the canonical sgRNA. To this end, each of the modification site MS3 to MS5 in the canonical sgRNA was further subdivided into three sections (see FIG. 2A). By combining one or more of these modifications, engineered gRNAs (see Table 10 in Example 1.2.1) were produced, and the indel activity for each of them was tested. As target sequences for comparing the indel efficiency, two types of human endogenous DNA target sites comprising the PAM sequence recognized as the cleavage site by the CWCas12f1 protein were identified and used, and the specific nucleotide sequences are provided in Table 12 below.

**[Table 12]**

| Name | Target sequence (5'→3') | SEQ ID NO |
|---|---|---|
| Target-1 | [TTTG]CACACACACAGTGGGCTACC | 358 |
| Target-2 | [TTTG]CATCCCCAGGACACACACAC | 359 |

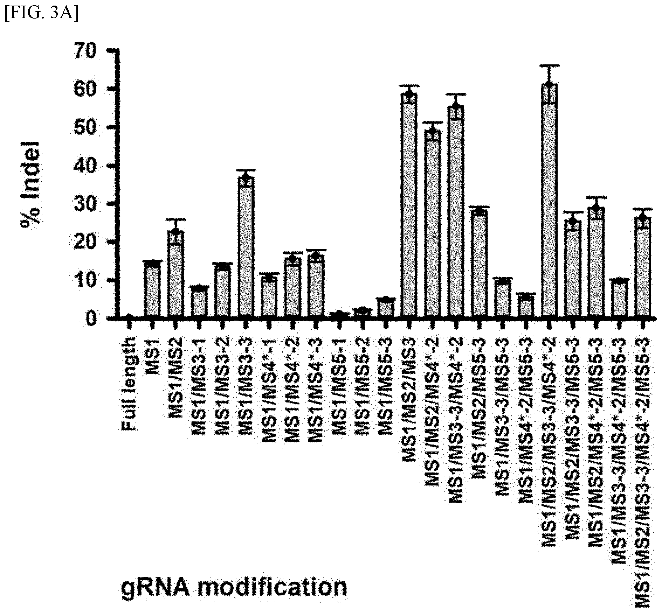

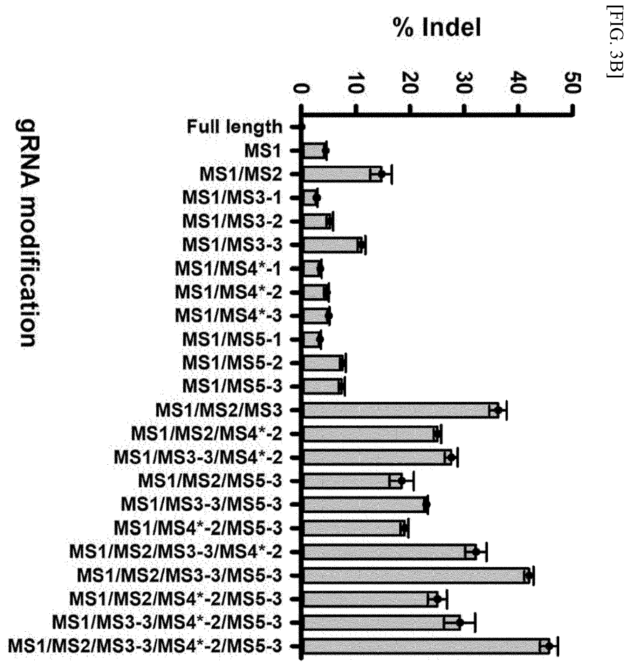

As a result, as shown in FIGS. 3A and 3B, the gene editing system comprising the canonical sgRNA (full length) and the wild-type CWCas12f1 protein did not cause cleavage of the target strand, but the engineered gRNA used in the test affected the indel efficiency of the CWCas12f1 protein for the target nucleic acid depending on its nucleotide sequence and target sequence.

Specifically, in the gene editing system comprising the CWCas12f1 protein for the target sequence 1 (Target-1; SEQ ID NO: 358), gRNAs to which MS1/MS2/MS3, MS1/MS2/MS4*-2, MS1/MS3-3/MS4*-2, and MS1/MS2/MS3-3/MS4*-2 modifications were applied exhibited high indel efficiency of about 50% to 65%, and gRNAs to which MS1/MS3-3, MS1/MS2/MS5-3, MS1/MS2/MS3-3/MS5-3, MS1/MS2/MS4*-2/MS5-3, and MS1/MS2/MS3-3/MS4*-2/MS5-3 modifications were applied exhibited indel efficiency of about 30% to 40% ['slash (/)' means 'and'; see FIG. 3A].

Next, in the gene editing system comprising the CWCas12f1 protein for the target sequence 2 (Target-2; SEQ ID NO: 359), gRNAs to which MS1/MS2/MS3, MS1/MS2/MS3-3/MS4*-2, MS1/MS2/MS3-3/MS5-3, and MS1/MS2/MS3-3/MS4*-2/MS5-3 modifications were applied exhibited indel efficiency of about 35% to 45%, and gRNAs to which MS1/MS2/MS4*-2, MS1/MS3-3/MS4*-2, MS1/MS2/MS5-3, MS1/MS3-3/MS5-3, MS1/MS4*-2/MSS-3, MS1/MS2/MS4*-2/MSS-3, and MS1/MS3-3/MS4*-2/MSS-3 modifications were applied exhibited indel efficiency of about 15% to 20%. (see FIG. 3B).

### Example 1.2.3. Comparison of indel activity of engineered RNA based on mature form sgRNA

Next, to obtain a highly efficient engineered single guide RNA for the CWCas12f1 protein, 5'-CUUCACUGAUAAAGUGGAGAACCGCUUCACCAAAAGCUGUCCCuuagGGGAUUA GAACUUGAGUGAAGGUGGGCUGCUUGCAUCAGCCUAAUGUCGAGAAGUGCUU UCUUCGGAAAGUAACCCUCGAAACAAAUUCAUUUgaaaGAAUGAAGGAAUGCAA CNNNNNNNNNNNNNNNNNNNN-3' (SEQ ID NO: 337), which is a mature form (hereinafter referred to as 'MF') sgRNA, and engineered gRNAs having partial modifications in the nucleotide sequence of the MF sgRNA were produced (see Table 11), and the indel efficiency of the CWCas12f1 gene editing system caused by them was measured.

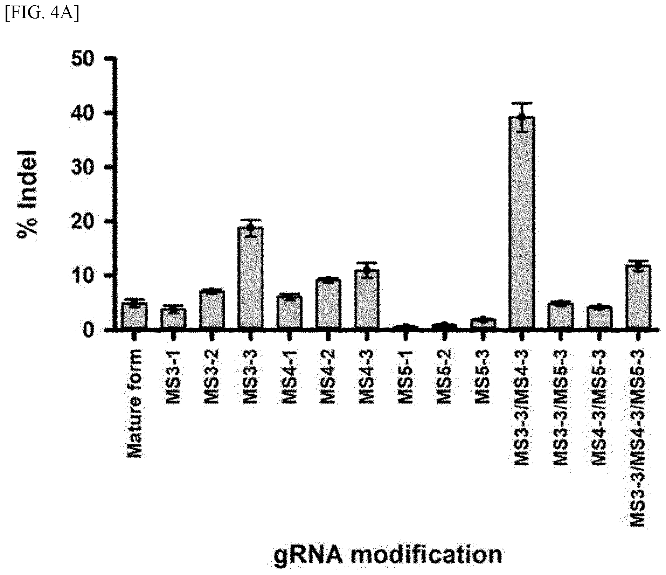

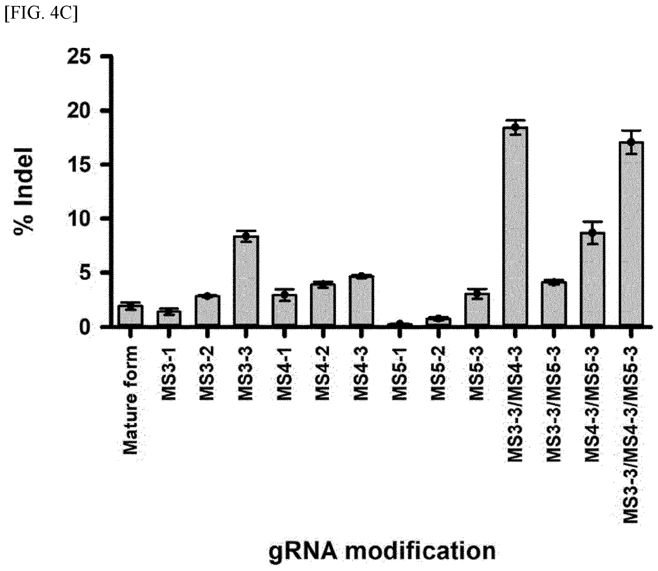

As a result, most of the thus obtained engineered gRNAs exhibited improved indel efficiency compared to the canonical sgRNA, and in particular, the gRNA with MS3-3/MS4-3 modifications (SEQ ID NO: 347) exhibited indel efficiency of about 40% and about 20% in Target-1 (SEQ ID NO: 358) and Target-2 (SEQ ID NO: 359), respectively (see FIGS. 4A and 4C).

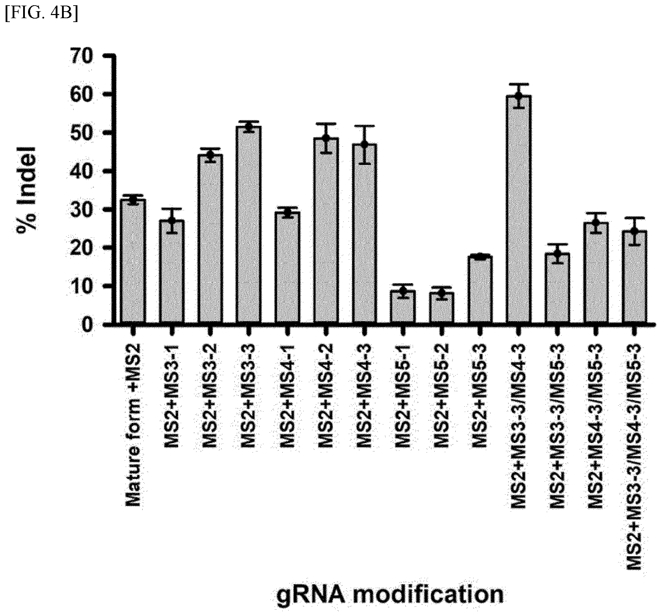

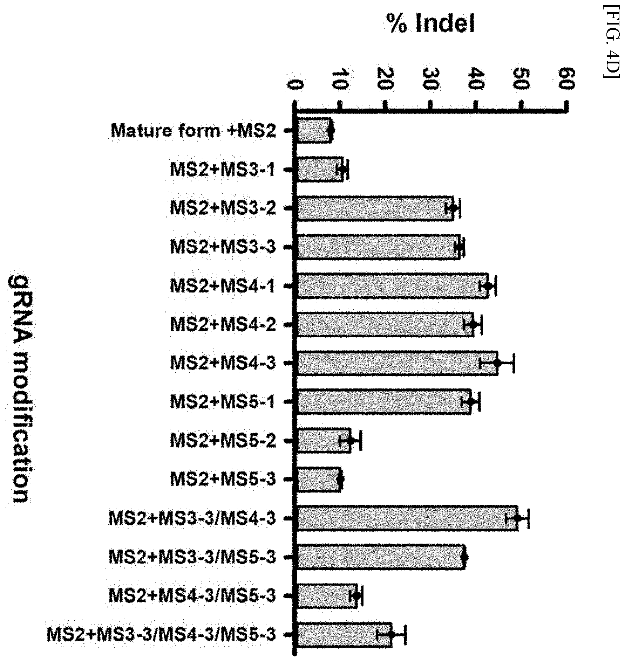

In addition, in a case where a U-rich tail (U₄AU₄) was added to the 3'-end (MS2) of each of the engineered gRNAs (SEQ ID NOs: 338 to 350, Table 11) having partial modifications in the nucleotide sequence of the MF sgRNA, such gRNAs exhibited higher indel efficiency than the MF sgRNA (see FIGS. 4B and 4D). Even when a U-rich tail (U₄AU₄) was added to the 3'-end of the gRNA(SEQ ID NO: 347) to which the MS3-3/MS4-3 modifications were applied, the indel efficiency was about 60% and about 50% in Target-1 and Target-2, respectively, confirming that indel efficiency was significantly increased when the modification at MS2 was further combined (FIGS. 4B and 4D).

Taken together, the results of the Examples 1.2.2 and 1.2.3 lead to the conclusion that as compared with a case where almost no nucleic acid cleavage activity is observed when the gene editing system based on Cas12f1 or a variant thereof of the present disclosure comprises a canonical guide RNA, significantly increased cleavage activity for the target gene or target nucleic acid is achieved by a modification where a U-rich tail is added to the 3'-end (MS2) of the engineered gRNA having a modification, in which at least one or more nucleotide sequences are deleted or substituted, or the canonical sgRNA nucleotide sequence.

Based on the experimental results, the gRNA to which MS1/MS2/MS3 modifications have been applied (Cas12f1 ver3.0; SEQ ID NO: 315), the gRNA to which MS2/MS3/MS4 modifications have been applied (Cas12f1 ver4.0; SEQ ID NO: 316), or the gRNA to which MS2/MS3/MS4/MS5 modifications have applied (Cas12f1 ver4.1; SEQ ID NO: 317) was used in the following examples to analyze USH2A gene editing efficiency depending on each guide sequence.

### Example 2. Selection of guide sequence for USH2A gene editing

### Example 2.1. Selection of protospacer sequence for designing guide sequence

As described above, type 2 (more specifically, type 2A) Usher syndrome can be effectively treated by deleting exon 13 of the USH2A gene (for example, exon 13 skipping). Accordingly, to achieve skipping of exon 13 of the USH2A gene including the c.2276G>T mutation and/or the c.2299delG mutation, target regions, which may compri se target sequences, were set as a region 3600 bp upstream of and a region 14440 bp downstream of exon 13, respectively, and protospacer sequences were selected for the entire double strand of USH2A DNA in the corresponding regions. For convenience, the upstream region was referred to as the F region, which is an abbreviation for the front region, and the downstream region was referred to as the R region, which is an abbreviation for the rear region.

The selected protospacer sequences are presented in Table 13 below along with the PAM sequences. To easily distinguish each protospacer sequence, the protospacer sequences present in the F region are numbered with F, and the protospacer sequences present in the R region are numbered with R.

**[Table 13]**

| Region | No. | Name (Oligo) | PAM (TTTR) | Protospacer sequence (5'→3') | SEQ ID NO |
|---|---|---|---|---|---|
| F | 1 | PS-USH2A-F02 | TTTG | TGTCTCGTCTATCTTGAATG | 397 |
| F | 2 | PS-USH2A-F03 | TTTG | TGTTCGTATCATCTGCAGTA | 398 |
| F | 3 | PS-USH2A-F05 | TTTG | AGAGTAAGATTGGCCCCCTA | 399 |
| F | 4 | PS-USH2A-F06 | TTTA | ATTTAGCTTTAATATACAAC | 400 |
| F | 5 | PS-USH2A-F07 | TTTA | ATATACAACTGTTTGCGATG | 401 |
| F | 6 | PS-USH2A-F09 | TTTG | TTAAAGAGAAAAAGAGCTCC | 402 |
| F | 7 | PS-USH2A-F10 | TTTA | AATGAGCACATTTGTTAAAA | 403 |
| F | 8 | PS-USH2A-F12 | TTTA | ATAAAAGGTTAAGCTGAGTA | 404 |
| F | 9 | PS-USH2A-F13 | TTTA | TACTCAGCTTAACCTTTTATTA | 405 |
| F | 10 | PS-USH2A-F15 | TTTG | GGGTGAGAACATTTAAGATC | 406 |
| F | 11 | PS-USH2A-F16 | TTTA | TAATGTGTACATATATCAAA | 407 |
| F | 12 | PS-USH2A-F17 | TTTA | ATTATACCTTCGTGAAGCTG | 408 |
| F | 13 | PS-USH2A-F22 | TTTA | TATTACTTCTATTTAAAGGA | 409 |
| F | 14 | PS-USH2A-F23 | TTTA | GAATAACCTTACTTGTCAGA | 410 |
| F | 15 | PS-USH2A-F24 | TTTA | TTTCTTTGCCTTGTAATACC | 411 |
| F | 16 | PS-USH2A-F25 | TTTG | CATCTAAACATTTACTATTC | 412 |
| F | 17 | PS-USH2A-F26 | TTTA | GATGCAAAATACTTCCTTTA | 413 |
| F | 18 | PS-USH2A-F27 | TTTG | GAAACTATCTAAAGGAAGTA | 414 |
| F | 19 | PS-USH2A-F30 | TTTA | AATTGCAATTATAGCTTGAA | 415 |
| F | 20 | PS-USH2A-FA01 | TTTA | AAAGGTGAGGATGGGAAAATG | 416 |
| F | 21 | PS-USH2A-FA02 | TTTA | TGAAGTTCATCGCAAACAGTTG | 417 |
| F | 22 | PS-USH2A-FA03 | TTTA | ATTATTACTTAATGCAAAGA | 418 |
| F | 23 | PS-USH2A-FA04 | TTTG | CATTAAGTAATAATTAAAAA | 419 |
| F | 24 | PS-USH2A-FA05 | TTTA | AAATTATAGTAGAATTACATA | 420 |
| F | 25 | PS-USH2A-FA06 | TTTA | AGATCTAATCTCTTAGCAA | 421 |
| F | 26 | PS-USH2A-FA07 | TTTG | ATATATGTACACATTATAAA | 422 |
| F | 27 | PS-USH2A-FA08 | TTTA | TGGCAGACAACATGATGTTTTG | 423 |
| F | 28 | PS-USH2A-FA09 | TTTA | TTTAATTATACCTTCGTGAAG | 424 |
| F | 29 | PS-USH2A-FA10 | TTTA | ACAGTGATGAATGACTCACC | 425 |
| F | 30 | PS-USH2A-FA11 | TTTA | ATTTCAATAAGGAAAATAAA | 426 |
| F | 31 | PS-USH2A-FA12 | TTTA | TCCATATATATACATATATA | 427 |
| F | 32 | PS-USH2A-FA13 | TTTA | GGAAATGCTTTTCTACATATG | 428 |
| F | 33 | PS-USH2A-FA14 | TTTA | TTTTCCTTATTGAAATTAAA | 429 |
| F | 34 | PS-USH2A-FA15 | TTTA | AAAATAACTGTATTGTTAAC | 430 |
| F | 35 | PS-USH2A-FA16 | TTTG | AACTATTAAAATTGGTCTAC | 431 |
| F | 36 | PS-USH2A-FA17 | TTTA | AAACTGATATGATATGAATC | 432 |
| F | 37 | PS-USH2A-FA18 | TTTA | ATAGTTCAAAATGAGTCATA | 433 |
| F | 38 | PS-USH2A-FA19 | TTTA | AAATTTGCAGCAATAGTGG | 434 |
| F | 39 | PS-USH2A-FA20 | TTTG | AATATGCCATACATATTCC | 435 |
| F | 40 | PS-USH2A-FA21 | TTTA | AAAATATGCACAGTGCTTA | 436 |
| F | 41 | PS-USH2A-FA22 | TTTA | AAAATTAATCTTAAAATAAG | 437 |
| F | 42 | PS-USH2A-FA23 | TTTA | AATTCATGGATATTTGGAAA | 438 |
| F | 43 | PS-USH2A-FA24 | TTTA | TCACCTAAACTTAAATCTCTG | 439 |
| F | 44 | PS-USH2A-FA25 | TTTA | AAGAGGTATGTTCTGAGTCA | 440 |
| F | 45 | PS-USH2A-FA26 | TTTA | TATTAATTGAAAATGATAAA | 441 |
| F | 46 | PS-USH2A-FA27 | TTTG | TATGCTCCTCTATTTTATCA | 442 |
| F | 47 | PS-USH2A-FA28 | TTTA | AGTAATTAATATAAATAAAA | 443 |
| F | 48 | PS-USH2A-FA29 | TTTG | TGAAAACAGCATATACACTTA | 444 |
| F | 49 | PS-USH2A-FA30 | TTTA | GATAGTTTCCAAATATCCATG | 445 |
| R | 1 | PS-USH2A-R01 | TTTG | TTAACTTAGGTAACTTCTCC | 446 |
| R | 2 | PS-USH2A-R02 | TTTG | GAAATAAAATTTGTAGAAGC | 447 |
| R | 3 | PS-USH2A-R04 | TTTA | GATAAAACTCAGCCGATCGG | 448 |
| R | 4 | PS-USH2A-R05 | TTTA | CTTCAAGTGTAGAAATTGAG | 449 |
| R | 5 | PS-USH2A-R07 | TTTG | AGTTGAATATCCATACAATG | 450 |
| R | 6 | PS-USH2A-R08 | TTTG | GCATTGTATGGATATTCAAC | 451 |
| R | 7 | PS-USH2A-R09 | TTTA | TTCAGAGATTTAGTTCATCA | 452 |
| R | 8 | PS-USH2A-R10 | TTTA | TATAGAAATACCTAGAATTG | 453 |
| R | 9 | PS-USH2A-R11 | TTTG | ATGGCATGTGGAAACAATTC | 454 |
| R | 10 | PS-USH2A-R13 | TTTA | TTAATTTGATGGCATGTGGA | 455 |
| R | 11 | PS-USH2A-R14 | TTTG | TATATAATGAGATTAAACAG | 456 |
| R | 12 | PS-USH2A-R17 | TTTA | GTTAAAATCTTAAATGTAAG | 457 |
| R | 13 | PS-USH2A-R18 | TTTA | TAGACCCATATAACTCAGAG | 458 |
| R | 14 | PS-USH2A-R19 | TTTG | CTTGCCAGAGAAGGAGTAGA | 459 |
| R | 15 | PS-USH2A-R20 | TTTG | TTCTACTCCTTCTCTGGCAA | 460 |
| R | 16 | PS-USH2A-R22 | TTTG | GCATTGGTCAGTGTGTAAGA | 461 |
| R | 17 | PS-USH2A-R23 | TTTA | ATACTTACATCACAAAAAGA | 462 |
| R | 18 | PS-USH2A-R24 | TTTA | TGGAGTATCTAGTTATAATA | 463 |
| R | 19 | PS-USH2A-R26 | TTTA | TAATTCTACCACCAGCCACA | 464 |
| R | 20 | PS-USH2A-R27 | TTTA | TTAGCTACCTCTTAGTTATA | 465 |
| R | 21 | PS-USH2A-R29 | TTTG | TTTGGTTGGTTACCTCTGAG | 466 |
| R | 22 | PS-USH2A-R30 | TTTG | GTTGGTTACCTCTGAGCCAA | 467 |
| R | 23 | PS-USH2A-R31 | TTTA | TAAGTACGTGACACCCCTGG | 468 |
| R | 24 | PS-USH2A-R32 | TTTA | GGGACCATTTCATCAGGTAG | 469 |
| R | 25 | PS-USH2A-R34 | TTTA | CCTTCAGGTTAATCCTTTCA | 470 |
| R | 26 | PS-USH2A-R35 | TTTG | GCAACAAAGTCCTTTGTCTC | 471 |
| R | 27 | PS-USH2A-R36 | TTTG | ACTGTGTAGGAGACAAAGGA | 472 |
| R | 28 | PS-USH2A-R38 | TTTG | TACACCTACCAGATATCTAA | 473 |
| R | 29 | PS-USH2A-R39 | TTTA | ATCTACACCATGCATAAGAC | 474 |
| R | 30 | PS-USH2A-R40 | TTTA | CTTAGAAAGAAGGATGTATA | 475 |

The guide sequence (or spacer sequence) of the guide RNA was designed based on the selected protospacer sequence. The guide sequence is a sequence that binds complementarily to a target sequence, and such a guide sequence can be designed using the protospacer sequence. Since the protospacer sequence is a sequence complementary to the target sequence, correlation between the target sequence and the protospacer sequence is similar to correlation between the target sequence and the guide sequence. Due to these characteristics, in general, a guide sequence may be designed using a protospacer sequence. That is, a guide sequence that binds complementarily to a target sequence may be designed as a nucleotide sequence having basically the same nucleotide sequence as the protospacer sequence. Here, the guide sequence is designed by replacing T with U in the protospacer sequence. The guide sequence was designed using the selected protospacer sequence. Specific sequence information of the guide sequence, and the like, are presented in Tables 4 and 5 of the section "2.2. Spacer region comprising guide sequence."

### Example 2.2. Optimization of combination of guide sequence and scaffold sequence of guide RNA

A nucleotide sequence encoding the guide RNA designed based on the protospacer sequence selected in Example 2.1 was constructed into an expression cassette, which was immediately transfected to confirm indel efficiency by next generation sequencing (NGS) without the T7E1 assay. Here, the guide RNA was designed to include a U-rich tail sequence (for example, 5'-U₄AU₆-3') at the 3'-end.

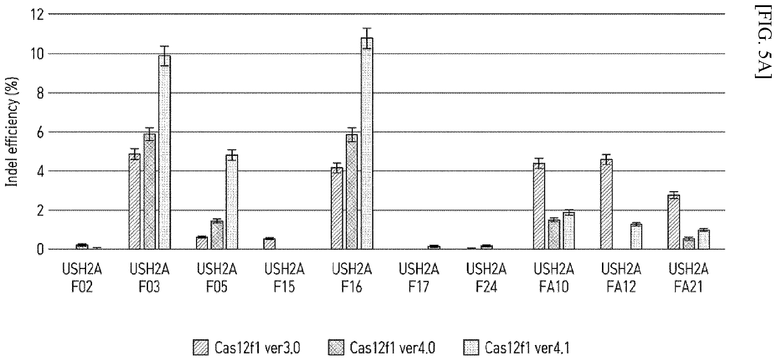

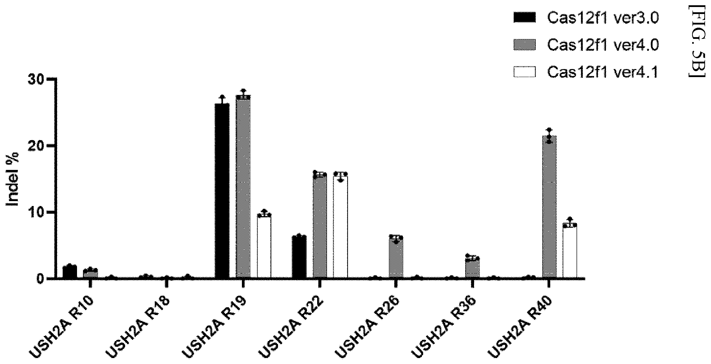

As a result, the indel efficiency was relatively lower in the F region compared to the R region, and it was confirmed that the indel efficiency of the Cas12f1 ver4.0 (SEQ ID NO: 316) cassette, which is an improved version of the Cas12f1 ver3.0 (SEQ ID NO: 315) cassette, was improved in most samples (see FIGS. 5A and 5B, and Table 14).

In addition, the Cas12f1 ver4.1 gRNA (SEQ ID NO: 317), which is an improved version of Cas12f1 ver4.0 gRNA, was produced and indel efficiency thereof was identified. As a result, for the target sequences located in the F region, ver4.1 showed higher indel efficiency than ver4.0; however, in the R region, on the contrary, ver4.0 showed higher indel efficiency than ver4.1 (see FIGS. 5A and 5B, and Table 14).

**[Table 14]**

| Name | Guide sequence | SEQ ID NO | Indel efficiency (%) | | |
|---|---|---|---|---|---|
| | | | Cas12f1 ver3.0 | Cas12f1 ver4.0 | Cas12f1 ver4.1 |
| GUIDE-USH2A-F02 | UGUCUCGUCUAUCUUGAAUG | 80 | - | 0.23 | 0.03 |
| GUIDE-USH2A-F03 | UGUUCGUAUCAUCUGCAGUA | 81 | 4.88 | 5.89 | 9.89 |
| GUIDE-USH2A-F05 | AGAGUAAGAUUGGCCCCCUA | 82 | 0.64 | 1.48 | 4.83 |
| GUIDE-USH2A-F 15 | GGGUGAGAACAUUUAAGAUC | 89 | 0.55 | - | - |
| GUIDE-USH2A-F16 | UAAUGUGUACAUAUAUCAAA | 90 | 4.19 | 5.87 | 10.78 |
| GUIDE-USH2A-F17 | AUUAUACCUUCGUGAAGCUG | 91 | 0.02 | 0.02 | 0.18 |
| GUIDE-USH2A-F24 | UUUCUUUGCCUUGUAAUACC | 94 | 0.04 | 0.19 | - |
| GUIDE-USH2A-FA10 | ACAGUGAUGAAUGACUCACC | 108 | 4.41 | 1.52 | 1.89 |
| GUIDE-USH2A-FA12 | UCCAUAUAUAUACAUAUAUA | 110 | 4.61 | - | 1.29 |
| GUIDE-USH2A-FA20 | AAUAUGCCAUACAUAUUCC | 118 | 2.78 | 0.58 | 1.03 |
| GUIDE-USH2A-R10 | UAUAGAAAUACCUAGAAUUG | 136 | 1.65 | 1.20 | - |
| GUIDE-USH2A-R18 | UAGACCCAUAUAACUCAGAG | 141 | 0.46 | - | - |
| GUIDE-USH2A-R19 | CUUGCCAGAGAAGGAGUAGA | 142 | 26.08 | 27.24 | 9.48 |
| GUIDE-USH2A-R22 | GCAUUGGUCAGUGUGUAAGA | 144 | 6.32 | 16.03 | 15.79 |
| GUIDE-USH2A-R26 | UAAUUCUACCACCAGCCACA | 147 | - | 6.41 | - |
| GUIDE-USH2A-R36 | ACUGUGUAGGAGACAAAGGA | 155 | - | 3.06 | - |
| GUIDE-USH2A-R40 | CUUAGAAAGAAGGAUGUAUA | 158 | - | 21.35 | 8.07 |

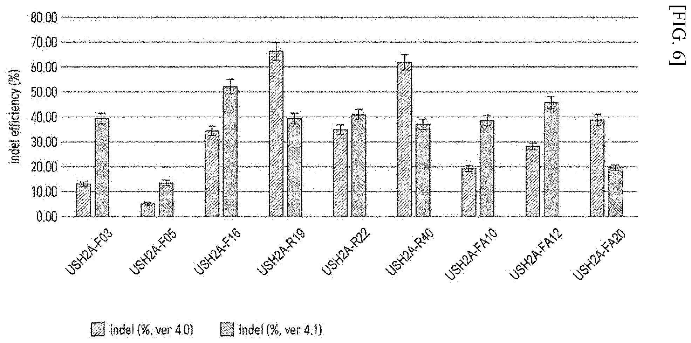

Based on the results of the indel efficiency experiment for the Cas12f1 ver4.0 cassette and Cas12f1 ver4.1 cassette, Cas12f1 ver. 4.1 was selected as the scaffold sequence for the guide sequences GUIDE-USH2A-F03, -F16, and -FA12, and Cas12f1 ver4.0 was selected as the scaffold sequence for the guide sequences GUIDE-USH2A-R19 and -R40 (FIGS. 5A and 5B, and Table 14). Using the thus selected guide sequences, indel efficiency was measured again. As a result, GUIDE-USH2A-F16 and -FA12 were finally selected as the guide sequences for Cas12f1 ver4.1, and GUIDE-USH2A-R19 and -R40 were selected as the guide sequences for Cas12f1 ver4.0 (see FIG. 6).

### Example 3. Confirmation of deletion of exon 13 region in USH2A gene

Using combinations of F16, FA12, and R19, R40, which are guide RNA sets that showed high indel efficiency in Example 2, deletion of exon 13 region in the USH2A gene was checked.

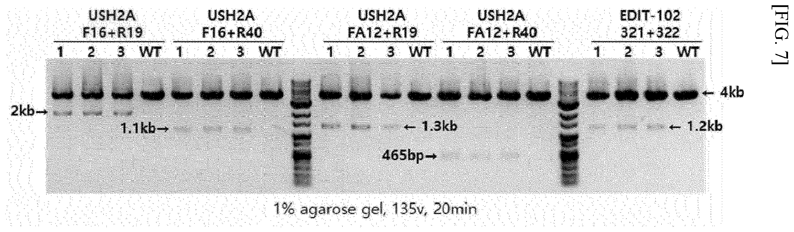

As a result, deletion bands were observed in all combinations of guide sequences, as shown in FIG. 7. Specifically, the deletion bands were observed at 2004 bp for the combination of F16 and R19, at 1167 bp for the combination of F16 and R40, at 1302 bp for the combination of FA12 and R19, and at 465 bp for the combination of FA12 and R40. Taking the results of the main band amplification and the deletion bands together, it was confirmed that the combination of F16 and R19 and the combination of FA12 and R19 showed high deletion efficiency (FIG. 7).

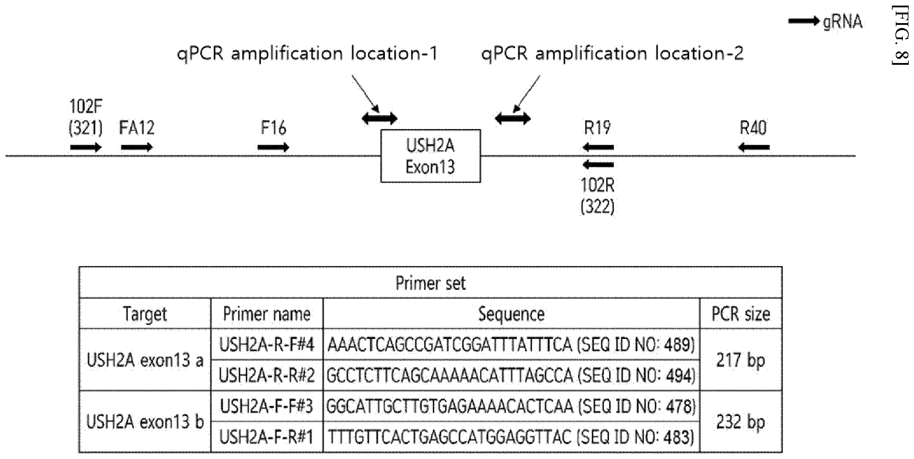

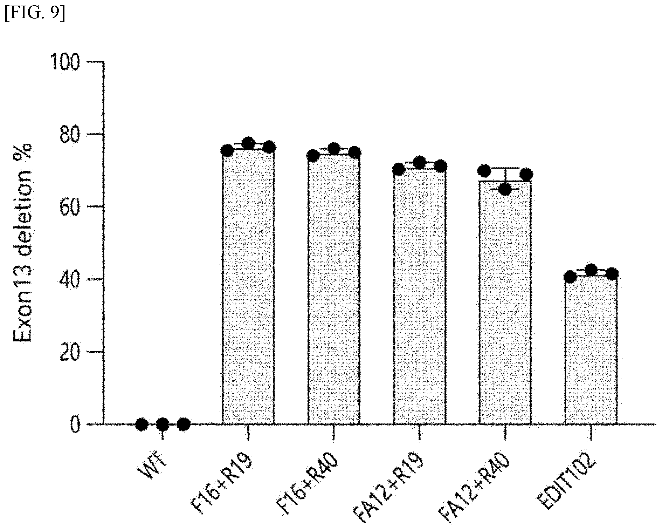

In addition, analysis was performed using qPCR to confirm the degree of deletion. The primer sequences and amplification positions used in the qPCR analysis are shown in FIG. 8. As a result of the analysis, it was confirmed that the combination of F16 and R19, the combination of F16 and R40, the combination of FA12 and R19, and the combination of FA12 and R40 all showed high deletion efficiency of over 60% (see FIG. 9).

In the following examples, optimization of the guide sequence was performed to further improve the deletion efficiency obtained so far.

### Example 4. Optimization of length of guide sequence to improve deletion efficiency

Each guide sequence (F16, FA12, R19, R40) selected through Examples 2 and 3 was modified to have a guide sequence of 19- to 25-mer length based on the PAM adjacent to the protospacer sequence, and vectors expressing the same were produced, respectively, to compare the indel efficiency depending on a length of the guide sequence. As for the scaffold sequence, Cas12f1 ver4.1 was used for F16 and FA12, and Cas12f1 ver4.0 was used for R19 and R40, as selected in Example 2.2. Information on each guide sequence used in this experiment is presented in Table 15 below.

**[Table 15]**

| Name | Guide sequence(5'→3') | Length | SEQ ID NO |
|---|---|---|---|
| GUIDE-USH2A-F16 | UAAUGUGUACAUAUAUCAAA | 20 | 90 |
| GUIDE-USH2A-F16-21mer | UAAUGUGUACAUAUAUCAAAA | 21 | 159 |
| GUIDE-USH2A-F 16-22mer | UAAUGUGUACAUAUAUCAAAAC | 22 | 160 |
| GUIDE-USH2A-F16-23mer | UAAUGUGUACAUAUAUCAAAACA | 23 | 161 |
| GUIDE-USH2A-F 16-25mer | UAAUGUGUACAUAUAUCAAAACAUC | 25 | 162 |
| GUIDE-USH2A-FA12 | UCCAUAUAUAUACAUAUAUA | 20 | 110 |
| GUIDE-USH2A-FA12-23mer | UCCAUAUAUAUACAUAUAUAUUA | 23 | 163 |
| GUIDE-USH2A-FA12-25mer | UCCAUAUAUAUACAUAUAUAUUAUG | 25 | 164 |
| GUIDE-USH2A-R19-19mer | CUUGCCAGAGAAGGAGUAG | 19 | 165 |
| GUIDE-USH2A-R19 | CUUGCCAGAGAAGGAGUAGA | 20 | 142 |
| GUIDE-USH2A-R19-21mer | CUUGCCAGAGAAGGAGUAGAA | 21 | 166 |
| GUIDE-USH2A-R19-22mer | CUUGCCAGAGAAGGAGUAGAAC | 22 | 167 |
| GUIDE-USH2A-R19-23mer | CUUGCCAGAGAAGGAGUAGAACA | 23 | 168 |
| GUIDE-USH2A-R19-24mer | CUUGCCAGAGAAGGAGUAGAACAA | 24 | 169 |
| GUIDE-USH2A-R19-25mer | CUUGCCAGAGAAGGAGUAGAACAAA | 25 | 170 |
| GUIDE-USH2A-R40 | CUUAGAAAGAAGGAUGUAUA | 20 | 158 |
| GUIDE-USH2A-R40-21mer | CUUAGAAAGAAGGAUGUAUAA | 21 | 171 |
| GUIDE-USH2A-R40-22mer | CUUAGAAAGAAGGAUGUAUAAA | 22 | 172 |
| GUIDE-USH2A-R40-24mer | CUUAGAAAGAAGGAUGUAUAAAUC | 24 | 173 |
| GUIDE-USH2A-R40-25mer | CUUAGAAAGAAGGAUGUAUAAAUCA | 25 | 174 |

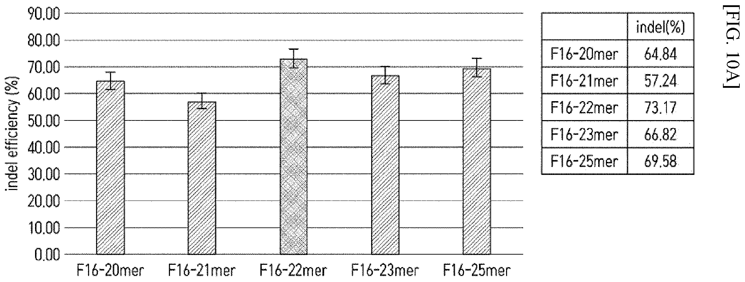

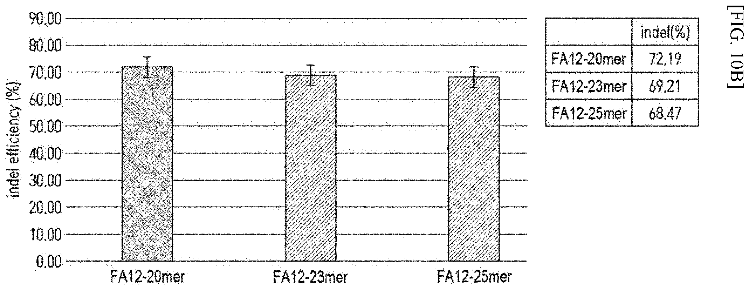

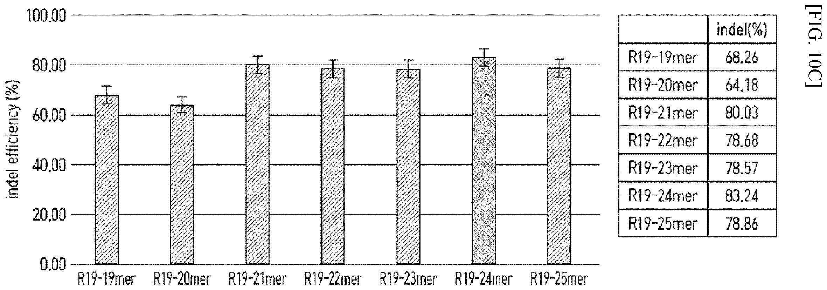

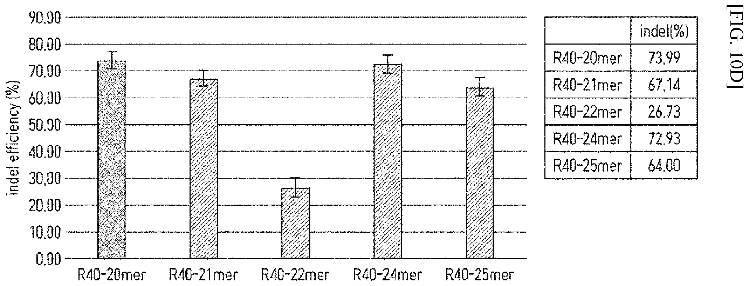

As a result, the guide sequence F16 showed the highest indel efficiency of 73% in 22mer (see FIG. 10A), and the guide sequence FA12 showed the highest indel efficiency of 72.19% in 20mer (see FIG. 10B). In addition, the guide sequence R19 showed the highest indel efficiency of 83% in 24mer (see FIG. 10C), and the guide sequence R40 showed the highest indel efficiency of 73.99% in 20mer (see FIG. 10D). Although indel efficiency of about 70% or more was observed throughout a length range of 19 to 25mer, since the combination with the shortest fragment length, which results in cleavage by deletion, is more efficient, the 22mer-length F16 (SEQ ID NO: 160) and the 24mer-length R19 (SEQ ID NO: 169) were finally selected as the guide sequences.

### Example 5. Optimization of U-rich tail sequence to improve indel efficiency

U-rich tail containing multiple uridine residues at the 3'-end of the guide RNA (for example, the 3'-end of the guide sequence) can contribute to stabilization of the guide RNA and improvement of indel efficiency. In order to compare the indel efficiency depending on a sequence of the U-rich tail, U₄AU₆ or U₆ was added as a U-rich tail to the 3'-end of the guide RNA, respectively, and indel efficiency thereof was evaluated. The results are disclosed in Table 16 below.

**[Table 16]**

| Sample name | Indel (%) | | | Average indel (%) |
|---|---|---|---|---|
| | #1 | #2 | #3 | |
| Cas12f1 ver4.1 USH2AF16 U4AU6 | 20.73 | 23.72 | 11.94 | 18.80 |
| Cas12f1 ver4.1 USH2AF16 U6 | 18.07 | 16.16 | 6.24 | 13.49 |
| Cas12f1 ver4.1 USH2AF12 U4AU6 | 9.03 | 14.74 | 11.41 | 11.73 |
| Cas12f1 ver4.1 USH2AF12 U6 | 10.71 | 10.55 | 4.6 | 8.62 |
| Cas12f1 ver4.0 USH2AF19 U4AU6 | 43.55 | 32.82 | 25.76 | 34.04 |
| Cas12f1 ver4.0 USH2AF19 U6 | 40.57 | 28.10 | 18.71 | 29.13 |
| Cas12f1 ver4.0 USH2AF40 U4AU6 | 23.43 | 22.67 | 13.79 | 19.97 |
| Cas12f1 ver4.0 USH2AF40 U6 | 24.78 | 20.42 | 12.14 | 19.11 |

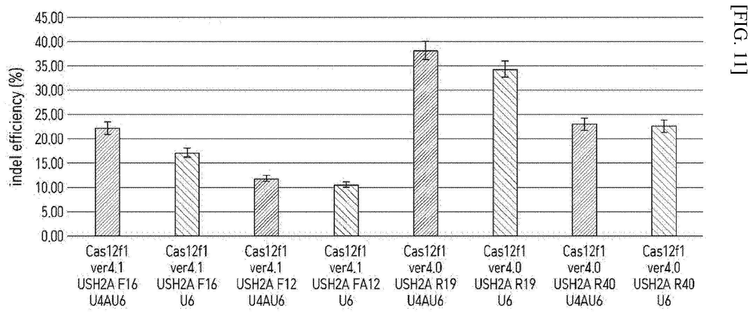

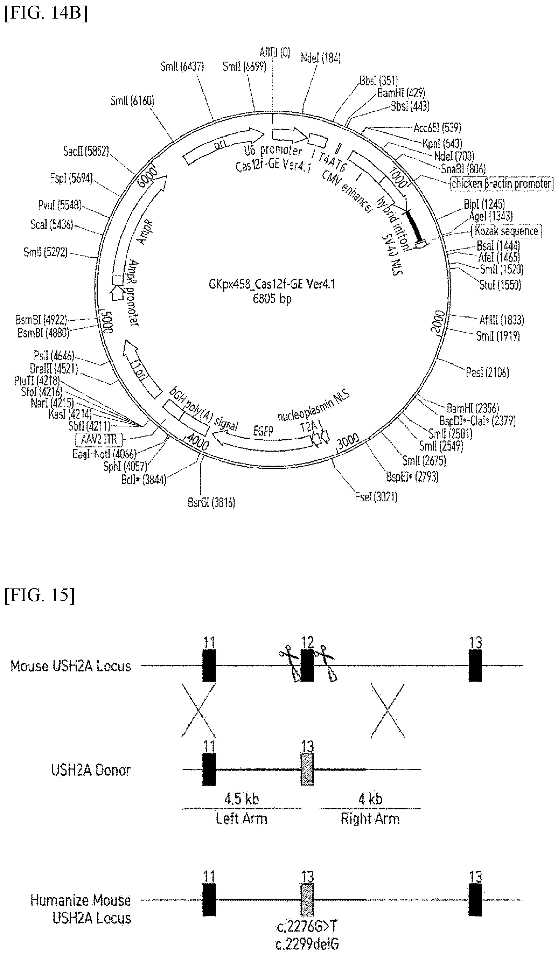

As a result of verifying the indel efficiency depending on a sequence of the U-rich tail, U₄AU₆ showed a significantly higher effect of improving the indel efficiency in all of F16, FA12, R19, and R40 (see FIGS. 11 and 15). According to the experimental results, U₄AU₆ was selected as a U-rich tail sequence.

### Example 6. Confirmation of deletion effect of exon 13 in USH2A gene mutant cell line

### Example 6.1. Confirmation of deletion effect in 661W-USH2A cell line

The effect of the USH2A gene editing system of the present disclosure was confirmed in the 661W-USH2A cell line. The cell line is a USH2A humanized 661W cell line in which intron 12, exon 13, and intron 13 (a part thereof) of the wt661W USH2A locus have been replaced with intron 12, exon 13 (including c.2276G>T and c.2299delG mutations) and intron 13 (a part thereof) of the human USH2A gene. The production method therefor is schematically illustrated in FIG. 15. In order to delete the mutated regions in the cell, two guide RNAs targeting the target sequences in the F region and the R region were used in combination, respectively. Specifically, for the guide sequences, a combination of F16 and R19 and a combination of F16 and R40 were used. EDIT102, which is a therapeutic agent for type 2 Usher syndrome from Editas, was used as a positive control. The nucleotide sequences of the guide sequences included in EDIT102 is as follows:
321 guide sequence, 5'-GAAATTAAATGATATGCCTTAG-3'; 322 guide sequence, 5'-GTGTGATTTGCTTGCCAGAGA-3' .

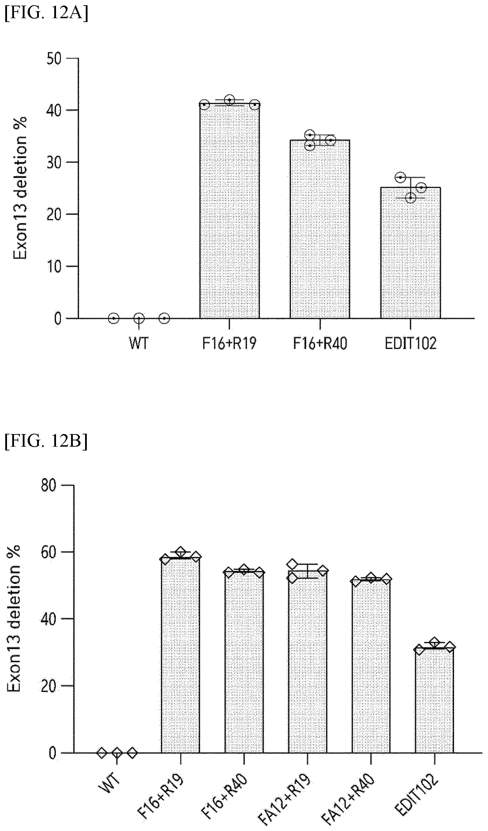

As a result, a large deletion effect of more than 30% was confirmed for both the combination of F16 and R19 and the combination of F16 and R40, and such a deletion effect was particularly high as compared with the positive control EDIT102 (see FIG. 12A).

### Example 6.2. Confirmation of deletion effect in ARPE19/HPV16-USH2A cell line

The effect of the USH2A gene editing system of the present disclosure was confirmed in ARPE19/HPV16-USH2A cell line. The cell line is such that exon 13 of the wtARPE19/HPV19 USH2A gene locus has been converted to have c.2276G>T and c.2299delG mutations. To delete the mutated regions in the cell, two guide RNAs targeting the target sequences in the F region and the R region were used in combination, respectively. Specifically, for the guide sequences, a combination of F16 and R19, a combination of F16 and R40, a combination of FA12 and R19, and a combination of FA12 and R40 were used. EDIT102 was used as a positive control.

As a result, a large deletion effect of more than 50% was identified for all four types of guide sequence combination, and such a deletion effect was particularly high as compared with the positive control EDIT102 (see FIG. 12B).

### Example 7. Confirmation of indel effect at target sites in vivo

For the guide sequences whose indel and/or deletion effects were identified in the above-described examples, it was identified whether a significant level of indel efficiency was observed even in a case where they are systemically injected into actual animals. DNAs encoding the guide RNAs, which comprise F16 (SEQ ID NO: 90), FA12 (SEQ ID NO: 110), R10 (SEQ ID NO: 136), and R22 (SEQ ID NO: 144), respectively, as guide sequences, and the Cas12f1 molecule were packaged into adeno-associated virus (AAV; Serotype 5) to produce AAVs expressing the USH2A gene editing system.

Specifically, three vectors, including a vector containing a nucleotide sequence encoding each guide RNA and Cas12f1 protein, a pHelper vector required for AAV virus production, and a REP/CAP vector, were transfected into HEK293T cells to produce AAV, and AAV particles were obtained through iodixanol gradient purification. The produced AAV was injected into the tail vein of mice at a dose of 5 × 10¹⁰ VG/g, and then liver tissues were extracted therefrom at intervals of 4, 6, and 12 weeks to analyze the editing efficiency for the target gene.

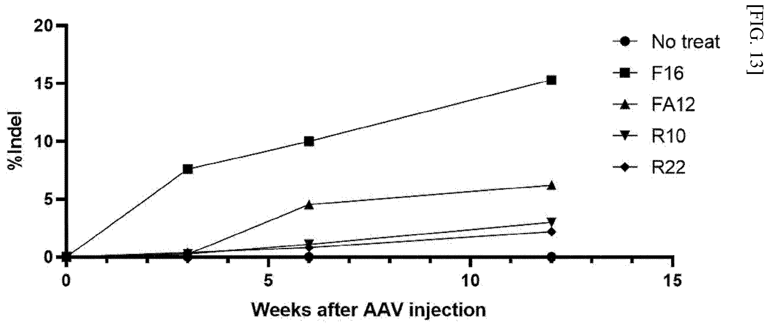

As a result, it was determined that the USH2A gene editing system of the present disclosure, consisting of the guide RNA comprising the selected guide sequence and the Cas12f1 protein, exhibited significant indel activity *in vivo,* as shown in FIG. 13.

### Conclusion

As demonstrated in the examples, the USH2A gene editing system of the present disclosure, which comprises two guide RNAs with optimized guide sequences and Cas12f1 or a variant protein thereof that recognizes a target sequence, can recognize target sequences present in the upstream and downstream regions of exon 13 in the USH2A gene, cleave the same, and delete exon 13 (that is, exon skipping), thereby inducing the production of Usherin protein capable of performing a normal function. This highly efficient exon 13 deletion effect was achieved by engineering of the scaffold region of the guide RNA, which enhances gene editing efficiency, and the optimized guide sequence. Furthermore, due to its miniaturized site, even in a case of further comprising a configuration such as shRNA that can enhance deletion efficiency, efficient delivery and expression *in vivo* can be achieved with a delivery vehicle such as AAV

### Experimental Methods and Materials

### Experimental Example 1. Expression and purification of Cas12f1 protein

The gene produced in Example 1.1 was expressed and the protein was purified by the following method. First, the nucleic acid construct was cloned into the pMAL-c2 plasmid vector and transformed into BL21 (DE3) *E. coli* cells. The transformed *E. coli* colonies were grown in LB broth at 37°C until the optical density reached 0.7. The transformed E. *coli* cells were cultured overnight at 18°C in the presence of 0.1 mM isopropylthio-β-D-galactoside. Thereafter, the cultured cells were collected by centrifugation at 3,500 g for 30 minutes, and the collected cells were resuspended in a buffer containing 20 mM Tris-HCl (pH 7.6), 500 mM NaCl, 5 mM β-mercaptoethanol, and 5% glycerol. The cells were lysed in a lysis buffer and then disrupted by sonication. The sample containing the disrupted cells was centrifuged at 15,000 g for 30 minutes, and the supernatant obtained was filtered through a 0.45 µm syringe filter (Millipore). The filtered supernatant was loaded onto a Ni²⁺-affinity column using an FPLC purification system (KTA Purifier, GE Healthcare). The bound fractions were eluted with a gradient of 80-400 mM imidazole, 20 mM Tris-HCl (pH 7.5).

The eluted proteins were cleaved by treatment with TEV protease for 16 hours. The cleaved proteins were purified on a heparin column with a linear gradient of 0.15-1.6 M NaCl. The recombinant Cas12f1 protein purified on the heparin column was dialyzed against a solution of 20 mM Tris pH 7.6, 150 mM NaCl, 5 mM β-mercaptoethanol, and 5% glycerol. The dialyzed protein was purified by passing it through an MBP column, and then repurified on a monoS column (GE Healthcare) or EnrichS with a linear gradient of 0.5-1.2 M NaCl.

The repurified proteins were collected and dialyzed against a solution of 20 mM Tris pH 7.6, 150 mM NaCl, 5 mM β-mercaptoethanol, and 5% glycerol to purify the gene editing protein (endonuclease) used in the present disclosure. The concentration of the produced gene editing protein was quantified by the Bradford quantitative method using bovine serum albumin (BSA) as a standard and measured electrophoretically on a coomassie blue-stained SDS-PAGE gel.

### Experimental Example 2. Preparation of guide RNA

The guide RNA (gRNA) and engineered gRNA used in Example 1.2 were prepared by the following method. First, the gRNA or engineered gRNA was chemically synthesized from the pre-designed gRNA for its production, and then a PCR amplicon containing the synthesized gRNA sequence and the T7 promoter sequence was prepared. The ligation of the U-rich tail to the 3'-end of the engineered gRNA was performed using Pfu PCR Master Mix (Biofact) in the presence of a sequence-modified primer and a gRNA plasmid vector. The PCR amplicon was purified using the HiGene^{™}Gel & PCR Purification System (Biofact).

Modification of the second stem region and the tracrRNA-crRNA complementarity region in the scaffold sequence of the engineered gRNA was performed by cloning synthetic oligonucleotides (Macrogen), which deliver the modified sequences, into the gRNA encoding vector linearized using ApoI and BamHI restriction enzymes.

In addition, modification of the first stem region in the scaffold sequence of the engineered gRNA was performed by PCR amplification of the canonical or engineered template plasmid vector using a forward primer targeting the 5'-end portion of tracrRNA and a reverse primer targeting the U6 promoter region. The PCR amplification was performed by Q5 Hot Start high-fidelity DNA polymerase (NEB), and the PCR products were ligated using KLD Enzyme Mix (NEB). The ligated PCR products were transformed into DH5α *E. coli.* The mutagenesis was confirmed by Sanger sequencing analysis.

The modified plasmid vectors were purified using the NucleoBond^{®} Xtra Midi EF kit (MN). 1 µg of the purified plasmid was used as a template for mRNA synthesis using T7 RNA polymerase (NEB) and NTPs (Jena Bioscience). The engineered gRNA was purified against the above-prepared Cas12f1 protein using the Monarch^{®} RNA cleanup kit (NEB), aliquoted into cryogenic vials, and stored in liquid nitrogen.

Next, amplicons of the canonical gRNA and engineered gRNA were prepared. For this purpose, the template DNA plasmids of the canonical gRNA and the engineered gRNA were subjected to PCR amplification using a U6-complementary forward primer and a reverse primer complementary to the protospacer sequence using KAPA HiFi HotStart DNA polymerase (Roche) or Pfu DNA polymerase (Biofact). The PCR amplification products were purified using the Higene^{™}Gel & PCR purification system (Biofact), and the amplicons of the canonical gRNA and engineered gRNA were obtained.

Using the PCR amplicon as a template, *in vitro* transcription was performed using NEB T7 polymerase. The *in vitro* transcription product was treated with DNase I (NEB), and then purified using the Monarch RNA Cleanup Kit (NEB) to obtain gRNA. Thereafter, a plasmid vector containing the pre-designed gRNA sequence and T7 promoter sequence was prepared according to the T-blunt plasmid (Biofact) cloning method.

After the guide RNA sequence comprising the T7 promoter sequence in the vector was purified by double cutting both ends of the guide RNA, and the resultant product was subjected to *in vitro* transcription using T7 polymerase (NEB). The *in vitro* transcription product was treated with DNase I (NEB), and purified using the Monarch RNA Cleanup Kit (NEB) to obtain gRNA.

### Experimental Example 3. Production of Ribonucleoprotein Particle (RNP)

The gene editing system of the present disclosure may be a ribonucleoprotein (RNP) formed by interaction between one gene editing protein (endonuclease) and a guide RNA (gRNA) or a RNP formed by interaction between two gene editing proteins and gRNA.

For the production of a RNP, the gene editing protein purified by the method of Experimental Example 1 and the gRNA or engineered gRNA prepared by the method of Experimental Example 2 were co-incubated at concentrations of 300 nM and 900 nM, respectively, for 10 minutes at room temperature to obtain ribonucleoprotein particles (RNPs).

### Experimental Example 4. Design and production of vector for gRNA engineering

The gene editing proteins CWCas12f1, Un1Cas12f1, and variant proteins thereof were human codon-optimized for expression in human cells, and oligonucleotides of the codon-optimized Cas12f1 genes were produced.

In addition, an oligonucleotide comprising the nucleotide sequence of the produced Cas12f1 gene while comprising a nuclear localization signal (NLS) sequence and a linker sequence at each of the 5'-end and 3'-end, respectively, was synthesized (Bionics), and a polynucleotide of a human codon-optimized Cas12f1 or Cas12f1 variant nucleic acid construct for cleavage of a target gene or target nucleic acid was synthesized. The polynucleotide of the codon-optimized Cas12f1 nucleic acid construct was operably linked to a plasmid comprising a sequence encoding eGFP linked to a chicken β-actin (CBA) promoter and a self-cleavage T2A peptide (2A), and then cloned.

In addition, template DNA for the canonical guide RNA used in this experiment was synthesized (Twist Bioscience) and cloned into the pTwist Amp plasmid vector for replication. Template DNA for the engineered guide RNA was produced using the enzyme cloning technique and cloned into the pTwist Amp plasmid for replication.

Using the plasmid as a template, a U6-complementary forward primer and a protospacer sequence complementary reverse primer were used to prepare the amplicon of the canonical guide RNA or engineered guide RNA. As needed, the prepared amplicon was cloned into a T-blunt plasmid (Biofact) for replication.

In addition, to prepare the engineered dual guide RNA, oligonucleotides encoding the engineered tracrRNA and engineered crRNA were digested with restriction enzymes BamHI and HindIII (NEB) and cloned into the pSilencer 2.0 vector (ThermoFisher Scientific).

Engineered gRNAs showing relatively high efficiency for Cas12f1 were selected and named "Cas12f1 ver3.0," "Cas12f1 ver4.0," and "Cas12f1 ver4.1," respectively. Template DNAs encoding them were synthesized and cloned into the pTwist Amp plasmid vector (Twist Bioscience). As needed, the vector was used as a template for amplification of the gRNA encoding sequence using a U6-complementary forward primer and a protospacer-complementary reverse primer.

The vector expressing components of the gene editing system of the present disclosure was prepared by cloning a polynucleotide encoding the wild-type Cas12f1 gRNA or the engineered gRNA into the vector comprising the human codon-optimized Cas12f1 gene or a nucleic acid construct comprising the same using the Gibson assembly method.

Specifically, as a vector expressing the gene editing system, an adeno-associated virus (AAV) inverted terminal repeat plasmid vector was produced in which 1) a sequence encoding eGFP linked to a chicken β-actin (CBA) promoter and a self-cleaving T2A peptide (2A), 2) a polynucleotide of the human codon-optimized nucleic acid construct encoding the Cas12f1 protein or the engineered Cas12f1 protein, and 3) the gRNA for the wild-type Cas12f1 or the engineered gRNA of the present disclosure are operably linked to each other.

Here, the transcription of the nucleic acid construct encoding the Cas12f1 variant protein or a homologous protein thereof and the guide RNA was promoted by the CBA and U6 promoters, respectively. In addition, the AAV plasmid vector (AAV vector) may be appropriately altered in terms of eGFP, the number of engineered gRNAs, and/or addition of effector proteins, and the like, depending on the purpose of gene editing or modification.

For mass production of the AAV vector, the AAV vector and helper plasmid were transduced into HEK 293T cells. The transduced HEK293 T cells were cultured in DMEM medium containing 2% FBS. Recombinant pseudo-typed AAV vector stocks were generated using PEI coprecipitation with PEIpro (Polyplus-transfection) and triple-transfection of plasmids at equal molar ratios. After 72 hours of incubation, the cells were lysed, and the AAV vectors were purified from the lysate by iodixanol step gradient ultra-centrifugation.

### Experimental Example 5. Cell transfection

Cell transfection for comparison of indel activity of the guide RNAs engineered in Example 1 was performed as follows.

HEK293T (ATCC CRL-11268), HeLa (ATCC CLL-2), U-2 OS (ATCC HTB-96), and K-562 (ATCC CCL-243) cells were cultured in DMEM medium supplemented with 10% heat-inactivated FBS, 1% penicillin/streptomycin, and 0.1 mM nonessential amino acids under a 5% CO₂ condition at 37°C.

For cell transfection of a nucleic acid construct for cleavage of a target gene or target nucleic acid, a vector comprising the same, or DNA encoding the engineered guide RNA, 1.0 × 10⁵ HEK293T cells were seeded 1 day before transfection. Cell transfection was performed by electroporation or lipofection. In a case of electroporation, 2-5 µg each of the nucleic acid construct, the plasmid vector comprising the same, or DNA encoding the engineered guide RNA was transfected into 4 × 10⁵ HEK293T cells using the Neon transfection system (Invitrogen). Electroporation was performed under the conditions of 1300 V, 10 mA, and 3 pulses. In a case of lipofection, 6-15 µl FuGene reagent (Promega) was mixed with 2-5 µg of the plasmid vector encoding Cas12f1 or a variant protein thereof and 1.5-5 µg of PCR amplicon for 15 minutes. The mixture (300 µl) was added to 1.5 ml DMEM medium plated with 1 × 10⁶ cells 1 day before transfection. The cells were cultured in the presence of the mixture for 1 to 10 days and then harvested. Genomic DNA of the cells was isolated manually using the PureHelix^{™} genomic DNA preparation kit (NanoHelix) or using the Maxwell RSC Cultured cells DNA Kit (Promega).

For cell transfection of the AAV vector comprising the nucleotide sequence encoding the gene editing protein, human HEK293T cells were transfected with the AAV vector at different multiplicity of infection (MOI) of 1, 5, 10, 50 and 100 as determined by quantitative PCR. The transfected HEK293T cells were cultured in DMEM medium containing 2% FBS. The cells were harvested for isolation of genomic DNA at different time points, for example, on day 1, day 3, day 5, and day 7.

In addition, the ribonucleoprotein (RNP) particles produced according to Experimental Example 3 were transfected into cells using electroporation or lipofection, and 1 day later, the engineered guide RNA was transfected into cells using electroporation.

Meanwhile, the comparative experiment of indel efficiency of the engineered Cas12f1 proteins in Examples 1.2.2 and 1.2.3 was performed as follows.

One day before transfection, HEK293T cells, which had been grown in 24-well plates to 80-90% confluency (based on 100cp dishes), were diluted 1/100 and passaged to prepare a volume of 500 µl. A total of 2 µg of DNA (vector + sgRNA transcription cassette targeting DY10) was used per transfection well. The experiment was repeated twice for each group. A transfection mixture was prepared which contains 1.5 µg of plasmid encoding the wild-type Cas12f1 or the engineered Cas12f1, 0.5 µg of the sgRNA transcription cassette, 200 µl of DMEM (excluding FBS and antibiotics), and 6 µl of FuGENE (Promega) reagent.

DNA and DMEM containing FuGENE reagent were mixed and vortexed, and then incubated for 15 minutes. 200 µl of the thus prepared transfection mixture was used to treat the cells prepared in a 24-well plate, and incubated at 37°C. After 72 hours, the supernatant was removed and cell lysis was performed.

### Experimental Example 6. Analysis of cleavage efficiency for nucleic acid

To analyze cleavage efficiency of the gene editing system for the target gene or target nucleic acid, the region comprising the protospacer in the genomic DNA isolated from HEK293T cells was subjected to PCR using target-specific primers in the presence of KAPA HiFi HotStart DNA polymerase (Roche). The amplification method was performed according to the manufacturer's instructions. The PCR amplicon, which is the amplification product comprising Illumina TruSeq HT dual indexes, was subjected to 150-bp pair-end sequencing using Illumina iSeq 100.

Indel frequency was calculated using MAUND provided at ^{┌}https://github.com/ibs-cge/maund_{┘}.

The PCR products were obtained using BioFACT^{™} Lamp Pfu DNA polymerase. The PCR products (100-300 µg) were allowed to react with 10 units of T7E1 enzyme (NEB) in a 25 µg reaction mixture at 37°C for 30 minutes. 20 µl of the reaction mixture was directly loaded onto a 10% acrylamide gel, and the cleaved PCR products were run in a TBE buffer system. The gel image was stained with ethidium bromide solution and digitized using a Printgraph 2 M gel imaging system (Atto). The digitized result was analyzed to evaluate the gene editing efficiency.

### Experimental Example 7. Analysis of cleavage activity for nucleic acid in cell

Analysis of the cleavage activity of the gene editing system for the target site of the target gene or target nucleic acid in a cell was performed as follows.

The adeno-associated virus (AAV) vector produced by the method according to Experimental Example 4 was transfected into HEK293T cells. After 3, 5, and 7 days, genomic DNA was obtained from the transfected HEK293T cells and purified using a Genomic DNA prep kit (Cat No.: 69504, QIAGEN). The target site of the target gene or target nucleic acid was amplified from the purified products by PCR, and the final PCR products were analyzed using targeted deep sequencing. For library generation, the target site was amplified using the KAPA HiFi HotStart PCR kit (Cat No.: KK2501, KAPA Biosystem). The library was sequenced using MiniSeq of the TruSeq HT Dual Index system (Illumina).

### Experimental Example 8. Extraction of genomic DNA

Extraction of gDNA was performed using a Genomic DNA Prep Kit (GCBL200, Nanohelix). The medium of the transfected cells in the 24-well was removed, 200 µl of trypsin was added to the well to detach the cells from the bottom, and then placed in a 1.5 ml tube. The centrifugation was performed at 300 × g for 5 minutes, and the supernatant was removed. 300 µl of NGD1 buffer and 2 µl of RNase A (50 mg/ml) were added to the tube, vortexing was performed for 1 minute. 8 µl of Proteinase K (10 mg/ml) was added thereto, and then the reaction was allowed to occur at 60°C for 10 minutes. Then, it was cooled on ice for 5 minutes. 300 µl of NPS buffer was added thereto, and then thorough mixing was performed. The mixture was incubated on ice for 5 minutes, and centrifuged at 12,000 rpm for 5 minutes. Next, the column was prepared according to the number of samples, 100 µl of MaxBinder solution was added thereto, and centrifugation was performed at 12,000 rpm for 30 seconds. The centrifuged supernatant was collected and placed in a new column, and centrifugation was performed at 12,000 rpm for 1 minute. The filtered solution was discarded. 500 µl of 80% ethanol was added to the column, centrifugation was performed at 10,000 rpm for 30 seconds, and the filtered solution was discarded. After washing with 80% ethanol twice, centrifugation was performed at 13,000 rpm for 3 minutes. The column was placed into a new 1.5 ml tube, 30 µl of EB solution was added dropwise to the center, the reaction was allowed to occur for 1 minute, and centrifugation was performed at 12,000 rpm for 2 minutes. The eluted gDNA was quantified and stored at 4°C.

### Experimental Example 9. PCR and gel purification

This experiment was performed using the GEL & PCR Purification System (GP104-200, Biofact). UB buffer was added to the PCR product in an amount equivalent to 3 times the volume of the PCR product and thorough mixing was performed. Then, isopropanol was added thereto in an amount equivalent to 2 times the volume of the PCR product and thorough mixing was performed. In a case of the gel, the gel of the corresponding band was cut and weighed. Then, UB buffer was added thereto in an amount equivalent to 3 times the weight of the gel. The gel was dissolved by incubation at 65°C for 10 minutes, and then isopropanol was added thereto in an amount equivalent to 1 time the gel volume and thorough mixing was performed. The column was prepared, 200 µl of HelpB buffer was added to the column, and centrifugation was performed at 13,000 rpm for 30 seconds. Then, the filtered solution was discarded. The reaction solution was added to the column, and centrifugation was performed at 7,000 rpm for 1 minute. Then, the filtered solution was discarded. 750 µl of 80% EtOH was added thereto, and centrifugation was performed at 13,000 rpm for 30 seconds. Then, the filtered solution was discarded. After repeating the process twice, centrifugation was performed at 13,000 rpm for 3 minutes. The centrifuged column was placed in a 1.5 ml tube, 30 µl of EB buffer was added dropwise to the center, and the reaction was allowed to occur at room temperature for 1 minute. Centrifugation was performed at 13,000 rpm for 1 minute. The DNA collected in the 1.5 ml tube was quantified and stored at 4°C.

### Experimental Example 10. Preparation of DNA cassette

To confirm indel efficiency of the guide sequences of Cas12f1, a cassette containing the U6 promoter, scaffold sequence, guide sequence, and U-rich tail sequence (T₄AT₆) was amplified by PCR and used. The process was performed as follows.

### 1) Selection of spacer

The spacer was selected from the 20mer sequence followed by TTTA or TTTG, which are PAM of Cas12f1, and spacers whose sequences end with T were excluded. In addition, to minimize off-target effects, the spacers were designed using CRISPR RGEN TOOL by classifying them with less than 2 mismatches. In addition, the reverse complement sequence comprising a DR (direct repeat) and U-rich sequence was used as an R primer.

### 2) PCR

The PCR was performed under the composition and condition shown in Table 17 below.

**[Table 17]**

| Reagent composition | | | PCR condition | | |
|---|---|---|---|---|---|
| 2x pfu PCR Master mix | 200 µl | | 95°C | 5 min | - |
| hU6 F primer | 20 µl | | 95°C | 20 s | 35 cycles |
| Target oligo(R) | 20 µl | | 58°C | 40 s | |
| Template | 1 µl (400 ng) | | 72°C | 45 s | |
| Distilled water | 159 µl | | 72°C | 5 min | - |
| Total | 400 µl | | - | | |

### 3) Gel analysis

1% agarose gel was prepared, and the size marker and PCR products were added to the gel. Electrophoresis was performed to confirm the amplified size.

### 4) Purification and quantification

After confirming the amplified size, the gel was purified according to Experimental Example 9 to quantify the PCR products.

### Experimental Example 11. Construction of vector for dual guide RNA

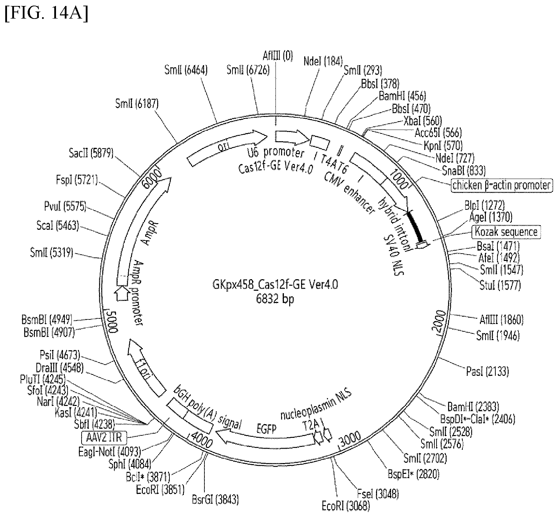

To produce Cas12f1 dual gRNA vector, Cas12f1 ver4.0-GFP vector (FIG. 14A) or Cas12f1 ver4.1-GFP (FIG. 14B) was used as a backbone vector, and the procedure was carried out as follows. The restriction enzyme ends of the vector to be cloned were confirmed, and dual gRNA oligos suitable for cloning into the Bbs I restriction enzyme site of the vector were designed and custom-made. The custom-made oligos were each diluted to a concentration of 100 pmol. 4.5 µl each of the diluted forward and reverse primers were placed into a PCR tube, and 1 µl of 10X annealing buffer was added thereto to adjust the total volume to 10 µl. Then, annealing was performed under the conditions of 95 °C for 5 minutes and -1 °C/min from 95 °C to 4 °C.

Cas12f1 ver4.0 orver4.1 dual gRNA vector was prepared and incubated at 500 rpm, 37°C for 2 hours under the digestion conditions in Table 18 below.

**[Table 18]**

| Reagent | Volume |
|---|---|
| NEB 10X 3.1 buffer | 5 *µ*ℓ |
| Cas12f1 ver4.0 or ver4.1 vector | 5 *µ*g |
| BbsI | 5 *µ*ℓ |
| Distilled water | Amount to make total volume of 50 *µ*ℓ |
| Total | 50 *µ*ℓ |

After digestion, the digested vector was obtained through electrophoresis and gel elution. Ligation was performed using the digested vector and annealed oligo (see Table 19 below for ligation conditions).

**[Table 19]**

| Reagent | Volume |
|---|---|
| 2X Rapid ligation buffer | 10 *µ*ℓ |
| Annealed oligos | 6 *µ*g |
| Vector digested with BbsI | 2 *µ*ℓ |
| T4 Ligase | 2 *µ*ℓ |
| Total | 20 *µ*ℓ |

After ligation, transformation was performed on DH5α. After incubation on an LB plate, positive colonies were confirmed through colony PCR and then incubated in 3 ml LB medium. After miniprep, sequencing was performed to confirm whether the final sequences matched.

**Experimental Example 12. DH5α transformation**

The vector produced in Experimental Example 11 was transformed into *E. coli* to produce the vector. DH5α competent cells were taken out and thawed on ice. The ligated vector was added up to 1/10 of the amount of DH5α, and the incubation was performed on ice for 30 minutes. After heat shock at 42°C for 30 s, cooling was performed on ice for 2 minutes. Incubation was performed using 100 µl of LB medium or S.O.C medium at 37°C for 1 hour. The cells were spread on LB plates warmed to room temperature (containing ampicillin or kanamycin depending on the vector) and incubated at 37°C for 14 to 16 hours.

### Experimental Example 13. Collection of plasmid vector

For transfection or Sanger sequencing, the vector-transformed DH5α was used. Plasmid Mini prep kit (PM105-200, Biofact) was used according to the manufacturer's instructions. The culture medium of the vector-transformed DH5α was placed in a 1.5 ml tube, and centrifuged at 13,000 rpm for 5 minutes. After centrifugation, the supernatant was discarded, and the pellet was sufficiently dispersed by vortexing. 350 µl of B1 buffer was added thereto, and the tube was shaken to ensure sufficient reaction. Next, 350 µl of A1 buffer containing RNase A was added thereto, and the tube was inverted until the blue color disappeared. Then, centrifugation was performed at 13,000 rpm for 5 minutes. The column was prepared, 200 µl of HelpB buffer was added thereto, and the solution was centrifuged at 13,000 rpm for 30 seconds. Then, the filtered solution was removed. 750 µl of the centrifuged supernatant was added to the prepared column, centrifugation was performed at 7,000 rpm for 1 minute, and the filtered solution was discarded. 750 µl of 80% EtOH was added thereto, centrifugation was performed at 13,000 rpm for 30 s, and the filtered solution was discarded. This process was repeated twice. After repeating the process twice, centrifugation was performed at 13,000 rpm for 3 minutes. The centrifuged column was placed into a 1.5 ml tube, 30 µl of EB buffer was added dropwise to the center, and the reaction was allowed to occur at room temperature for 1 minute. Centrifugation was performed at 13,000 rpm for 1 minute, and the plasmid vectors collected in the 1.5 ml tube were quantified and stored at -20°C.

### Experimental Example 14. Production of humanized cell line with USH2A exon 13 mutation

To confirm gene deletion effects of the USH2A gene editing system according to an example of the present disclosure, 661W-USH2A cell line or ARPE19/HPV16-USH2A cell line having a mutation in the USH2A gene was produced.

The 661W-USH2A cell line is a USH2A humanized 661W cell line in which intron 12, exon 13, and part of intron 13 of the wt661W USH2A locus have been replaced with intron 12, exon 13 (including c.2276G>T and c.2299delG mutations) and intron 13 (a part thereof) of the human USH2A gene by the inducing homology directed repair (HDR) method (see FIG. 15).

The ARPE19/HPV16-USH2A cell line is a cell line produced to include c.2276G>T and c.2299delG mutations in exon 13 of the wtARPE19/HPV19 USH2A locus by the HDR method.

### Experimental Example 15. Cell culture

For use in the experiments, HEK293T cells were cultured in DMEM medium containing 10% FBS and 1% penicillin-streptomycin, and AREP-19/HPV-16 cells were cultured in DEME/F12 medium containing 10% FBS and 1% penicillin-streptomycin. When the cell confluency reached 80% or more, HEK293T cells were passaged at a ratio of 1/15, and AREP-19/HPV-16 cells were passaged at a ratio of 1/4.

### Experimental Example 16. Transfection (HEK293T and ARPE19-HPV cells)

The day before transfection, HEK293T and ARPE19-HPV cells (80% confluency) cultured in 100 mm dishes were treated with trypsin to detach from the bottom of the dish. The detached cells were placed in 50 ml of pre-warmed medium (see Experimental Example 15 for its composition) and slowly dissolved with a pipette. 24-well plates were prepared according to the number of samples and repetitions, and 500 µl of cell suspension medium was added to each well (1/100 dilution). Then, incubation was performed overnight in a CO₂ incubator at 37°C until transfection.

The next day, when the cell confluency reached approximately 70% to 80%, 200 µl of the 500 µl medium per well was removed and the plates were placed in the incubator. 1.5 ml tubes were prepared according to the number of samples, and 200 µl of Opti-MEM was added to each tube. 1.5 µg of Cas12f1 DNA and 0.5 µg of gRNA (or a vector containing nucleic acids encoding Cas12f1 DNA and two gRNAs) were added to the tube containing Opti-MEM, and vortexed for 5 seconds (nucleic acid mixture). Then, the nucleic acid mixture and FuGENE HD were added at a ratio of 1:3, and reaction was allowed to occur at room temperature for 20 minutes (that is, in a case where the nucleic acid mixture was 2 µg, 6 µl of FuGENE HD was administered). The 24-well plate was taken out from the incubator, and 200 µl of the solution containing the nucleic acid mixture and FuGENE HD was gently added along the well wall. After shaking the plate sufficiently in an S shape, it was incubated in a CO₂ incubator at 37°C for 72 hours. After 72 hours, the cells were harvested and gDNA was extracted therefrom according to Experimental Example 8.

### Experimental Example 17. Next generation sequencing (NGS)

NGS analysis to confirm indel efficiency for the target was performed over a total of three PCR rounds.

The conditions for conducting the first PCR for each region are disclosed in Tables 20 to 29 below.

**[Table 20]**

| Fa region of USH2A (F01 to F 10) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-F-F#2 (10 pmol) | 0.5 *µ*ℓ | 0.5 µM | 98°C | 20 s | 32 cycles |
| USH2A-F-R#1 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 21]**

| Fb region of USH2A (F 11 to F20) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-F-F#2 (10 pmol) | 0.5 *µ*ℓ | 0.5 µM | 98°C | 20 s | 32 cycles |
| USH2A-F-R#1 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 22]**

| Fc region of USH2A (F21 to F30) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-F-F#7 (10 pmol) | 0.5 *µ*ℓ | 0.5 µM | 98°C | 20 s | 32 cycles |
| USH2A-F-R#8 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 23]**

| Ra region of USH2A (R01 to R06) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-R-F#1 (10 pmol) | 0.5 *µ*ℓ | 0.5 µM | 98°C | 20 s | 32 cycles |
| USH2A-R-R#2 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 24]**

| Rb region of USH2A (R07 to R14) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-R-F#4 (10 pmol) | 0.5 *µ*ℓ | 0.5 µM | 98°C | 20 s | 32 cycles |
| USH2A-R-R#3 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 25]**

| Rc region of USH2A (R15 to R20) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-R-F#3 (10 pmol) | 0.5 *µ*ℓ | 0.5 µM | 98°C | 20 s | 32 cycles |
| USH2A-R-R#1 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 26]**

| Rd region of USH2A (R21 to R30) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | |
| USH2A-R-F#8 (10 pmol) | 0.5 *µ*ℓ | 0.5 µM | 98°C | 20 s | 32 cycles |
| USH2A-R-R#8 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 27]**

| Re region of USH2A (R31 to R36, R40) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-R-F#9 (10 pmol) | 0.5 *µ*ℓ | 0.5 µM | 98°C | 20 s | 32 cycles |
| USH2A-R-R#9 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

**[Table 28]**

| Rf region of USH2A (R37 to R39) | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| USH2A-R-F#10 (10 pmol) | 0.5 *µ*ℓ | 0.5 µM | 98°C | 20 s | 32 cycles |
| USH2A-R-R#10 (10 pmol) | 0.5 *µ*ℓ | 0.5 µM | 61°C | 15 s | |
| Template (gDNA) | 1 *µ*ℓ | - | 72°C | 45 s | |
| (Each sample) | (100 ng) | - | 72°C | 5 min | - |
| Distilled water | up to 10 *µ*ℓ | - | - | | |
| Total | 10 *µ*ℓ | - | | | |

The first PCR resulted in a band of approximately 450 to 500 bp, and the second PCR was performed using this PCR product as a template. The conditions for performing the second PCR are disclosed in Table 29 below.

**[Table 29]**

| USH2A 2nd PCR | | | PCR condition | | |
|---|---|---|---|---|---|
| PCR mixture composition | Volume | Concentration | | | |
| 2X KAPA HiFi PCR mix | 5 *µ*ℓ | 1X | 95°C | 5 min | - |
| Forward primer (10 pmol) | 0.5 *µ*ℓ | 0.5 µM | 98°C | 20 s | 33 cycles |
| Reverse primer (10 pmol) | 0.5 *µ*ℓ | 0.5 µM | 60°C | 15 s | |
| Template (1st PCR product) | 1 *µ*ℓ | - | 72°C | 30 s | |
| Distilled water | *3 µ*ℓ | - | 72°C | 3 min | - |
| Total | 10 *µ*ℓ | - | | - | |

After the second PCR, the sample was loaded onto a 2% agarose gel to confirm whether the band was properly displayed within 250 bp. If the band was not properly displayed, the cause was determined. Then, the process was restarted from the first PCR. If the correct band was confirmed, the third PCR was performed using the second PCR product as a template. Here, if the concentration of the second PCR product was high, distilled water was added to adjust the concentration. The conditions for the third PCR are disclosed in Table 30 below.

**[Table 30]**

| USH2A 3rd PCR | | | PCR condition | | |
|---|---|---|---|---|---|
| PCR mixture composition | Volume | Concentration | | | |
| 2X pfu PCR Master mix | 5 *µ*ℓ | 1X | 95°C | 5 min | - |
| Forward primer (10 pmol) | 0.5 *µ*ℓ | 0.5 µM | 95°C | 20 s | 33 cycles |
| Reverse primer (10 pmol) | 0.5 *µ*ℓ | 0.5 µM | 60°C | 40 s | |
| Template (2nd PCR product) | 1 *µ*ℓ | - | 72°C | 45 s | |
| Distilled water | *3 µ*ℓ | - | 72°C | 3 min | |
| Total | 10 *µ*ℓ | - | - | | |

The primers used in each PCR are disclosed in Table 31 below.

**[Table 31]**

| Purpose | Target | No. | Name | Direction | Sequence (5'→3') | SEQ ID NO |
|---|---|---|---|---|---|---|
| 1 st PCR | USH2A F | 1 | USH2A-F-F#1 | F | AGGATTAAACCAAAA ATTGCCCTGGA | 476 |
| 1 st PCR | USH2A F | 2 | USH2A-F-F#2 | F | CACCATGCTGTACAAT AGAGCTCCAG | 477 |
| 1 st PCR | USH2A F | 3 | USH2A-F-F#3 | F | GGCATTGCTTGTGAG AAAACACTCAA | 478 |
| 1 st PCR | USH2A F | 4 | USH2A-F-F#4 | F | AGAGCTCCAGCATAT GTAACAGAAACA | 479 |
| 1 st PCR | USH2A F | 5 | USH2A-F-F#7 | F | TGCCTTAGGTGAGTC ATTCATCACTG | 480 |
| 1 st PCR | USH2A F | 6 | USH2A-F-F#8 | F | AGAACTTGCCTTCATT GGAGTTCTTGAA | 481 |
| 1 st PCR | USH2A F | 7 | USH2A-F-F#10 | F | TGAGTTCCTGAGTATG TTTTTGACTC | 482 |
| 1 st PCR | USH2A F | 8 | USH2A-F-R#1 | R | TTTGTTCACTGAGCC ATGGAGGTTAC | 483 |
| 1 st PCR | USH2A F | 9 | USH2A-F-R#3 | R | TGTTTCTGTTACATAT GCTGGAGCTC | 484 |
| 1 st PCR | USH2A F | 10 | USH2A-F-R#4 | R | AATTTGTTCACTGAG CCATGGAGGTT | 485 |
| 1 st PCR | USH2A F | 11 | USH2A-F-R#8 | R | TCCAGGGCAATTTTT GGTTTAATCCT | 486 |
| 1 st PCR | USH2A R | 12 | USH2A-R-F#1 | F | GAGTGTGATTCCTTG GGGACATTACC | 487 |
| 1 st PCR | USH2A R | 13 | USH2A-R-F#3 | F | TGGCTAAATGTTTTTG CTGAAGAGGC | 488 |
| 1 st PCR | USH2A R | 14 | USH2A-R-F#4 | F | AAACTCAGCCGATCG GATTTATTTCA | 489 |
| 1 st PCR | USH2A R | 15 | USH2A-R-F#8 | F | AGCAAAGAATCCAGC CTAGGATAATTGG | 490 |
| 1 st PCR | USH2A R | 16 | USH2A-R-F#9 | F | CCAGGGGTGTCACGT ACTTATAAAATGA | 491 |
| 1 st PCR | USH2A R | 17 | USH2A-R-F#10 | F | CAAAGTCCTTTGTCT CCTACACAGTCAA | 492 |
| 1 st PCR | USH2A R | 18 | USH2A-R-R#1 | R | TACACACTGACCAAT GCCAAAGGAAA | 493 |
| 1 st PCR | USH2A R | 19 | USH2A-R-R#2 | R | GCCTCTTCAGCAAAA ACATTTAGCCA | 494 |
| 1 st PCR | USH2A R | 20 | USH2A-R-R#3 | R | ATTGGCTGACAGGAC AACAATTAGCA | 495 |
| 1 st PCR | USH2A R | 21 | USH2A-R-R#8 | R | TCTTCCTGTCTTCTGG GATACTTACCAC | 496 |
| 1 st PCR | USH2A R | 22 | USH2A-R-R#9 | R | GGACCAAAGGAAC AAATGTTTGTAACT | 497 |
| 1 st PCR | USH2A R | 23 | USH2A-R-R#10 | R | GAGAGCCACAAAGAT AAAGGAAAGAGCA | 498 |
| 1 st PCR | USH2A R | 24 | USH2A-R-R#11 | R | TTCTAATTCCTGAGTC CTGACTGCAG | 499 |
| 2nd PCR | USH2A F | 1 | USH2A-F-F#1 miseq F | F | CACTCTTTCCCTACAC GACGCTCTTCCGATCT AGGATTAAACCAAAA ATTGCCCTGGA | 500 |
| 2nd PCR | USH2A F | 2 | USH2A-F-F#2 miseq F | F | CACTCTTTCCCTACAC GACGCTCTTCCGATCT CACCATGCTGTACAAT AGAGCTCCAG | 501 |
| 2nd PCR | USH2A F | 3 | USH2A-F-F#3 miseq F | F | CACTCTTTCCCTACAC GACGCTCTTCCGATCT | 502 |
| | | | | | GGCATTGCTTGTGAG AAAACACTCAA | |
| 2nd PCR | USH2A F | 4 | USH2A-F-F#4 miseq F | F | CACTCTTTCCCTACAC GACGCTCTTCCGATCT AGAGCTCCAGCATAT GTAACAGAAACA | 503 |
| 2nd PCR | USH2A F | 5 | USH2A-F-F#5 miseq F | F | CACTCTTTCCCTACAC GACGCTCTTCCGATCT CTGAAACTTTGTACTC AGCTTAACCT | 504 |
| 2nd PCR | USH2A F | 6 | USH2A-F-F#6 miseq F | F | CACTCTTTCCCTACAC GACGCTCTTCCGATCT TTTTTCCCAGCTTCAC GAAGGTATAATT | 505 |
| 2nd PCR | USH2A F | 7 | USH2A-F-F#9 miseq F | F | CACTCTTTCCCTACAC GACGCTCTTCCGATCT CTTTGCCTTGTAATAC CCTTTTATC | 506 |
| 2nd PCR | USH2A F | 8 | USH2A-F-F#10 miseq F | F | CACTCTTTCCCTACAC GACGCTCTTCCGATCT TGAGTTCCTGAGTATG TTTTTGACTC | 507 |
| 2nd PCR | USH2A F | 9 | USH2A-F-R#3 miseq R | R | GTGACTGGAGTTCAG ACGTGTGCTCTTCCG ATCTTGTTTCTGTTAC ATATGCTGGAGCTC | 508 |
| 2nd PCR | USH2A F | 10 | USH2A-F-R#4 miseq R | R | GTGACTGGAGTTCAG ACGTGTGCTCTTCCG ATCTAATTTGTTCACT GAGCCATGGAGGTT | 509 |
| 2nd PCR | USH2A F | 11 | USH2A-F-R#5 miseq R | R | GTGACTGGAGTTCAG ACGTGTGCTCTTCCG ATCTAGACGAGACAC AAACAATGCTACTGC | 510 |
| 2nd PCR | USH2A F | 12 | USH2A-F-R#6 miseq R | R | GTGACTGGAGTTCAG ACGTGTGCTCTTCCG ATCTCAACTGTTTGCG ATGAACTTCATAA | 511 |
| 2nd PCR | USH2A F | 13 | USH2A-F-R#7 miseq R | R | GTGACTGGAGTTCAG ACGTGTGCTCTTCCG ATCTCTGGAGCTCTAT TGTACAGCATGGTG | 512 |
| 2nd PCR | USH2A F | 14 | USH2A-F-R#9 miseq R | R | GTGACTGGAGTTCAG ACGTGTGCTCTTCCG ATCTCATTGCTTGTCA TCTTGTGTGACTCA | 513 |
| 2nd PCR | USH2A F | 15 | USH2A-F-R#10 miseq R | R | GTGACTGGAGTTCAG ACGTGTGCTCTTCCG ATCTCACCTAAACTTA AATCTCTGACAAGTA AGGT | 514 |
| 2nd PCR | USH2A F | 16 | USH2A-F-R#11 miseq R | R | GTGACTGGAGTTCAG ACGTGTGCTCTTCCG ATCTGTACATATATCA AAACATCATGTTGTCT GCC | 515 |
| 2nd PCR | USH2A R | 17 | USH2A-R-F#2 miseq F | F | CACTCTTTCCCTACAC GACGCTCTTCCGATCT ACATTTTCAGTGCAC AATGACATTCC | 516 |
| 2nd PCR | USH2A R | 18 | USH2A-R-F#3 miseq F | F | CACTCTTTCCCTACAC GACGCTCTTCCGATCT TGGCTAAATGTTTTTG CTGAAGAGGC | 517 |
| 2nd PCR | USH2A R | 19 | USH2A-R-F#4 miseq F | F | CACTCTTTCCCTACAC GACGCTCTTCCGATCT AAACTCAGCCGATCG GATTTATTTCA | 518 |
| 2nd PCR | USH2A R | 20 | USH2A-R-F#5 miseq F | F | CACTCTTTCCCTACAC GACGCTCTTCCGATCT AACTTCTCCCTGTTTC TGGTTTGTGG | 519 |
| 2nd PCR | USH2A R | 21 | USH2A-R-F#6 miseq F | F | CACTCTTTCCCTACAC GACGCTCTTCCGATCT ACCTAGAATTGTTTCC ACATGCCATCA | 520 |
| 2nd PCR | USH2A R | 22 | USH2A-R-F#7 miseq F | F | CACTCTTTCCCTACAC GACGCTCTTCCGATCT GACCCCATCTATGGCT CTCCTTACAT | 521 |
| 2nd PCR | USH2A R | 23 | USH2A-R-F#8 miseq F | F | CACTCTTTCCCTACAC GACGCTCTTCCGATCT AGCAAAGAATCCAGC CTAGGATAATTGG | 522 |
| 2nd PCR | USH2A R | 24 | USH2A-R-F#9 miseq F | F | CACTCTTTCCCTACAC GACGCTCTTCCGATCT CCAGGGGTGTCACGT ACTTATAAAATGA | 523 |
| 2nd PCR | USH2A R | 25 | USH2A-R-F#11 miseq F | F | CACTCTTTCCCTACAC GACGCTCTTCCGATCT CTAGGATAATTGGGCC ATGCTTTTCC | 524 |
| 2nd PCR | USH2A R | 26 | USH2A-R-F#12 miseq F | F | CACTCTTTCCCTACAC GACGCTCTTCCGATCT ATAATTCTACCACCAG CCACAACAGA | 525 |
| 2nd PCR | USH2A R | 27 | USH2A-R-F#13 miseq F | F | CACTCTTTCCCTACAC GACGCTCTTCCGATCT TTTGGCAACAAAGTC CTTTGTCTC | 526 |
| 2nd PCR | USH2A R | 28 | USH2A-R-F#14 miseq F | F | CACTCTTTCCCTACAC GACGCTCTTCCGATCT CTTATCCGTTGTTTAA CAGCTGTGCT | 527 |
| 2nd PCR | USH2A R | 29 | USH2A-R-F#15 miseq F | F | CACTCTTTCCCTACAC GACGCTCTTCCGATCT TTTCTCTACATGGGTA TATGGCCACC | 528 |
| 2nd PCR | USH2A R | 30 | USH2A-R-F#16 miseq F | F | CACTCTTTCCCTACAC GACGCTCTTCCGATCT TCATCAGGTAGAAGC AAGGTGGTAAG | 529 |
| 2nd PCR | USH2A R | 31 | USH2A-R-F#17 miseq F | F | CACTCTTTCCCTACAC GACGCTCTTCCGATCT ACAGCCTAAATGACA GATACAGCACA | 530 |
| 2nd PCR | USH2A R | 32 | USH2A-R-F#18 miseq F | F | CACTCTTTCCCTACAC GACGCTCTTCCGATCT TCCTTCCAATGAAAG ACCCAATCCAT | 531 |
| 2nd PCR | USH2A R | 33 | USH2A-R-R#1 miseq R | R | GTGACTGGAGTTCAG ACGTGTGCTCTTCCG ATCTTACACACTGACC AATGCCAAAGGAAA | 532 |
| 2nd PCR | USH2A R | 34 | USH2A-R-R#2 miseq R | R | GTGACTGGAGTTCAG ACGTGTGCTCTTCCG | 533 |
| | | | | | ATCTGCCTCTTCAGCA AAAACATTTAGCCA | |
| 2nd PCR | USH2A R | 35 | USH2A-R-R#4 miseq R | R | GTGACTGGAGTTCAG ACGTGTGCTCTTCCG ATCTTGAAATAAATCC GATCGGCTGAGTTT | 534 |
| 2nd PCR | USH2A R | 36 | USH2A-R-R#5 miseq R | R | GTGACTGGAGTTCAG ACGTGTGCTCTTCCG ATCTAAATGTAAGGA GAGCCATAGATGGGG | 535 |
| 2nd PCR | USH2A R | 37 | USH2A-R-R#6 miseq R | R | GTGACTGGAGTTCAG ACGTGTGCTCTTCCG ATCTACAGGACAACA ATTAGCACAGCTGTT | 536 |
| 2nd PCR | USH2A R | 38 | USH2A-R-R#7 miseq R | R | GTGACTGGAGTTCAG ACGTGTGCTCTTCCG ATCTTACTCCTTCTCT GGCAAGCAAATCAC | 537 |
| 2nd PCR | USH2A R | 39 | USH2A-R-R#10 miseq R | R | GTGACTGGAGTTCAG ACGTGTGCTCTTCCG ATCTGAGAGCCACAA AGATAAAGGAAAGAG CA | 538 |
| 2nd PCR | USH2A R | 40 | USH2A-R-R#11 miseq R | R | GTGACTGGAGTTCAG ACGTGTGCTCTTCCG ATCTTTCTAATTCCTG AGTCCTGACTGCAG | 539 |
| 2nd PCR | USH2A R | 41 | USH2A-R-R#12 miseq R | R | GTGACTGGAGTTCAG ACGTGTGCTCTTCCG ATCTCTTACCACCTTG CTTCTACCTGATGA | 540 |
| 2nd PCR | USH2A R | 42 | USH2A-R-R#13 miseq R | R | GTGACTGGAGTTCAG ACGTGTGCTCTTCCG ATCTGGAAGGACCAA AGGGAACAAATGTTT | 541 |
| 2nd PCR | USH2A R | 43 | USH2A-R-R#14 miseq R | R | GTGACTGGAGTTCAG ACGTGTGCTCTTCCG ATCTGGAGATTTACTT CAAGTGTAGAAATTG AGTC | 542 |
| 2nd PCR | USH2A R | 44 | USH2A-R-R#15 miseq R | R | GTGACTGGAGTTCAG ACGTGTGCTCTTCCG | 543 |
| | | | | | ATCTTGTGCTGTATCT GTCATTTAGGCTGT | |
| 2nd PCR | USH2A R | 45 | USH2A-R-R#16 miseq R | R | GTGACTGGAGTTCAG ACGTGTGCTCTTCCG ATCTTTGACTGTGTAG GAGACAAAGGACTT | 544 |
| 2nd PCR | USH2A R | 46 | USH2A-R-R#17 miseq R | R | GTGACTGGAGTTCAG ACGTGTGCTCTTCCG ATCTGCTTGTTTTGGT TTACTTAGAAAGAAG GATG | 545 |

After completing the third PCR, the sample was loaded onto a 2% agarose gel to identify the bands. The completed PCR products were pooled in equal amounts (5 µl each) and then subjected to PCR purification.

PCR purification was performed using the GEL & PCR Purification System (GP 104-200, Biofact). UB buffer equivalent to 5 times the volume of the PCR products was added to the PCR products and thorough mixing was performed. The column was prepared, 200 µl of HelpB buffer was added to the column, centrifugation was performed at 13,000 rpm for 30 seconds, and the filtered solution was discarded. The reaction solution was added to the column, centrifugation was performed at 7,000 rpm for 1 minute, and the filtered solution was discarded. 750 µl of 80% ethanol was added thereto, centrifugation was performed at 13,000 rpm for 30 seconds, and the filtered solution was discarded. After repeating the process twice, centrifugation was performed at 13,000 rpm for 3 minutes. The centrifuged column was placed into a 1.5 ml tube, 100 µl of EB buffer was added dropwise to the center, and the reaction was allowed to occur at room temperature for 1 minute. Centrifugation was performed at 13,000 rpm for 1 minute. The DNA collected in the 1.5 ml tube was quantified to obtain a concentration of 15 ng/µl and stored at 4°C until NGS analysis.

### Experimental Example 18. T-blunt end cloning

For vectorization of the cassette or sequencing of the PCR product, the target cassette or PCR product was cloned into T-vector according to the manufacturer's instructions using the All in one PCR cloning kit (VT202-020, Biofact). Cloning was performed using the product or cassette DNA that was designed so that a length of DNA did not exceed 2 kb. A mixture having the composition disclosed in Table 32 below was prepared and the ligation reaction was performed.

**[Table 32]**

| Reagent | Volume |
|---|---|
| 6X All in one buffer | 1 *µ*ℓ |
| All in one vector | 1 *µ*g |
| PCR product or cassette | 4 *µ*ℓ |
| Total | 6 *µ*ℓ |

The mixture was incubated for 30 minutes, and then transformation was performed on competent cells (*E. coli*). The above-mentioned description of the present disclosure is for illustrative purposes, and those skilled in the art to which the present disclosure pertains will understand that the present disclosure may be easily modified into other specific forms without changing the technical idea or essential characteristics of the present disclosure. Therefore, it should be understood that the embodiments described are illustrative in all respects and not restrictive.

## Claims

1. A system for editing a USH2A gene, comprising:
an endonuclease comprising a Cas12f1 molecule or a nucleic acid encoding the endonuclease;
a first guide RNA comprising a first guide sequence capable of hybridizing to a target sequence of contiguous 15 to 30 bp in length, wherein the target sequence is located in a region 5000 bp upstream of USH2A exon 13 and is adjacent to a protospacer-adjacent motif (PAM) sequence recognized by the Cas12f1 molecule, or a nucleic acid encoding the first guide RNA; and
a second guide RNA comprising a second guide sequence capable of hybridizing to a target sequence of contiguous 15 to 30 bp in length, wherein the target sequence is located in a region 14500 bp downstream of USH2A exon 13 and is adj acent to a PAM sequence recognized by the Cas12f1 molecule, or a nucleic acid encoding the second guide RNA.

2. The system of claim 1, wherein the system induces deletion of exon 13 in the USH2A gene in a cell.

3. The system of claim 1, wherein the system is for treatment of type 2AUsher syndrome.

4. The system of claim 1, wherein the USH2A exon 13 comprises at least one mutation that causes Usher syndrome.

5. The system of claim 1, wherein the target sequence located in a region 5000 bp upstream of USH2A exon 13 comprises a nucleotide sequence selected from the group consisting of SEQ ID NOs: 1 to 49, and/or
the target sequence located in a region 14500 bp downstream of the USH2A exon 13 comprises a nucleotide sequence selected from the group consisting of SEQ ID NOs: 50 to 79.

6. The system of claim 1, wherein the first guide sequence comprises a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or
the second guide sequence comprises a sequence of contiguous 15 to 20 nucleotides in a nucleotide sequence selected from the group consisting of SEQ ID NOs: 446 to 475, or a sequence that is different by 5 or fewer nucleotides from the contiguous nucleotide sequence, wherein thymine (T) in the contiguous nucleotide sequence is substituted with uracil (U).

7. The system of claim 1, wherein the first guide sequence comprises a nucleotide sequence selected from the group consisting of SEQ ID NOs: 80 to 128 and SEQ ID NOs: 159 to 164, and/or
the second guide sequence comprises a nucleotide sequence selected from the group consisting of SEQ ID NOs: 129 to 158 and SEQ ID NOs: 165 to 174.

8. The system of claim 1, wherein the first or second guide RNA comprises a U-rich tail sequence linked to the 3' end of the guide sequence, in which the U-rich tail is represented by 5'-(UₘV)ₙUₒ-3', wherein V is each independently A, C, or G; m and o are integers between 1 to 20; and n is an integer between 0 to 5.

9. The system of claim 1, wherein the first or second guide RNA comprises an engineered scaffold region, and the engineered scaffold region comprises a nucleotide sequence having at least 50% sequence identity to a scaffold region of a wild-type Cas12f1 guide RNA sequence, in which the scaffold region sequentially comprises, from the 5'-end, a first stem-loop region, a second stem-loop region, a third stem-loop region, a fourth stem-loop region, and a tracrRNA-crRNA complementarity region, and comprises at least one modification selected from the group consisting of the following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

10. The system of claim 9, wherein the wild-type Cas 12f1 guide RNA comprises tracrRNA comprising the nucleotide sequence of SEQ ID NO: 175 and crRNA comprising the nucleotide sequence of SEQ ID NO: 176.

11. The system of claim 9, wherein the engineered scaffold region comprises a sequence having at least 80% sequence identity to a sequence represented by Formula (I): 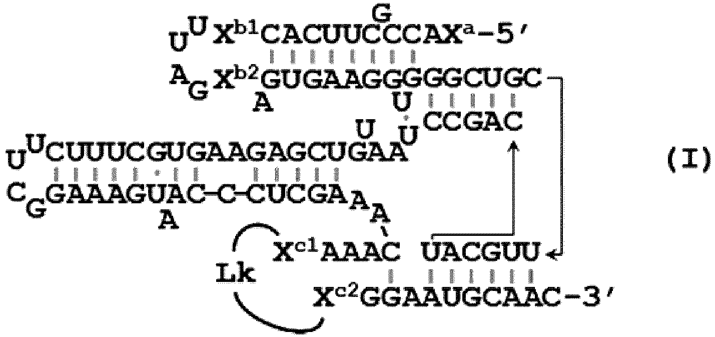 in Formula (I),
X^{a} comprises the nucleotide sequence of SEQ ID NO: 178 or a nucleotide sequence having the sequence of SEQ ID NO: 178 from which 1 to 20 nucleotides are deleted,
X^{b1} comprises the nucleotide sequence of SEQ ID NO: 189 or a nucleotide sequence having the sequence of SEQ ID NO: 189 from which 1 to 13 nucleotides are deleted,
X^{b2} comprises the nucleotide sequence of SEQ ID NO: 193 or a nucleotide sequence having the sequence of SEQ ID NO: 193 from which 1 to 14 nucleotides are deleted,
X^{c1} comprises the nucleotide sequence of SEQ ID NO: 203 or a nucleotide sequence having the sequence of SEQ ID NO: 203 from which 1 to 28 nucleotides are deleted,
X^{c2} comprises the nucleotide sequence of SEQ ID NO: 222 or a nucleotide sequence having the sequence of SEQ ID NO: 222 from which 1 to 27 nucleotides are deleted, and
Lk is a polynucleotide linker of 2 to 20 nucleotides in length or absent.

12. The system of claim 11, wherein in a case where three or more consecutive uracil (U) residues are present in the X^{c1} sequence, the X^{c1} sequence comprises a modification in which at least one U residue thereof is replaced with A, G or C.

13. The system of claim 11, wherein the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

14. The system of claim 11, wherein the sequence 5'-X^{b1}UUAGX^{b2}-3' in Formula (I) is selected from the group consisting of SEQ ID NOs: 198 to 202 and 5'-UUAG-3'.

15. The system of claim 11, wherein the sequence 5'-X^{c1}-Lk-X^{c2}-3' in Formula (I) is selected from the group consisting of SEQ ID NOs: 244 to 250 and 5'-Lk-3'.

16. The system of claim 9, wherein the scaffold region comprises an engineered tracrRNA consisting of a nucleotide sequence selected from the group consisting of SEQ ID NOs: 251 to 296 and/or
an engineered crRNA consisting of a nucleotide sequence selected from the group consisting of SEQ ID NOs: 297 to 304.

17. The system of claim 1, wherein the first or second guide RNA is a dual guide RNA or a single guide RNA.

18. The system of claim 1, wherein the first or second guide RNA comprises a scaffold region sequence of a nucleotide sequence selected from the group consisting of SEQ ID NOs: 313 to 350.

19. The system of claim 1, wherein the Cas12f1 molecule comprises an amino acid sequence having at least 70% sequence identity with an amino acid sequence selected from the group consisting of SEQ ID NOs: 360 to 364 and SEQ ID NOs: 370 to 377.

20. The system of claim 1, wherein the endonuclease forms a ribonucleoprotein (RNP) with the first guide RNA or the second guide RNA.

21. A vector system, comprising at least one vector wherein the at least one vector comprises:
a first nucleic acid construct to which a nucleotide sequence encoding an endonuclease is operably linked, the endonuclease comprising a Cas12f1 molecule;
a second nucleic acid construct to which a nucleotide sequence encoding a first guide RNA is operably linked, the first guide RNA comprising a first guide sequence capable of hybridizing to a target sequence of contiguous 15 to 30 bp in length, wherein the target sequence is located in a region 5000 bp upstream of USH2A exon 13 and is adjacent to a protospacer-adjacent motif (PAM) sequence recognized by the Cas12f1 molecule; and
a third nucleic acid construct to which a nucleotide sequence encoding a second guide RNA is operably linked, the second guide RNA comprising a second guide sequence capable of hybridizing to a target sequence of contiguous 15 to 30 bp in length, wherein the target sequence is located in a region 14500 bp downstream of USH2A exon 13 and is adjacent to a PAM sequence recognized by the Cas12f1 molecule.

22. The vector system of claim 21, wherein the vector system induces deletion of exon 13 in a USH2A gene in a cell.

23. The vector system of claim 21, wherein the USH2A exon 13 comprises at least one mutation that causes Usher syndrome.

24. The vector system of claim 21, wherein the nucleic acid constructs are contained in the same or different vectors.

25. The vector system of claim 21, wherein the nucleic acid constructs are contained in one vector.

26. The vector system of claim 21, wherein the target sequence located in a region 5000 bp upstream of USH2A exon 13 comprises a nucleotide sequence selected from the group consisting of SEQ ID NOs: 1 to 49, and/or
the target sequence located in a region 14500 bp downstream of USH2A exon 13 comprises a nucleotide sequence selected from the group consisting of SEQ ID NOs: 50 to 79.

27. The vector system of claim 21, wherein the first guide sequence comprises a sequence of contiguous 15 to 22 nucleotides from a nucleotide sequence selected from the group consisting of SEQ ID NOs: 397 to 445, or a sequence that is different by 5 or fewer nucleotides in the contiguous nucleotide sequence, wherein thymine (T) in the contiguous nucleotide sequence is substituted with uracil (U), and/or
the second guide sequence comprises a sequence of contiguous 15 to 20 nucleotides in a nucleotide sequence selected from the group consisting of SEQ ID NOs: 446 to 475, or a sequence that is different by 5 or fewer nucleotides in the contiguous nucleotide sequence, wherein thymine (T) in the contiguous nucleotide sequence is substituted with uracil (U).

28. The vector system of claim 21, wherein the first guide sequence comprises a nucleotide sequence selected from the group consisting of SEQ ID NOs: 80 to 128 and SEQ ID NOs: 159 to 164, and/or
the second guide sequence comprises a nucleotide sequence selected from the group consisting of SEQ ID NOs: 129 to 158 and SEQ ID NOs: 165 to 174.

29. The vector system of claim 21, wherein the first or second guide RNA comprises a U-rich tail sequence linked to the 3' end of the guide sequence, in which the U-rich tail is represented by 5'-(UₘV)ₙUₒ-3', wherein V is each independently A, C, or G; m and o are integers between 1 to 20; and n is an integer between 0 to 5.

30. The vector system of claim 21, wherein the first or second guide RNA comprises an engineered scaffold region, wherein the engineered scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region and a tracrRNA-crRNA complementarity region, and comprises at least one modification selected from the group consisting of the following (1) to (4):
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

31. The vector system of claim 30, wherein the wild-type Cas12f1 guide RNA comprises tracrRNA comprising the nucleotide sequence of SEQ ID NO: 175 and crRNA comprising the nucleotide sequence of SEQ ID NO: 176.

32. The vector system of claim 30, wherein the engineered scaffold region comprises a sequence having at least 80% sequence identity to a sequence represented by Formula (I): 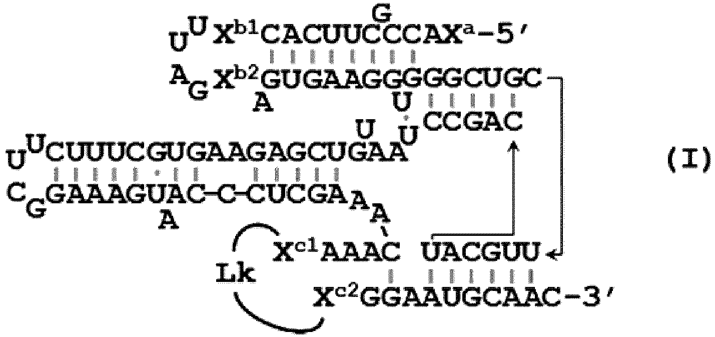 in Formula (I),
X^{a} comprises the nucleotide sequence of SEQ ID NO: 178 or a nucleotide sequence having the sequence of SEQ ID NO: 178 from which 1 to 20 nucleotides are deleted,
X^{b1} comprises the nucleotide sequence of SEQ ID NO: 189 or a nucleotide sequence having the sequence of SEQ ID NO: 189 from which 1 to 13 nucleotides are deleted,
X^{b2} comprises the nucleotide sequence of SEQ ID NO: 193 or a nucleotide sequence having the sequence of SEQ ID NO: 193 from which 1 to 14 nucleotides are deleted,
X^{c1} comprises the nucleotide sequence of SEQ ID NO: 203 or a nucleotide sequence having the sequence of SEQ ID NO: 203 from which 1 to 28 nucleotides are deleted,
X^{c2} comprises the nucleotide sequence of SEQ ID NO: 222 or a nucleotide sequence having the sequence of SEQ ID NO: 222 from which 1 to 27 nucleotides are deleted, and
Lk is a polynucleotide linker of 2 to 20 nucleotides in length or absent.

33. The vector system of claim 32, wherein in a case where three or more consecutive uracil (U) residues are present in the X^{c1} sequence, the system comprises a modification in which at least one U residue thereof is replaced with A, G or C.

34. The vector system of claim 32, wherein the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

35. The vector system of claim 32, wherein the sequence 5'-X^{b1}UUAGX^{b2}-3' in Formula (I) is selected from the group consisting of SEQ ID NOs: 198 to 202 and 5'-UUAG-3'.

36. The vector system of claim 32, wherein the sequence 5'-X^{c1}-Lk-X^{c2}-3' in Formula (I) is selected from the group consisting of SEQ ID NOs: 244 to 250 and 5'-Lk-3'.

37. The vector system of claim 32, wherein the Lk comprises a nucleotide sequence selected from the group consisting of 5'-GAAA-3', 5'-UUAG-3', 5'-UGAAAA-3', 5'-UUGAAAAA-3', 5'-UUCGAAAGAA-3' (SEQ ID NO: 240), 5'-UUCAGAAAUGAA-3' (SEQ ID NO: 241), 5'-UUCAUGAAAAUGAA-3' (SEQ ID NO: 242), and 5'-UUCAUUGAAAAAUGAA-3' (SEQ ID NO: 243).

38. The vector system of claim 30, wherein the scaffold region comprises an engineered tracrRNA consisting of a nucleotide sequence selected from the group consisting of SEQ ID NOs: 251 to 296 and/or
an engineered crRNA consisting of a nucleotide sequence selected from the group consisting of SEQ ID NOs: 297 to 304.

39. The vector system of claim 21, wherein the first or second guide RNA comprises a scaffold region sequence of a nucleotide sequence selected from the group consisting of SEQ ID NOs: 313 to 350.

40. The vector system of claim 21, wherein the Cas12f1 molecule comprises an amino acid sequence having at least 70% sequence identity with an amino acid sequence selected from the group consisting of SEQ ID NOs: 360 to 364 and SEQ ID NOs: 370 to 377.

41. The vector system of claim 21, wherein the vector further comprises a promoter or enhancer.

42. The vector system of claim 41, wherein the promoter is U6 promoter, EFS promoter, EF1-α promoter, H1 promoter, 7SK promoter, CMV promoter, LTR promoter, Ad MLP promoter, HSV promoter, SV40 promoter, CBA promoter, or RSV promoter.

43. The vector system of claim 21, wherein the vector is selected from the group consisting of a retrovirus vector, a lentivirus vector, an adenovirus vector, an adeno-associated virus vector, a vaccinia virus vector, a poxvirus vector, a herpes simplex virus vector, and a phagemid vector.

44. The vector system of claim 21, wherein the vector is selected from the group consisting of plasmid, naked DNA, DNA complex, mRNA (transcript), and amplicon.

45. A recombinant virus produced by the vector system of any one of claims 21 to 44.

46. A composition comprising the system of any one of claims 1 to 20, the vector system of any one of claims 21 to 44, or the recombinant virus of claim 45.

47. The composition of claim 46, wherein the composition is a pharmaceutical composition.

48. A method for inducing deletion of a segment comprising exon 13 in a USH2A gene in a cell, comprising bringing, into contact with the cell, the system of any one of claims 1 to 20, the vector system of any one of claims 21 to 44, or the recombinant virus of claim 45.

49. A method for treating a subject having a disease associated with a mutation in exon 13 of the USH2A gene, comprising bringing, into contact with the subject, the system of any one of claims 1 to 20, the vector system of any one of claims 21 to 44, or the recombinant virus of claim 45.

50. A method for modifying a gene of a cell, comprising bringing, into contact with the cell, the system of any one of claims 1 to 20, the vector system of any one of claims 21 to 44, or the recombinant virus of claim 45.

51. The method of any one of claims 48 to 50, wherein the recombinant virus is an adeno-associated virus (AAV).

52. The method of claim 48 or 50, wherein the cell is a stem cell, or a cell from the eye or inner ear of a mammal.

53. The method of claim 48 or 50, wherein the cell is derived from a subject having Usher syndrome.

54. The method of claim 48 or 50, wherein the bringing-into-contact occurs ex vivo or in vivo.

55. A stem cell genetically modified by the method of claim 48 or 50.

56. The stem cell of claim 55, wherein the stem cell is for treating type 2AUsher syndrome.

57. A guide RNA, comprising a spacer region, which comprises a guide sequence capable of hybridizing to a target sequence in a USH2A (Usherin) gene, and a scaffold region, wherein the guide sequence comprises (i) a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, wherein thymine (T) in the contiguous nucleotide sequence is substituted with uracil (U), and/or (ii) a sequence of contiguous 15 to 20 nucleotides from a nucleotide sequence selected from the group consisting of SEQ ID NOs: 446 to 475, or a sequence that is different by 5 or fewer nucleotides in the contiguous nucleotide sequence, wherein thymine (T) in the contiguous nucleotide sequence is substituted with uracil (U).

58. The guide RNA of claim 57, wherein the guide sequence comprises a nucleotide sequence selected from the group consisting of SEQ ID NOs: 80 to 128 and SEQ ID NOs: 159 to 164, and/or
the guide sequence comprises a nucleotide sequence selected from the group consisting of SEQ ID NOs: 129 to 158 and SEQ ID NOs: 165 to 174.

59. The guide RNA of claim 57, wherein the guide RNA comprises a U-rich tail sequence linked to the 3' end of the guide sequence, in which the U-rich tail is represented by 5'-(UₘV)ₙUₒ-3', wherein V is each independently A, C, or G; m and o are integers between 1 to 20; and n is an integer between 0 to 5.

60. The guide RNA of claim 57, wherein the scaffold region comprises a nucleotide sequence having at least 50% sequence identity to a scaffold region of a wild-type Cas12f1 guide RNA sequence, which sequentially comprises, from the 5'-end, a first stem-loop region, a second stem-loop region, a third stem-loop region, a fourth stem-loop region and a tracrRNA-crRNA complementarity region, and comprises at least one modification selected from the group consisting of the following (1) to (4) with respect to the wild-type Cas12f1 guide RNA sequence:
(1) deletion of at least a part of the first stem-loop region;
(2) deletion of at least a part of the second stem-loop region;
(3) deletion of at least a part of the tracrRNA-crRNA complementarity region; and
(4) replacement of one or more uracil (U) residues with A, G, or C in three or more consecutive U residues when the consecutive U residues are present in the tracrRNA-crRNA complementarity region.

61. The guide RNA of claim 60, wherein the wild-type Cas12f1 guide RNA comprises tracrRNA comprising the nucleotide sequence of SEQ ID NO: 175 and crRNA comprising the nucleotide sequence of SEQ ID NO: 176.

62. The guide RNA of claim 60, wherein the scaffold region comprises a sequence having at least 80% sequence identity to a sequence represented by Formula (I): 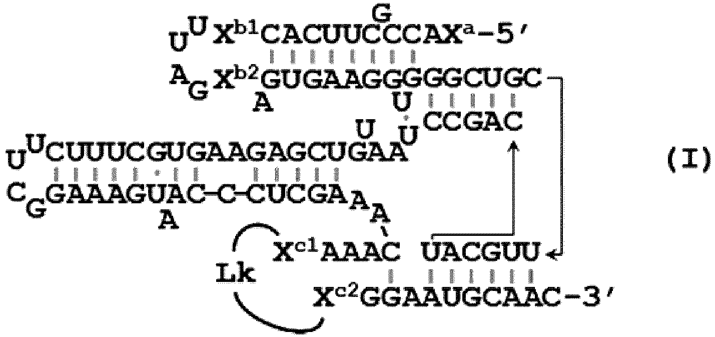 in Formula (I),
X^{a} comprises the nucleotide sequence of SEQ ID NO: 178 or a nucleotide sequence having the sequence of SEQ ID NO: 178 from which 1 to 20 nucleotides are deleted,
X^{b1} comprises the nucleotide sequence of SEQ ID NO: 189 or a nucleotide sequence having the sequence of SEQ ID NO: 189 from which 1 to 13 nucleotides are deleted,
X^{b2} comprises the nucleotide sequence of SEQ ID NO: 193 or a nucleotide sequence having the sequence of SEQ ID NO: 193 from which 1 to 14 nucleotides are deleted,
X^{c1} comprises the nucleotide sequence of SEQ ID NO: 203 or a nucleotide sequence having the sequence of SEQ ID NO: 203 from which 1 to 28 nucleotides are deleted,
X^{c2} comprises the nucleotide sequence of SEQ ID NO: 222 or a nucleotide sequence having the sequence of SEQ ID NO: 222 from which 1 to 27 nucleotides are deleted, and
Lk is a polynucleotide linker of 2 to 20 nucleotides in length or absent.

63. The guide RNA of claim 62, wherein in a case where three or more consecutive uracil (U) residues are present in the X^{c1} sequence, the X^{c1} sequence comprises a modification in which at least one U residue thereof is replaced with A, G or C.

64. The guide RNA of claim 62, wherein the deletion in the nucleotide sequence of X^{a}, the deletion in the nucleotide sequences of X^{b1} and X^{b2}, and/or the deletion in the nucleotide sequences of X^{c1} and X^{c2} comprises deletion of one or more pairs of complementary nucleotides.

65. The guide RNA of claim 62, wherein the sequence 5'-X^{b1}UUAGX^{b2}-3' in Formula (I) is selected from the group consisting of SEQ ID NOs: 198 to 202 and 5'-UUAG-3'.

66. The guide RNA of claim 62, wherein the sequence 5'-X^{c1}-Lk-X^{c2}-3' in Formula (I) is selected from the group consisting of SEQ ID NOs: 244 to 250 and 5'-Lk-3'.

67. The guide RNA of claim 62, wherein the scaffold region comprises an engineered tracrRNA consisting of a nucleotide sequence selected from the group consisting of SEQ ID NOs: 251 to 296 and/or
an engineered crRNA consisting of a nucleotide sequence selected from the group consisting of SEQ ID NOs: 297 to 304.

68. The guide RNA of claim 57, wherein the guide RNA is a single guide RNA.

69. The guide RNA of claim 57, wherein the guide RNA comprises a scaffold region sequence of a nucleotide sequence selected from the group consisting of SEQ ID NOs: 313 to 350.

70. The guide RNA of claim 57, wherein the guide RNA comprises a scaffold region sequence of a nucleotide sequence selected from the group consisting of SEQ ID NOs: 315 to 317.

71. A nucleic acid molecule encoding the guide RNA of any one of claims 57 to 70.

72. A composition comprising at least one guide RNA of any one of claims 57 to 70.

73. A composition comprising at least one guide RNA of any one of claims 57 to 70 and an endonuclease comprising a Cas12f1 molecule.

74. The composition of claim 72 or 73, wherein the composition comprises two or more guide RNAs, of which (i) at least one guide RNA comprises a sequence of contiguous 15 to 22 nucleotides from a nucleotide sequence selected from the group consisting of SEQ ID NOs: 397 to 445, or a sequence that is different by 5 or fewer nucleotides from the contiguous nucleotide sequence, and (ii) at least one other guide RNA comprises a sequence of contiguous 15 to 20 nucleotides from a nucleotide sequence selected from the group consisting of SEQ ID NOs: 446 to 475, or a sequence that is different by 5 or fewer nucleotides from the contiguous nucleotide sequence.
